# EUROPÄISCHE PATENTSCHRIFT

(11) **EP 2 379 529 B1**
(45) Veröffentlichungstag und Bekanntmachung des Hinweises auf die Patenterteilung: **10.06.2015**
(21) Anmeldenummer: 09784087.0
(22) Anmeldetag: 17.12.2009
(51) Int. Cl.: C07D 401/14, C07D 413/14, C07D 471/04, C07D 498/20, A61K 31/551, A61K 31/506, A61K 31/537, A61P 25/06, A61P 5/30, A61P 25/02, A61P 1/00, A61P 9/00

(54) **CGRP-ANTAGONISTEN**
CGRP-ANTAGONISTS
ANTAGONISTES DU CGRP

(30) Priorität: 19.12.2008 EP 08172320
(43) Veröffentlichungstag der Anmeldung: 26.10.2011
(73) Patentinhaber: BOEHRINGER INGELHEIM INTERNATIONAL GMBH, 55216 Ingelheim am Rhein (DE)
(72) Erfinder: GOTTSCHLING, Dirk, 55216 Ingelheim am Rhein (DE); DOODS, Henri, 55216 Ingelheim am Rhein (DE); HEIMANN, Annekatrin, 55216 Ingelheim am Rhein (DE); MUELLER, Stephan Georg, 55216 Ingelheim am Rhein (DE); RUDOLF, Klaus, 55216 Ingelheim am Rhein (DE); SCHAENZLE, Gerhard, 55216 Ingelheim am Rhein (DE); STENKAMP, Dirk, 55216 Ingelheim am Rhein (DE)
(74) Vertreter: Simon, Elke Anna Maria
(86) Internationale Anmeldenummer: PCT/EP2009/067360
(87) Internationale Veröffentlichungsnummer: WO 2010/070022

(56) Entgegenhaltungen:
- WO-A1-03/032997
- WO-A2-2006/031513
- WO-A2-2006/041830
- WO-A2-2008/060568
- US-A1- 2008 113 966
- PAONE D V ET AL: "Calcitonin gene-related peptide receptor antagonists for the treatment of migraine: A patent review" EXPERT OPINION ON THERAPEUTIC PATENTS 2009 INFORMA HEALTHCARE GBR, Bd. 19, Nr. 12, Dezember 2009 (2009-12), Seiten 1675-1713, XP002572832 ISSN: 1354-3776

## Beschreibung

### HINTERGRUND DER ERFINDUNG

WO03/032997 offenbart 2,4,5-trisubstituerte Pyrimidinderivate als Inhibitoren von Proteinkinasen.

WO2008/060568 offenbart amid-substituierte Aryl-piperidine als CGRP-Inhibitoren.

WO2006/031513 offenbart Aryl-spirolactame als CGRP-Antagonisten.

WO2006/041830 offenbart Spiropiperidine als CGRP-Antagonisten.

US2008/113966 offenbart Imidazo-azepine und Triazolo-azepine als CGRP-Antagonisten.

### NEUE VERBINDUNGEN

Gegenstand der vorliegenden Erfindung sind die neuen Verbindungen der allgemeinen Formel I 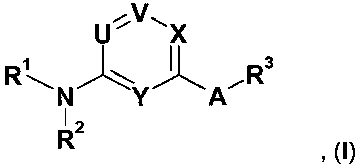 in der **A, U, V, X, Y, R¹**, **R²** und **R³** wie nachstehend erwähnt definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

Die neuen Verbindungen der allgemeinen Formel I besitzen CGRP-antagonistische Eigenschaften und eignen sich daher insbesondere zur Behandlung von Migräne.

### DETAILLIERTE BESCHREIBUNG DER ERFINDUNG

In der obigen allgemeinen Formel **I** bedeuten in einer ersten Ausführungsform
- **A**: -NH, -N(C(O)-C₁₋₃-Alkyl)-, S, O, SO, SO₂,
- **R¹** und **R²**: zusammen mit dem Stickstoffatom, an das sie gebunden sind, eine Gruppe ausgewählt aus 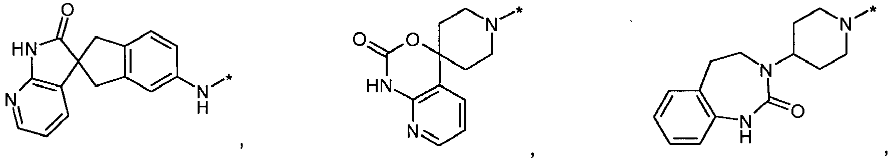 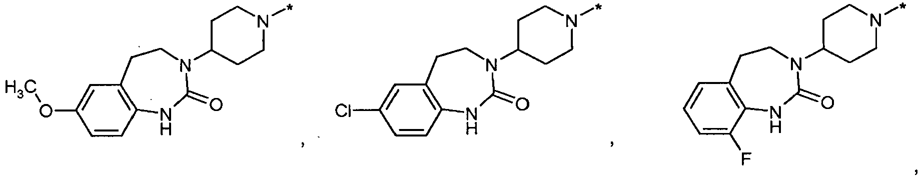 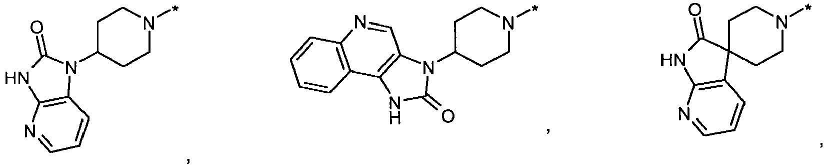 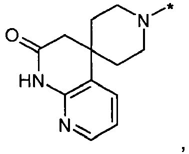 bedeutet,
- **R³**: eine Gruppe ausgewählt aus 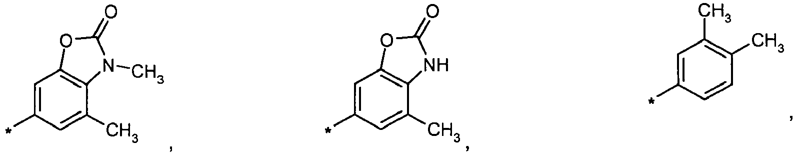 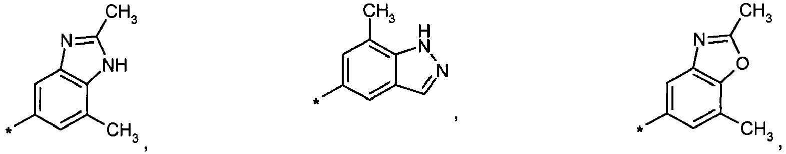 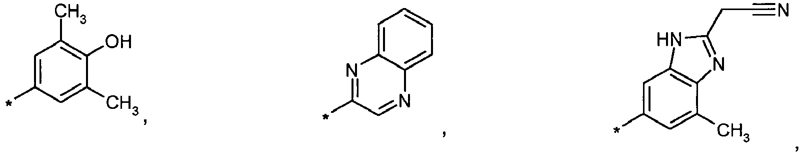 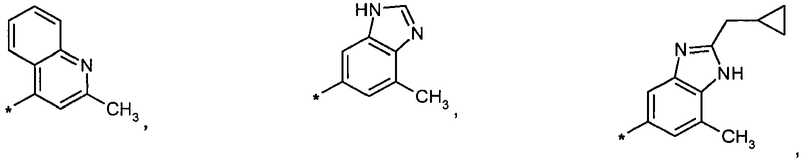 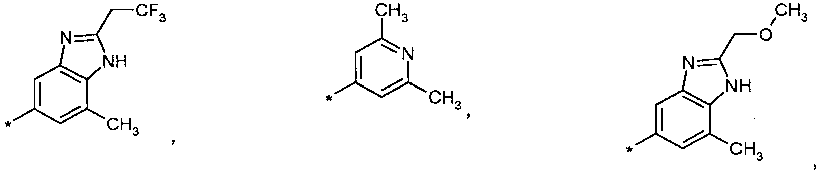 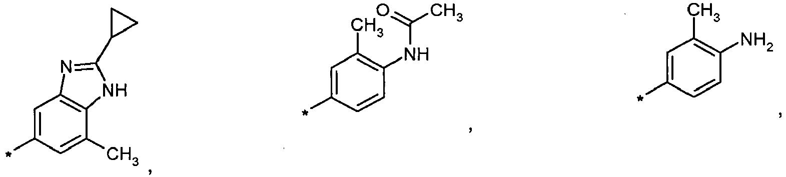 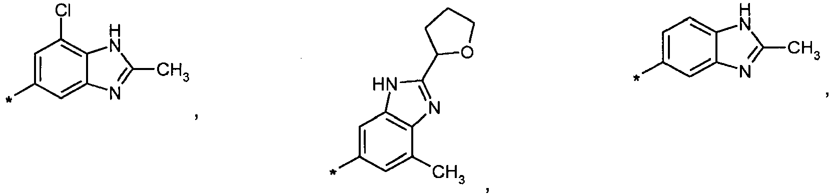 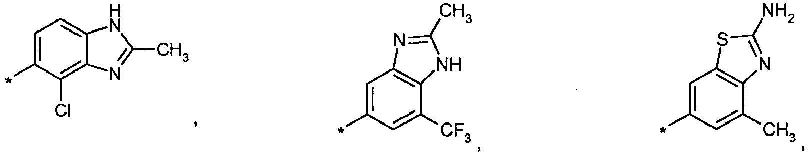 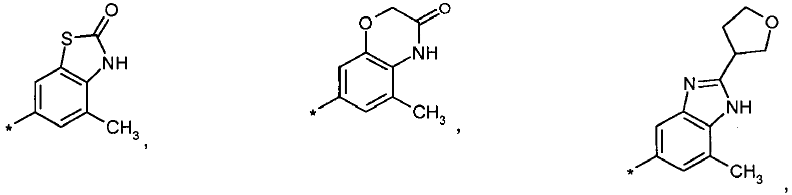 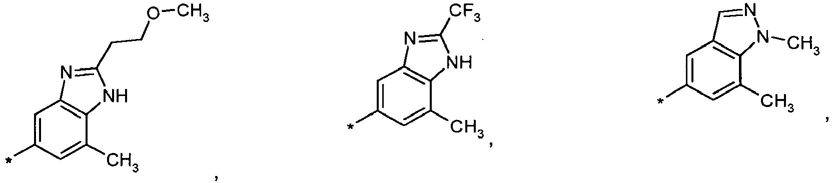 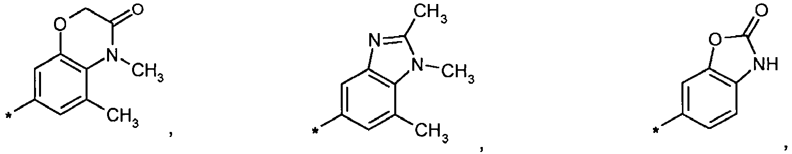 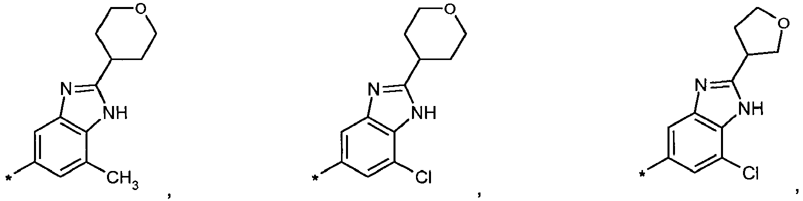 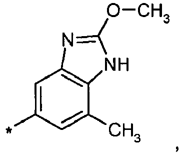
bedeutet,
**U-V-X** eine Gruppe ausgewählt aus -N=(C-**R⁵**)-N=, -N=(C-**R⁵**)-(C-**R⁶**)=, -(N-Oxid)=(C-**R⁵**)-(C-**R⁶**)=, und
- **Y**: N oder CH,
- **R⁵**: (a) H,
(b) -N**R^{5.1}R^{5.2}**,
(c) Halogen, C₃₋₆-Cycloalkyl-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
- **R^{5.1}**: H,
- **R^{5.2}**: H,
- **R⁶**: (a) H,
(b) -CN,

bedeutet,

deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine zweite Ausführungsform der vorliegenden Erfindung besteht in den Verbindungen der obigen allgemeinen Formel **I**, in denen **A, R¹**, **R²** und **R³** wie voranstehend unter der ersten Ausführungsform definiert sind und der Ring 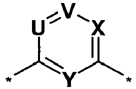 eine Gruppe ausgewählt aus 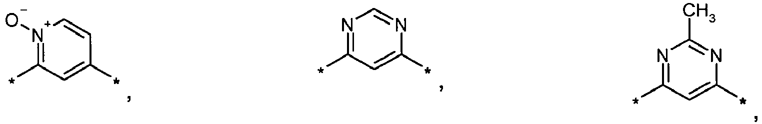 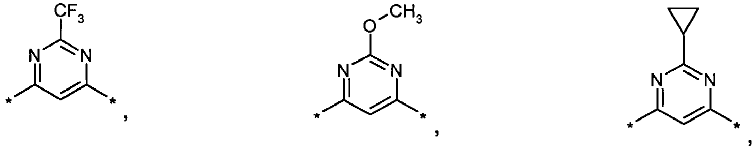 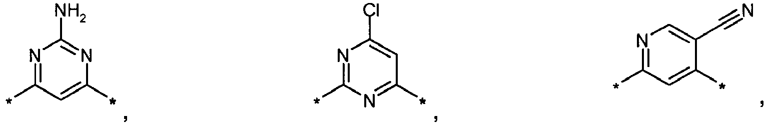 bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine dritte Ausführungsform der vorliegenden Erfindung besteht in den Verbindungen der obigen allgemeinen Formel **I**, in denen **U, V, X, Y, R¹**, **R²** und **R³** wie voranstehend unter der ersten oder zweiten Ausführungsform definiert sind und
- **A**: -O- oder -NH- bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine vierte Ausführungsform der vorliegenden Erfindung besteht in den Verbindungen der allgemeinen Formel **Ia** 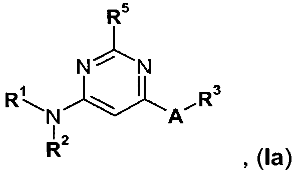 in der
- **A**: -NH- oder -O-,
- **R¹** und **R²**: zusammen mit dem Stickstoffatom, an das sie gebunden sind, eine Gruppe ausgewählt aus 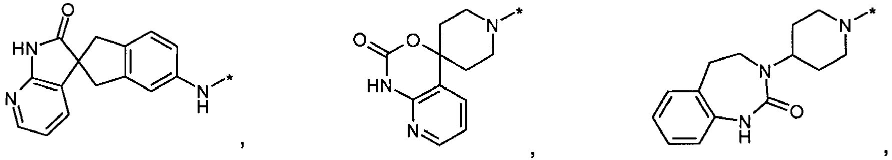 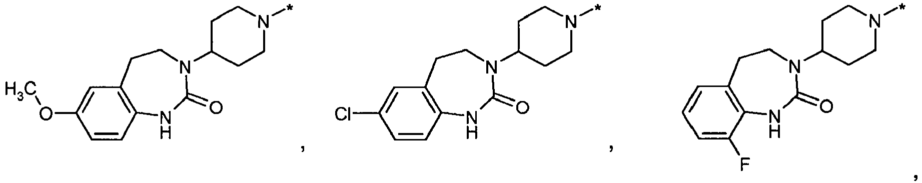 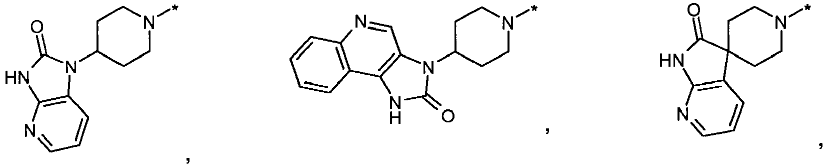 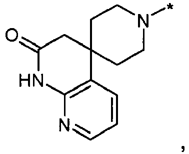
- **R³**: eine Gruppe ausgewählt aus

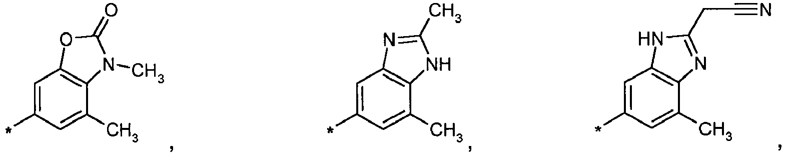

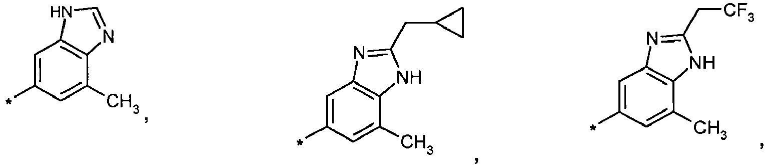

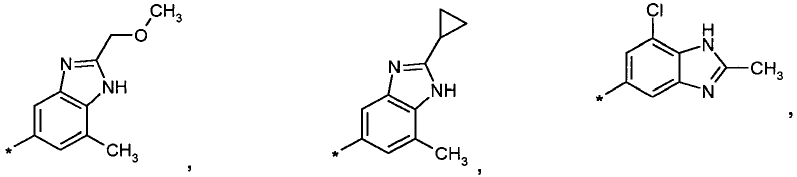

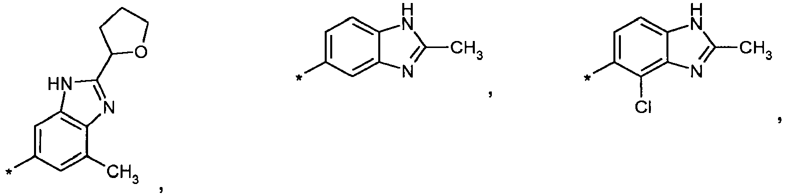

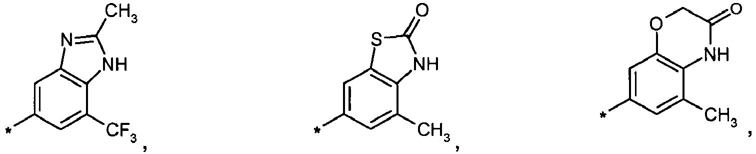

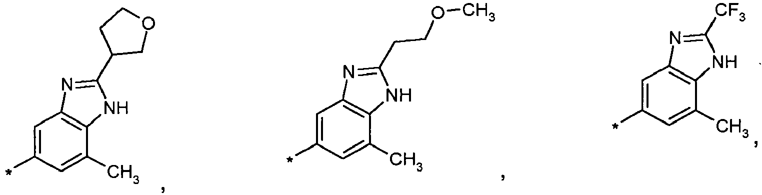

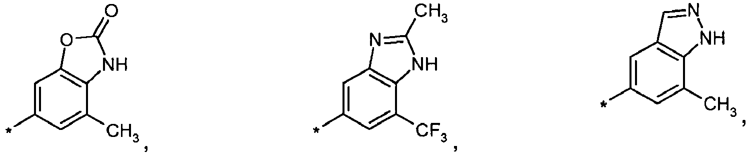

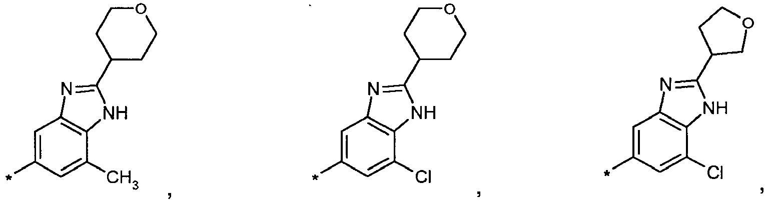

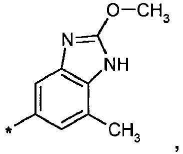

- **R⁵**: H, Cl, CH₃, CF₃, -O-CH₃ oder Cyclopropyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Als ganz besonders bevorzugte Verbindungen der obigen allgemeinen Formel **I** seien beispielsweise folgende Verbindungen genannt:

| **Nr.** | **Struktur** |
|---|---|
| (1) | 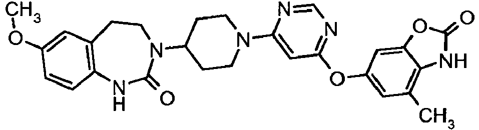 |
| (2) | 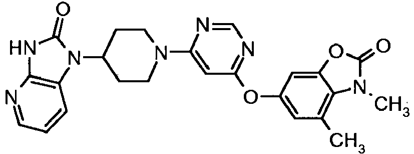 |
| (3) | 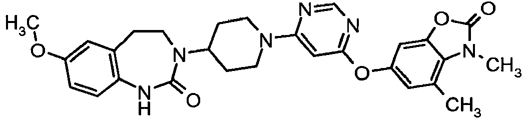 |
| (4) | 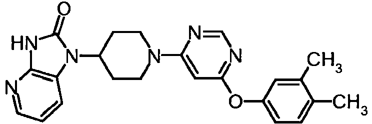 |
| (5) | 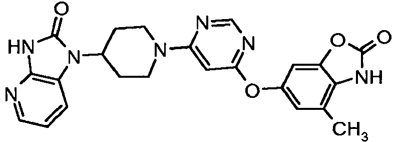 |
| (6) | 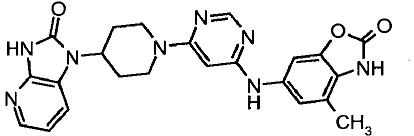 |
| (7) | 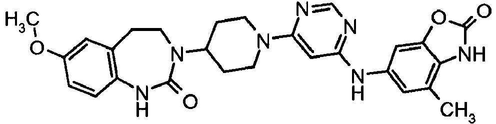 |
| (8) | 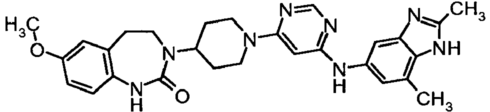 |
| (9) | 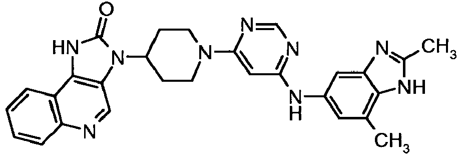 |
| (10) | 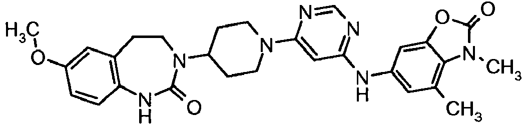 |
| (11) | 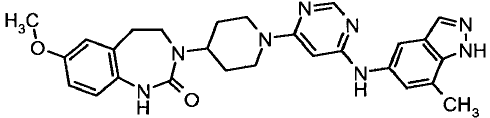 |
| (12) | 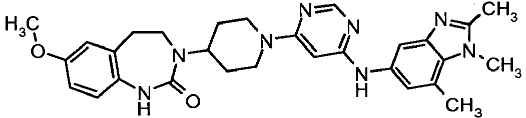 |
| (13) | 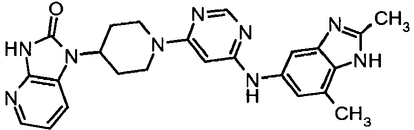 |
| (14) | 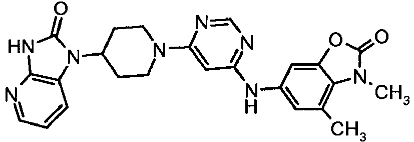 |
| (15) | 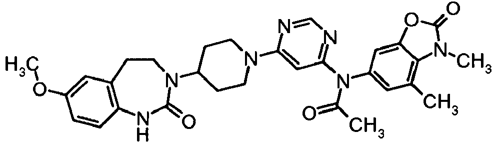 |
| (16) | 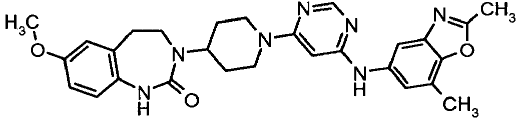 |
| (17) | 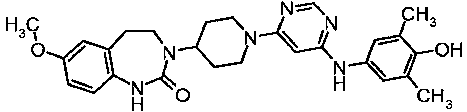 |
| (18) | 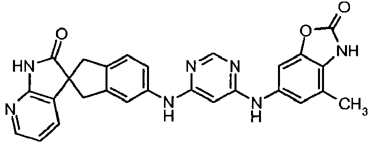 |
| (19) | 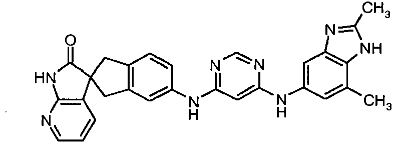 |
| (20) | 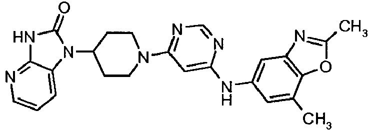 |
| (21) | 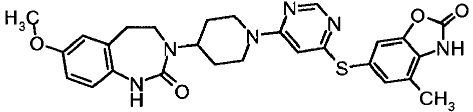 |
| (22) | 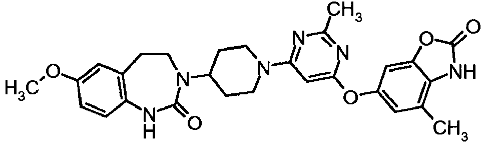 |
| (23) | 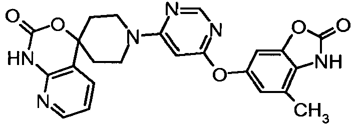 |
| (24) | 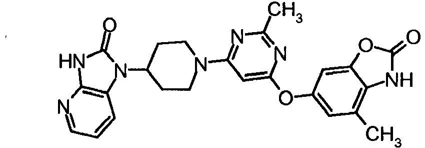 |
| (25) | 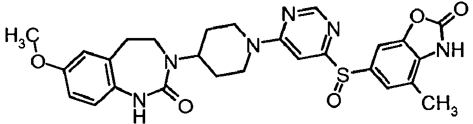 |
| (26) | 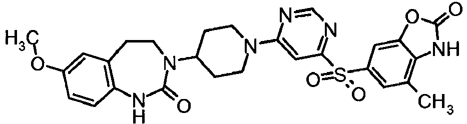 |
| (27) | 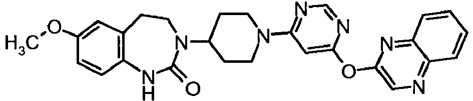 |
| (28) | 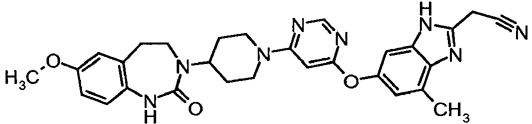 |
| (29) | 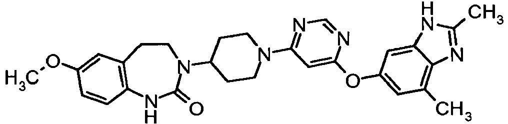 |
| (30) | 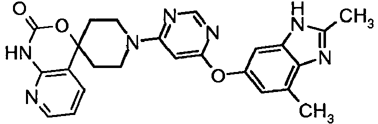 |
| (31) | 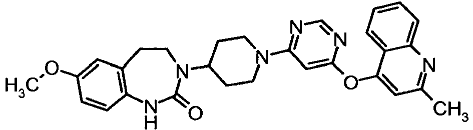 |
| (32) | 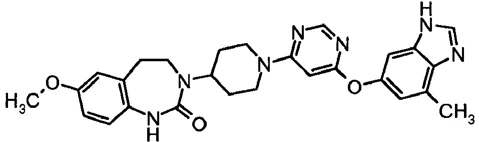 |
| (33) | 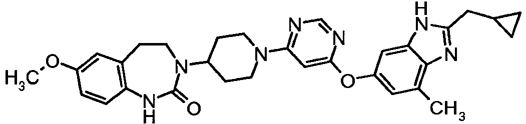 |
| (34) | 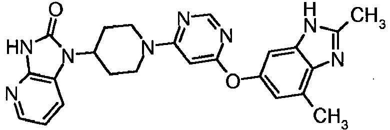 |
| (35) | 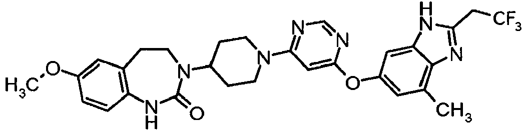 |
| (36) | 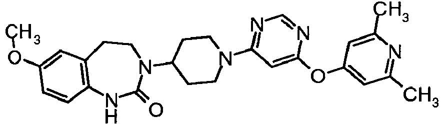 |
| (37) | 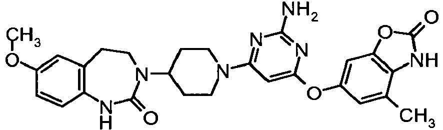 |
| (38) | 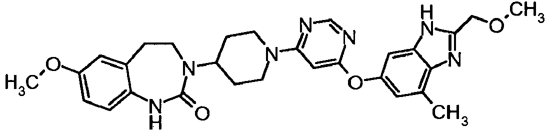 |
| (39) | 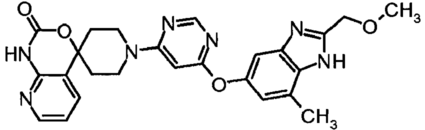 |
| (40) | 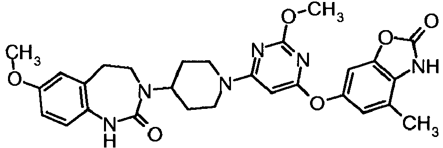 |
| (41) | 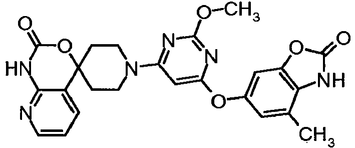 |
| (42) | 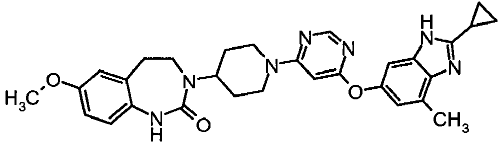 |
| (43) | 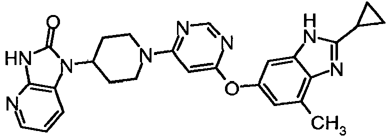 |
| (44) | 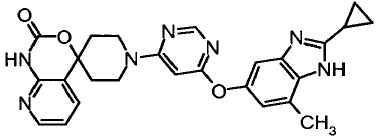 |
| (45) | 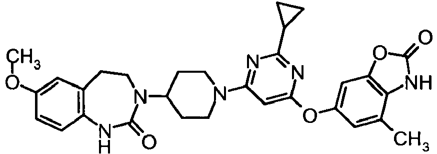 |
| (46) | 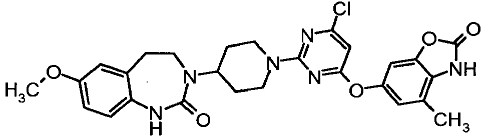 |
| (47) | 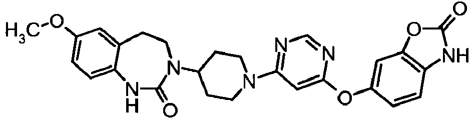 |
| (48) | 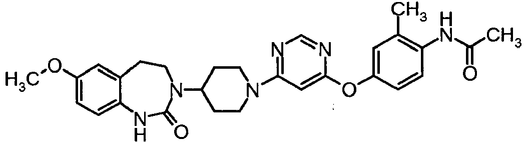 |
| (49) | 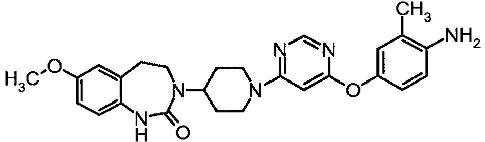 |
| (50) | 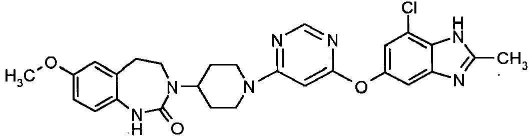 |
| (51) | 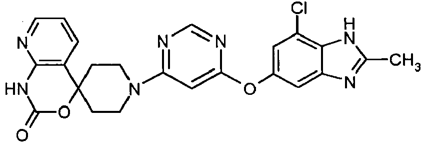 |
| (52) | 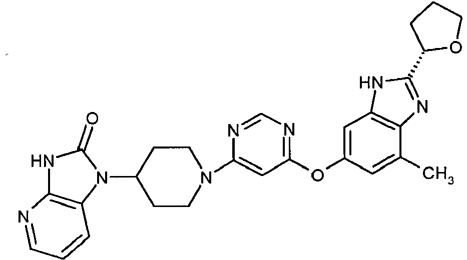 |
| (53) | 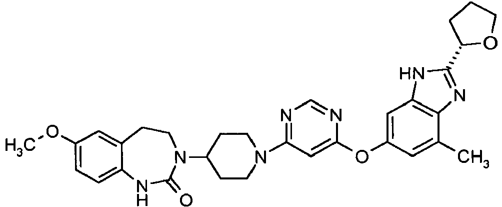 |
| (54) | 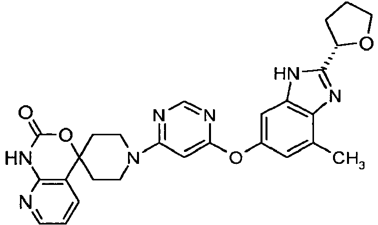 |
| (55) | 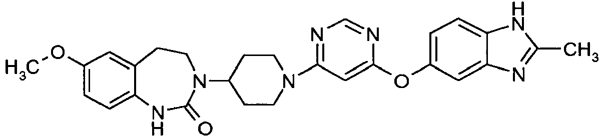 |
| (56) | 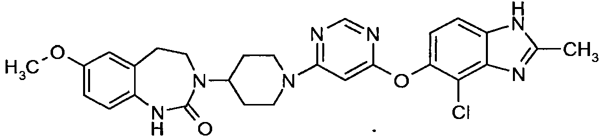 |
| (57) | 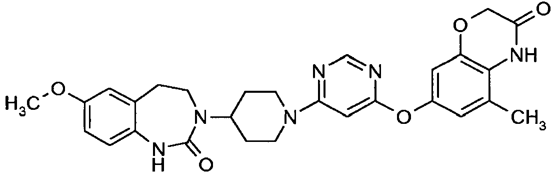 |
| (58) | 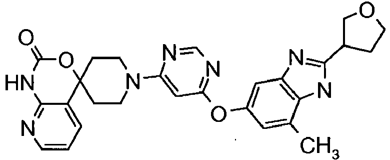 |
| (59) | 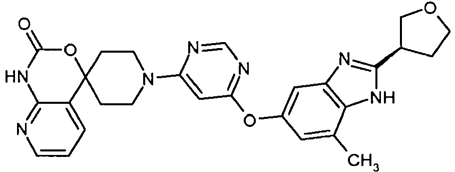 |
| (60) | 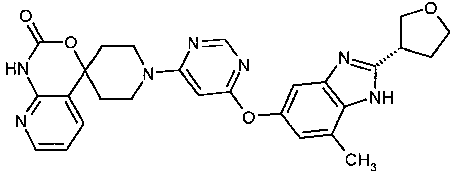 |
| (61) | 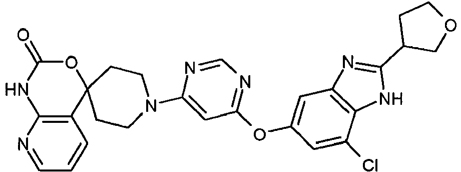 |
| (62) | 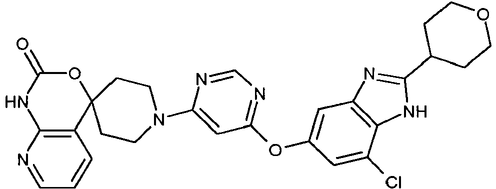 |
| (63) | 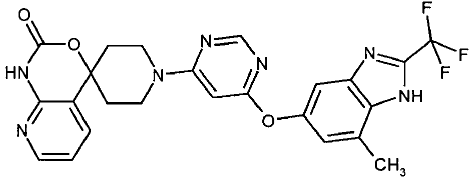 |
| (64) | 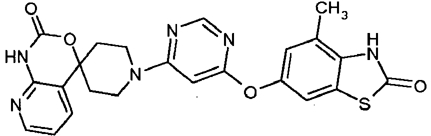 |
| (65) | 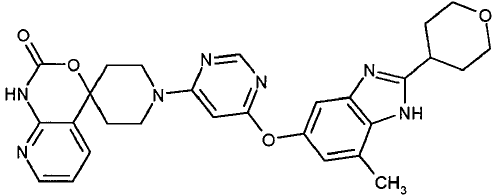 |
| (66) | 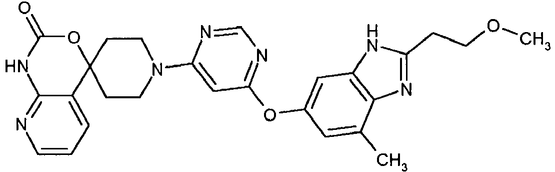 |
| (67) | 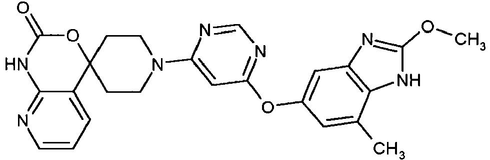 |
| (68) | 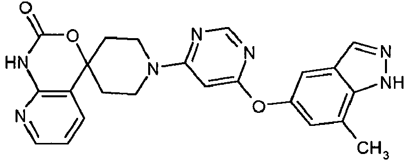 |
| (69) | 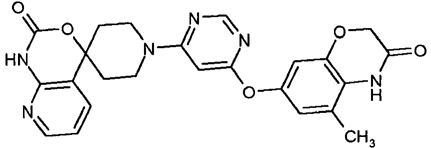 |
| (70) | 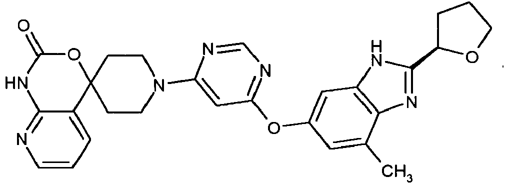 |
| (71) | 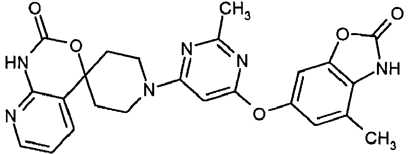 |
| (72) | 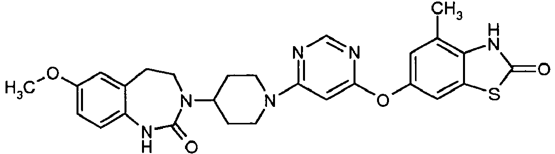 |
| (73) | 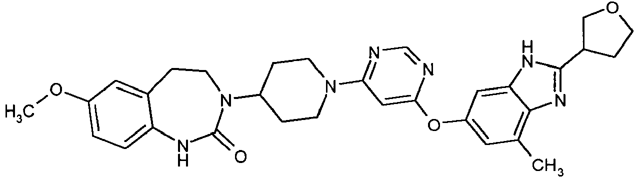 |
| (74) | 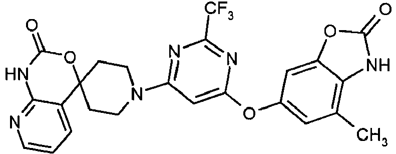 |
| (75) | 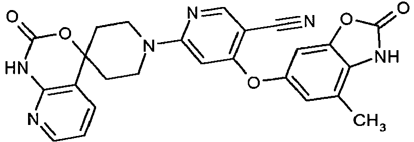 |
| (76) | 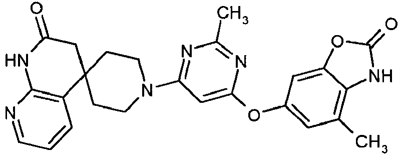 |
| (77) | 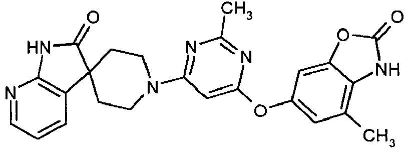 |
| (78) | 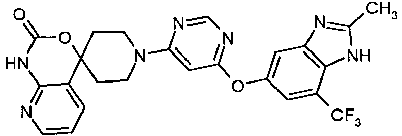 |
| (79) | 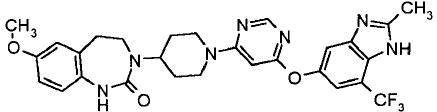 |
| (80) | 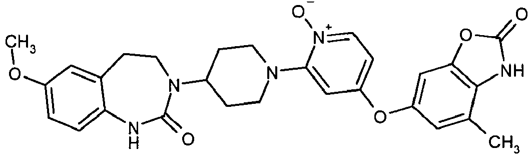 |

deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

### VERWENDETE BEGRIFFE UND DEFINITIONEN

Die vorliegende Beschreibung der Erfindung soll in Übereinstimmung mit den Gesetzmäßigkeiten und Regeln der chemischen Bindungen aufgefasst werden.

Die Verbindungen, die von dieser Erfindung umfasst sind, sind jene, die auch chemisch stabil sind.

Soweit nicht anders angegeben, sind alle Substituenten voneinander unabhängig. Sollten an einer Gruppe z. B. mehrere C₁₋₄-Alkylgruppen als Substituenten sein, so könnte, im Fall von drei Substituenten C₁₋₄-Alkyl unabhängig voneinander einmal Methyl, einmal Ethyl und einmal *n*-Propyl bedeuten.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden. Beispielsweise wird ein Phenyl-Rest wie folgt dargestellt:

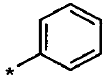

Weiterhin wird das auf den Verknüpfungspunkt folgende Atom des Substituenten als das Atom mit der Positionsnummer 1 verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₃-Alkyl" (auch soweit sie Bestandteil anderer Reste sind) werden verzweigte und unverzweigte Alkylgruppen mit 1 bis 3 Kohlenstoffatomen, unter dem Begriff "C₁₋₄-Alkyl" werden verzweigte und unverzweigte Alkylgruppen mit 1 bis 4 Kohlenstoffatomen und unter dem Begriff "C₁₋₆-Alkyl" werden verzweigte und unverzweigte Alkylgruppen mit 1 bis 6 Kohlenstoffatomen verstanden. Beispielsweise werden hierfür genannt: Methyl, Ethyl, *n*-Propyl, *iso*-Propyl, *n*-Butyl, *iso*-Butyl, sec-Butyl, *tert*-Butyl, Pentyl, Neopentyl oder *n*-Hexyl. Gegebenenfalls werden für die vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl und Butyl alle denkbaren isomeren Formen der jeweiligen Reste. So umfasst beispielsweise Propyl *n*-Propyl und *iso*-Propyl, Butyl umfasst *iso*-Butyl, sec-Butyl und *tert*-Butyl etc.

Unter dem Begriff "C₁₋₆-Alkylen" (auch soweit sie Bestandteil anderer Reste sind) werden verzweigte und unverzweigte Alkylengruppen mit 1 bis 6 Kohlenstoffatomen, unter dem Begriff "C₁₋₄-Alkylen" werden verzweigte und unverzweigte Alkylengruppen mit 1 bis 4 Kohlenstoffatomen, unter dem Begriff "C₁₋₃-Alkylen" werden verzweigte und unverzweigte Alkylengruppen mit 1 bis 3 Kohlenstoffatomen und unter dem Begriff "C₀₋₃-Alkylen" werden verzweigte und unverzweigte Alkylengruppen mit 0 bis 3 Kohlenstoffatomen verstanden. Beispielsweise werden hierfür genannt: Methylen, Ethylen, Propylen, 1-Methylethylen, Butylen, 1-Methylpropylen, 1,1-Dimethylethylen, 1,2-Dimethylethylen, Pentylen, 1,1-Dimethylpropylen, 2,2-Dimethylpropylen, 1,2-Dimethylpropylen, 1,3-Dimethylpropylen oder Hexylen. Sofern nicht anders beschrieben, umfasst die Definition Propylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1,1-Dimethylethylen, 1,2-Dimethylethylen. Die Definition für C₀-Alkylen bedeutet eine Bindung.

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen.

Bevorzugt sind Alkenylgruppen mit 2 bis 4 Kohlenstoffatomen. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc..

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc..

Unter dem Begriff "C₃₋₆-Cycloalkyl" (auch soweit sie Bestandteil anderer Reste sind) werden cyclische Alkylgruppen mit 3 bis 6 Kohlenstoffatomen und unter dem Begriff "C₅₋₆-Cycloalkyl" werden cyclische Alkylgruppen mit 5 bis 6 Kohlenstoffatomen verstanden. Beispielsweise werden hierfür genannt: Cyclopropyl, Cyclobutyl, Cyclopentyl oder Cyclohexyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "C₅₋₆-Cycloalkenyl" (auch soweit sie Bestandteil anderer Reste sind) werden cyclische Alkenylgruppen mit 5 oder 6 Kohlenstoffatomen verstanden, die eine ungesättigte Bindung enthalten. Beispielsweise werden hierfür genannt: Cyclopentenyl oder Cyclohexenyl. Soweit nicht anders beschrieben, können die cyclischen Alkenylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Heterocyclyl" oder "Heterocyclus" werden, soweit in den Definitionen nicht anders beschrieben, stabile 5-, 6- oder 7-gliedrige monocyclische oder 8-, 9-, 10-oder 11-gliedrige bicyclische heterocyclische Ringsysteme verstanden, die in mindestens einem Ring kein aromatisches Ringsystem bilden und neben Kohlenstoffatomen ein bis vier Heteroatome tragen können, die ausgewählt sind aus der Gruppe bestehend aus Stickstoff, Sauerstoff und Schwefel. Dabei können sowohl Stickstoffatome als auch Schwefelatome optional oxidiert sein und Stickstoffatome können quaternisiert sein. Der heterocyclische Ring kann eine oder zwei Carbonyl-, Thiocarbonyl- oder Cyaniminogruppen benachbart zu einem Stickstoffatom enthalten. Die voranstehend genannten Heterocyclen können über ein Kohlenstoffatom oder über ein Stickstoffatom mit dem restlichen Molekül verknüpft sein.

Soweit nicht anders beschrieben, können die Heterocyclen substituiert sein mit einem oder mehreren Resten ausgewählt aus der Gruppe bestehend aus:
(a) OH, NO₂, CN, OCF₃, OCHF₂, OCH₂F, NH₂,
(b) Halogen, vorzugsweise Fluor oder Chlor,
(c) C₁₋₆-Alkyl, vorzugsweise C₁₋₃-Alkyl, besonders bevorzugt Ethyl, Methyl, *iso-*Propyl oder *tert*-Butyl,
(d) -SO₂-O-C₁₋₃-Alkyl, vorzugsweise -O-Methyl,
(e) -O-C₁₋₃-Alkyl, vorzugsweise -O-Methyl oder -O-Ethyl,
(f) COOH, COO-C₁₋₃-Alkyl, vorzugsweise CO-O-Methyl oder CO-O-Ethyl, wobei die Reste gleich oder verschieden sein können.

Als Beispiele seien folgende Verbindungen genannt, sollen jedoch nicht auf diese beschränkt sein: Azetidin, Oxetan, Thietan, Thietandioxid, Tetrahydrofuran, Dihydrofuran, Dioxalan, Imidazolidin, Imidazolin, Imidazolidinon, Dihydroimidazolon, Oxazolin, Oxazolidin, Oxazolidinon, Pyrrolidinon, Dihydropyrazol, Pyrrolidin, Pyrrolin, Morpholin, Tetrahydropyridin, Dihydropyran, Tetrahydropyran, Dioxan, Piperazin, Piperidin, Piperazinon, Piperidinon, Pyran, Thiomorpholin-S-oxid, Thiomorpholin-S-dioxid, Thiomorpholin, Dihydrooxazin, Morpholindion, Morpholinthion, Perhydrothiazindioxid, ε-Caprolactam, Oxazepanon, Diazepanon, Thiazepanon, Perhydroazepin, Dihydrochinazolinon, Dihydroindol, Dihydroisoindol, Benzoxazolon, Benzimidazolon, Chromanon, Tetrahydrochinolin, Tetrahydrobenzoxazol, Tetrahydrobenzisoxazol, Tetrahydrobenzthiophen, Tetrahydrothieno-pyridin, Tetrahydrobenzofuran, Tetrahydro-oxazolopyridin, Tetrahydro-isoxazolopyridin.

Bevorzugt im Sinne dieser Erfindung seien folgende Heterocyclen:

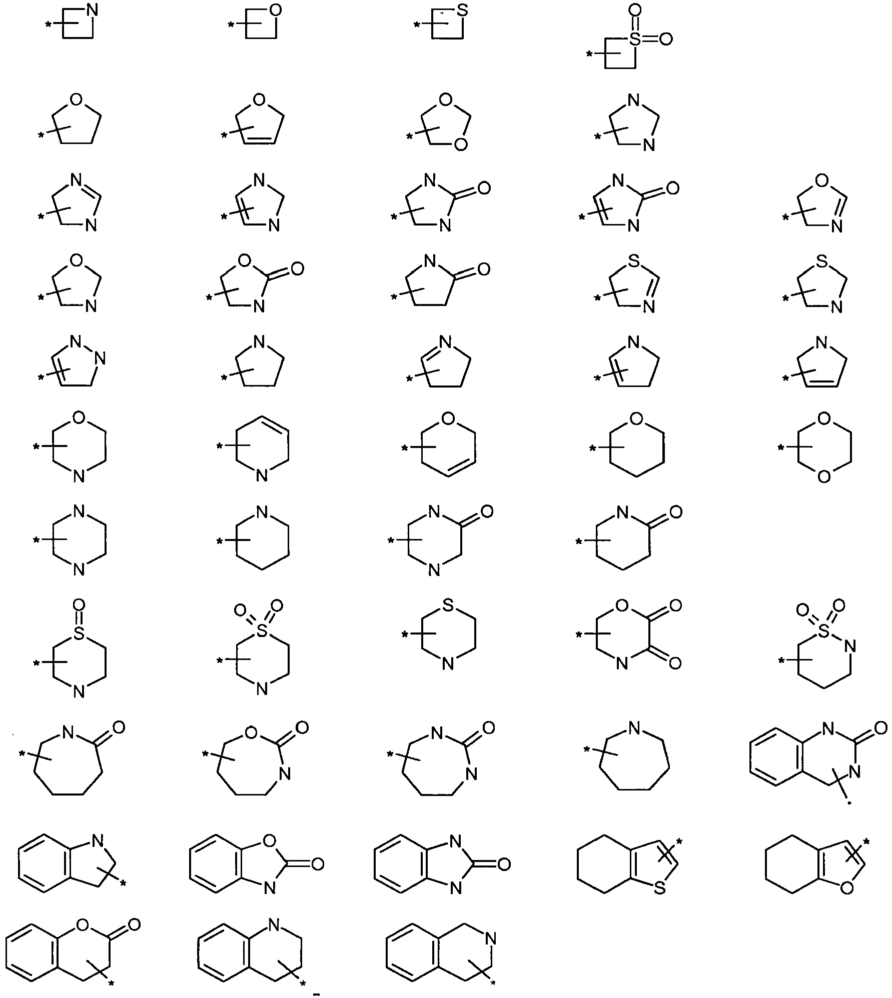

Unter dem Begriff "Aryl" (auch soweit sie Bestandteil anderer Reste sind) werden monocyclische aromatische Ringsysteme mit 6 Kohlenstoffatomen oder bicyclische aromatische Ringsysteme mit 10 Kohlenstoffatomen verstanden. Beispielsweise werden hierfür Phenyl, 1-Naphthyl oder 2-Naphthyl genannt; bevorzugter Arylrest ist Phenyl. Soweit nicht anders beschrieben, können die Aromaten substituiert sein mit einem oder mehreren Resten ausgewählt aus der Gruppe bestehend aus:
(a) OH, NO₂, CN, OCF₃, OCHF₂, OCH₂F, NH₂,
(b) Halogen, vorzugsweise Fluor oder Chlor,
(c) C₁₋₆-Alkyl, vorzugsweise C₁₋₃-Alkyl, besonders bevorzugt Ethyl, Methyl, *iso-*Propyl oder *tert*-Butyl,
(d) -SO₂-O-C₁₋₃-Alkyl, vorzugsweise -O-Methyl,
(e) -O-C₁₋₃-Alkyl, vorzugsweise -O-Methyl oder -O-Ethyl,
(f) COOH, CO-O-C₁₋₃-Alkyl, vorzugsweise CO-O-Methyl oder CO-O-Ethyl, wobei die Reste gleich oder verschieden sein können.

Unter dem Begriff "Heteroaryl" werden stabile fünf- oder sechsgliedrige heterocyclische Aromaten oder 8- bis 10-gliedrige bicyclische Heteroarylringe verstanden, die in jedem Ring ein, zwei oder drei Heteroatome, ausgewählt aus der Gruppe Sauerstoff, Schwefel und Stickstoff, enthalten können und zusätzlich so viele konjugierte Doppelbindungen enthalten, dass ein aromatisches System gebildet wird. Als Beispiele für fünf- oder sechsgliedrige heterocyclische Aromaten werden genannt, sollen aber nicht auf diese beschränkt sein:
Furan, Pyrrol, Thiophen, Pyrazol, Imidazol, Oxazol, Thiazol, Isothiazol, Isoxazol, Oxadiazol, Triazol, Tetrazol, Furazan, Thiadiazol, Pyridin, Pyrimidin, Pyrazin, Pyridazin, Triazin.

Bevorzugt im Sinne dieser Erfindung seien folgende fünfgliedrige heterocyclische Aromaten:

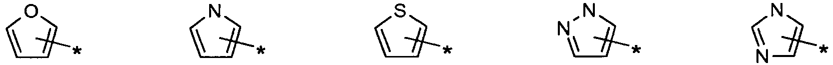

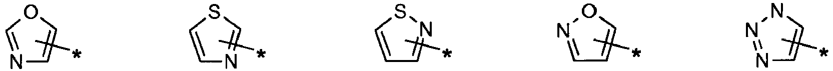

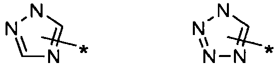

Bevorzugt im Sinne dieser Erfindung seien folgende sechsgliedrige heterocyclische Aromaten:

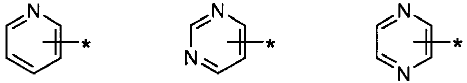

Als Beispiele für 9- oder 10-gliedrige bicyclische Heteroarylringe werden genannt, sollen aber nicht auf diese beschränkt sein:
Indol, Isoindol, Indazol, Indolizin, Benzofuran, Benzthiophen, Benzimidazol, Benzoxazol, Benzthiazol, Benztriazol, Benzisoxazol, Benzisothiazol, Chinolin, Isochinolin, Cinnolin, Phtalazin, Chinoxalin, Chinazolin, Pyridopyrimidin, Pyridopyrazin, Pyridopyridazin, Pyrimidopyrimidin, Pteridin, Purin, Chinolizin, Benzoxazolcarbonitril, Chinolin, Isochinolin, Chinolizin, Pteridin, Purin, Chinolizin, Benzoxazol-carbonitril.

Bevorzugt im Sinne dieser Erfindung seien folgende bicyclische Heteroarylringe:

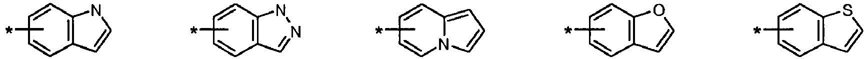

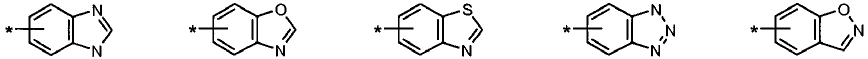

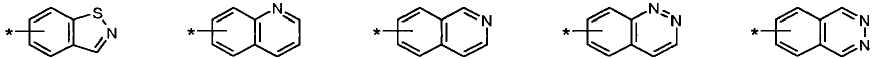

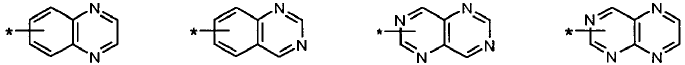

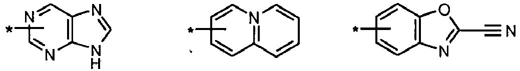

Soweit nicht anders beschrieben, können die voranstehend genannten Heteroaryle substituiert sein mit einem oder mehreren Resten ausgewählt aus der Gruppe bestehend aus:
(a) OH, NO₂, CN, OCF₃, OCHF₂, OCH₂F, NH₂,
(b) Halogen, vorzugsweise Fluor oder Chlor,
(c) C₁₋₆-Alkyl, vorzugsweise C₁₋₃-Alkyl, besonders bevorzugt Ethyl, Methyl, *iso-*Propyl oder *tert*-Butyl,
(d) -SO₂-O-C₁₋₃-Alkyl, vorzugsweise -O-Methyl,
(e) -O-C₁₋₃-Alkyl, vorzugsweise -O-Methyl oder -O-Ethyl,
(f) COOH, CO-O-C₁₋₃-Alkyl, vorzugsweise CO-O-Methyl oder CO-O-Ethyl, wobei die Reste gleich oder verschieden sein können.

Bicyclische Heteroarylringe können dabei vorzugsweise im Phenylrest substituiert sein.

Unter dem Begriff "Halogen" werden Fluor-, Chlor-, Brom- oder lodatome verstanden.

Verbindungen der allgemeinen Formel **I** können Säuregruppen besitzen, hauptsächlich Carboxylgruppen, und/oder basische Gruppen wie z.B. Aminofunktionen. Verbindungen der allgemeinen Formel **I** können deshalb als innere Salze, als Salze mit pharmazeutisch verwendbaren anorganischen Säuren wie beispielsweise Bromwasserstoffsäure, Phosphorsäure, Salpetersäure, Salzsäure, Schwefelsäure, Methansulfonsäure, Ethansulfonsäure, Benzolsulfonsäure, *p*-Toluolsulfonsäure oder organischen Säuren wie beispielsweise Äpfelsäure, Bernsteinsäure, Essigsäure, Fumarsäure, Maleinsäure, Mandelsäure, Milchsäure, Weinsäure, Zitronensäure oder als Salze mit pharmazeutisch verwendbaren Basen wie Alkali- oder Erdalkalimetallhydroxiden, beispielsweise Natriumhydroxid oder Kaliumhydroxid, oder Carbonaten, Ammoniak, Zink- oder Ammoniumhydroxiden oder organischen Aminen wie z.B. Diethylamin, Triethylamin, Ethanolamin, Diethanolamin, Triethanolamin, Cyclohexylamin, Dicyclohexylamin u.a. vorliegen.

Die erfindungsgemäßen Verbindungen können als Racemate vorliegen, sofern sie nur ein Chiralitätselement besitzen, sie können aber auch als reine Enantiomere, d.h. in (R)- oder (S)-Form gewonnen werden.

Verbindungen mit einer substituierten Kohlenstoff-Doppelbindung können sowohl in der *E-*als auch Z-Form vorliegen.

Die folgenden stickstoffhaltigen Heteroaryle können in unterschiedlichen tautomeren Formen vorliegen:

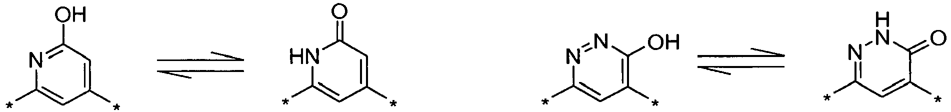

Das bedeutet, dass die die jeweils dargestellte Verbindung nicht auf eine tautomere Form beschränkt ist, sondern alle tautomeren Formen umfasst.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enantiomere, aus denen sich die erwähnten Racemate zusammensetzen.

Gegenstand der Erfindung sind die jeweiligen Verbindungen gegebenenfalls in Form der einzelnen optischen Isomere, Mischungen der einzelnen Enantiomere oder Racemate, in Form der Tautomere sowie in Form der freien Basen oder der entsprechenden Säureadditionssalze mit pharmakologisch unbedenklichen Säuren.

### HERSTELLVERFAHREN

Gegenstand der Erfindung ist weiterhin ein Verfahren zur Herstellung der Verbindungen der allgemeinen Formel **I**, in der die Substituenten **A, U, V, X, Y, R¹**, **R²** und **R³** wie voranstehend erwähnt definiert sind.

Einige Methoden zur Herstellung der erfindungsgemäßen Verbindungen der allgemeinen Formel **I** 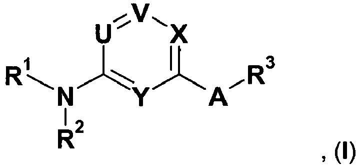 in der **A, U, V, X, Y, R¹**, **R²** und **R³** wie voranstehend erwähnt definiert sind, sind in den folgenden Syntheseschemata und Beispielen dargestellt.

In einigen Fällen kann die Reihenfolge bei der Durchführung der Reaktionsschemata variiert werden, um die Reaktionen zu vereinfachen oder um unerwünschte Nebenprodukte zu verhindern. Die nachfolgenden Beispiele sind genannt, um die Erfindung vollständig verständlich zu machen. Die Beispiele sollen der Anschaulichkeit der Erfindung dienen und sollen die Erfindung in keinster Weise einschränken.

Die erfindungsgemäßen Verbindungen können gemäß den dargestellten Schemata und spezifischen Beispielen oder entsprechenden Modifikationen davon hergestellt werden. Von diesen Reaktionen können auch Abwandlungen eingesetzt werden, die einem Fachmann bekannt sind, hier aber nicht detailliert beschrieben sind. Die allgemeinen Verfahren zur Herstellung der erfindungsgemäßen Verbindungen erschließen sich einem Fachmann beim Anblick der folgenden Schemata.

Ausgangsverbindungen sind kommerziell verfügbar oder werden gemäß Verfahren hergestellt, die in der Literatur beschrieben sind, in dem Fachgebiet dem Fachmann bekannt sind oder wie sie hierin beschrieben sind. Vor Durchführung der Reaktion können in den Verbindungen entsprechende funktionelle Gruppen durch übliche Schutzreste geschützt werden. Diese Schutzgruppen können auf einer geeigneten Stufe innerhalb der Reaktionssequenz nach für den Fachmann geläufigen Methoden wieder abgespalten werden.

Bei den nachstehend beschriebenen Umsetzungen können gegebenenfalls vorhandene reaktive Gruppen wie Hydroxy-, Carboxy-, Amino-, Alkylamino-, Amid- oder Iminogruppen während der Umsetzung durch übliche Schutzgruppen geschützt werden, welche nach der Umsetzung wieder abgespalten werden.

Beispielsweise kommt
- als Schutzrest für eine Hydroxygruppe die Methoxy-, Benzyloxy-,Trimethylsilyl-, Acetyl-, Benzoyl-, tert.-Butyl-, Trityl-, Benzyl- oder Tetrahydropyranylgruppe,
- als Schutzreste für eine Carboxylgruppe die Trimethylsilyl-, Methyl-,Ethyl-, tert.-Butyl-, Benzyl- oder Tetrahydropyranylgruppe, und
- als Schutzreste für eine Amidgruppe die N-Methoxymethyl- (MOM), N-Benzyloxymethyl- (BOM), N-(Trimethylsilyl)ethoxymethyl (SEM), N-*tert*-Butyldimethyl-siloxymethyl, N-tert-Butyldimethylsilyl- (TBDMS), N-Triisopropylsilyl- (TIPS), N-Benzyl-, N-4-Methoxybenzyl- (PMB), N-Triphenylmethyl- (Tr), N-tert-Butoxycarbonyl- (BOC), N-Benzyloxycarbonyl- (Cbz), N-Trimethylsilylethylsulfonyl- (SES)
- als Schutzrest für eine Amino-, Alkylamino- oder Iminogruppe die Acetyl-, Trifluor-acetyl-, Benzoyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe,
in Betracht.

Weitere Schutzgruppen und deren Abspaltung sind in T.W. Greene, P.G.M. Wuts, "Protective Groups in Organic Synthesis", Wiley, 1991 und 1999 beschrieben.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0°C und 100°C, vorzugsweise bei Temperaturen zwischen 10°C und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0°C und 50°C, vorzugsweise jedoch bei Raumtemperatur, und bei einem Wasserstoffdruck von 1 bis 7 bar,vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0°C und 50°C, vorzugsweise jedoch bei Raumtemperatur, erfolgen.

Die Abspaltung einer Methoxygruppe erfolgt zweckmäßigerweise in Gegenwart von Bortribromid in einem Lösungsmittel wie Methylenchlorid bei Temperaturen zwischen -35°C und -25°C.

Die Abspaltung eines 2,4-Dimethoxybenzylrestes erfolgt vorzugsweise in Trifluoressigsäure in Gegenwart von Anisol.

Die Abspaltung eines tert.-Butyl- oder tert.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder *n*-Butylamin in einem Lösungsmittel wie Methanol, Ethanol, Isopropanol, Toluol∼Wasser oder Dioxan bei Temperaturen zwischen 20°C und 50°C.

Die Abspaltung eines Methoxymethyl-Rests kann in Gegenwart einer Säure wie konzentrierter Salsäure in einem Lösungsmittel wie Dimethoxyethan durchgeführt werden. Alternativ kann eine Säure wie Trifluoressigsäure auch ohne Lösungsmittel eingesetzt werden.

Die Abspaltung eines N-(Trimethylsilyl)ethoxymethyl-Rests kann in Gegenwart von Tetrabutylammoniumfluorid und 1,3-Dimethyl-3,4,5,6-Tetrahydro-2(1*H*)-Pyrimidon durchgeführt werden. Alternativ kann die SEM-Schutzgruppe auch mit einer Säure wie Chlorwasserstoff in einem organischen Lösungsmittel wie Dioxan oder Ethanol durchgeführt werden.

Die Abspaltung eines Allyloxycarbonylrestes erfolgt durch Behandlung mit einer katalytischen Menge Tetrakis-(triphenylphosphin)-palladium(0) vorzugsweise in einem Lösungsmittel wie Tetrahydrofuran und vorzugsweise in Gegenwart eines Überschusses von einer Base wie Morpholin bei Temperaturen zwischen 0°C und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo-[2.2.2]octan bei Temperaturen zwischen 20°C und 70°C.

Die folgenden Verfahren zur Herstellung der erfindungsgemäßen Verbindungen der allgemeinen Formel **I** und ihrer Vorstufen haben sich besonders bewährt:

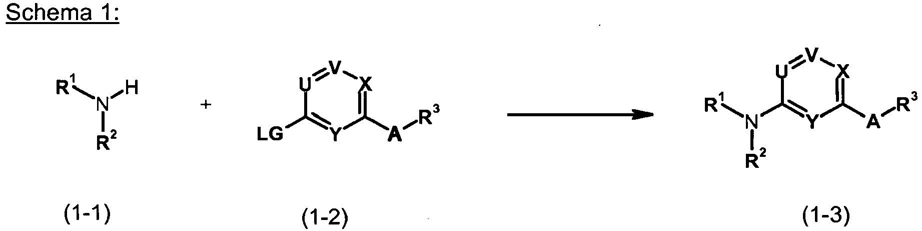

Die Herstellung einer Verbindung der allgemeinen Formel (1-3), in der **A, U, V, X, Y, R¹**, **R²** und **R³** wie voranstehend erwähnt definiert sind, kann durch Reaktion eines Amins oder Anilins der allgemeinen Formel (1-1), in der **R¹** und **R²** wie eingangs erwähnt definiert sind, mit einer elektronenarmen Verbindung der allgemeinen Formel (1-2), in der **U, V, X, Y, A** und **R³** wie eingangs erwähnt definiert sind und **LG** eine Austrittsgruppe darstellt, erfolgen. Als Austrittsgruppe **LG** können Halogenide, vorzugsweise Chloride und Bromide, -SO₂CH₃, -OSO₂CH₃, -OSO₂C₆H₄-CH₃ oder -S-CH₃ (-S-CH₃ erfordert die weitere Umsetzung mit einem organischen Peroxid, um in die eigentliche Austrittsgruppe überführt werden zu können) etc. fungieren, müssen aber nicht auf diese beschränkt sein. Ganz besonders bevorzugt ist die Verwendung von Chloriden.

Die Reaktion kann über eine nucleophile aromatische Substitution in einem inerten Lösungsmittel unter Verwendung einer Hilfsbase in einem Temperaturbereich von 0°C bis zum Rückfluss des Lösungsmittels erfolgen. Nucleophile aromatische Substitutionen werden in einem geeigneten, inerten Lösungsmittel, wie Tetrahydrofuran, Toluol, Xylol, Dialkylformamide (besonders bevorzugt Dimethylformamid), cyclische Amide (besonders bevorzugt N-Methylpyrrolidon), 1,4-Dioxan, Acetonitril oder in Lösungsmittelgemischen durchgeführt. Als geeignete Hilfsbasen können tertiäre Amine wie Triethylamin oder Ethyldiisopropylamin, Alkalimetallcarbonate wie Kaliumcarbonat oder Natriumcarbonat Natriumhydrid (NaH) oder Lithiumdiisopropylamid (LDA) verwendet werden. Dabei muss das verwendete inerte Lösungsmittel kompatibel sein mit der verwendeten Base. Bevorzugt wird die Reaktion in Dimethylformamid, bei Temperaturen zwischen Raumtemperatur und Rückfluss des Lösungsmittels, in Gegenwart einer tertiären Aminbase durchgeführt.

Alternativ können Strukturen der allgemeinen Form (1-3), in der **A, U, V, X, Y, R¹**, **R²** und **R³** wie voranstehend erwähnt definiert sind, über Übergangsmetall-katalysierte Reaktionen synthetisiert werden. Dabei kann ein Amin oder Anilin der allgemeinen Formel (1-1), in der **R¹** und **R²** wie eingangs erwähnt definiert sind, mit einer Verbindung der allgemeinen Formel (1-2), in der **A, U, V, X, Y,** und **R³** wie eingangs erwähnt definiert sind und **LG** eine Austrittsgruppe darstellt, in einem inerten Lösungsmittel bei Anwesenheit eines Katalysators und einer Hilfsbase reagieren. Für den Katalysator kann zusätzlich ein geeigneter Ligand verwendet werden. Als Austrittsgruppe **LG** können Chloride, Bromide, Iodide, Trifluoracetate, Trifluormethansulfonate, Methansulfonate und Toluolsulfonate fungieren, müssen aber nicht auf diese beschränkt sein. Als inerte Lösungsmittel können Xylol, Tetrahydrofuran, Dimethylformamid, Dimethoxyethan, Toluol, Benzol, *tert*-Butanol, 1,4-Dioxan, Acetonitril oder Lösungsmittelgemische verwendet werden. Bevorzugtes Lösungsmittel ist Xylol. Geeignete Basen sind besonders Amin-Basen wie z.B. Triethylamin oder Diisopropylethylamin oder auch anorganische Basen wie Cäsiumcarbonat, Cäsiumacetat, Kaliumcarbonat, Kalium-*tert*-Butylat, Natriumcarbonat, Natrium-*tert*-Butylat oder Kaliumphosphat. Bevorzugte Reaktionstemperaturen sind von RT bis Rückfluss des Lösungsmittels bei Normaldruck. Typische Katalysatoren sind z.B. Übergangsmetall-Katalysatoren, wie z.B. Palladium-Katalysatoren der Art Tris(dibenzylidenaceton)-dipalladium(0), Tetrakis-(triphenylphosphin)-palladium(0), Palladium-(II)-acetat, Pd(PPh₃)₂Cl₂, Pd(CH₃CN)₂Cl₂, Pd(dppf)Cl₂ oder Palladium(II)-chlorid. Typische Liganden sind z.B. Triphenylphosphin, Triphenylarsen, 2,2'-Bis(dipenylphosphino)-1,1'-binaphtyl (BINAP), 2-Di-*tert*-Butylphosphino-2',4',6'-triisopropylbiphenyl (XPhos), 4,5-Bis-diphenyl-phosphanyl-9,9-dimethyl-9H-Xanthen (XantPhos), oder 2-(Di-*tert*-butylphosphino)-biphenyl.

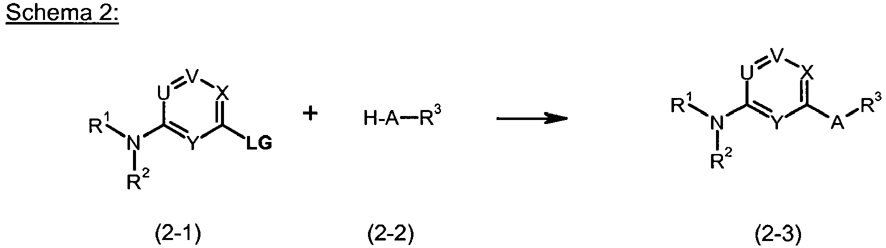

Die Herstellung einer Verbindung der allgemeinen Formel (2-3), in der **U, V, X, Y, R¹**, **R²** und **R³** wie voranstehend erwähnt definiert sind und **A** ein Schwefel- oder Sauerstoffatom oder die Gruppe -NH- darstellt, kann - wie in Schema 2 gezeigt - durch Kupplung einer nucleophilen Verbindung der allgemeinen Formel (2-2), in der **A** ein Schwefel-, Stickstoff- oder Sauerstoffatom darstellt und **R³** wie eingangs erwähnt definiert ist, mit einer Verbindung der allgemeinen Formel (2-1), in der **U, V, X, Y, R¹** und **R²** wie voranstehend erwähnt definiert sind und **LG** eine Austrittsgruppe darstellt, erfolgen. Als Austrittsgruppe **LG** können Halogenide wie z. B.Chloride, Bromide und Fluoride, -SO₂CH₃, -OSO₂CH₃, - NO₂, -OSO₂C₆H₄-CH₃ oder -S-CH₃ (-S-CH₃ erfordert die weitere Umsetzung mit einem organischen Peroxid, um in die eigentliche Austrittsgruppe überführt werden zu können) etc. fungieren, müssen aber nicht auf diese beschränkt sein. Ganz besonders bevorzugt ist die Verwendung von Chloriden als Austrittsgruppe **LG.**

Die Reaktion kann über eine nucleophile aromatische Substitution in einem inerten Lösungsmittel unter Verwendung einer Hilfsbase in einem Temperaturbereich von 0°C bis zum Rückfluss des Lösungsmittels erfolgen. Nucleophile aromatische Substitutionen werden in einem geeigneten, inerten Lösungsmittel, wie Tetrahydrofuran, Toluol, Xylol, Dialkylformamide (besonders bevorzugt Dimethylformamid), cyclische Amide (besonders bevorzugt N-Methylpyrrolidon), 1,4-Dioxan, Acetonitril oder in Lösungsmittelgemischen durchgeführt. Als geeignete Hilfsbasen können beispielsweise tertiäre Amine wie Triethylamin oder Ethyldiisopropylamin, Alkoholate der Alkali- und Erdalkalimetalle wie Natrium-tert.-Butylat oder Kalium-tert.-Butylat, Alkalimetallcarbonate wie Kaliumcarbonat, Cäsiumcarbonat oder Natriumcarbonat, Natriumhydrid (NaH) oder Lithiumdiisopropylamid (LDA) verwendet werden. Dabei muss das verwendete inerte Lösungsmittel kompatibel sein mit der verwendeten Base. Bevorzugt wird die Reaktion in N-Methylpyrrolidon bei Temperaturen zwischen Raumtemperatur und Rückfluss des Lösungsmittels in Gegenwart von Kalium-*tert*-Butylat durchgeführt.

Alternativ können Strukturen der allgemeinen Form (2-3), in der **U, V, X, Y, R¹**, **R²** und **R³** wie voranstehend erwähnt definiert sind und **A** ein Schwefel- oder Sauerstoffatom oder die Gruppe -N-H- darstellt, über Übergangsmetall-katalysierte Reaktionen nach bekannten Verfahren synthetisiert werden (siehe z. B Alex R. Muci und Stephen L. Buchwald, Topics in Current Chemistry 2002, 219, 133-209.). Dabei kann eine nucleophile Verbindung der allgemeinen Formel (2-2), in der **A** ein Schwefel-, Stickstoff- oder Sauerstoffatom darstellt und **R³** wie eingangs erwähnt definiert ist, mit einer Verbindung der allgemeinen Formel (2-1), in der **U, V, X, Y, R¹** und **R³** wie eingangs erwähnt definiert sind und **LG** eine Austrittsgruppe darstellt, in einem inerten Lösungsmittel bei Anwesenheit eines Katalysators und einer Hilfsbase reagieren. Für den Katalysator kann zusätzlich ein geeigneter Ligand verwendet werden. Als Austrittsgruppe **LG** können Chloride, Bromide, Iodide, Trifluoracetate, Trifluormethansulfonate, Methansulfonate und Toluolsulfonate fungieren, müssen aber nicht auf diese beschränkt sein.

Die Reaktion wird in einem geeigneten Lösungsmittelgemisch durchgeführt, das ein hinreichendes Lösungsvermögen für alle beteiligten Reaktanden hat, wobei auch heterogene Durchführungen möglich sind (z. B. Einsatz fast unlöslicher Basen). Vorzugsweise wird die Reaktion in polaren aprotischen oder protischen Lösungsmitteln durchgeführt. Bevorzugt werden ein oder Gemische mehrerer Lösungsmittel der Gruppe Tetrahydrofuran, 1,4-Dioxan, Isopropanol, tert.-Butanol, Toluol oder Xylol eingesetzt.

Als Hilfsbasen gut geeignet sind z. B. Hydroxide, Alkoholate und Fluoride der Alkali- und Erdalkalimetalle, Carbonate, Hydrogencarbonate und Phosphate der Alkalimetalle und ihre Gemische. Besonders geeignet sind die Basen der Gruppe Kaliumphosphat, Natriumphosphat, Kalium-tert.-Butylat, Natrium-tert.-Butylat, Cäsium-tert.-Butylat, Lithium-tert.-Butylat sowie die entsprechenden Isopropylate. Dabei wird die Base üblicherweise mindestens in äquimolarer Stoffmenge bezogen auf die Verbindung (2-2) eingesetzt, vorzugsweise werden 1.2 bis 3 Äquivalente Base verwendet.

Die Reaktion kann bei Temperaturen zwischen RT und dem Siedepunkt des verwendeten Lösungsmittels beim verwendeten Druck durchgeführt werden. Um eine schnellere Reaktion zu erzielen, ist die Durchführung bei erhöhten Temperaturen im Bereich von 0 C bis 240°C bevorzugt. Besonders bevorzugt ist der Temperaturbereich von RT bis 200°C, insbesondere von 50 bis 150°C. Die Konzentration der Reaktanden kann in weiten Bereichen variiert werden. Zweckmäßigerweise wird die Reaktion in einer möglichst hohen Konzentration durchgeführt, wobei die Löslichkeiten der Reaktionspartner und Reagenzien im jeweiligen Reaktionsmedium beachtet werden müssen.

Typische Katalysatoren sind z.B. Übergangsmetall-Katalysatoren, wie z.B. Palladium-Katalysatoren der Art Tris(dibenzylidenaceton)-dipalladium(0), Tetrakis-(triphenyl-phosphin)-palladium(0), Palladium-(II)-acetat, Pd(PPh₃)₂Cl₂, Pd(CH₃CN)₂Cl₂, Pd(dppf)Cl₂, Palladium(II)-chlorid oder Kupfer(I)-Trifluormethansulfonat (s. J. F. Marcoux, S. Doye, S. L. Buchwald J. Amer. Chem. Soc. 1997, 119, 10539-10540). Typische Liganden für Palladium-katalysierte Kupplungen sind z.B. Triphenylphosphin, Triphenylarsen, BINAP, XPhos, XantPhos, oder 2-(Di-*tert*-butylphosphino)biphenyl (siehe z. B. Angew. Chemie. Int. Ed. 2006, 45, 4321-4326). Die Auswahl des Katalysators und der Liganden muss dabei jeweils auf die elektronischen und sterischen Eigenschaften der beiden Reaktionspartner abgestimmt werden.

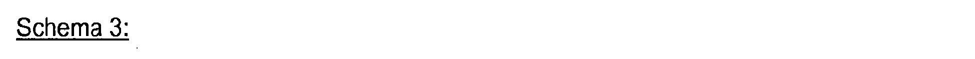

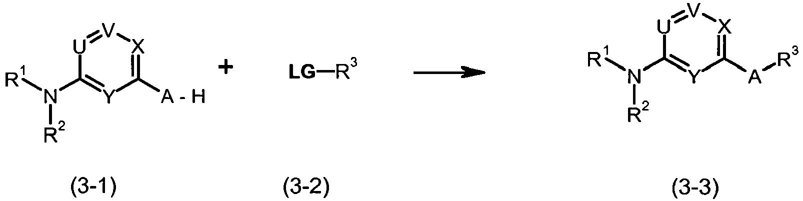

Die Herstellung einer Verbindung der allgemeinen Formel (3-3), in der **U, V, X, Y, R¹**, **R²** und **R³** wie voranstehend erwähnt definiert sind und **A** ein Schwefel- oder Sauerstoffatom oder die Gruppe -N-H- darstellt, kann - wie in Schema 3 gezeigt - durch Kupplung einer Verbindung der allgemeinen Formel (3-2), in der **R³** wie eingangs erwähnt definiert ist und **LG** eine Austrittsgruppe darstellt, mit einer Verbindung der allgemeinen Formel (3-1), in der **U, V, X, Y, R¹** und **R²** wie voranstehend erwähnt definiert sind und **A** ein Schwefel- oder Sauerstoffatom oder die Gruppe -N-H- darstellt, erfolgen. Als Austrittsgruppe **LG** können Halogenide, vorzugsweise Bromide und Iodide, -SO₂CF₃ etc. fungieren, müssen aber nicht auf diese beschränkt sein.

Die Reaktion wird in einem inerten Lösungsmittel unter Verwendung einer Hilfsbase in einem Temperaturbereich von 0°C bis zum Rückfluss des Lösungsmittels durchgeführt. Als Lösungsmittel eignen sich z. B. Tetrahydrofuran, Toluol, Xylol, Dialkylformamide (besonders bevorzugt Dimethylformamid), cyclische Amide (besonders bevorzugt N-Methylpyrrolidon), 1,4-Dioxan, Acetonitril oder Gemische aus diesen Lösungsmitteln. Als geeignete Hilfsbasen können tertiäre Amine wie Triethylamin oder Ethyldiisopropylamin, Alkalimetallcarbonate wie Kaliumcarbonat oder Natriumcarbonat Natriumhydrid (NaH) oder Lithiumdiisopropylamid (LDA) verwendet werden. Dabei muss das verwendete inerte Lösungsmittel kompatibel sein mit der verwendeten Base. Bevorzugt wird die Reaktion in Dimethylformamid, bei Temperaturen zwischen Raumtemperatur und Rückfluss des Lösungsmittels, in Gegenwart einer tertiären Aminbase durchgeführt.

Ist **A** ein Sauerstoffatom, so kann die Hydroxy-Gruppe in eine gute Austrittsgruppe überführt werden. Dies kann durch Verwendung eines Phosphin-Reagenzes (welches auch Festphasen gebunden sein kann) wie z.B. Triphenylphosphin, Trimethylphosphin, Triisopropylphosphit oder Tributyl-phosphin in Kombination mit einem Azodicarboxylat wie z.B. Diethyl-azodicarboxylat (DEAD), Düsopropylazodicarboxylat (DIAD), Di-*tert*-butylazodicarboxylat (DBAD), N,N,N',N'-Tetraisopropylazodicarboxylamid (TIPA), 1,1'-(Azodicarbonyl)-dipiperidin (ADDP) oder N,N,N',N'-Tetramethylazodicarboxylamid (TMAD) geschehen. Ebenso können fluorierte Varianten des Phosphin-Reagenzes und der Azo-Verbindung eingesetzt werden (für eine Zusammenfassung siehe Chem. Asian J. 2007**,** Eur. J. Org. Chem. 2004, 2763-2772.). Auch die Verwendung eines Phosphin-Ylids, wie z. B. (Cyanomethylen)-trimethylphosphin (CMMP) oder (Cyanomethylen)-tributylphosphin (CMBP) ist möglich (Tetrahedron Lett. 1996, 37, 2459-2462.). Zur Durchführung dieser Reaktion eignen sich insbesondere polare, aprotische Lösungsmittel wie z.B. Tetrahydrofuran, Toluol, Xylol, Benzol, 1,4-Dioxan, Dimethoxyethan, Acetonitril, Dichlormethan, Dichlorethan oder Lösungsmittelgemische; es kann aber auch ohne Lösungsmittel gearbeitet werden. Die Reaktion wird üblicherweise in einem Temperaturbereich von 0°C bis zum Rückfluss des Lösungsmittels, falls ohne Lösungsmittel gearbeitet wird in einem Temperaturbereich von RT bis zum Rückfluss der Azo-Verbindung, durchgeführt.

Alternativ können Strukturen der allgemeinen Form (3-3), in der **A, U, V, X, Y, R¹**, **R²** und **R³** wie voranstehend erwähnt definiert sind, über Übergangsmetall-katalysierte Reaktionen synthetisiert werden. Dabei kann eine Verbindung der allgemeinen Formel (3-2), in der **R³** wie eingangs erwähnt definiert ist und **LG** eine Austrittsgruppe darstellt, mit einer Verbindung der allgemeinen Formel (3-1), in der **R¹** und **R²** wie eingangs erwähnt definiert sind, in einem inerten Lösungsmittel bei Anwesenheit eines Katalysators und einer Hilfsbase reagieren.

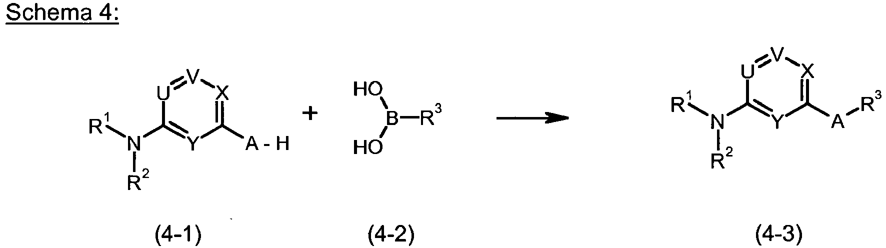

Die Herstellung einer Verbindung der allgemeinen Formel (4-3), in der **U, V, X, Y, R¹**, **R²** und **R³** wie voranstehend erwähnt definiert sind und **A** ein Sauerstoffatom oder die Gruppe -N-H- darstellt, kann - wie in Schema 4 gezeigt - durch Kupplung einer Boronsäure (4-2) mit einer Verbindung der allgemeinen Formel (4-1), in der **U, V, X, Y, R¹** und **R²** wie voranstehend erwähnt definiert sind und **A** ein Sauerstoffatom oder die Gruppe -NH- darstellt, unter Zusatz eines Kupfer(II)-Katalysators und einer Hilfsbase erfolgen (siehe z.B. D. M. T. Chan, K. L. Monaco, R.-P. Wang, M. P. Winters Tetrahedron Lett. 1998, 39, 2933-2936; D. A. Evans, J. L. Katz, T. R. West Tetrahedron Lett. 1998, 39, 2937-2940; P. Y. S. Lam, C. G. Clark, S. Saubern, J. Adams, M. P. Winters, D. M. T. Chan, A. Combs Tetrahedron Lett. 1998" 39, 2941-2944). Die Reaktion wird in einem inerten Lösungsmittel in einem Temperaturbereich von 0°C bis zum Rückfluss des Lösungsmittels durchgeführt. Als Lösungsmittel eignen sich z. B. Dichlormethan oder Dichlorethan. Als Hilfsbasen sind tertiäre Amine wie Triethylamin oder Ethyldiisopropylamin oder Pyridin besonders geeignet. Bevorzugt wird die Reaktion unter Verwendung von Kupfer(II)-Acetat als Katalysator in Dichlormethan, bei Temperaturen zwischen Raumtemperatur und Rückfluss des Lösungsmittels, in Gegenwart von Triethylamin oder Pyridin durchgeführt.

Die Verbindungen der in Schema 1 gezeigten allgemeinen Formel (1-2), in der **U, V, X, Y, R¹**, **R²** und **R³** wie voranstehend erwähnt definiert sind und **A** ein Schwefel- oder Sauerstoffatom oder die Gruppe -N-H- darstellt, können nach den unter Schema 2, 3 und 4 beschriebenen Verfahren analog hergestellt werden.

Verbindungen der allgemeinen Formel **(I),** in der **U, V, X, Y, R¹**, **R²** und **R³** wie voranstehend erwähnt definiert sind und **A** eine Gruppe ausgewählt aus -S(=O)- (Sulfoxid) oder -SO₂- (Sulfon) darstellt, können zweckmäßigerweise durch Oxidation der jeweiligen Thioether-Verbindungen hergestellt werden. Als Oxidationsmittel eignen sich beispielsweise Peroxide wie Wasserstoffperoxid, 4-Chlorperbenzoesäure oder Periodsäure, gegebenenfalls können Lewissäuren dem Reaktionsgemisch zugesetzt werden. Die Reaktion wird in einem geeigneten Lösungsmittelgemisch durchgeführt, das ein hinreichendes Lösungsvermögen für alle beteiligten Reaktanden hat. Bevorzugte Lösungsmittel sind Dichlormethan oder Acetonitril. Die Reaktion kann bei Temperaturen zwischen dem Festpunkt und dem Siedepunkt des verwendeten Lösungsmittels durchgeführt werden.

Verbindungen der allgemeinen Formel **(I),** in der **U, V, X, Y, R¹**, **R²** und **R³** wie voranstehend erwähnt definiert sind und **A** für -N(C₁-₃-Alkyl), -N(C(O)-C₁₋₃-Alkyl) steht, können zweckmäßigerweise durch Alkylierung- oder Acylierung der jeweiligen Biarylamine (1-3) nach in der Fachliteratur beschrieben Verfahren (siehe z.B. J. March, Advanced Organic Reactions, Reactions Mechanism, and Structure, 4th Edition, John Wiley & Sons, Chichester/New York/Brisbane/Toronto/Singapore, 1992 und darin zitierte Literatur) dargestellt werden.

Die Verbindungen der allgemeinen Formeln (2-2), (3-2) und (4-2) sind entweder kommerziell verfügbar oder können nach Verfahren synthetisiert werden, die im Fachgebiet der organischen Chemie bekannt oder in der Fachliteratur beschrieben sind (siehe z.B. J. March, Advanced Organic Reactions, Reactions Mechanism, and Structure, 4th Edition, John Wiley & Sons, Chichester/New York/Brisbane/Toronto/Singapore, 1992 und darin zitierte Literatur). Die Verwendung von Übergangsmetallen und Organo-Metall-Verbindungen für die Synthese ist detailiert in Monographien beschrieben. (siehe z.B. L. Brandsma, S.F. Vasilevsky, H.D. Verkruijsse, Application of Transition Metals Catalysts in Organic Synthesis, Springer-Verlag, Berlin/Heidelberg, 1999; M. Schlosser, Organometallics in Synthesis, John Wiley & Sons, Chichester/ New York/ Brisbane/ Toronto/ Singapor, 1994, P.J. Stang, F. Diederich, Metal-Catalyzed Cross-Coupling Reactions, Wiley-VCH, Weinheim, 1997 and darin enthaltene Referenzen.)

In einigen Fällen kann das Endprodukt weiter derivatisiert werden, z.B. durch Manipulation der Substituenten. Diese Manipulationen können unter anderem diejenigen sein, die dem Fachmann allgemein bekannt sind wie Oxidation, Reduktion, Alkylierung, Acylierung und Hydrolyse, müssen allerdings nicht auf diese beschränkt sein.

Die erfindungsgemäßen neuen Verbindungen der allgemeinen Formel **I** können ein oder mehrere Chiralitätszentren enthalten. Sind beispielsweise zwei Chiralitätszentren vorhanden, dann können die Verbindungen in Form zweier diastereomerer Antipodenpaare auftreten. Die Erfindung umfasst die einzelnen Isomere ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel **I** fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)- oder (-)-Camphersulfonsäure, bzw. einer optisch aktiven Base, beispielsweise mit (*R*)-(+)-I-Phenylethylamin, (*S*)-(-)-I-Phenylethylamin oder (S)-Brucin, entstehen.

Nach einem üblichen Verfahren zur Isomerentrennung wird das Racemat einer Verbindung der allgemeinen Formel **I** mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (R)- oder (S)-Enantiomer bzw. ein Gemisch zweier optisch aktiver, unter die allgemeine Formel **I** fallender, diastereomerer Verbindungen wird auch dadurch erhalten, dass man die oben beschriebenen Synthesen mit jeweils einer geeigneten (*R*)- bzw. (*S*)-konfigurierten Reaktionskomponente durchführt.

Die neuen Verbindungen der allgemeinen Formel **I** und deren physiologisch verträgliche Salze weisen wertvolle pharmakologische Eigenschaften auf, die auf ihre selektiven CGRP-antagonistischen Eigenschaften zurück gehen. Ein weiterer Gegenstand der Erfindung sind diese Verbindungen enthaltende Arzneimittel, deren Verwendung und deren Herstellung.

Die voranstehend genannten neuen Verbindungen und deren physiologisch verträgliche Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität der voranstehend genannten Verbindungen zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC Membranen (~ 20 µg Proteinmenge) werden für 180 Minuten bei Raumtemperatur mit 50 pM ¹²⁵I-Iodotyrosyl-Calcitonin-Gene-Related Peptide und ansteigenden Konzentrationen der Testsubstanzen in einem Gesamtvolumen von 250 µl inkubiert (Assay Puffer: 10 mM Tris, 50 mM NaCl, 5 mM MgCl₂, 1mM EDTA, pH=7.4). Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM BIBN4096BS während der Inkubation definiert.

Die Analyse der Konzentrations-Bindungskurven erfolgt mit Hilfe einer computergestützten nichtlinearen Kurvenanpassung.

Die eingangs erwähnten Verbindungen zeigen in dem beschriebenen Test Kᵢ-Werte ≤ 50 µM.

### B. CGRP-Antagonismus in SK-N-MC-Zellen

SK-N-MC Zellen (∼1000 Zellen pro well) werden in Anwesenheit von steigenden Konzentrationen von CGRP und verschiedenen Konzentrationen der Testsubstanz für 30 Minuten inkubiert.

Die cAMP-Gehalte der Proben werden mittels AlphaScreen cAMP assay kit (Perkin Elmer) bestimmt und die pA₂-Werte antagonistisch wirkender Substanzen graphisch ermittelt.

Die erfindungsgemäßen Verbindungen zeigen in dem beschriebenen *in vitro*-Testmodell CGRP-antagonistische Eigenschaften in einem Dosisbereich zwischen 10⁻¹² bis 10⁻⁴ M.

Um zu zeigen, dass die Verbindungen der allgemeinen Formel **I** mit unterschiedlichen Strukturelementen gute bis sehr gute CGRP-antagonistische Aktivitäten zeigen, werden in der folgenden Tabelle die Aktivitätswerte-Werte angegeben, die gemäß dem voranstehenden Versuchsprotokoll erhalten wurden.

| **Nr.** | **CGRP Bindung Kᵢ (nM)** |
|---|---|
| (1) | 2 |
| (2) | 20 |
| (3) | 23 |
| (4) | 175 |
| (5) | 3 |
| (6) | 4 |
| (7) | 5 |
| (8) | 12 |
| (9) | 37 |
| (10) | 49 |
| (11) | 54 |
| (12) | 61 |
| (13) | 68 |
| (14) | 81 |
| (15) | 102 |
| (16) | 187 |
| (17) | 198 |
| (18) | 227 |
| (19) | 241 |
| (20) | 481 |
| (21) | 2 |
| (22) | 5 |
| (23) | 1 |
| (24) | 3 |
| (25) | 18 |
| (26) | 45 |
| (27) | 207 |
| (28) | 4 |
| (29) | 3 |
| (30) | 25 |
| (31) | 129 |
| (32) | 15 |
| (33) | 8 |
| (34) | 18 |
| (35) | 16 |
| (36) | 61 |
| (37) | 65 |
| (38) | 10 |
| (39) | 7 |
| (40) | 8 |
| (41) | 3 |
| (42) | 2 |
| (43) | 6 |
| (44) | 1 |
| (45) | 81 |
| (46) | 194 |
| (47) | 47 |
| (48) | 96 |
| (49) | 154 |
| (50) | 11 |
| (51) | 3 |
| (52) | 27 |
| (53) | 5 |
| (54) | 2 |
| (55) | 89 |
| (56) | 29 |
| (57) | 35 |
| (58) | 2 |
| (59) | 2 |
| (60) | 2 |
| (61) | 2 |
| (62) | 2 |
| (63) | 2 |
| (64) | 2 |
| (65) | 3 |
| (66) | 6 |
| (67) | 6 |
| (68) | 4 |
| (69) | 4 |
| (70) | 5 |
| (71) | 8 |
| (72) | 3 |
| (73) | 3 |
| (74) | 29 |
| (75) | 23 |
| (76) | 26 |
| (77) | 21 |
| (78) | 8 |
| (79) | 11 |
| (80) | 11 |

### INDIKATIONSGEBIETE

Aufgrund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne-, Cluster-Kopfschmerz sowie Spannungskopfschmerzen. Weiterhin beeinflussen die erfindungsgemäßen Verbindungen auch die folgenden Erkrankungen positiv: Nicht-insulinabhängigen Diabetes mellitus ("NIDDM"), cardiovaskuläre Erkrankungen, Morphintoleranz, Clostridiumtoxin-bedingte Durchfallerkrankungen, Erkrankungen der Haut, insbesondere thermische und strahlenbedingte Hautschäden inklusive Sonnenbrand, Lichen, Prurigo, pruriginöse Toxidermien sowie schwerer Juckreiz, entzündliche Erkrankungen, z.B. entzündliche Gelenkerkrankungen (Osteoarthritis, rheumatoide Arthritis, neurogene Arthritis), generalisierter Weichteilrheumatismus (Fibromyalgie), neurogene Entzündungen der oralen Mucosa, entzündliche Lungenerkrankungen, allergische Rhinitis, Asthma, COPD, Erkrankungen, die mit einer überschießenden Gefäßerweiterung und dadurch bedingter verringerter Gewebedurchblutung einhergehen, z.B. Schock und Sepsis, chronische Schmerzerkrankungen, wie z.B. diabetische Neuropathien, durch Chemotherapien induzierte Neuropathien, HIV-induzierte Neuropathien, postherpetische Neuropathien durch Gewebetrauma induzierte Neuropathien, trigeminale Neuralgien, temporomandibuläre Dysfunktionen, CRPS (complex regional pain syndrome), Rückenschmerzen, und viszerale Erkrankungen, wie z.B. irritable bowel syndrome (IBS), inflammatory bowel syndrome. Darüber hinaus zeigen die erfindungsgemäßen Verbindungen eine lindernde Wirkung auf Schmerzzustände im allgemeinen. Die Symptomatik menopausaler, durch Gefäßerweiterung und erhöhten Blutfluss verursachter Hitzewallungen östrogendefizienter Frauen sowie hormonbehandelter Prostatakarzinompatienten und Kastraten wird durch die CGRP-Antagonisten der vorliegenden Anwendung präventiv und akut-therapeutisch günstig beeinflusst, wobei sich dieser Therapieansatz vor der Hormonsubstitution durch Nebenwirkungsarmut auszeichnet.

Vorzugsweise eignen sich die erfindungsgemäßen Verbindungen zur akuten und prophylaktischen Behandlung von Migräne- und Cluster-Kopfschmerz, zur Behandlung des irritable bowel syndroms (IBS) und zur präventiven und akut-therapeutischen Behandlung von Hitzewallungen östrogendefizienter Frauen.

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0.01 bis 10 mg/kg Körpergewicht, vorzugsweise 0.1 bis 10 mg/kg Körpergewicht, jeweils ein- bis dreimal täglich.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatomen durch Tritium, in RIA- und ELISA-Assays und als diagnostische bzw. analytische Hilfsmittel in der Neurotransmitter-Forschung.

### KOMBINATIONEN

Als Kombinationspartner denkbare Wirkstoffklassen sind z.B. Antiemetica, Prokinetica, Neuroleptica, Antidepressiva, Neurokinin-Antagonisten, Anticonvulsiva, Histamin-H1-Rezeptorantagonisten, β-Blocker, α-Agonisten und α-Antagonisten, Ergotalkaloiden, schwachen Analgetica, nichtsteroidalen Antiphlogistica, Corticosteroiden, Calcium-Antagonisten, 5-HT_{1B/1D}-Agonisten oder andere Antimigränemitteln, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/- Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Aceclofenac, Acemetacin, Acetylsalicylsäure, Acetaminophen (Paracetamol), Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phenylbutazon, Piroxicam, Sulfasalazin, Zomepirac oder deren pharmazeutisch verträgliche Salze sowie Meloxicam und andere selektive COX2-Inhibitoren, wie beispielsweise Rofecoxib, Valdecoxib, Parecoxib, Etoricoxib und Celecoxib, in Betracht sowie Substanzen, die frühere oder spätere Schritte in der Prostaglandin-Synthese inhibieren oder Prostaglandinrezeptor Antagonisten wie z.B. EP2-Rezeptor Antagonisten und IP-Rezeptor Antagonisten.

Weiterhin können Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Pregabalin, Duloxetin, Topiramat, Riboflavin, Montelukast, Lisinopril, Micardis, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Imipramine, Venlafaxine, Lidocain oder Diltiazem und andere 5-HT_{1B/1D}-Agonisten wie z.B. Almotriptan, Avitriptan, Eletriptan, Frovatriptan, Naratriptan, Rizatriptan, Sumatriptan und Zolmitriptan verwendet werden.

Außerdem können CGRP-Antagonisten mit Vanilloid-Rezeptor Antagonisten, wie z.B. VR-1 Antagonisten, Glutamatrezeptor Antagonisten, wie z.B. mGlu5-Rezeptor Antagonisten, mGlu1-Rezeptor Antagonisten, iGlu5-Rezeptor Antagonisten, AMPA-Rezeptor Antagonisten, Purinrezeptor Blockern, wie z.B. P2X3 Antagonisten, NO-Synthase Inhibitoren, wie z.B. iNOS Inhibitoren, Calciumkanal-Blockern, wie z.B. PQ-typ Blockern, N-typ Blockern, Kaliumkanalöffnern, wie z.B. KCNQ Kanalöffnern, Natriumkanal Blockern, wie z.B. PN3 Kanal Blockern, NMDA-Rezeptor Antagonisten, Acid-sensing Ionenkanal Antagonisten, wie z.B. ASIC3 Antagonisten, Bradykinin Rezeptor Antagonisten wie z.B. B1-Rezeptor Antagonisten, Cannabinoid-Rezeptor Agonisten, wie z.B. CB2 Agonisten, CB1 Agonisten, Somatostatin-Rezeptor Agonisten, wie z.B. sst2 Rezeptor Agonisten gegeben werden. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

### DARREICHUNGSFORMEN

Die erfindungsgemäß hergestellten Verbindungen können entweder alleine oder gegebenenfalls in Kombination mit anderen Wirksubstanzen zur Behandlung von Migräne intravenös, subkutan, intramuskulär, intraartikulär, intrarektal, intranasal, durch Inhalation, topisch, transdermal oder oral erfolgen, wobei zur Inhalation insbesondere Aerosolformulierungen geeignet sind. Die Kombinationen können entweder simultan oder sequentiell verabreicht werden.

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0.1 bis 90 Gew.-%, bevorzugt 0.5 bis 50 Gew.-% der Gesamtzusammensetzung liegen, d.h. in Mengen die ausreichend sind, um den voranstehend angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel **I** gemäß der obigen bevorzugten Ausführungsformen.

Besonders bevorzugt ist es, wenn die Verbindungen der Formel **I** oral verabreicht werden, ganz besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie *p*-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnussoder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der Formel **I** inhalativ verabreicht werden, besonders bevorzugt ist es, wenn die Verabreichung ein oder zweimal täglich erfolgt. Hierzu müssen die Verbindungen der Formel **I** in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### EXPERIMENTELLER TEIL

Für die hergestellten Verbindungen liegen in der Regel ¹H-NMR und/oder Massenspektren vor. Wenn nicht anders angegeben, werden R_{f}-Werte unter Verwendung von DC-Fertigplatten Kieselgel 60 F254 (E. Merck, Darmstadt, Artikel-Nr. 1.05714) ohne Kammersättigung bestimmt.

Die bei den Fliessmitteln angegebenen Verhältnisse beziehen sich auf Volumeneinheiten der jeweiligen Lösungsmittel. Die angegebenen Volumeneinheiten bei NH₃ beziehen sich auf eine konzentrierte Lösung von NH₃ in Wasser.

Soweit nicht anders vermerkt sind die bei den Aufarbeitungen der Reaktionslösungen verwendeten Säure-, Basen- und Salzlösungen wässrige Systeme der angegebenen Konzentrationen. Zu chromatographischen Reinigungen wird Kieselgel der Firma Millipore (MATREX™, 35 bis 70 µm) verwendet.

Die angegebenen HPLC-Daten werden unter nachstehend angeführten Parametern und unter Verwendung der aufgeführten Säulen erhoben:
Verwendete Säulen:
(Säulentemperatur: 30°C; Injektionsvolumen: 5 µL; Detektion bei 254 nm)

| | |
|---|---|
| S1 | Zorbax-Säule (Agilent Technologies), SB (Stable Bond) C18; |
| | 3.5 µm; 4.6 x 75 mm |
| S2 | Zorbax-Säule (Agilent Technologies), SB (Stable Bond) C18; |
| | 1.8 µm; 3.0 x 30 mm |
| S3 | Zorbax-Säule (Agilent Technologies), SB (Stable Bond) |
| | C18; 5 µm; 4.6 x 75 mm |
| S4 | X-Bridge(Waters) C18; |
| | 2.5 µm; 3.0 x 30 mm, |
| S5 | Sunfire C18 (Waters); |
| | 3.5 µm; 4.6 x 75 mm |
| S6 | Symmetry C18 (Waters); |
| | 3.5 µm; 4.6 x 75 mm |

### Verwendete Lösungsmittel:

### Saure Bedingungen:

Lösungsmittel A: Wasser (mit 0.1% Ameisensäure)
Lösungsmittel B: Acetonitril (mit 0.1% Ameisensäure)
(Die prozentualen Angaben beziehen sich auf das Gesamtvolumen.)

### Gradienten:

| Gradient | Zeit [min] | %A | %B |
|---|---|---|---|
| **G1** (1.6 mL/min) | 0.00 | 95 | 5 |
| | 0.10 | 95 | 5 |
| | 1.75 | 5 | 95 |
| | 1.90 | 5 | 95 |
| | 1.95 | 95 | 5 |
| | 2.00 | 95 | 5 |

| Gradient | Zeit [min] | %A | %B |
|---|---|---|---|
| **G2** (1.6 mL/min) | 0.00 | 95 | 5 |
| | 4.50 | 10 | 90 |
| | 5.00 | 10 | 90 |
| | 5.50 | 95 | 5 |

| Gradient | Zeit [min] | %A | %B |
|---|---|---|---|
| **G3** (1.6 mL/min) | 0.00 | 95 | 5 |
| | 4.00 | 50 | 50 |
| | 4.50 | 10 | 90 |
| | 5.00 | 10 | 90 |
| | 5.50 | 95 | 5 |

| Gradient | Zeit [min] | %A | %B |
|---|---|---|---|
| **G4** (1.6 mL/min) | 0.00 | 95 | 5 |
| | 1.00 | 10 | 90 |
| | 2.50 | 50 | 50 |
| | 2.75 | 95 | 5 |

| Gradient | Zeit [min] | %A | %B |
|---|---|---|---|
| **G5** (1.4 mL/min) | 0,00 | 95 | 5 |
| | 1.80 | 10 | 90 |
| | 2.00 | 10 | 90 |
| | 2.20 | 95 | 5 |

| Gradient | Zeit [min] | %A | %B |
|---|---|---|---|
| **G6** | 0,00 | 95 | 5 |
| (1.6 mL/min) | 2.00 | 50 | 50 |
| | 2.25 | 10 | 90 |
| | 2.50 | 10 | 90 |
| | 2.75 | 95 | 5 |

Trennungen mit Methanol : Lösungsmittel A: Wasser (mit 0.2% HCOOH)
Lösungsmittel B: Methanol
(Die prozentualen Angaben beziehen sich auf das Gesamtvolumen.)

| Gradient | Zeit [min] | %A | %B |
|---|---|---|---|
| **G7** (1.6 mL/min) | 0.00 | 95 | 5 |
| | 4.50 | 10 | 90 |
| | 6.50 | 10 | 90 |
| | 7.00 | 95 | 5 |

Basische Bedingungen:
Lösungsmittel A: Wasser (mit 0.1% NH4OH)
Lösungsmittel B: Acetonitril (mit 0.1% NH4OH)
(Die prozentualen Angaben beziehen sich auf das Gesamtvolumen.)

| Gradient | Zeit [min] | %A | %B |
|---|---|---|---|
| **basisch_1** (0.8 mL/min) | 0.00 | 95 | 5 |
| | 9.00 | 10 | 90 |
| | 10.00 | 10 | 90 |
| | 11.00 | 95 | 5 |

| Gradient | Zeit [min] | %A | %B |
|---|---|---|---|
| **basisch_2** (1.4 mL/min) | 0.00 | 95 | 5 |
| | 1.80 | 10 | 90 |
| | 2.00 | 10 | 90 |
| | 2.20 | 95 | 5 |

### Methoden:

Die jeweilig verwendete HPLC-Methode ergibt sich aus Kombination der zuvor beschriebenen Säulen und Gradienten:

| | Säule | Gradient |
|---|---|---|
| Methode A | S1 | G1 |
| Methode B | S2 | G1 |
| Methode C | S1 | G2 |
| Methode D | S1 | G3 |
| Methode E | S2 | G4 |
| Methode F | S4 | G5 |
| Methode G | S5 | G2 |
| Methode H | S4 | basisch_1 |
| Methode I | S2 | G6 |
| Methode J | S6 | G2 |
| Methode K | S4 | basisch_2 |
| Methode L | S5 | G3 |
| Methode M | S1 | G7 |

Bei präparativen HPLC-Reinigungen werden in der Regel die gleichen Gradienten verwendet, die bei der Erhebung der analytischen HPLC-Daten benutzt wurden. Die Sammlung der Produkte erfolgt massengesteuert, die Produkt enthaltenden Fraktionen werden vereinigt und gefriergetrocknet.

Falls nähere Angaben zur Konfiguration fehlen, bleibt offen, ob es sich um reine Enantiomere handelt oder ob partielle oder gar völlige Racemisierung eingetreten ist.

In den Versuchsbeschreibungen werden die folgenden Abkürzungen verwendet:

| | |
|---|---|
| AcOH | Essigsäure |
| BINAP | 2,2'-Bis(diphenylphosphino)-1,1'-Binaphthyl |
| BOC | *tert.*-Butyloxycarbonyl |
| Cyc | Cyclohexan |
| CDI | 1,1'-Carbonyldimidazol |
| DCM | Dichlormethan |
| DIPE | Diisopropylether |
| DIPEA | Diisopropylethylamin |
| DMF | *N,N*-Dimethylformamid |
| DMSO | Dimethylsulfoxid |
| dppf | 1,1'-Bis(diphenyl-phosphino)ferrocen |
| d. Th. | der Theorie |
| EI | Elektronenstoß-Ionisation (bei MS) |
| ESI | Elektrospray-Ionisation (bei MS) |
| EtOAc | Essigsäureethylester |
| EtOH | Ethanol |
| E-Wasser | Entionisiertes Wasser |
| FM | Fließmittel |
| HCl | Chlorwasserstoff |
| HCOOH | Ameisensäure |
| HPLC | High Performance Liquid Chromatography |
| HPLC-MS | HPLC gekoppelte Massenspektrometrie |
| i.vac. | in vacuo (im Vakuum) |
| konz. | konzentriert |
| MeOH | Methanol |
| MS | Massenspektrometrie |
| MW | Molekulargewicht [g/mol] |
| NaOH | Natriumhydroxid |
| NH₄OH | Ammoniumhydroxid (wässrige Ammoniak-Lösung, 30%) |
| NMP | *N*-Methyl-2-pyrrolidin |
| Pd₂dba₃ | Bis(dibenzylidenaceton)-palladium(0) |
| PE | Petrolether |
| R_{f} | Retentionsindex (bei DC) |
| RT | Raumtemperatur |
| Rₜ | Retentionszeit (bei HPLC) |
| TEA | Triethylamin |
| TFA | Trifluoressigsäure |
| THF | Tetrahydrofuran |
| ULTS | Umluft-Trockenschrank |

### Herstellung der Ausgangsverbindungen:

### Intermediat 1

### 7-Methoxy-3-piperidin-4-yl-1,3,4,5-tetrahydro-1,3-benzodiazepin-2-on

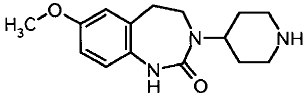

Diese Verbindung und deren Vorstufen wurde wie in US 6,313,097 beschrieben hergestellt.

### Intermediat 2

### 6-(6-Chlor-pyrimidin-4-yloxy)-4-methyl-3H-benzoxazol-2-on

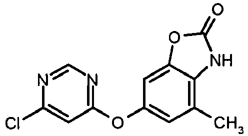

### Stufe 1: 4-Methyl-2-oxo-2,3-dihydrobenzo[d]oxazol-6-ylboronsäure

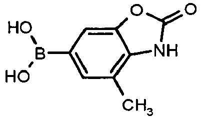

Unter Argonatmosphäre wurden 1.00 g (4.39 mmol) 6-Brom-4-methyl-3H-benzoxazol-2-on, 1.71 g (6.60 mmol) Bis-(pinacolato)-diboran, 0.359 g (0.440 mmol) 1,1'-Bis(diphenyl-phosphino)ferrocen-palladium(II)dichlorid und 1.30 g (13.2 mmol) Kaliumacetat zu 3.0 mL DMSO gegeben und 2 h bei 120°C gerührt. Der Ansatz wurde auf Eiswasser gegossen und mehrmals mit DCM extrahiert. Die vereinigten organischen Phasen wurden mit Wasser und gesättigter Natriumchlorid-Lösung gewaschen und i.vac. eingeengt. Der Rückstand wurde in DMF gelöst und mittels präparativer HPLC-MS gereinigt. Die Produkt enthaltenden Fraktionen wurden vereinigt, mit 1 N wässriger Natronlauge schwach alkalisch gestellt und i.vac. eingeengt. Der Rückstand wurde in Wasser aufgenommen und der Niederschlag abgesaugt. Das Filtrat wurde mit 1 N wässriger Salzsäure-Lösung leicht sauer gestellt, der Niederschlag wurde abgesaugt und i.vac. getrocknet.

| | |
|---|---|
| Ausbeute: | 0.3 g (36% d.Th.) |
| ESI-MS: | m/z = 192 (M-H)⁻ |
| Rₜ(HPLC): | 0.89 min (Methode B) |

### Stufe 2: 6-Hydroxy-4-methyl-3H-benzoxazol-2-on

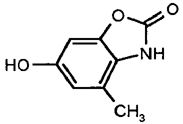

77 mg (0.40 mmol) 4-Methyl-2-oxo-2,3-dihydrobenzo[d]oxazol-6-ylboronsäure wurden in 2.0 mL Eisessig gelöst und mit 0.20 mL (2.3 mmol) Wasserstoffperoxid-Lösung (35% in Wasser) versetzt und kurz verrührt. Das Reaktionsgemisch wurde 1 h bei RT stehen gelassen. Der entstandene Niederschlag wurde abgesaugt, mit Wasser gewaschen und i.vac. getrocknet.

| | |
|---|---|
| Ausbeute: | 40 mg (61% d.Th.) |
| ESI-MS: | m/z = 164 (M-H)⁻ |
| Rₜ(HPLC): | 1.07 min (Methode B) |

### Stufe 3: 6-(6-Chlor-pyrimidin-4-yloxy)-4-methyl-3H-benzoxazol-2-on

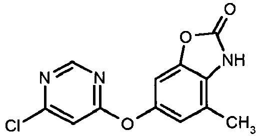

0.10 g (0.60 mmol) 6-Hydroxy-4-methyl-3H-benzoxazol-2-on in 1.5 mL DMF wurden mit 0.10 g (0.70 mmol) Kaliumcarbonat versetzt und 15 min bei RT gerührt. Anschließend wurden 0.10 g (0.60 mmol) 4,6-Dichlorpyrimidin zu der Reaktionsmischung gegeben und über Nacht bei RT gerührt. Dann wurde Wasser hinzugefügt und der entstandene Niederschlag abgesaugt, gewaschen und i.vac. getrocknet.

| | |
|---|---|
| Ausbeute: | 90 mg (56% d.Th.) |
| ESI-MS: | m/z = 278(M+H)⁺ |
| Rₜ(HPLC): | 1.29 min (Methode B) |

### Intermediat 3

### 6-(6-Chlor-pyrimidin-4-yloxy)-3,4-dimethyl-3H-benzoxazol-2-on

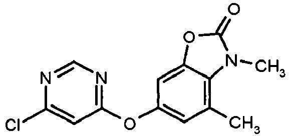

### Stufe 1: 3,4-Dimethyl-6-(4,4,5,5-tetramethyl-[1,3,2]dioxaborolan-2-yl)-3H benzoxazol-2-on

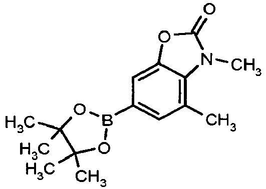

Unter Argonatmosphäre wurden 3.20 g (13.4 mmol) 6-Brom-3,4-dimethyl-3H-benzoxazol-2-on, 5.00 g (19.3 mmol) Bis-(pinacolato)-diboran, 0.90 g (1.1 mmol) 1,1'-Bis(diphenyl-phosphino)ferrocen-palladium(II)dichlorid und 3.94 g (40.1 mmol) Kaliumacetat in 10.0 mL DMSO zusammen gegeben und 2 h bei 120°C gerührt. Der Ansatz wurde mit EtOAc verdünnt und mit Wasser und gesättigter Natriumchlorid-Lösung gewaschen. Die organische Phase wurde eingeengt und mittels Flash-Chromatographie gereinigt. Die Produkt enthaltenden Fraktionen wurden vereinigt und eingeengt. Der Rückstand wurde mit PE verrieben und abgesaugt. Der erhaltene Feststoff wurde PE nachgewaschen und i.vac. getrocknet.

| | |
|---|---|
| Ausbeute: | 2.40 g (62% d.Th.) |
| ESI-MS: | m/z = 290 (M+H)⁺ |
| Rₜ(HPLC): | 1.66 min (Methode B) |

### Stufe 2: 6-Hydroxy-3,4-dimethyl-3H-benzoxazol-2-on

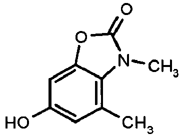

0.20 g (0.70 mmol) 3,4-Dimethyl-6-(4,4,5,5-tetramethyl-[1,3,2]dioxaborolan-2-yl)-3H-benzoxazol-2-on wurden in 2.0 mL Eisessig gelöst und mit 0.40 mL (4.6 mmol) Wasserstoffperoxid-Lösung (35% in Wasser) versetzt und verrührt. Der entstandene Niederschlag wurde abgesaugt, mit Wasser gewaschen und i.vac. getrocknet.

| | |
|---|---|
| Ausbeute: | 122 mg (98% d.Th.) |
| ESI-MS: | m/z = 180 (M-H)⁻ |
| Rₜ(HPLC): | 1.09 min (Methode B) |

### Stufe 3: 6-(6-Chlor-pyrimidin-4-yloxy)-3,4-dimethyl-3H-benzoxazol-2-on

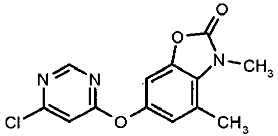

Zu 0.12 g (0.70 mmol) 6-Hydroxy-3,4-dimethyl-3H-benzoxazol-2-on in 5.0 ml DMF wurden 30 mg (0.70 mmol) Natriumhydrid (55%, Suspension in Mineralöl) zugegeben und 10 min bei RT gerührt. Anschließend wurden 0.10 g (0.70 mmol) 4,6-Dichlorpyrimidin hinzugefügt und 1 h bei RT gerührt. Dann wurde Wasser und EtOAc zugesetzt, die organische Phase abgetrennt, über Natriumsulfat getrocknet, filtriert und eingeengt.

| | |
|---|---|
| Ausbeute: | 260 mg (quantitativ) |
| ESI-MS: | m/z = 292 (M+H)⁺ |
| Rₜ(HPLC): | 1.50 min (Methode B) |

### Intermediat 4a

### 1-Piperidin-4-yl-1,3-dihydroimidazo[4,5-b]pyridin-2-on

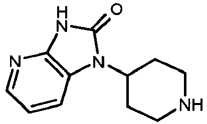

### Stufe 1: 4-(2-Chlor-pyridin-3-yl-amino)-piperidin-1-carbonsäurebenzylester

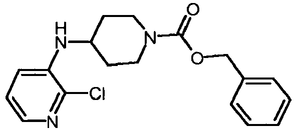

Zu 930 g (3.99 mol) *N*-Benzyloxycarbonyl-4-piperidon und 466 g (3.63 mol) 2-Chlor-3-aminopydridin in 9.5 L Isopropylacetat wurden bei ca. 15°C 560 mL (7.25 mol) TFA zugetropft. 922 g (4.35 mol) Natriumtriacetoxyborhydrid wurden portionsweise zugegeben. Es wurde bis zur Vollständigkeit der Reaktion nachgerührt. Bei RT wurde die Reaktionsmischung mit 860 mL Natronlauge (2 mol/L) versetzt. Die organische Phase wurde abgetrennt, mit 5 L Wasser gewaschen und eingeengt.

| | |
|---|---|
| Ausbeute: | 1250 g (roh 100% d.Th.) |
| ESI-MS: | m/z = 346 (M+H)⁺ |

### Stufe 2: 4-[1-(2-Chlor-pyridin-3-yl)-ureido]-piperidin-1-carbonsäurebenzylester

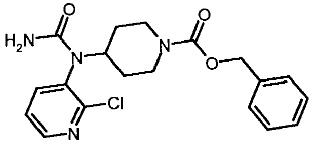

530 mL (6.1 mol) Chlorsulfonylisocyanat wurden in 6 L THF vorgelegt und auf -15°C abgekühlt. Zu diesem Gemisch wurde anschließend eine Lösung von 1.25 kg (3.63 mol) 4-(2-Chlor-pyridin-3-yl-amino)-piperidin-1-carbonsäurebenzylester in 7 L THF innerhalb einer Stunde so zugetropft, dass die Temperatur der Reaktionsmischung -7°C nicht überstieg. Es wurde 90 Minuten bei ca. -8°C nachgerührt und anschließend 700 mL Wasser innerhalb von 30 Minuten zugetropft. Die Mischung wurde 30 Minuten bei ca. 10°C nachgerührt und anschließend mit 8.1 L Natronlauge (2 mol/L) langsam versetzt. Das Reaktionsgemisch wurde anschließend auf 50°C erwärmt und die Phasen getrennt. Die organische Phase wurde mit 2 L Wasser gewaschen. Danach wurden aus der organischen Phase 10 L Lösemittel abdestilliert, 15 L Butylacetat zum Rückstand zugegeben und hiervon nochmals 8 L abdestilliert. Durch langsames Abkühlen auf 0°C wurde das Produkt kristallisiert. Der Niederschlag wurde abgesaugt, mit 2 L Butylacetat gewaschen und bei 40°C getrocknet.

| | |
|---|---|
| Ausbeute: | 1108 g (78.8% d.Th.) |
| ESI-MS: | m/z = 389/391 (M+H)⁺ |

### Stufe 3: 4-(2-Oxo-2,3-dihydro-imidazo[4,5-b]pyridin-1-yl)-piperidin-1-carbonsäure-Benzylester

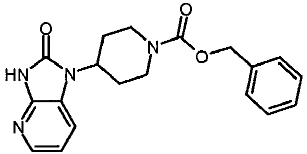

1108 g (2.85 mol) 4-[1-(2-Chlor-pyridin-3-yl)-ureido]-piperidin-1-carbonsäurebenzylester wurden mit 720 g (8.57 mol) Natriumhydrogencarbonat in 14.5 L tert-Amylalkohol unter Rückfluss erhitzt. Hierbei wurden 3 L Lösungsmittel abdestilliert. Das Reaktionsgemisch wurde auf 35°C abgekühlt und mit 11 mL Wasser versetzt. Anschließend wurden 13 g (0.058 mol) Palladiumacetat und 49 g (0.115 mol) 1,4-Bis-(diphenylphosphino)-butan (DPPB) zugegeben und auf Rückflusstemperatur erhitzt. Es wurde bei 100°C bis zur Vollständigkeit der Reaktion nachgerührt, auf RT abgekühlt und 7.5 L Wasser zugegeben. Die organische Phase wurde abgetrennt, mit 5 L Wasser gewaschen und anschließend eingeengt. Der ölige Rückstand wurde zweimal mit je 3 L Isopropylacetat versetzt und abdestilliert. Anschließend wurde der Rückstand in 7 L Isopropylacetat heiß gelöst und langsam auf RT abgekühlt. Der ausgefallene Feststoff wurde abgesaugt, mit je 2 L Isopropylacetat und tert.-Butyl-methylether gewaschen und bei 50°C getrocknet.

| | |
|---|---|
| Ausbeute: | 690 g (69% d.Th.) |
| ESI-MS: | m/z = 353 (M+H)⁺ |

### Stufe 4: 1-Piperidin-4-yl-1,3-dihydro-imidazo[4,5-b]pyridin-2-on

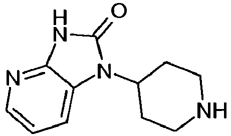

690 g (1.96 mol) 4-(2-Oxo-2,3-dihydro-imidazo[4,5-*b*]pyridin-1-yl)-piperidin-1-carbonsäure-benzylester wurden in 5.4 L Methanol gelöst und unter Zusatz von 46 g Pd/C (10%ig; 6.6 Gew.%) bei 60°C und einem Wasserstoffdruck von 60 psi bis zur vollständigen Wasserstoffaufnahme hydriert. Der Katalysator wurde abfiltriert. Vom Filtrat wurden 4 L Methanol abdestilliert. Es wurden 2 L Methylcyclohexan zugegeben und weitere 1.5 L Lösungsmittel abdestilliert. Die so erhaltene Suspension wurde abgesaugt, der Rückstand mit Methylcyclohexan gewaschen und bei 40°C getrocknet.

| | |
|---|---|
| Ausbeute: | 446 g (100% d.Th.) |
| ESI-MS: | m/z = 219 (M+H)⁺ |

### Intermediat 4b

### 1-Piperidin-4-yl-1,3-dihydroimidazo[4,5-b]pyridin-2-on-dihydrochlorid

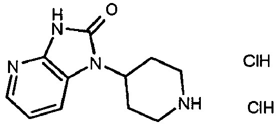

Diese Verbindung und ihre Vorstufen wurden wie in der WO 2005/013894 beschrieben synthetisiert.

### Intermediat 5

### 4-Chlor-6-(3,4-dimethyl-phenoxy)-pyrimidin

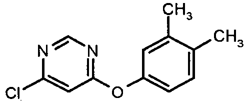

Zu 617 mg (5.00 mmol) 3,4-Dimethylphenol in 10 ml DMF wurden 218 mg (5.00 mmol) Natriumhydrid (55%, Suspension in Mineralöl) gegeben und 10 min bei RT gerührt. Anschließend wurden 768 mg (5.00 mmol) 4,6-Dichlorpyrimidin hinzugefügt und 48 h bei RT gerührt. Zu der Reaktionsmischung wurde Wasser und EtOAc zugesetzt, die organische Phase abgetrennt, über Natriumsulfat getrocknet, filtriert und eingeengt. Der Rückstand wurde i.vac. getrocknet.

| | |
|---|---|
| Ausbeute: | 1.3 g (94% d.Th.) |
| Reinheit: | 85% |
| ESI-MS: | m/z = 235/37 (Cl) (M+H)⁺ |
| Rₜ(HPLC): | 1.64 min (Methode B) |

### Intermediat 6

### 6-(6-Chlor-pyrimidin-4-ylamino)-4-methyl-3H-benzoxazol-2-on

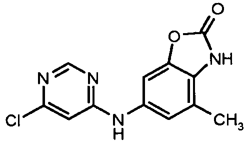

### Stufe 1: 4-Methyl-2-oxo-2,3-dihydro-benzoxazol-6-carbonsäuremethylester

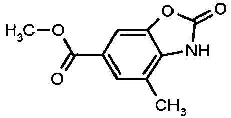

12 g (53 mmol) 6-Brom-4-methyl-3H-benzoxazol-2-on, 30 mg (0.13 mmol) Palladium(II)-acetat, 1.0 g (1.8 mmol) dppf und 12 g (0.14 mol) Natriumacetat wurden in 600 mL MeOH vorgelegt und unter einem Druck von 5 bar in Kohlenmonoxidatmosphäre 8 h bei 100°C reagieren gelassen und anschließend auf RT abgekühlt. Der ausgefallene Niederschlag wurde abgesaugt und die Mutterlauge fast bis zur Trockene einrotiert, mit Eiswasser versetzt und der entstandene Niederschlag abgesaugt, mit Wasser gewaschen und getrocknet.

| | |
|---|---|
| Ausbeute: | 11 g (82% d.Th.) |
| ESI-MS: | m/z = 206 (M-H)⁻ |
| R_{f}: | 0.30 (Kieselgel: PE / EtOAc = 2/1) |

### Stufe 2: 4-Methyl-2-oxo-2,3-dihydro-benzoxazol-6-carbonsäure

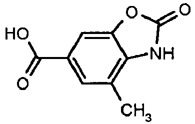

Zu 5.00 g (24.1 mmol) 4-Methyl-2-oxo-2,3-dihydro-benzoxazol-6-carbonsäuremethylester in 50 mL MeOH wurden 20 mL 4N wässrige Natronlauge gegeben und 24 h bei RT gerührt. Anschließend wurden 10 mL 4N wässrige Natronlauge hinzugefügt und weitere 8 h bei RT gerührt. Dann wurde das MeOH i.vac. entfernt, Wasser hinzugefügt und unter Eiskühlung mit konz. Salzsäure-Lösung angesäuert, bis sich ein Niederschlag bildete. Dieser wurde abgesaugt, mit Wasser nachgewaschen und i.vac. getrocknet.

| | |
|---|---|
| Ausbeute: | 4.6 g (99% d.Th.) |
| Rₜ(HPLC): | 1.96 min (Methode C) |

### Stufe 3: 4-Methyl-2-oxo-2,3-dihydro-benzoxazol-6-carbonsäureazid

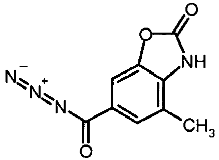

1.00 g (5.20 mmol) 4-Methyl-2-oxo-2,3-dihydro-benzoxazol-6-carbonsäure, 3.00 ml (41.1 mmol) Thionylchlorid, 20 mL DCM und 1.0 mL DMF wurden zusammen gegeben und 4 h bei 40°C gerührt. Der Reaktionsansatz wurde bis zur Trockene eingeengt und mit Toluol coevaporiert. Der Rückstand wurde in Aceton gelöst, mit 340 mg (5.20 mmol) Natriumazid versetzt und 1 h bei RT gerührt. Anschließend wurde Wasser hinzugefügt, der entstandene Niederschlag abgesaugt und getrocknet.

| | |
|---|---|
| Ausbeute: | 1.40 g (81% d.Th.) |
| Reinheit: | 65% |
| ESI-MS: | m/z = 217 (M-H)⁻ |
| Rₜ(HPLC): | 1.30 min (Methode B) |

### Stufe 4: (4-Methyl-2-oxo-2,3-dihydro-benzoxazol-6-yl)-carbaminsäure-tert-butylester

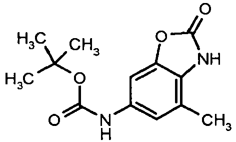

1.00 g (2.98 mmol) 4-Methyl-2-oxo-2,3-dihydro-benzoxazol-6-carbonsäureazid wurden in 15 mL *tert*-Butanol 2.5 unter Rückfluss gerührt. Nach dem Abkühlen auf RT wurde das Lösungsmittel i.vac. entfernt, der Rückstand in Methanol aufgenommen und über Alox filtriert. Das Filtrat wurde eingeengt und der Rückstand getrocknet.

| | |
|---|---|
| Ausbeute: | 500 mg (64% d.Th.) |
| ESI-MS: | m/z = 263 (M-H)⁻ |
| Rₜ(HPLC): | 1.30 min (Methode B) |

### Stufe 5: 6-Amino-4-methyl-3H-benzoxazol-2-on

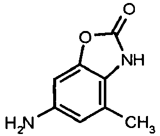

Zu 230 mg (0.900 mmol) (4-Methyl-2-oxo-2,3-dihydro-benzoxazol-6-yl)-carbaminsäure-*tert*-butylester in 5.0 ml DCM wurden 400µL (5.30 mmol) TFA gegeben und 1.5 h bei RT gerührt. Der Reaktionsansatz wurde eingeengt und der Rückstand zwischen halbgesättigter Natriumhydrogencarbonat-Lösung und EtOAc verteilt. Die organische Phase wurde über Natriumsulfat getrocknet, filtriert und eingeengt.

| | |
|---|---|
| Ausbeute: | 120 mg (84% d. Th.) |
| ESI-MS: | m/z = 165 (M+H)⁺ |
| Rₜ(HPLC): | 0.23 min (Methode B) |

### Stufe 6: 6-(6-Chlor-pyrimidin-4-ylamino)-methyl-3H-benzoxazol-2-on

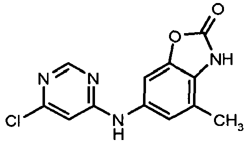

400 mg (2.40 mmol) 6-Amino-4-methyl-3H-benzoxazol-2-on, 400 mg (2.70 mmol) 4,6-Dichlorpyrimidin und 1.10 mL (8.00 mmol) TEA in 5 mL 1-Butanol wurden 1 h bei 100°C gerührt. Nach dem Abkühlen auf RT wurde der Reaktionsansatz eingeengt und der Rückstand mit Wasser und gesättigter Natriumhydrogencarbonat-Lösung gewaschen. Die organische Phase wurde über Natriumsulfat getrocknet, filtriert und eingeengt.

| | |
|---|---|
| Ausbeute: | 500 mg (58% d.Th.) |
| Reinheit: | 78% |
| ESI-MS: | m/z = 277 (M+H)⁺ |
| Rₜ(HPLC): | 1.35 min (Methode B) |

### Intermediat 7

### (6-Chlor-pyrimidin-4-yl)-(2,7-dimethyl-1H-benzimidazol-5-yl)-amin

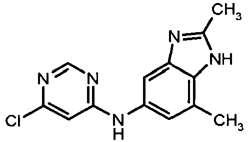

### Stufe 1: N-(2-Methyl-4,6-dinitro-phenyl)-acetamid

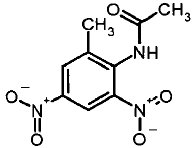

Zu 400 mL rauchender Salpetersäure, die bei -5°C gerührt wurde, wurden 50.0 g (0.257 mol) N-(2-Methyl-4-nitro-phenyl)-acetamid in kleinen Portionen derart eingetragen, dass die Temperatur im Reaktionsgefäß nicht über 0°C anstieg. Nach beendeter Zugabe wurde das Reaktionsgemisch 15 min bei 0°C gerührt und anschließend auf 2 kg EisWasser-Gemisch gegeben. Das als Feststoff ausgefallene Produkt wurde abgesaugt, mit Wasser gewaschen und getrocknet. Es wurde direkt für die nächste Stufe eingesetzt.

| | |
|---|---|
| Ausbeute: | 55.3 (90% d.Th.) |
| R_{f}: | 0.77 (Kieselgel: DCM) |

### Stufe 2: N-(2,4-Diamino-6-methyl-phenyl)-acetamid

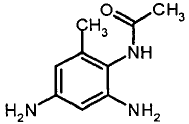

Ein Gemisch aus 45.0 g (0.188 mol) N-(2-Methyl-4,6-dinitro-phenyl)-acetamid und 4.50 g Palladium auf Kohle (Pd/C 10%) in 1.400 L Methanol wurde 2 h bei RT unter Wasserstoffatmosphäre hydriert. Der Katalysator wurde abgesaugt und das Filtrat i.vac. bis zur Trockene eingeengt. Der Rückstand wurde in Diethylether verrieben, abgesaugt und getrocknet. Das erhaltene Rohprodukt wurde direkt für die nächste Stufe eingesetzt.

### Stufe 3: 2,7-Dimethyl-benzimidazol-5-ylamin

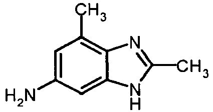

33.0 g (0.184 mol) N-(2,4-Diamino-6-methyl-phenyl)-acetamid wurden in 400 mL Eisessig 1.5 h bei 100°C gerührt. Der Eisessig wurde i.vac. entfernt und der verbliebene Rückstand mit Ether verrieben, abgesaugt und getrocknet. Die so erhaltene freie Base wurde in methanolischer Salzsäure aufgenommen und stehen gelassen. Das langsam als Niederschlag ausfallende Hydrochlorid wurde abgesaugt, mit Aceton gewaschen und getrocknet.

| | |
|---|---|
| Ausbeute: | 22.6 g (76% d.Th.) als freie Base |
| ESI-MS: | m/z = 161 (M)⁺ |

### Stufe 4: (6-Chlor-pyrimidin-4-yl)-(2,7-dimethyl-1H-benzimidazol-5-yl)-amin

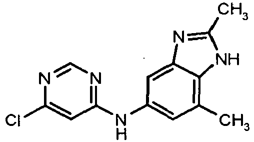

Unter Stickstoffatmosphäre wurden 3.00 g (15.2 mmol) 2,7-Dimethyl-benzimidazol-5-yl-amin-hydrochlorid, 2.30 g (15.4 mmol) 4,6-Dichlorpyrimidin und 6.50 mL (47.0 mmol) TEA in 15 mL 1-Butanol 3 h bei 100°C gerührt. Nach dem Abkühlen auf RT wurde der Reaktionsansatz eingeengt, der Rückstand in DCM verrührt und der entstandene Niederschlag abgesaugt und getrocknet.

| | |
|---|---|
| Ausbeute: | 3.10 g (75% d.Th.) |
| ESI-MS: | m/z = 274/6 (Cl)(M+H)⁺ |
| Rₜ(HPLC): | 0.84 min (Methode B) |

### Intermediat 8

### 3-Piperidin-4-yl-1,3-dihydro-imidazo[4,5-c]chinolin-2-on

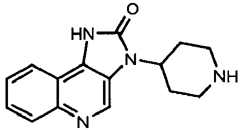

### Stufe 1: 3-Bromchinolin-1-oxid

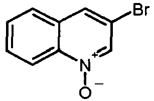

Zu einer auf 5°C gekühlten Lösung von 85 g (0.41 mol) 3-Bromchinolin in 100 mL DCM wurde eine Lösung aus 72%iger 3-Chlorperbenzoesäure (98 g, 0.41 mol) gelöst in 1 L Dichlormethan zugetropft. Dabei wurde darauf geachtet, dass die Temperatur der Reaktionsmischung nicht über 10°C anstieg. Nach Beendigung der Zugabe wurde 5 h gerührt, dann wurde nochmals eine Lösung von 72%iger 3-Chlorperbenzoesäure (25 g, 0.10 mol) gelöst in 200 mL DCM, über Natriumsulfat getrocknet und abfiltriert) zugetropft und über Nacht bei RT gerührt. Es wurde gesättigte wässrige Natriumcarbonat-Lösung zugegeben, die Phasen getrennt und die organische Phase über Natriumsulfat getrocknet. Die Lösung wurde über Aktivkohle filtriert und anschließend i.vac. eingeengt.

| | |
|---|---|
| Ausbeute: | 224 g (99% d.Th.) |
| ESI-MS: | m/z = 223/225 (Br) |
| R_{f}= | 0.15 (Kieselgel, PE / EtOAc = 2:1) |

### Stufe2: 3-Brom-4-nitrochinolin-1-oxid

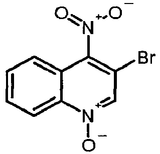

Eine Lösung aus 190 g (0.85 mol) 3-Bromchinolin-1-oxid in 500 mL konzentrierter Schwefelsäure wurde auf 90°C erhitzt. Dann wurden 120 g (1.2 mol) Kaliumnitrat in kleinen Portionen über einen Zeitraum von 100 min derart zugegeben, dass die Temperatur der Reaktion nicht über 95°C anstieg. Der Ansatz wurde 3 h bei 90°C gerührt; man ließ auf RT abkühlen und goss die Mischung auf Eis. Das ausgefallene Produkt wurde abfiltriert und der Filterkuchen mit Wasser gewaschen. Der Rückstand wurde in Dichlormethan gelöst und mit gesättigter, wässriger Natriumhydrogencarbonat-Lösung gewaschen, bis die Lösung alkalisch reagierte. Die Phasen wurden getrennt und die wässrige Phase nochmals mit Dichlormethan extrahiert. Die vereinigten organischen Phasen wurden über Natriumsulfat getrocknet und i.vac. eingeengt. Nach Zerkleinerung des Rückstandes und ausgiebiger Trocknung i.vac. wurde das Produkt als gelber Feststoff erhalten.

| | |
|---|---|
| Ausbeute: | 269 g (46% d.Th.) |
| ESI-MS: | m/z = 268/270 (M+H)⁺ (Br) |
| R_{f}= | 0.77 (Kieselgel, EtOAc) |

### Stufe 3 (1-Benzylpiperidin-4-yl)-(4-nitro-1-oxychinolin-3-yl)-amin

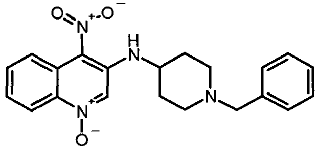

Zu 320 mL (1.54 mol) 4-Amino-1-benzylpiperidin wurden 104 g (0.387 mol) 3-Brom-4-nitrochinolin-1-oxid gegeben. Dann wurden 500 mL THF hinzugefügt und die Mischung bis zur vollständigen Lösung der Substanzen etwas erwärmt. Anschließend wurde 3 h bei 70°C gerührt und die Reaktionsmischung darauf i.vac. eingeengt. Der erhaltene Rückstand wurde in 2.5 L Dichlormethan gelöst und mit gesättigter wässriger Natriumhydrogencarbonat-Lösung gewaschen. Die wässrige Phase wurde nochmals mit 300 mL Dichlormethan extrahiert. Anschließend wurden die organischen Phasen vereinigt, über Natriumsulfat getrocknet und i.vac. eingeengt. Der Rückstand wurde in 250 mL Methanol gelöst. Das als Feststoff ausgefallene Produkt wurde abgesaugt und i.vac. getrocknet.

| | |
|---|---|
| Ausbeute: | 104 g (71% d.Th.) |
| ESI-MS: | m/z = 379 (M+H)⁺ |
| R_{f}: | 0.75 (Kieselgel, EtOAc) |

### Stufe 4 N³-(1-Benzylpiperidin-4-yl)chinolin-3,4-diamin

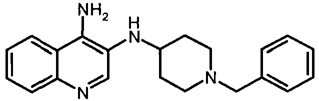

Zu einer Lösung von 76 g (0.20 mol) (1-Benzylpiperidin-4-yl)-(4-nitro-1-oxychinolin-3-yl)-amin in 1.0 L THF wurden 12 g Rhodiumkohle (5%, wasserfeucht) gegeben. Die Reaktion wurde 4.5 h unter Wasserstoffatmosphäre (50 psi) bei RT geschüttelt. Der Katalysator wurde abfiltriert und das Lösungsmittel i.vac. entfernt. Wegen seiner Oxidationsempfindlichkeit wurde das Rohprodukt sogleich für die nächste Stufe eingesetzt.

| | |
|---|---|
| Ausbeute: | 66.0 g (99% d.Th.) |
| R_{f}: | 0.30 (Kieselgel, DCM/MeOH/Cyc/NH₄OH = 70:15:15:2) |

### Stufe 5 3-(1-Benzylpiperidin-4-yl)-1,3-dihydroimidazo[4,5-c]chinolin-2-on

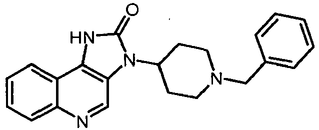

Zu einer Lösung von 9.0 g (27 mmol) *N*³-(1-Benzylpiperidin-4-yl)-chinolin-3,4-diamin in 100 mL DMF wurden 23 g (0.14 mol) 1,1'-Carbonyldiimidazol gegeben. Die Mischung wurde auf 100°C erhitzt und 1.5 h bei dieser Temperatur gerührt. Nach Abkühlen der Reaktionsmischung wurde diese auf 300 mL Wasser gegossen. Der ausgefallene Feststoff wurde abfiltriert, mit Wasser gewaschen und bei 30°C i.vac. getrocknet. Der Rückstand wurde mit Diethylether verrieben, abgesaugt und das Festprodukt i.vac. getrocknet.

| | |
|---|---|
| Ausbeute: | 7.42 g (77% d.Th.) |
| ESI-MS: | m/z = 359 (M+H)⁺ |

### Stufe 6 3-Piperidin-4-yl-1,3-dihydroimidazo[4,5-c]chinolin-2-on

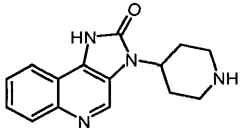

Eine Mischung aus 44 g (0.12 mol) 3-(1-Benzylpiperidin-4-yl)-1,3-dihydroimidazo-[4,5-c]chinolin-2-on und 10 g Palladium auf Kohle (Pd/C 10%) in 500 mL Methanol wurde 16 h bei 50°C in einer Wasserstoff-Atmosphäre von 50 psi hydriert. Nach Filtration des Reaktionsgemisches wurde das Lösungsmittel im Vakuum entfernt. Durch Zugabe von Isopropanol fiel das Produkt als Niederschlag aus. Dieser wurde abfiltriert und anschließend im Vakuum getrocknet.

| | |
|---|---|
| Ausbeute: | 31.2 g (95% d.Th.) |
| ESI-MS: | m/z = 269 (M+H)⁺ |
| R_{f}: | 0.20 (Kieselgel, DCM/MeOH/Cyc/NH₄OH = 70:15:15:2) |

### Intermediat 9

### 6-(6-Chlor-pyrimidin-4-ylamino)-3,4-dimethyl-3H-benzoxazol-2-on

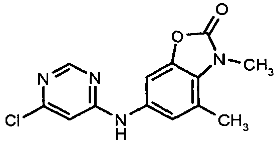

### Stufe 1: 3,4-Dimethyl-2-oxo-2,3-dihydro-benzoxazol-6-carbonsäureazid

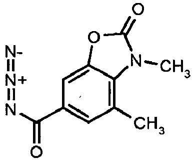

550 mg (2.50 mmol) 4-Methyl-2-oxo-2,3-dihydro-benzoxazol-6-carbonsäureazid und 400 mg (2.90 mmol) Kaliumcarbonat wurden in DMF 20 min bei RT gerührt. Anschließend wurden 0.200 mL (3.21 mmol) Methyljodid hinzugefügt und 1.5 h bei RT gerührt. Zum Reaktionsansatz wurde Wasser gegeben, der entstandene Niederschlag wurde abgesaugt und getrocknet.

| | |
|---|---|
| Ausbeute: | 530 mg (91% d.Th.) |
| ESI-MS: | m/z = 233 (M+H)⁺ |
| Rₜ(HPLC): | 1.39 min (Methode B) |

### Stufe 2: (3,4-Dimethyl-2-oxo-2,3-dihydro-benzoxazol-6-yl)-carbaminsäure-tert-butylester

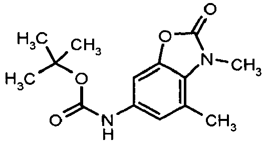

520 mg (2.20 mmol) 3,4-Dimethyl-2-oxo-2,3-dihydro-benzoxazol-6-carbonsäureazid in 10 mL *tert*-Butanol wurden 3.5 h zum Rückfluss erhitzt. Nach Abkühlen auf RT wurde der Reaktionsansatz i.vac. eingeengt und der Rückstand in Diethylether verrührt. Der entstandene Niederschlag wurde abgesaugt und getrocknet.

| | |
|---|---|
| Ausbeute: | 550 mg (79% d.Th.) |
| Reinheit: | 90% |
| ESI-MS: | m/z = 296 (M+NH₄)⁺ |
| Rₜ(HPLC): | 1.44 min (Methode B) |

### Stufe 3: 6-Amino-3,4-dimethyl-3H-benzoxazol-2-on

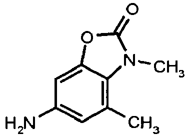

Zu 500 mg (1.80 mmol) (3,4-Dimethyl-2-oxo-2,3-dihydro-benzoxazol-6-yl)-carbaminsäure-*tert*-butylester in 10 mL DCM wurden 500 µL (6.58 mmol) TFA gegeben und 1.5 h bei RT gerührt. Anschließend wurde der Reaktionsansatz eingeengt, der Rückstand alkalisch gestellt und mit DCM ausgeschüttelt. Die organische Phase wurde über Natriumsulfat getrocknet, filtriert und eingeengt.

| | |
|---|---|
| Ausbeute: | 305 mg (95% d.Th.) |
| ESI-MS: | m/z = 179 (M+H)⁺ |
| Rₜ(HPLC): | 0.39 min (Methode B) |

### Stufe 4: 6-(6-Chlor-pyrimidin-4-ylamino)-3,4-dimethyl-3H-benzoxazol-2-on

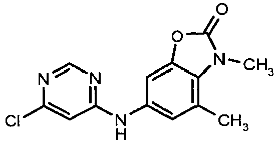

300 mg (1.70 mmol) 6-Amino-3,4-dimethyl-3H-benzoxazol-2-on, 276 mg (1.90 mmol) 4,6-Dichlorpyrimidin und 0.77 mL (60 µmol) TEA wurden in 5.0 mL 1-Butanol zusammen gegeben und 1 h bei 100°C gerührt. Der Reaktionsansatz wurde auf RT abgekühlt, eingeengt und der Rückstand in Diethylether verrieben. Der Niederschlag wurde mit Wasser gewaschen und i.vac. getrocknet.

| | |
|---|---|
| Ausbeute: | 325 mg (66% d.Th.) |
| ESI-MS: | m/z = 291/3 (Cl) (M+H)⁺ |
| Rₜ(HPLC): | 1.28 min (Methode B) |

### Intermediat 10

### (6-Chlor-pyrimidin-4-yl)(7-methyl-1H-indazol-5-yl)-amin

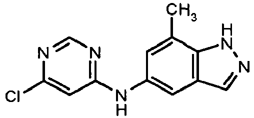

500 mg (3.40 mmol) 4,6-Dichlorpyrimidin, 500 mg (3.40 mmol) 7-Methyl-1 H-indazol-5-yl-amin und 1.30 mL (7.60 mmol) DIPEA wurden in 5.0 mL DMF zusammen gegeben und 5 h unter Rückfluss gekocht. Der Reaktionsansatz wurde auf RT abgekühlt und i.vac. eingeengt. Der Rückstand wurde in EtOAc aufgenommen und mehrmals mit gesättigter Natriumchlorid-Lösung extrahiert. Die organischen Phasen wurden vereinigt, über Magnesiumsulfat getrocknet, filtriert und eingeengt. Der Rückstand wurde mit DIPE verrührt, abgesaugt und i.vac. getrocknet.

| | |
|---|---|
| Ausbeute: | 800 mg (92% d.Th.) |
| ESI-MS: | m/z = 258/260 (Cl) (M-H)⁻ |

### Intermediat 11

### (6-Iod-pyrimidin-4-yl)-(1,2,7-trimethyl-1H-benzimidazol-5-yl)-amin

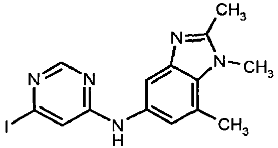

### Stufe 1: (4-Brom-2-methyl-6-nitro-phenyl)-methyl-amin

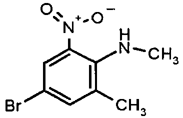

Zu 20.0 g (86.6 mmol) 4-Brom-2-methyl-6-nitroanilin in 250 mL DMF wurden portionsweise 3.80 g (86.6 mmol) Natriumhydrid (55%, Suspension in Mineralöl) unter leichter Kühlung zugegeben und 30 min nachgerührt. Anschließend wurden langsam 5.40 mL (86.6 mmol) Methyljodid zugetropft, die Kühlung entfernt und über Nacht bei RT gerührt. Dann wurde unter Kühlung langsam Wasser zugegeben und mehrmals mit EtOAc extrahiert. Die organischen Phasen wurden vereinigt, über Natriumsulfat getrocknet, filtriert und i.vac. eingeengt. Der Rückstand wurde aus DIPE umkristallisiert, abgekühlt und der Niederschlag abgesaugt und getrocknet.

| | |
|---|---|
| Ausbeute: | 17.0 g (80% d.Th.) |
| ESI-MS: | m/z = 244/46 (Br) (M+) |
| Rₜ(HPLC): | 1.63 min (Methode B) |

### Stufe 2: 5-Brom-3,N²-dimethyl-benzol-1,2-diamin

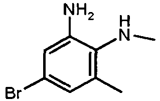

1.50 g (5.50 mmol) (4-Brom-2-methyl-6-nitro-phenyl)-methylamin wurden mit 150 mg Platinkohle in 20 mL EtOAc in einer 50 psi Wasserstoffatmosphäre 2.5 h bei RT hydriert. Der Katalysator wurde abgesaugt und die Lösung einrotiert.

| | |
|---|---|
| Ausbeute: | 1.30 g (93% d.Th.) |
| Reinheit: | 85% |
| ESI-MS: | m/z = 215/17 (Br) (M+H)⁺ |
| Rₜ(HPLC): | 0.89 min (Methode B) |

### Stufe 3: 5-Brom-1,2,7-trimethyl-1H-benzimidazol

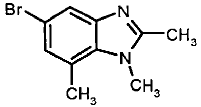

Zu 1.3 g (5.4 mmol) 5-Brom-3,*N*²-dimethyl-benzol-1,2-diamin in 5.0 mL Eisessig wurden 0.60 mL (6.5 mmol) Essigsäureanhydrid zugegeben und 30 min auf 130°C erwärmt. Anschließend wurde konz. Ammoniak langsam zugetropft und 10 min nachgerührt. Der Niederschlag wurde abgesaugt und getrocknet.

| | |
|---|---|
| Ausbeute: | 1.40 g (93% d.Th.) |
| Reinheit: | 85% |
| ESI-MS: | m/z = 239/41 (Br) (M+H)⁺ |
| Rₜ(HPLC): | 0.94 min (Methode B) |

### Stufe 4: 5-Azid-1,2,7-trimethyl-1H-benzimidazol

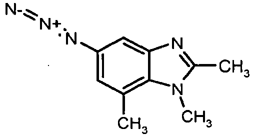

0.59 g (2.50 mmol) 5-Brom-1,2,7-trimethyl-1*H*-benzimidazol, 0.32 g (4.9 mmol) Natriumazid, 75 mg (0.50 mmol) Trans-(1R,2R)-*N,N'*-Bismethyl-1,2-cyclohexan-diamin, 50 mg (0.30 mmol) Kupfer(I)jodid und 50 mg (0.30 mmol) L-Ascorbinsäure Natriumsalz wurden in 2.0 ml Ethanol-Wasser-Gemisch (EtOH / Wasser = 7/3) gegeben und 1 h bei 100°C gerührt. Anschließend wurde das EtOH abdestilliert und der Rückstand mit Wasser versetzt. Der entstandene Niederschlag wurde abgesaugt, mit Wasser und Diethylether gewaschen und getrocknet.

| | |
|---|---|
| Ausbeute: | 0.30 g (60% d.Th.) |
| ESI-MS: | m/z = 202 (M+H)⁺ |
| Rₜ(HPLC): | 0.85 min (Methode B) |

### Stufe 5: 1,2,7-Trimethyl-1H-benzimidazol-5-ylamin Hydrochlorid

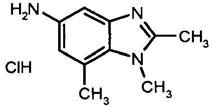

Unter einer Stickstoffatmosphäre wurden 200 mg (1.00 mmol) 5-Azid-1,2,7-trimethyl-1*H*-benzimidazol, 420 mg (1.50 mmol) Tris(2-carboxyethyl)phosphin-hydrochlorid, 1.5 mL Dioxan und 0.5 mL E-Wasser zusammen gegeben und bei RT über Nacht gerührt. Anschließend wurde der Reaktionsansatz sauer gestellt und mit EtOAc extrahiert. Die wässrige Phase wurde mit wässriger Natronlauge alkalisch gestellt und mit DCM extrahiert. Die organische Phase wurde über Natriumsulfat getrocknet, filtriert und eingeengt. Der Rückstand wurde mit etherischer Salzsäure versetzt und das als Niederschlag ausgefallene Hydrochlorid abgesaugt und getrocknet.

| | |
|---|---|
| Ausbeute: | 155 mg (74% d.Th.) |
| ESI-MS: | m/z = 176 (M+H)⁺ |
| Rₜ(HPLC): | 0.27 min (Methode B) |

### Stufe 6: (6-Iod-pyrimidin-4-yl)(1,2,7-trimethyl-1H-benzimidazol-5-yl)-amin

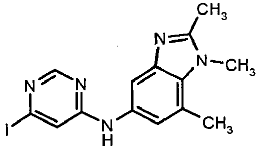

150 mg (0.700 mmol) 1,2,7-Trimethyl-1*H*-benzimidazol-5-ylamin Hydrochlorid, 260 mg (0.800 mmol) 4,6-Diiodopyrimidin, 0.300 mL (2.00 mmol) TEA und 5.0 mL 1-Butanol wurden zusammen gegeben und 1 h bei RT gerührt. Der Reaktionsansatz wurde eingeengt und der Rückstand mit DCM und 1 N wässriger Natronlauge ausgeschüttelt. Die organische Phase wurde über Natriumsulfat getrocknet, filtriert und eingeengt.

| | |
|---|---|
| Ausbeute: | 286 mg (85% d.Th.) |
| Reinheit: | 80% |
| ESI-MS: | m/z = 380 (M+H)⁺ |
| Rₜ(HPLC): | 0.97 min (Methode B) |

### Intermediat 12

### N-(-Chlor-pyrimidin-4-yl)-N-(3,4-dimethyl-2-oxo-2,3-dihydro-benzoxazol-6-yl)-acetamid

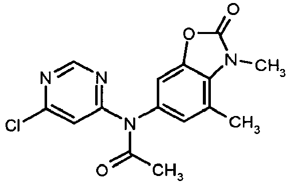

100 mg (0.300 mmol) 6-(6-Chlor-pyrimidin-4-ylamino)-3,4-dimethyl-3H-benzoxazol-2-on wurden zusammen mit 1.00 ml (10.6 mmol) Acetanhydrid in ein Mikrowellengefäß gegeben und 20 min bei 150°C gerührt. Der Reaktionsansatz wurde mit Wasser verdünnt und mit DCM extrahiert. Die organische Phase wurde abgetrennt, getrocknet und eingeengt. Der Rückstand wurde mittels präparativer HPLC gereinigt. Die Produkt enthaltenden Fraktionen wurden vereinigt und eingeengt.

| | |
|---|---|
| Ausbeute: | 70 mg (61% d.Th.) |
| ESI-MS: | m/z = 333/5 (Cl) (M+H)⁺ |
| Rₜ(HPLC): | 1.31 min (Methode B) |

### Intermediat 13

### (6-Chlor-pyrimidin-4-yl)-(2,7-dimethyl-benzoxazol-5-yl)-amin

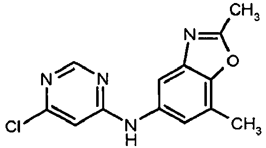

### Stufe 1: 2,4-Diamino-6-methyl-phenol

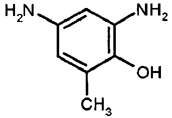

11.0 g (50.0 mmol) 4,6-Dinitro-o-kresol wurden mit 2.0 g Raney-Nickel in 100 mL MeOH unter einer 5 bar Wasserstoffatmosphäre 4 h bei RT hydriert. Anschließend wurde der Kataysator abgesaugt und das Filtrat einrotiert.
Ausbeute: 7.5 g (98% d.Th.)

### Stufe 2: 2,7-Dimethyl-benzoxazol-5-ylamin

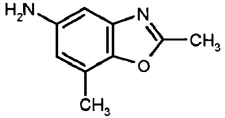

1.00g (7.20 mmol) 2,4-Diamino-6-methyl-phenol, 5.00 mL Essigsäure und 15.0 mL (81.8 mmol) Triethylorthoacetat wurden zusammen gegeben und unter Rückfluss gekocht. Der Reaktionsansatz wurde über Nacht auf RT abgekühlt und eingeengt. Der Rückstand wurde mit 0.5 N Natronlauge und EtOAc ausgeschüttelt und die organische Phase eingeengt. Der Kolbenrückstand wurde mit Diethylether verrieben und der entstandene Niederschlag abgesaugt. Die Mutterlauge wurde eingeengt und der Rückstand mittels Flash-Chromatographie gereinigt. Die Produkt enthaltenden Fraktionen wurden vereinigt und eingeengt.

| | |
|---|---|
| Ausbeute: | 600 mg (26% d.Th.) |
| Reinheit: | 50% |
| ESI-MS: | m/z = 162 (M+H)⁺ |
| Rₜ(HPLC): | 0.70 min (Methode B) |

### Stufe 3: (6-Chlor-pyrimidin-4-yl)-(2,7-dimethyl-benzoxazol-5-yl)-amin

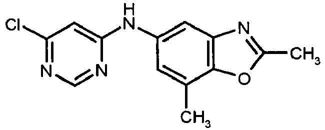

600 mg (1.90 mmol) 2,7-Dimethyl-benzoxazol-5-ylamin, 400 mg (2.70 mmol) 4,6-Dichlorpyrimidin und 1.50 mL (11.0 mmol) TEA wurden in 8.0 mL 1-Butanol vereinigt und 2 h bei 90°C gerührt. Der Reaktionsansatz wurde auf RT abgekühlt und mittels präparativer HPLC gereinigt. Die Produkt enthaltenden Fraktionen wurden vereinigt, eingeengt und der Rückstand mit Diethylether verrieben, abgesaugt und getrocknet.

| | |
|---|---|
| Ausbeute: | 120 mg (24% d.Th.) |
| ESI-MS: | m/z = 275/7 (Cl) (M+H)⁺ |
| Rₜ(HPLC): | 1.33 min (Methode B) |

### Intermediat 14

### 4-(6-Chlor-pyrimidin-4-ylamino)-2,6-dimethyl-phenol

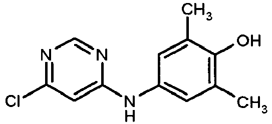

### Stufe 1: 4-Amino-2,6-dimethyl-phenol

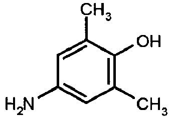

3.00 g (18.0 mmol) 2,6-Dimethyl-4-nitrophenol wurden mit 0.50 g Palladium auf Kohle (Pd/C, 10%) in 50 mL MeOH in einer 3 bar Wasserstoffatmosphäre bei RT hydriert. Nach beendeter Reaktion wurde der Katalysator abgesaugt und das Filtrat einrotiert. Der Rückstand wurde mit DIPE verrieben und der Niederschlag abgesaugt und i.vac. getrocknet.

| | |
|---|---|
| Ausbeute: | 2.10 g (85% d.Th.) |
| ESI-MS: | m/z = 138 (M+H)⁺ |
| R_{f}: | 0.5 (Kieselgel: DCM/MeOH/Cyc/NH₄OH = 70:15:15:2) |

### Stufe 2: 4-(6-Chlor-pyrimidin-4-ylamino)-2,6-dimethyl-phenol

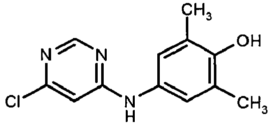

250 mg (1.68 mmol) 4,6-Dichlorpyrimidin, 240 mg (1.75 mmol) 4-Amino-2,6-dimethyl-phenol und 0.650 mL (3.78 mmol) DIPEA in 5 mL DMF wurden zusammen gegeben und 5 h unter Rückfluss gekocht. Der Reaktionsansatz wurde eingeengt, der Rückstand in EtOAC aufgenommen und mit gesättigter Natriumchlorid-Lösung extrahiert. Die organische Phase wurde über Magnesiumsulfat getrocknet, filtriert und eingeengt. Der Rückstand wurde mit DIPE verrührt, der entstandene Niederschlag abgesaugt und i.vac. getrocknet.

| | |
|---|---|
| Ausbeute: | 260 mg (62% d.Th.) |
| ESI-MS: | m/z = 250/252 (Cl) (M+H)⁺ |
| R_{f}: | 0.3 (Kieselgel: PE / EtOAc = 2:1) |

### Intermediat 15

### 5-Amino-1,3-dihydrospiro[inden-2,3'-pyrrolo[2,3-b]pyridin]-2'1'H-on

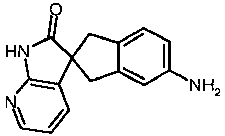

Diese Verbindung und deren Vorstufen wurden analog zur WO 2006/31513 synthetisiert.

### Intermediat 16

### 6-(6-Chlor-pyrimidin-4-ylsulfanyl)-4-methyl-3H-benzoxazol-2-on

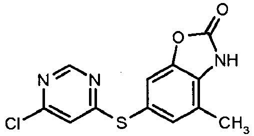

### Stufe 1: 4-Methyl-2-oxo-2,3-dihydro-benzoxazol-6-sulfonsäurechlorid

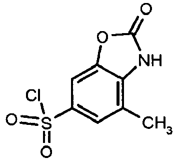

1.10 g (6.60 mmol) 4-Methyl-3H-benzoxazol-2-on in 5.0 mL (75 mmol) Chlorsulfonsäure wurden 2 h bei RT gerührt. Anschließend wurde der Reaktionsansatz sehr langsam auf viel Eis getropft und bei RT nachgerührt. Der entstandene Niederschlag wurde abgesaugt und getrocknet.

| | |
|---|---|
| Ausbeute: | 1.4 g (85% d.Th.) |
| ESI-MS: | m/z = 247 (M)⁺ |
| Rₜ: | 1.25 min (Methode B) |

### Stufe 2: 6-Mercapto-4-methyl-3H-benzoxazol-2-on

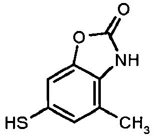

Unter einer Stickstoffatmosphäre wurde zu 1.30 g (19.9 mmol) Zink und 2.40 mL (19.5 mmol) Dichlordimethylsilan in 20 mL 1,2-Dichlorethan langsam eine Lösung aus 1.40 g (5.60 mmol) 4-Methyl-2-oxo-2,3-dihydro-benzoxazol-6-sulfonsäurechlorid und 1.60 g (16.8 mmol) *N,N*-Dimethylacetamid in 1,2-Dichlorethan zugetropft. Der Reaktionsansatz wurde 2 h bei 75°C gerührt. Nach dem Erkalten wurde filtriert und das Filtrat eingeengt. Der verbliebene Rückstand wurde mit MeOH versetzt, der ausgefallene Niederschlag filtriert und das Filtrat eingeengt. Der Rückstand wurde als Rohprodukt weiter umgesetzt.

| | |
|---|---|
| Ausbeute: | 1.0 g (quantitativ) |
| ESI-MS: | m/z = 180 (M-H)⁻ |
| Rₜ: | 1.23 (Methode B) |

### Stufe 3: 6-(6-Chlor-pyrimidin-4-ylsulfanyl)-4-methyl-3H-benzoxazol-2-on

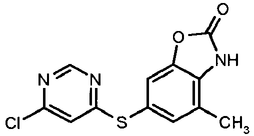

820 mg (5.50 mmol) 4,6-Dichlorpyrimidin, 1.00 g (5.50 mmol) 6-Mercapto-4-methyl-3H-benzoxazol-2-on und 5.00 mL (29.1 mmol) DIPEA wurden in 20 mL DCM zusammen gegeben und über Nacht bei 40°C gerührt. Der Reaktionsansatz wurde eingeengt, der Rückstand in EtOAc aufgenommen und mit Natriumhydrogencarbonat-Lösung versetzt. Der entstandene Niederschlag wurde abgesaugt, mit viel Wasser gewaschen und getrocknet.

| | |
|---|---|
| Ausbeute: | 185 mg (9% d.Th.) |
| Reinheit: | 80% |
| ESI-MS: | m/z = 294 (M+H)⁺ |

### Intermediat 17

### 6-(6-Chlor-2-methyl-pyrimidin-4-yloxy)-4-methyl-3H-benzoxazol-2-on

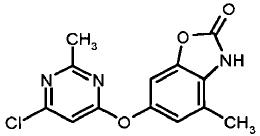

### Stufe 4: 6-(6-Chlor-2-methyl-pyrimidin-4-yloxy)-4-methyl-3H-benzoxazol-2-on

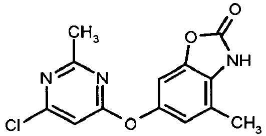

Zu 100 mg (0.575 mmol) 6-Hydroxy-4-methyl-3H-benzoxazol-2-on in 1.5 mL DMF wurden 100 mg (0.724 mmol) Kaliumcarbonat und 110 mg (0.655 mmol) 4,6-Dichlor-2-methyl-pyrimidin zugefügt und 2 h bei RT gerührt. Anschließend wurden 15 mL Wasser hinzugefügt und über Nacht bei RT nachgerührt. Der entstandene Niederschlag wurde abgesaugt, mit Wasser gewaschen und i.vac. getrocknet.

| | |
|---|---|
| Ausbeute: | 145 mg (86% d.Th.) |
| ESI-MS: | m/z = 292(M+H)⁺ |
| Rₜ(HPLC): | 1.26 min (Methode B) |

### Intermediat 18

### Spiro[benzo[d][1,3]oxazin-4,4'-piperidin]-2(1H)-on hydrochlorid

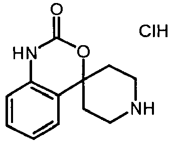

Diese Verbindung und deren Vorstufen wurden wie in US 6,436,962 beschrieben synthetisiert.

| | |
|---|---|
| ESI-MS: | m/z = 219 (M+H)⁺ |
| R_{f}: | 0.14 (Kieselgel, DCM/Cyc/MeOH/ NH₄OH = 70/15/15/2) |

### Intermediat 19

### 2-(6-Chlor-pyrimidin-4-yloxy)-chinoxalin

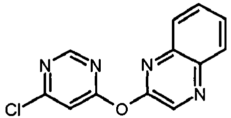

Zu 0.10 g (0.68 mmol) 2-Hydroxychinoxalin in 1.5 mL DMF wurden 40 mg (0.92 mmol) Natriumhydrid (55%, Suspension in Mineralöl) gegeben und 15 min nachgerührt. Anschließend wurden 0.11 g (0.69 mmol) 4,6-Dichlorpyrimidin zugegeben und über Nacht bei RT nachgerührt. Das Reaktionsgemisch wurde mittels präparativer HPLC-MS gereinigt. Die Produkt enthaltenden Fraktionen wurden vereinigt, das Acetonitril i.vac. entfernt, und das ausgefallene Produkt wurde abgesaugt und getrocknet.

| | |
|---|---|
| Ausbeute: | 24 mg (13% d.Th.) |
| ESI-MS: | m/z = 259 (M+H)⁺ |

### Intermediat 20

### 4-Amino-3-methyl-5-nitro-phenol

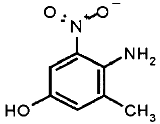

### Stufe 1: Essigsäure-4-acetylamino-3-methyl-phenylester

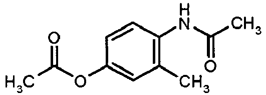

4.66 g (37.5 mmol) 4-Amino-3-methylphenol, 5.00 mL Eisessig, 0.500 g (6.09 mmol) Natriumacetat und 7.87 mL (83.3 mmol) Acetanhydrid wurden zusammen gegeben und 1 h bei 125°C gerührt. Nach dem Abkühlen auf RT wurde Eiswasser zum Reaktionsansatz hinzugefügt. Der ausgefallene Niederschlag wurde abgesaugt, mit Wasser nachgewaschen und im HV getrocknet.

| | |
|---|---|
| Ausbeute: | 5.20 g (67% d.Th.) |
| ESI-MS: | m/z = 208 (M+H)⁺ |
| Rₜ(HPLC): | 0.92 min (Methode B) |

### Stufe 2: Essigsäure-4-acetylamino-3-methyl-5-nitro-phenylester

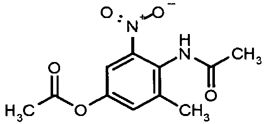

Zu 0.60 mL (14.4 mmol) Salpetersäure (90%) wurden unter Eiskühlung 748 mg (3.61 mmol) Essigsäure-4-acetylamino-3-methyl-phenylester portionsweise zugegeben. Nach beendeter Zugabe wurde die Kühlung entfernt und 2 h bei RT nachgerührt. Dann wurde der Reaktionsansatz auf Eiswasser gegossen, der ausgefallene Niederschlag abgesaugt, mit Wasser nachgewaschen und im HV getrocknet.

| | |
|---|---|
| Ausbeute: | 550 mg (60% d.Th.) |
| ESI-MS: | m/z = 253 (M+H)⁺ |
| Rₜ(HPLC): | 0.98 min (Methode B) |

### Stufe 3: 4-Amino-3-methyl-5-nitro-phenol

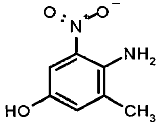

20.4 g (80.7 mmol) Essigsäure-4-acetylamino-3-methyl-5-nitro-phenylester in 100 mL halbkonzentrierter wässriger Salzsäure wurden 2 h unter Rückfluss gekocht. Anschließend wurde der Reaktionsansatz eingeengt, der Rückstand mit Aceton verrieben und abgesaugt. Nach dem Waschen mit Aceton und Diethylether wurde der Niederschlag im HV getrocknet. Die Mutterlauge wurde eingeengt, der Rückstand in Wasser aufgenommen und gefriergetrocknet.

| | |
|---|---|
| Ausbeute: | 6.00 g (44% d.Th.) |
| ESI-MS: | m/z = 169 (M+H)⁺ |
| Rₜ(HPLC): | 0.96 min (Methode B) |

### Intemediat 21

### 3-{1-[6-(3,4-Diamino-5-methyl-phenoxy)-pyrimidin-4-yl]-piperidin-4-yl}-7-methoxy-1,3,4,5-tetrahydro-benzo[d][1,3]diazepin-2-on

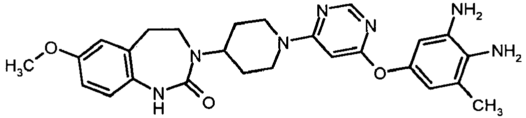

### Stufe 1: 4-(6-Chlor-pyrimidin-4-yloxy)-2-methyl-6-nitro-phenylamin

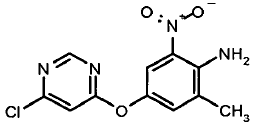

Zu 0.922 g (6.00 mmol) 4,6-Dichlorpyrimidin und 1.11 g (6.60 mmol) 4-Amino-3-methyl-5-nitro-phenol in 1.5 mL DMF wurden 1.66 g (12.0 mmol) Kaliumcarbonat zugegeben und 6 h bei 50°C gerührt. Dann wurde der Ansatz auf Eiswasser gegeben und mehrmals mit EtOAc extrahiert. Die vereinigten organischen Phasen wurden über Natriumsulfat getrocknet, eingeengt und mittels Flash-Chromatographie gereinigt. Die Produkt enthaltenden Fraktionen wurden vereinigt und eingeengt. Der Rückstand wurde mit EtOAc verrieben, abgesaugt und im HV getrocknet.

| | |
|---|---|
| Ausbeute: | 0.50 g (30% d.Th.) |
| ESI-MS: | m/z = 281 (M+H)⁺ |
| Rₜ(HPLC): | 1.43 min (Methode B) |

### Stufe 2: 3-{1-[6-(4-Amino-3-methyl-5-nitro-phenoxy)-pyrimidin-4-yl]-piperidin-4-yl}-7-methoxy-1,3,4,5-tetrahydro-benzo[d][1,3]diazepin-2-on

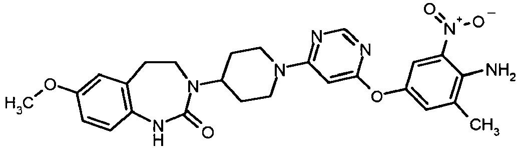

496 mg (1.80 mmol) 7-Methoxy-3-piperidin-4-yl-1,3,4,5-tetrahydro-1,3-benzodiazepin-2-on, 480 mg (1.71 mmol) 4-(6-Chlor-pyrimidin-4-yloxy)-2-methyl-6-nitro-phenylamin und 0.697 mL (4.00 mmol) DIPEA wurden in 10.0 mL DMF zusammen gegeben und über Nacht bei RT gerührt. Der Reaktionsansatz wurde auf Eiswasser gegeben, der ausgefallene Niederschlag abgesaugt, mit Wasser gewaschen und im HV getrocknet.

| | |
|---|---|
| Ausbeute: | 840 mg (90% d.Th.) |
| ESI-MS: | m/z = 520 (M+H)⁺ |
| Rₜ(HPLC): | 1.45 min (Methode B) |

### Stufe 3: 3-{1-[6-(3,4-Diamino-5-methyl-phenoxy)-pyrimidin-4-yl]-piperidin-4-yl}-7-methoxy-1,3,4,5-tetrahydro-benzo[d][1,3]diazepin-2-on

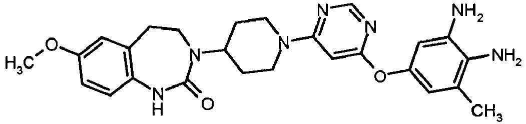

830 mg (1.60 mmol) 3-{1-[6-(4-Amino-3-methyl-5-nitro-phenoxy)-pyrimidin-4-yl]-piperidin-4-yl}-7-methoxy-1,3,4,5-tetrahydro-benzo[d][1,3]diazepin-2-on in 20 mL MeOH wurden mit 100 mg Palladium auf Kohle (Pd/C 10%) versetzt und unter einer Wasserstoffatmosphäre von 50 psi 8 h hydriert. Nach dem Absaugen des Katalysators wurde das Filtrat eingeengt und der Rückstand als Rohprodukt weiter umgesetzt.

| | |
|---|---|
| Ausbeute: | 800 mg (quantitativ) |

### Intermediat 22

### 3,4-Diamino-5-methyl-phenol

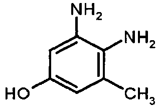

6.00 g (35.7 mmol) 4-Amino-3-methyl-5-nitro-phenol in 200 mL MeOH wurde mit 0.600 g Palladium auf Kohle (Pd/C 10%) versetzt und unter einer Wasserstoffatmosphäre von 60 psi 4 h bei 50°C hydriert. Der Katalysator wurde abgesaugt, das Filtrat eingeengt und der Rückstand im HV getrocknet.

| | |
|---|---|
| Ausbeute: | 4.90 g (99% d.Th.) |
| ESI-MS: | m/z = 139 (M+H)⁺ |
| Rₜ(HPLC): | 0.26 min und 0.69 min (Methode H) (Salzeffekte) |

### Intermediat 23

### 6-(6-Chlor-pyrimidin-4-yloxy)-2,4-dimethyl-1H-benzimidazol

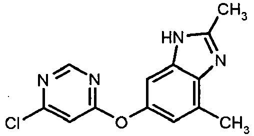

### Stufe 1: 2,7-Dimethyl-3H-benzimidazol-5-ol

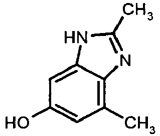

0.553 g (4.00 mml) 3,4-Diamino-5-methyl-phenol in 5.00 mL Eisessig wurden 3 h gekocht. Anschließend wurde der Reaktionsansatz eingeengt und im HV getrocknet.

| | |
|---|---|
| Ausbeute: | 0.65 g (quantitativ) |
| ESI-MS: | m/z = 163 (M+H)⁺ |
| Rₜ(HPLC): | 0.50 min (Methode I) |

### Stufe 2: 6-(6-Chlor-pyrimidin-4-yloxy)-2,4-dimethyl-1H-benzimidazol

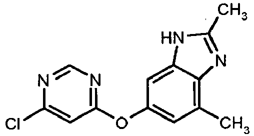

Zu 0.676 g (4.40 mmol) 4,6-Dichlorpyrimidin und 0.649 g (4.00 mmol) 2,7-Dimethyl-3H-benzimidazol-5-ol in 5.00 mL DMF wurden 1.66 g (12.0 mmol) Kaliumcarbonat gegeben und 8 h bei 50°C gerührt. Dann wurde der Ansatz auf Eiswasser gegeben und mehrmals mit EtOAc extrahiert. Die vereinigten organischen Phasen wurden über Natriumsulfat getrocknet, eingeengt und mittels präparativer HPLC-MS gereinigt. Die Produkt enthaltenden Fraktionen wurden vereinigt und das Acetonitril eingeengt. Der Rückstand wurde mit 1 N wässriger Natronlauge alkalisch gestellt und mit EtOAc extrahiert. Die organische Phase wurde über Natriumsulfat getrocknet, filtriert, eingeengt und im HV getrocknet.

| | |
|---|---|
| Ausbeute: | 0.50 g (41% d. Th.) |
| ESI-MS: | m/z = 275 (M+H)⁺ |
| Rₜ(HPLC): | 0.90 min (Methode B) |

### Intermediat 24

### Spiro[piperidin-4,4'-pyrido[2,3-d][1,3]oxazin]-2'(1'H)-on Hydrochlorid

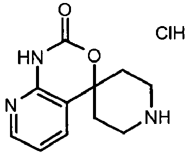

Diese Verbindung und ihre Vorstufen wurden wie in der WO 2006/99268 beschrieben synthetisiert.

| | |
|---|---|
| ESI-MS: | m/z = 220 (M+H)⁺ |
| Rₜ(HPLC): | 0.90 min (Methode C) |

### Intermediat 25

### 4-(6-Chlor-pyrimidin-4-yloxy)-2-methyl-chinolin

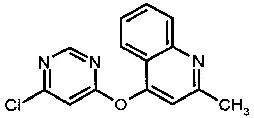

Zu 0 10 g (0.68 mmol) 2-Hydroxy-2-methylchinolin in 1.5 mL DMF wurden 36 mg (0.83 mmol) Natriumhydrid (55%, Suspension in Mineralöl) gegeben und 15 min nachgerührt. Anschließend wurden 97 mg (0.63 mmol) 4,6-Dichlorpyrimidin zugegeben und das Gemisch über Nacht bei RT gerührt. Das Reaktionsgemisch wurde mit Wasser versetzt und über Nacht stehen gelassen. Der entstandene Niederschlag wurde abgesaugt, mit Wasser gewaschen und i.vac. getrocknet.

| | |
|---|---|
| Ausbeute: | 140 mg (65% d.Th.) |
| Reinheit: | 80% |
| ESI-MS: | m/z = 272 (M+H)⁺ |
| Rₜ(HPLC): | 1.03 min (Methode B) |

### Intermediat 26

### 6-(6-Chlor-pyrimidin-4-yloxy)-4-methyl-1H-benzimidazol

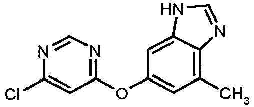

### Stufe 1: 7-Methyl-3H-benzimidazol-5-ol

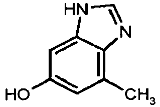

276 mg (2.00 mmol) 3,4-Diamino-5-methyl-phenol in 3.00 mL Ameisensäure (98%) wurden 1 h unter Rückfluss gekocht. Der Reaktionsansatz wurde anschließend eingeengt und der Rückstand im HV getrocknet.

| | |
|---|---|
| Ausbeute: | 295 mg (quantitativ) |
| ESI-MS: | m/z = 149 (M+H)⁺ |
| Rₜ(HPLC): | 0.36 min (Methode B) |

### Stufe 2: 6-(6-Chlor-pyrimidin-4-yloxy)-4-methyl-1H-benzimidazol

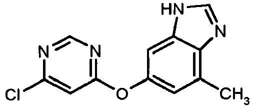

Zu 307 mg (2.00 mmol) 4,6-Dichlorpyrimidin und 295 mg (2.00 mmol) 7-Methyl-3H-benzimidazol-5-ol in 1.50 mL DMF wurden 0.830 g (6.00 mmol) Kaliumcarbonat gegeben und das Gemisch 8 h bei 50°C gerührt. Dann wurde der Ansatz auf Eiswasser gegeben und mehrmals mit EtOAc extrahiert. Die vereinigten organischen Phasen wurden über Natriumsulfat getrocknet, eingeengt und mittels präparativer HPLC-MS gereinigt. Die Produkt enthaltenden Fraktionen wurden vereinigt und das Acetonitril eingeengt. Der Rückstand wurde mit 1 N wässriger Natronlauge alkalisch gestellt und mit EtOAc extrahiert. Die organische Phase wurde über Natriumsulfat getrocknet, filtriert, eingeengt und im HV getrocknet.

| | |
|---|---|
| Ausbeute: | 120 mg (23% d.Th.) |
| ESI-MS: | m/z = 261 (M+H)⁺ |
| Rₜ(HPLC): | 0.90 min (Methode B) |

### Intermediat 27

### 6-Chlor-N-(2,6-dimethylpyridin-4-yl)pyrimidin-4-amin

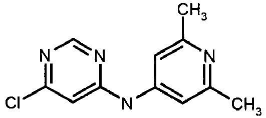

0.12 g (0.97 mmol) 2,6-Dimethyl-4-hydroxypyridin und 55 mg (1.26 mmol) Natriumhydrid (60% Dispersion in Mineralöl) wurden in 1.5 mL DMF gelöst und 15 min gerührt. 0.15 g (0.98 mmol) 4,6-Dichlorpyrimidin wurden zugegeben und über Nacht bei RT gerührt. Der Reaktionsansatz wurde mit einer 4M Salzsäure angesäuert, filtriert und mittels präparativer HPLC-MS gereinigt. Die Produkt enthaltenden Fraktionen wurden vereinigt und gefriergetrocknet.

| | |
|---|---|
| Ausbeute: | 105 mg (46% d.Th.) |
| ESI-MS: | m/z = 236/238 (Cl) (M+H)⁺ |
| Rₜ(HPLC): | 0.78 (Methode B) |

### Intermediat 28

### 6-(2-Amino-6-chlorpyrimidin-4-yloxy)-4-methylbenzo[d]oxazol-2(3H)-on

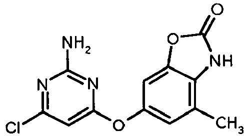

### Stufe 1: 4-Methylbenzo[d]oxazol-2(3H)-on

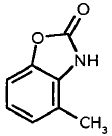

50.0 g (0.39 mol) 2-Amino-m-cresol und 0.21 L (1.23 mol) DIPEA in 400 mL Dichlormethan wurden auf 0°C abgekühlt. Eine Mischung aus 76.0 g (0.45 mol) CDI in 1.2 L Dichlormethan wurde zugetropft und über Nacht bei RT gerührt. Der Reaktionsansatz wurde mit einer 1 M KHSO₄-Lösung und Wasser gewaschen. Die organische Phase wurde abgetrennt und i.vac. eingeengt. Der Rückstand wurde mit kochendem Essigsäureethylester versetzt und während des Abkühlens wurde PE zugetropft. Der entstandene Niederschlag wurde abgesaugt, mit PE nachgewaschen und getrocknet.

| | |
|---|---|
| Ausbeute: | 42.62 g (73% d.Th.) |
| ESI-MS: | m/z = 150 (M+H)⁺ |

### Stufe 2: 6-Acetyl-4-methylbenzo[d]oxazol-2(3H)-on

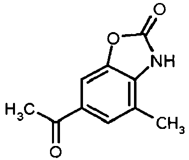

20.0 g (149.99 mmol) Aluminium(III)chlorid wurden unter Kühlung mit 5.0 mL DMF versetzt. Nach 15 min Rühren wurden zunächst 5.0 g (33.52 mmol) 4-Methylbenzo[d]-oxazol-2(3H)-on und anschließend 5.0 mL (52.90 mmol) Acetanhydrid eingetragen und für 1 h bei 80°C gerührt. Die abgekühlte Reaktionsmischung wurde mit Wasser versetzt. Der entstandene Niederschlag wurde abgesaugt, nachgewaschen und getrocknet.

| | |
|---|---|
| Ausbeute: | 4.40 g (58% d.Th.) |
| ESI-MS: | m/z = 192 (M+H)⁺ |
| Rₜ(HPLC): | 1.02 min (Methode B) |

### Stufe 3: 4-Methyl-2-oxo-2,3-dihydrobenzo[d]oxazol-6-yl-acetat

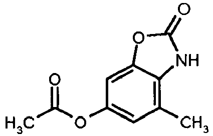

Unter einer Stickstoffatmosphäre wurden 4.40 g (19.56 mmol) 6-Acetyl-4-methylbenzo[d]-oxazol-2(3H)-on in 50.0 mL AcOH eingetragen und mit 4.65 g (26.93 mmol) Metachlorperbenzoesäure versetzt. Nachdem das Gemisch über Nacht bei 50°C gerührt hat, wurden nochmals 1.0 g (5.79 mmol) Metachlorperbenzoesäure zugeben und weitere 3 h bei 60°C gerührt. Die Reaktionsmischung wurde i.vac. eingeengt und der Rückstand in Essigsäureethylester aufgenommen. Die organische Phase wurde mit gesättigter Na₂SO₃-Lösung und gesättigter NaHCO₃-Lösung gewaschen und anschließend eingeengt.

| | |
|---|---|
| Ausbeute: | 4.20 g (93% d.Th.) |
| ESI-MS: | m/z = 208 (M+H)⁺ |
| Rₜ(HPLC): | 1.07 min (Methode B) |

### Stufe 4: 6-Hydroxy-4-methylbenzo[d]oxazol-2(3H)-on

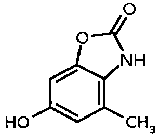

4.20 g (18.24 mmol) 4-Methyl-2-oxo-2,3-dihydrobenzo[d]oxazol-6-yl-acetat in 50.0 mL einer 4M Salzsäure wurden 3 h bei 80°C gerührt. Der Reaktionsansatz wurde auf RT abgekühlt und der ausgefallene Niederschlag abgesaugt. Die erhaltene Substanz wurde chromatographisch aufgereinigt. Die Produkt enthaltenden Fraktionen wurden vereinigt und i.vac. eingeengt.

| | |
|---|---|
| Ausbeute: | 2.10 g (70% d.Th.) |
| ESI-MS: | m/z = 166 (M+H)⁺ |
| Rₜ(HPLC): | 0.75 min (Methode B) |

### Stufe 5: 6-(2-Amino-6-chlorpyrimidin-4-yloxy)-4-methylbenzo[d]oxazol-2(3H)-on

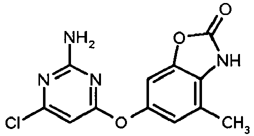

0.20 g (1.15 mmol) 6-Hydroxy-4-methylbenzo[d]oxazol-2(3H)-on und 0.19 g (1.16 mmol) 2-Amino-4,6-dichlorpyrimidin in 3.0 mL DMF wurden mit 0.22 g (1.59 mmol) Kaliumcarbonat versetzt und über Nacht bei 60°C gerührt. Anschließend wurde Wasser hinzugefügt und der entstandene Niederschlag abgesaugt, gewaschen und getrocknet.

Die Substanz wurde mittels präparativer HPLC-MS gereinigt. Die Produkt enthaltenden Fraktionen wurden vereinigt und i.vac. eingeengt.

| | |
|---|---|
| Ausbeute: | 70 mg (20% d.Th.) |
| ESI-MS: | m/z = 293 (M+H)⁺ |
| Rₜ(HPLC): | 1.22 min (Methode B) |

### Intermediat 29

### 6-(6-Chlorpyrimidin-4-yloxy)-2-(methoxymethyl)-4-methyl-1H-benzo[d]imidazol

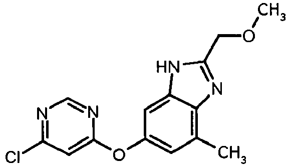

### Stufe 1: 2-(Methoxymethyl)-4-methyl-1H-benzo[d]imidazol-6-ol

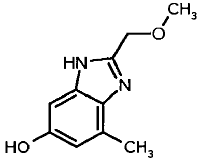

0.55 g (4.00 mmol) 3,4-Diamino-5-methyl-phenol und 1.57 mL (20.00 mmol) Methoxyessigsäure wurden für 1 h bei 120°C gerührt. Anschließend wurde das Reaktionsgemisch eingeengt und als Rohprodukt weiter umgesetzt.

| | |
|---|---|
| Ausbeute: | 0.77 g (quantitativ) |
| ESI-MS: | m/z = 193 (M+H)⁺ |
| Rₜ(HPLC): | 0.65 min (Methode B) |

### Stufe 2: 6-(6-Chlorpyrimidin-4-yloxy)-2-(methoxymethyl)-4-methyl-1H-benzo[d]-imidazol

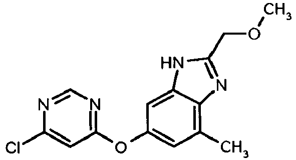

0.77 g (4.00 mmol) 2-(Methoxymethyl)-4-methyl-1H-benzo[d]imidazol-6-ol und 0.68 g (4.40 mmol) 4,6-Dichlorpyrimidin in 5.0 mL DMF wurden mit 1.66 g (12.0 mmol) Kaliumcarbonat versetzt und 8 h bei 70°C gerührt. Anschließend wurde Eiswasser hinzugefügt und die Substanz mit Essigsäureethylester extrahiert und i.vac. einrotiert. Der Rückstand wurde mittels präparativer HPLC-MS gereinigt. Die Produkt enthaltenden Fraktionen wurden vereinigt und i.vac. eingeengt.

| | |
|---|---|
| Ausbeute: | 0.10 g (7.5% d.Th.) |
| ESI-MS: | m/z = 305 (M+H)⁺ |
| Rₜ(HPLC): | 0.99 min (Methode B) |

### Intermediat 30

### 6-(6-Chlor-2-methoxypyrimidin-4-yloxy)-4-methylbenzo[d]oxazol-2(3H)-on

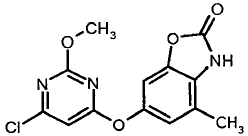

### Stufe 1: 2-Methoxypyrimidin-4,6-diol

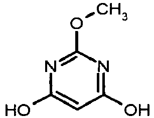

Unter einer Stickstoffatmosphäre wurden 3.10 mL (27.0 mmol) Dimethylmalonat, 3.00 g (27.1 mmol) o-Methylisoharnstoff Hydrochlorid und 15.0 mL (80.8 mmol) Natriummethanolat-Lösung (30%) 1.25 h unter Rückfluss gerührt. Der Reaktionsansatz wurde abgekühlt. Der ausgefallene Niederschlag wurde abgesaugt, nachgewaschen und getrocknet.

| | |
|---|---|
| Ausbeute: | 2.27 g (30% d.Th.) |
| ESI-MS: | m/z = 143 (M+H)⁺ |

### Stufe 2: 4,6-Dichlor-2-methoxypyrimidin

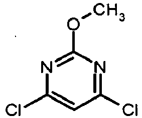

2.25 g (7.92 mmol) 2-Methoxypyrimidin-4,6-diol und 10.0 mL (108.92 mmol) Phosphoroxychlorid wurden 2 h gekocht. Der Reaktionsansatz wurde auf alkalisches Eiswasser gegossen und mit NaOH alkalisch gestellt. Die wässrige Phase wurde mit DCM extrahiert. Die organische Phase wurde abgetrennt, getrocknet und i.vac. eingeengt.

| | |
|---|---|
| Ausbeute: | 0.20 g (14% d.Th.) |
| ESI-MS: | m/z = 179 (M+H)⁺ |
| Rₜ(HPLC): | 1.38 min (Methode B) |

### Stufe 3: 6-(6-Chlor-2-methoxypyrimidin-4-yloxy)-4-methylbenzo[d]oxazol-2(3H)-on

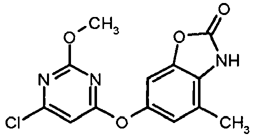

0.19 g (1.01 mmol) 4,6-Dichlor-2-methoxypyrimidin, 0.18 g (1.04 mmol) 6-Hydroxy-4-methyl-3H-benzoxazol-2-on und 0.15 g (1.09 mmol) Kaliumcarbonat in 1.5 mL DMF wurden 2 h bei RT gerührt. Anschließend wurde Wasser hinzugefügt und der entstandene Niederschlag abgesaugt, nachgewaschen und getrocknet.

| | |
|---|---|
| Ausbeute: | 145 mg (42% d.Th.) |
| ESI-MS: | m/z = 308/310 (Cl) (M+H)⁺ |
| Rₜ(HPLC): | 1.41 min (Methode B) |

### Intermediat 31

### 6-(6-Chlorpyrimidin-4-yloxy)-2-cyclopropyl-4-methyl-1H-benzo[d]imidazol

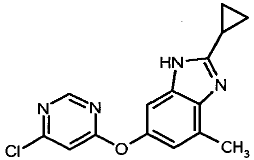

### Stufe 1: 2-Cyclopropyl-4-methyl-1H-benzo[d]imidazol-6-ol

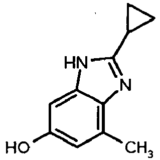

0.55 g (4.00 mmol) 3,4-Diamino-5-methyl-phenol und 1.67 mL (20.00 mmol) Cyclopropancarbonsäure wurden für 1 h bei 120°C gerührt. Anschließend wurde der Reaktionsansatz i.vac. eingeengt und als Rohprodukt weiter umgesetzt.

| | |
|---|---|
| Ausbeute: | 90 mg (12% d.Th.) |
| ESI-MS: | m/z = 189 (M+H)⁺ |

### Stufe 2: 6-(6-Chlorpyrimidin-4-yloxy)-2-cyclopropyl-4-methyl-1H-benzo[d]imidazol

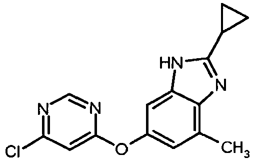

90 mg (0.48 mmol) 2-Cyclopropyl-4-methyl-1H-benzo[d]imidazol-6-ol und 77 mg (0.50 mmol) 4,6-Dichlorpyrimidin in 2.0 mL DMF wurden mit 70 mg (0.50 mmol) Kaliumcarbonat versetzt und 8 h bei 50°C gerührt. Anschließend wurde Eiswasser hinzugefügt und die Substanz wurde mit Essigsäureethylester extrahiert und i.vac. einrotiert.

| | |
|---|---|
| Ausbeute: | 120 mg (80% d.Th.) |
| ESI-MS: | m/z = 301 (M+H)⁺ |
| Rₜ(HPLC): | 1.02 min (Methode B) |

### Intermediat 32

### 6-(6-Chlor-2-cyclopropylpyrimidin-4-yloxy)-4-methylbenzo[d]oxazol-2(3H)-on

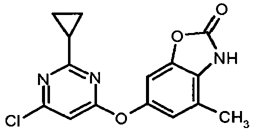

### Stufe 1: 2-Cyclopropylpyrimidine-4,6-diol

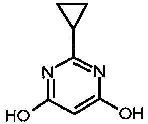

Unter einer Stickstoffatmosphäre wurden 1.50 mL (13.06 mmol) Dimethylmalonat, 1.60 g (13.27 mmol) Cyclopropancarboxamidin Hydrochlorid und 15.0 mL (80.80 mmol) Natriummethanolat-Lösung (30%) 1.25 h unter Rückfluss gerührt. Der Reaktionsansatz wurde leicht angesäuert und der ausgefallene Niederschlag wurde abgesaugt, nachgewaschen und getrocknet.

| | |
|---|---|
| Ausbeute: | 1.35 g (64% d.Th.) |
| ESI-MS: | m/z = 153 (M+H)⁺ |
| Rₜ(HPLC): | 0.75 min (Methode K) |

### Stufe 2: 4,6-Dichlor-2-cyclopropylpyrimidin

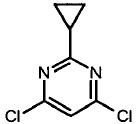

1.34 g (8.40 mmol) 2-Cyclopropylpyrimidin-4,6-diol und 15.0 mL (163.37 mmol) Phosphoroxychlorid wurden 2 h gekocht. Der Reaktionsansatz wurde auf Eiswasser gegossen und mit NaOH alkalisch gestellt. Der ausgefallene Niederschlag wurde abgesaugt, nachgewaschen und getrocknet.

| | |
|---|---|
| Ausbeute: | 1.02g (61% d.Th.) |
| Rₜ(HPLC): | 1.57 min (Methode B) |

### Stufe 3: 6-(6-Chlor-2-cyclopropylpyrimidin-4-yloxy)-4-methylbenzo[d]oxazol-2(3H)-on

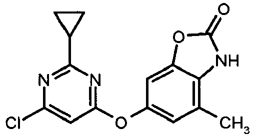

Zu 95 mg (0.55 mmol) 6-Hydroxy-4-methylbenzo[d]oxazol-2(3H)-on und 0.21 g (0.63 mmol) Cäsiumcarbonat in 1.5 mL DMF wurden nach 15 min 0.10 g (0.53 mmol) 4,6-Di-chlor-2-cyclopropylpyrimidin zugegeben und über Nacht bei RT gerührt. Der Reaktionsansatz wurde mittels präparativer HPLC-MS gereinigt. Die Produkt enthaltenden Fraktionen wurden vereinigt und gefriergetrocknet.

| | |
|---|---|
| Ausbeute: | 43 mg (24% d.Th.) |
| ESI-MS: | m/z = 318/320 (Cl) (M+H)⁺ |
| Rₜ(HPLC): | 1.47 min (Methode B) |

### Intermediat 33

### 6-(2,6-Dichlorpyrimidin-4-yloxy)-4-methylbenzo[d]oxazol-2(3H)-on

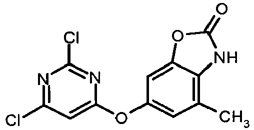

Zu 0.15 g (0.83 mmol) 6-Hydroxy-4-methylbenzo[d]oxazol-2(3H)-on und 0.32 g (0.98 mmol) Cäsiumcarbonat in 1.5 mL DMF wurden nach 15 min 0.15 g (0.82 mmol) 2,4,6-Trichlorpyrimidin zugegeben und 1.5 h bei RT gerührt. Der Reaktionsansatzh wurde mit Wasser versetzt. Der ausgefallene Niederschlag wurde abgesaugt, nachgewaschen und getrocknet.

| | |
|---|---|
| Ausbeute: | 210 mg (78% d.Th.) |
| ESI-MS: | m/z = 312/314/316 (Cl2) (M+H)⁺ |
| Rₜ(HPLC): | 1.41 min (Methode B) |

### Intermediat 34

### 6-(6-Chlorpyrimidin-4-yloxy)benzo[d]oxazol-2(3H)-on

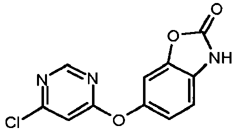

0.20 g (1.34 mmol) 4,6-Dichlorpyrimidin, 0.21 g (1.39 mmol) 6-Hydroxy-2-benzoxazolinon und 0.20. g (1.45 mmol) Kaliumcarbonat in 2.0 mL DMF wurden 2 h bei RT gerührt. Der Reaktionsansatz wurde mit Wasser versetzt. Der ausgefallene Niederschlag wurde abgesaugt, nachgewaschen und getrocknet.

| | |
|---|---|
| Ausbeute: | 0.23 g (65% d.Th.) |
| ESI-MS: | m/z = 264/266 (Cl) (M+H)⁺ |
| Rₜ(HPLC): | 3.21 min (Methode C) |

### Intermediat 35

### 7-Methoxy-3-(1-(6-(3-methyl-4-nitrophenoxy)pyrimidin-4-yl)piperidin-4-yl)-4,5-dihydro-1H-benzo[d][1,3]diazepin-2(3H)-on

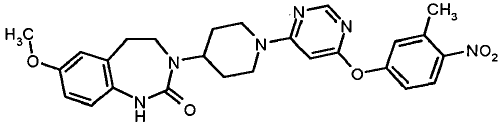

### Stufe 1: 4-Chlor-6-(3-methyl-4-nitrophenoxy)pyrimidin

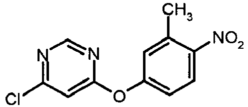

0.40 g (2.68 mmol) 4,6-Dichlorpyrimidin, 0.42 g (2.74 mmol) 3-Methyl-4-nitrophenol und 0.40 g (2.89 mmol) Kaliumcarbonat in 2.0 mL DMF wurden für 2 h bei RT gerührt. Der Reaktionsansatz wurde mit Wasser versetzt und der ausgefallene Niederschlag wurde abgesaugt, nachgewaschen und getrocknet.

| | |
|---|---|
| Ausbeute: | 660 mg (93% d.Th.) |
| ESI-MS: | m/z = 266/268 (Cl) (M+H)⁺ |
| Rₜ(HPLC): | 1.51 min (Methode B) |

### Stufe 2: 7-Methoxy-3-(1-(6-(3-methyl-4-nitrophenoxy)pyrimidin-4-yl)piperidin-4-yl)-4,5-dihydro-1H-benzo[d][1,3]diazepin-2(3H)-on

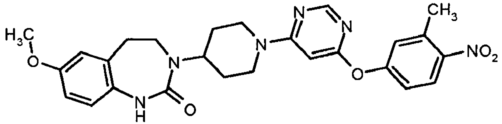

0.69 g (2.51 mmol) 7-Methoxy-3-piperidin-4-yl-1,3,4,5-tetrahydro-1,3-benzodiazepin-2-on, 0.66 g (2.48 mmol) 4-Chlor-6-(3-methyl-4-nitrophenoxy)pyrimidin und 1.0 mL (5.81 mmol) DIPEA in 1.0 mL DMF wurden über Nacht bei 40°C gerührt. Der Reaktionsansatz wurde mit Wasser versetzt. Der ausgefallene Niederschlag wurde abgesaugt, nachgewaschen und getrocknet.

| | |
|---|---|
| Ausbeute: | 0.93 g (74% d.Th.) |
| ESI-MS: | m/z = 505 (M+H)⁺ |
| Rₜ(HPLC): | 1.59 min (Methode B) |

### Intermediat 36

### 7-Chlor-5-(6-chlorpyrimidin-4-yloxy)-2-methyl-1H-benzo[d]imidazol

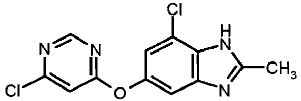

### Stufe 1: 2-Chlor-4-methoxy-6-nitroanilin

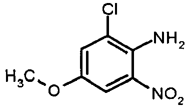

20.0 g (118.94 mmol) 4-Methoxy-2-nitroanilin und 10.0 mL Sulfurylchlorid wurden in 300.0 mL Essigsäure vorgelegt und 3 h bei RT gerührt. Der Reaktionsansatz wurde auf Wasser gegossen und der ausgefallene Niederschlag wurde abgesaugt, nachgewaschen und getrocknet. Die erhaltene Substanz wurde chromatographisch aufgereinigt. Die Produkt enthaltenden Fraktionen wurden vereinigt und i.vac. eingeengt.

| | |
|---|---|
| Ausbeute: | 2.40 g (10% d.Th.) |
| ESI-MS: | m/z = 203/205 (Cl) (M+H)⁺ |
| Rₜ(HPLC): | 1.41 min (Methode B) |

### Stufe 2: 3-Chlor-5-methoxybenzol-1,2-diamin

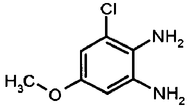

2.40 g (11.85 mmol) 2-Chlor-4-methoxy-6-nitroanilin wurden in 50.0 mL Essigsäureethylester vorgelegt und unter einer Wasserstoff-Atmosphäre unter Zusatz von 0.22 g Raney-Nickel bei RT und einem Wasserstoffdruck von 3 bar bis zur vollständigen Wasserstoffaufnahme hydriert. Der Katalysator wurde abfiltriert. Das Filtrat wurde i.vac. eingeengt.

| | |
|---|---|
| Ausbeute: | 2.00 g (98% d.Th.) |
| ESI-MS: | m/z = 173/175 (Cl) (M+H)⁺ |
| Rₜ(HPLC): | 0.89 min (Methode B) |

### Stufe 3: 7-Chlor-5-methoxy-2-methyl-1H-benzo[d]imidazol

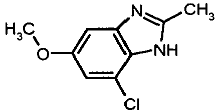

1.00 g (5.79 mmol) 3-Chlor-5-methoxybenzol-1,2-diamin und 20.0 mL Essigsäure wurden für 60 h unter Rückfluss gerührt. Der Reaktionsansatz wurde i.vac. eingeengt. Der Rückstand wurde mit einer wässrigen, gesättigten NaHCO₃-Lösung versetzt. Der Niederschlag wurde abgesaugt, nachgewaschen und getrocknet.

| | |
|---|---|
| Ausbeute: | 0.98 g (86% d.Th.) |
| ESI-MS: | m/z = 197/199 (Cl) (M+H)⁺ |
| Rₜ(HPLC): | 0.88 min (Methode B) |

### Stufe 4: 7-Chlor-5-hydroxy-2-methyl-1H-benzo[d]imidazol

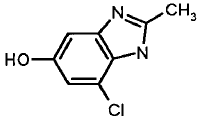

Unter Stickstoffatmosphäre wurden 0.95 g (4.83 mmol) 7-Chlor-5-methoxy-2-methyl-1 H-benzo[d]imidazol und 7.0 g (0.06 mol) Pyridin Hydrochlorid bei 180°C Badtemperatur 15 h gerührt. Der Reaktionsansatz wurde heiß auf Wasser gegossen und mit einer wässrigen K₂CO₃-Lösung alkalisch gestellt. Die Substanz wurde mit Essigsäureethylester extrahiert und mit Wasser gewaschen. Die organische Phase wurde abgetrennt, getrocknet und i.vac. eingeengt.

| | |
|---|---|
| Ausbeute: | 600 mg (68% d.Th.) |
| ESI-MS: | m/z = 183/185 (Cl) (M+H)⁺ |
| Rₜ(HPLC): | 0.69 min (Methode B) |

### Stufe 5: 7-Chlor-5-(6-chlorpyrimidin-4-yloxy)-2-methyl-1H-benzo[d]imidazol

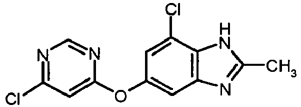

0.49 g (3.29 mmol) 4,6-Dichlorpyrimidin, 0.60 g (3.29 mmol) 7-Chlor-5-hydroxy-2-methyl-1 H-benzo[d]imidazol und 0.50 mg (3.62 mmol) Kaliumcarbonat in 2.0 mL DMF wurden für 2 h bei RT gerührt. Der Reaktionsansatz wurde mit Wasser versetzt und der ausgefallene Niederschlag abgesaugt, nachgewaschen und getrocknet.

| | |
|---|---|
| Ausbeute: | 0.78 g (80% d.Th.) |
| ESI-MS: | m/z = 295/297 (Cl) (M+H)⁺ |
| Rₜ(HPLC): | 1.02 min (Methode B) |

### Intermediat 37

### (S)-6-(6-Chlorpyrimidin-4-yloxy)-4-methyl-2-(tetrahydrofuran-2-yl)-1H-benzo[d]imidazol

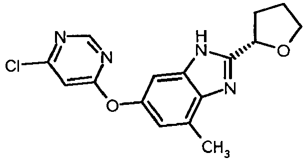

### Stufe 1: Tetrahydro-furan-2-carbonsäure 4-amino-3-methyl-5-nitro-phenylester

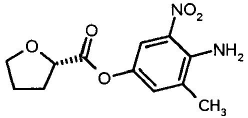

0.84 g (5.00 mmol) 4-Amino-3-methyl-5-nitro-phenol, 0.58 g (5.00 mmol) (*R*)-Tetrahdyro-furan-2-carbonsäure, 1.77 g (5.50 mmol) TBTU und 1.92 mL (11.00 mmol) DIPEA in 10.0 mL DMF wurden über Nacht bei RT gerührt. Der Reaktionsansatz wurde auf Eiswasser gegossen und mit Essigsäureethylester extrahiert. Die organische Phase wurde abgetrennt, getrocknet und i.vac. eingeengt. Der Rückstand wurde mittels präparativer HPLC-MS gereinigt. Die Produkt enthaltenden Fraktionen wurden teilweise i.vac. eingeengt und mit einer wässrigen 1 M Natronlauge neutralisiert. Der ausgefallene Niederschlag wurde abgesaugt, nachgewaschen und getrocknet.

| | |
|---|---|
| Ausbeute: | 0.62 g (47% d.Th.) |
| ESI-MS: | m/z = 267 (M+H)⁺ |
| Rₜ(HPLC): | 1.33 min (Methode B) |

### Stufe 2: (S)-4-Methyl-2-(tetrahydrofuran-2-yl)-1H-benzo[d]imidazol-6-ol

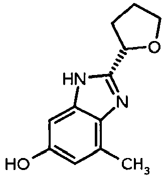

0.62 g (2.33 mmol) Tetrahydrofuran-2-carbonsäure 4-amino-3-methyl-5-nitro-phenylester wurden in 20.0 mL Essigsäure vorgelegt und unter einer Wasserstoff-Atmosphäre unter Zusatz von 0.50 g Raney-Nickel bei 50°C und einem Wasserstoffdruck von 60 psi bis zur vollständigen Wasserstoffaufnahme hydriert. Der Katalysator wurde abfiltriert. Das Filtrat wurde chromatographisch gereinigt. Die Produkt enthaltenden Fraktionen wurden vereinigt und i.vac. eingeengt.

| | |
|---|---|
| Ausbeute: | 140 mg (28% d.Th.) |
| ESI-MS: | m/z = 219 (M+H)⁺ |
| Rₜ(HPLC): | 0.75 min (Methode B) |

### Stufe 3: (S)-6-(6-Chlorpyrimidin-4-yloxy)-4-methyl-2-(tetrahydrofuran-2-yl)-1H-benzo[d]imidazol

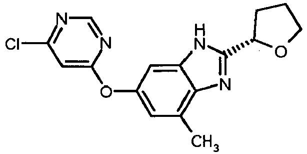

135 mg (0.62 mmol) (*S*)-4-Methyl-2-(tetrahydrofuran-2-yl)-1H-benzo[d]imidazol-6-ol und 97 mg (0.63 mmol) 4,6-Dichlorpyrimidin in 2.0 mL DMF wurden mit 97 mg (0.70 mmol) Kaliumcarbonat versetzt und über Nacht bei 50°C gerührt. Anschließend wurde Eiswasser hinzugefügt und mit Essigsäureethylester extrahiert. Die organische Phase wurde i.vac. einrotiert.

| | |
|---|---|
| Ausbeute: | 210 mg (quantitativ) |
| ESI-MS: | m/z = 331 (M+H)⁺ |
| Rₜ(HPLC): | 1.08 min (Methode B) |

### Intermediat 38

### 4-Chlor-5-(6-chlorpyrimidin-4-yloxy)-2-methyl-1H-benzo[d]imidazol

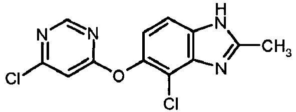

### Stufe 1: 5-Methoxy-2-methyl-1H-benzo[d]imidazol

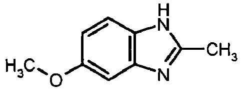

1.95 g (14.11 mmol) 4-Methoxy-o-phenylendiamin wurden in 30.0 mL Essigsäure für 2 h unter Rückfluss gerührt. Der Reaktionsansatz wurde i.vac. eingeengt. Der Rückstand wurde in einer wässrigen NaHCO₃-Lösung aufgenommen und filtriert. Das Filtrat wurde mit Dichlormethan extrahiert. Die organische Phase wurde abgetrennt, getrocknet und i.vac. eingeengt.

| | |
|---|---|
| Ausbeute: | 1.30 g (57% d.Th.) |
| ESI-MS: | m/z = 163 (M+H)⁺ |
| Rₜ(HPLC): | 0.69 min (Methode B) |

### Stufe 2: 4-Chlor-5-methoxy-2-methyl-1H-benzo[d]imidazol

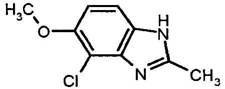

1.30 g (8.02 mmol) 5-Methoxy-2-methyl-1 H-benzo[d]imidazol und 0.70 mL (8.56 mmol) Sulfurylchlorid in 30.0 mL Essigsäure wurden 2 h gerührt. Es wurden nochmals 0.3 mL Sulfurylchlorid zugegeben und weitere 2 h nachgerührt. Der Reaktionsansatz wurde mit Wasser versetzt und i.vac. eingeengt. Der Rückstand wurde mit DIPE und Isopropanol verrührt, abgesaugt und getrocknet.

| | |
|---|---|
| Ausbeute: | 1.50 g (95% d.Th.) |
| ESI-MS: | m/z = 197/199 (Cl) (M+H)⁺ |
| Rₜ(HPLC): | 0.82 min (Methode B) |

### Stufe 3: 4-Chlor-5-hydroxy-2-methyl-1H-benzo[d]imidazol

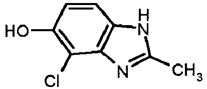

Unter Stickstoffatmosphäre wurden 1.50 g (7.63 mmol) 4-Chlor-5-methoxy-2-methyl-1 H-benzo[d]imidazol und 11.20 g (0.10 mol) Pyridin Hydrochlorid bei 180°C Badtemperatur 15 min gerührt. Der Reaktionsansatz wurde heiß auf Wasser gegossen. Die Substanz wurde mit Essigsäureethylester extrahiert und mit Wasser gewaschen. Die organische Phase wurde abgetrennt, getrocknet und i.vac. eingeengt. Der Rückstand wurde mit DIPE und Essigsäureethylester verrührt, abgesaugt und getrocknet.

| | |
|---|---|
| Ausbeute: | 0.66 g (47% d.Th.) |
| ESI-MS: | m/z = 183/185 (Cl) (M+H)⁺ |
| Rₜ(HPLC): | 0.43 min (Methode B) |

### Stufe 4: 4-Chlor-5-(6-chlorpyrimidin-4-yloxy)-2-methyl-1H-benzo[d]imidazol

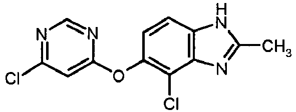

270 mg (1.81 mmol) 4,6-Dichlorpyrimidin, 330 mg (1.81 mmol) 4-Chlor-5-hydroxy-2-methyl-1H-benzo[d]imidazol und 270 mg (1.95 mmol) Kaliumcarbonat in 1.5 mL DMF wurden für 2 h bei RT gerührt. Der Reaktionsansatz wurde mit Wasser versetzt und der ausgefallene Niederschlag wurde abgesaugt, nachgewaschen und getrocknet.

| | |
|---|---|
| Ausbeute: | 400 mg (75% d.Th.) |
| ESI-MS: | m/z = 295/297 (Cl) (M+H)⁺ |
| Rₜ(HPLC): | 0.98 min (Methode B) |

### Intermediat 39

### 5-Chlor-7-(6-chlorpyrimidin-4-yloxy)-2H-benzo[b][1,4]oxazin-3(4H)-on

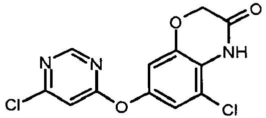

### Stufe 1: 5-Methyl-1,3-phenylen diacetat

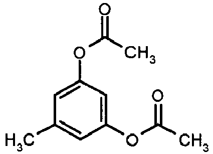

5.80 g (0.04 mol) 5-Methylresorcin Monohydrat, 20.0 mL (0.21 mol) Essigsäureanhydrid und 0.05 g 4-Dimethylaminopyridin wurden in 40.0 mL Essigsäure für 2.5 h bei 100°C gerührt. Der Reaktionsansatz wurde i.vac. eingeengt. Der Rückstand wurde in Dichlormethan aufgenommen und mit Wasser gewaschen. Die organische Phase abgetrennt, getrocknet und i.vac. eingeengt.

| | |
|---|---|
| Ausbeute: | 8.20 g (quantitativ) |
| ESI-MS: | m/z = 209 (M+H)⁺ |
| Rₜ(HPLC): | 3.79 min (Methode C) |
| R_{f}: | 0.6 (Kieselgel: PE/Essigester = 2:1) |

### Stufe 2: 5-Methyl-4-nitro-1,3-phenylendiacetat

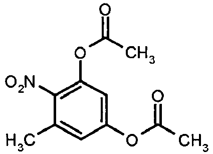

Unter Argonatmosphäre wurde 1.00 g (4.80 mmol) 5-Methyl-1,3-phenylendiacetat in 20.0 mL Acetonitril auf -30°C abgekühlt. 700 mg (5.27 mmol) Nitronium-Tetrafluorborat wurden zugegeben und für 1.5 h gerührt. Der Reaktionsansatz wurde auf Eiswasser gegossen und mit Diethylether extrahiert. Die organische Phase wurde abgetrennt, getrocknet und i.vac. eingeengt.

| | |
|---|---|
| Ausbeute: | 1.30 g (86 d.Th.) |
| ESI-MS: | m/z = 271 (M+H)⁺ |
| Rₜ(HPLC): | 1.50 min (Methode B) |

### Stufe 3: 5-Methyl-4-nitrobenzol-1,3-diol

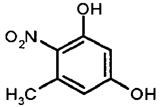

Eine Mischung aus 1.15 g (3.63 mmol) 5-Methyl-4-nitro-1,3-phenylen diacetat und 10.0 mL (10.0 mmol) 1 M wässriger Lithiumhydroxid-Lösung in 10.0 mL Methanol wurde über Nacht bei RT gerührt. Der Reaktionsansatz wurde mit 4M Salzsäure sauer gestellt und mit Dichlormethan extrahiert. Die organische Phase wurde abgetrennt, getrocknet und i.vac. eingeengt. Der Rückstand wurde chromatographisch aufgereinigt. Die Produkt enthaltenden Fraktionen wurden vereinigt und i.vac. eingeengt.

| | |
|---|---|
| Ausbeute: | 530 mg (86% d.Th.) |
| ESI-MS: | m/z = 168 (M+H)⁺ |
| Rₜ(HPLC): | 3.22 min (Methode C) |

### Stufe 4: 4-Amino-5-methylbenzol-1,3-diol

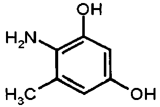

2.43 g (14.4 mmol) 5-Methyl-4-nitrobenzol-1,3-diol wurden in 200 mL Methanol vorgelegt und unter einer Wasserstoffatmosphäre und unter Zusatz von 0.20 g Pd/C (10%ig) bei 50°C und einem Wasserstoffdruck von 5 bar bis zur vollständigen Wasserstoffaufnahme hydriert. Der Katalysator wurde abfiltriert und das Filtrat i.vac. eingeengt.

| | |
|---|---|
| Ausbeute: | 2.00 g (quantitativ) |
| ESI-MS: | m/z = 140 (M+H)⁺ |
| Rₜ(HPLC): | 0.20 min (Methode B) |

### Stufe 5: 7-Hydroxy-5-methyl-2H-benzo[b][1,4]oxazin-3(4H)-on

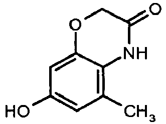

1.0 g (7.2 mmol) 4-Amino-5-methylbenzol-1,3-diol und 2.2 g (15.8 mmol) Kaliumcarbonat in 20.0 mL Acetonitril wurden im Eisbad gekühlt. 0.65 mL (7.9 mmol) Bromacetylchlorid wurden zugetropft und der Reaktionsansatz wurde 30 min bei RT und 1 h bei 70°C nachgerührt. Die Mischung wurde mit Wasser versetzt, mit wässriger Salzsäure angesäuert und mit Dichlormethan extrahiert. Die organische Phase wurde abgetrennt, getrocknet und i.vac. eingeengt.

| | |
|---|---|
| Ausbeute: | 620 mg (48% d.Th.) |
| ESI-MS: | m/z = 180 (M+H)⁺ |
| Rₜ(HPLC): | 0.83 min (Methode B) |

### Stufe 6: 7-(6-Chlorpyrimidin-4-yloxy)-5-methyl-2H-benzo[b][1,4]oxazin-3(4H)-on

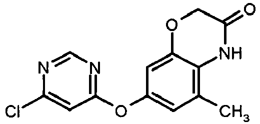

0.10 g (0.56 mmol) 7-Hydroxy-5-methyl-2H-benzo[b][1,4]oxazin-3(4H)-on und 0.10 g (0.73 mmol) Kaliumcarbonat wurden in 2.0 mL DMF vorgelegt. Nach 15 min wurden 86 mg (0.56 mmol) 4,6-Dichlorpyrimidin hinzugefügt und über das Wochenende bei RT gerührt. Der Reaktionsansatz wurde mit Wasser versetzt, die Substanz wurde mit Essigsäureethylester extrahiert und i.vac. eingeengt.

| | |
|---|---|
| Ausbeute: | 135 mg (79% d.Th.) |
| ESI-MS: | m/z = 292 (M+H)⁺ |
| Rₜ(HPLC): | 1.29 min (Methode B) |

### Intermediat 40

### 6-(6-Chlorpyrimidin-4-yloxy)-4-methyl-2-(tetrahydrofuran-3-yl)-1H-benzo[d]-imidazol

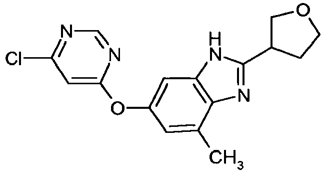

### Stufe 1: 4-Acetamido-3-methylphenylacetat

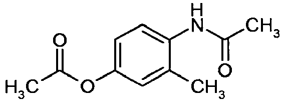

25.0 g (0.20 mol) 4-Amino-3-methylphenol, 42.5 mL (0.45 mol) Essigsäureanhydrid und 2.50 g Natriumacetat wurden in 25.0 mL Essigsäure für 1 h bei 125°C gerührt. Der Reaktionsansatz wurde mit Eiswasser versetzt und der ausgefallene Niederschlag wurde abgesaugt, nachgewaschen und getrocknet.

| | |
|---|---|
| Ausbeute: | 27.0 g (65% d.Th.) |
| ESI-MS: | m/z = 208 (M+H)⁺ |
| Rₜ(HPLC): | 0.95 min (Methode B) |

### Stufe 2: 4-Acetamido-3-methyl-5-nitrophenylacetat

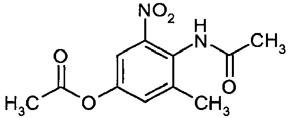

25.0 mL 90%ige Salpetersäure wurden vorgelegt und bei 10-15°C portionsweise 26.9 g (0.13 mol) 4-Acetamido-3-methylphenylacetat zugegeben. Der Reaktionsansatz wurde 1 h bei 18°C gerührt und anschließend mit Eiswasser versetzt und der ausgefallene Niederschlag wurde abgesaugt, nachgewaschen und getrocknet.

| | |
|---|---|
| Ausbeute: | 22.0 g (67% d.Th.) |
| ESI-MS: | m/z = 253 (M+H)⁺ |
| Rₜ(HPLC): | 1.05 min (Methode B) |

### Stufe 3: 4-Amino-3-methyl-5-nitrophenol

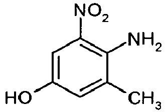

22.0 g (87.2 mmol) 4-Acetamido-3-methyl-5-nitrophenylacetat wurden in 100 mL halbkonzentrierter Salzsäure für 2 h gekocht. Der Reaktionsansatz wurde i.vac. eingeengt und getrocknet.

| | |
|---|---|
| Ausbeute: | 15.7 g (quantitativ) |
| ESI-MS: | m/z = 169 (M+H)⁺ |
| Rₜ(HPLC): | 0.96 min (Methode B) |

### Stufe 4: 3,4-Diamino-5-methylphenol

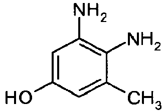

2.00 g (11.06 mmol) 4-Amino-3-methyl-5-nitrophenol wurden in 50 mL Methanol vorgelegt und unter einer Wasserstoffatmosphäre und unter Zusatz von 1.00 g Pd/C (10%ig) bei 50°C und einem Wasserstoffdruck von 5 bar bis zur vollständigen Wasserstoffaufnahme hydriert. Der Katalysator wurde abfiltriert und das Filtrat i.vac. eingeengt.

| | |
|---|---|
| Ausbeute: | 1.80 g (quantitativ) |
| ESI-MS: | m/z = 139 (M+H)⁺ |

### Stufe 5: 4-Methyl-2-(tetrahydro-3-yl)-1H-benzo[d]imidazol-6-ol

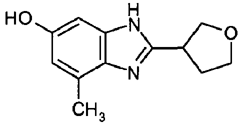

0.66 g (4.78 mmol) 3,4-Diamino-5-methylphenol wurden in wenig Methanol vorgelegt und 1.17 g (10.00 mmol) Tetrahydrofuran-3-carbonsäure zugegeben. Zunächst wurde Methanol abdestilliert und anschließend 2 h bei 140°C gerührt. Der Reaktionsansatz wurde in DMF/MeOH aufgenommen und chromatographisch gereinigt. Die Produkt enthaltenden Fraktionen wurden vereinigt und i.vac. bis zum wässrigen Rückstand eingeengt. Dieser wurde mit einer wässrigen 4M NaOH Lösung neutralisiert und mit Essigsäureethylester extrahiert. Die organische Phase wurde getrocknet und i.vac. eingeengt und getrocknet.

| | |
|---|---|
| Ausbeute: | 0.21 g (20% d.Th.) |
| ESI-MS: | m/z = 219 (M+H)⁺ |
| Rₜ(HPLC): | 0.73 min (Methode B) |

### Stufe 6: 6-(6-Chlorpyrimidin-4-yloxy)-4-methyl-2-(tetrahydrofuran-3-yl)-1H-benzo[d]-imidazol

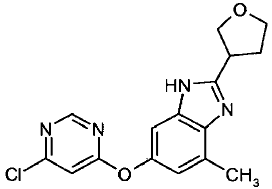

0.20 g (0.92 mmol)4-Methyl-2-(tetrahydro-3-yl)-1H-benzo[d]imidazol-6-ol und 0.15 g (1.00 mmol) 4,6-Dichlorpyrimidin wurden in 5.0 mL DMF vorgelegt. 0.42 mg (3.00 mmol) Kaliumcarbonat wurden hinzugefügt und über Nacht bei 50°C gerührt. Der Reaktionsansatz wurde mit Eiswasser versetzt. Die Substanz wurde mit Essigsäureethylester extrahiert und i.vac. eingeengt.

| | |
|---|---|
| Ausbeute: | 262 mg (20% d.Th.) |
| ESI-MS: | m/z = 331 (M+H)⁺ |
| Rₜ(HPLC): | 1.06 min (Methode B) |

### Intermediat 41

### 7-Chlor-5-(6-Chlorpyrimidin-4-yloxy)-2-(tetrahydrofuran-3-yl)-1H-benzo[d]imidazol

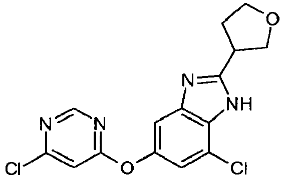

### Stufe 1: 4-Acetamido-3-chlorphenylacetat

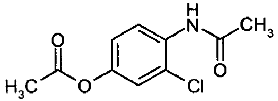

5.0 g (27.8 mmol) 4-Amino-3-chlorphenol Hydrochlorid und 2.6 g Natriumacetat wurden in 10.0 mL Essigsäure vorgelegt. Anschließend wurden 5.9 mL (62.09 mmol) Essigsäureanhydrid hinzugefügt und 2 h bei 125°C gerührt. Der Reaktionsansatz wurde mit Eiswasser versetzt, 1 h nachgerührt und der ausgefallene Niederschlag wurde abgesaugt, nachgewaschen und getrocknet.

| | |
|---|---|
| Ausbeute: | 5.20 g (82% d.Th.) |
| ESI-MS: | m/z = 228 (M+H)⁺ |
| Rₜ(HPLC): | 1.01 min (Methode B) |

### Stufe 2: N-(2-Chlor-4-hydroxy-6-nitrophenyl)acetamid

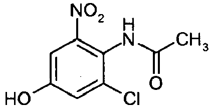

4.85 mL 90%ige Salpetersäure wurden vorgelegt und bei 10-15°C portionsweise 5.20 g (22.84 mmol) 4-Acetamido-3-chlorphenylacetat zugegeben. Der Reaktionsansatz wurde 2 h bei RT gerührt, anschließend mit Eiswasser versetzt und der ausgefallene Niederschlag wurde abgesaugt, nachgewaschen und getrocknet. Das Rohprodukt wurde chromatographisch gereinigt. Die Produkt enthaltenden Fraktionen wurden vereinigt und i.vac. eingeengt.

| | |
|---|---|
| Ausbeute: | 1.00 g (19% d.Th.) |
| ESI-MS: | m/z = 231 (M+H)⁺ |
| Rₜ(HPLC): | 1.03 min (Methode B) |

### Stufe 3: 4-Amino-3-chlor-5-nitrophenol

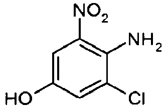

1.00 g (4.24 mmol) N-(2-Chlor-4-hydroxy-6-nitrophenyl)acetamid wurden in 10 mL halbkonzentrierter Salzsäure 2 h unter Rückfluss gekocht. Der Reaktionsansatz wurde i.vac. eingeengt und getrocknet.

| | |
|---|---|
| Ausbeute: | 0.80 g (98% d.Th.) |
| ESI-MS: | m/z = 189 (M+H)⁺ |
| Rₜ(HPLC): | 1.07 min (Methode B) |

### Stufe 4: N-(2-Chlor-4-hydroxy-6-nitrophenyl)tetrahydrofuran-3-carboxamid

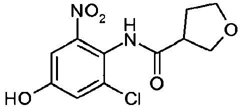

0.40 g (2.12 mmol) 4-Amino-3-chlor-5-nitrophenol wurden in 5 mL DCM vorgelegt. 0.44 mL DIPEA und 0.34 g Tetrahydrofuran-3-carbonylchlorid wurden hinzugegeben und bei RT 2 h gerührt. Es wurden weitere 0.34 g Tetrahydrofuran-3-carbonylchlorid zugegeben und über Nacht bei RT nachgerührt. Der Reaktionsansatz wurde mit DCM verdünnt und mit Wasser gewaschen. Die organische Phase wurde i.vac. eingeengt.
Ausbeute: 0.60 g (99% d.Th.)

### Stufe 5: N-(2-Amino-6-chlor-4-hydroxyphenyl)tetrahydrofuran-3-carboxamid

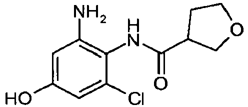

0.60 g (2.09 mmol) N-(2-Chlor-4-hydroxy-6-nitrophenyl)tetrahydrofuran-3-carboxamid wurden in 10 mL Essigsäureethylester vorgelegt und unter einer Wasserstoffatmosphäre und unter Zusatz von 0.20 g Raney/Nickel bei RT und einem Wasserstoffdruck von 3 bar bis zur vollständigen Wasserstoffaufnahme hydriert. Der Katalysator wurde abfiltriert und das Filtrat i.vac. eingeengt.

| | |
|---|---|
| Ausbeute: | 120 mg (22% d.Th.) |
| ESI-MS: | m/z = 257 (M+H)⁺ |
| Rₜ(HPLC): | 0.71 min (Methode B) |

### Stufe 6: 7-Chlor-2-(tetrahydrofuran-3-yl)-1H-benzo[d]imidazol-5-ol

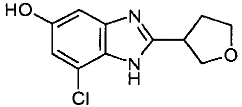

0.10 g (0.39 mmol) N-(2-Amino-6-chlor-4-hydroxyphenyl)tetrahydrofuran-3-carboxamid wurden in 20 mL Methanol vorgelegt, mit einer Spatelspitze p-Toluolsulfonsäure Monohydrat versetzt und 2 h gekocht. Der Reaktionsansatz wurde i.vac. eingeengt und chromatographisch gereinigt. Die Produkt enthaltenden Fraktionen wurden vereinigt und i.vac. eingeengt.

| | |
|---|---|
| Ausbeute: | 80 mg (86% d.Th.) |
| ESI-MS: | m/z = 239 (M+H)⁺ |
| Rₜ(HPLC): | 3.10 min (Methode M) |

### Stufe 7: 7-Chlor-5-(6-Chlorpyrimidin-4-yloxy)-2-(tetrahydrofuran-3-yl)-1H-benzo[d]-imidazol

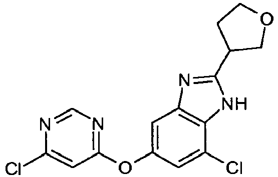

80 mg (0.34 mmol) 7-Chlor-2-(tetrahydrofuran-3-yl)-1 H-benzo[d]imidazol-5-ol und 51 mg (0.34 mmol) 4,6-Dichlorpyrimidin wurden in 2.0 mL DMF vorgelegt. 55 mg (0.40 mmol) Kaliumcarbonat wurden hinzugefügt und über Nacht bei RT gerührt. Der Reaktionsansatz wurde filtriert und chromatographisch gereinigt. Die Produkt enthaltenden Fraktionen wurden vereinigt und i.vac. eingeengt.

| | |
|---|---|
| Ausbeute: | 60 mg (51% d.Th.) |
| ESI-MS: | m/z = 351 (M+H)⁺ |
| Rₜ(HPLC): | 1.13 min (Methode B) |

### Intermediat 42

### 7-Chlor-5-(6-chlorpyrimidin-4-yloxy)-2-(tetrahydro-2H-pyran-4-yl)-1H-benzo[d]imidazol

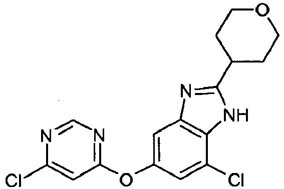

### Stufe 1: N-(2-Chlor-4-hydroxy-6-nitrophenyl)tetrahydro-2H-pyran-4-carboxamid

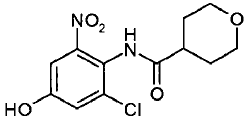

0.40 g (2.12 mmol) 4-Amino-3-chlor-5-nitrophenol wurden in 5 mL DCM vorgelegt. 0.44 mL (2.54 mmol) DIPEA und 0.26 mL (2.54 mmol) Tetrahydro-2H-pyran-4-carbonylchlorid wurden hinzugegeben und bei RT 2 h gerührt. Es wurden weitere 0.26 mL (2.54 mmol) Tetrahydro-2H-pyran-4-carbonylchlorid zugegeben und über Nacht bei RT nachgerührt. Der Reaktionsansatz wurde mit DCM verdünnt und mit Wasser gewaschen. Die organische Phase wurde i.vac. eingeengt.

| | |
|---|---|
| Ausbeute: | 0.60 g (94% d.Th.) |
| ESI-MS: | m/z = 299 (M-H)⁻ |
| Rₜ(HPLC): | 0.87 min (Methode B) |

### Stufe 2: N-(2-Amino-6-chlor-4-hydroxyphenyl)tetrahydro-2H-pyran-4-carboxamid

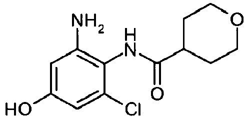

0.60 g (2.00 mmol) N-(2-Chlor-4-hydroxy-6-nitrophenyl)tetrahydro-2H-pyran-4-carboxamid wurden in 10 mL Essigsäureethylester vorgelegt und unter einer Wasserstoffatmosphäre und unter Zusatz von 0.20 g Raney/Nickel bei RT und einem Wasserstoffdruck von 3 bar bis zur vollständigen Wasserstoffaufnahme hydriert. Der Katalysator wurde abfiltriert und das Filtrat i.vac. eingeengt.

| | |
|---|---|
| Ausbeute: | 0.50 g (93% d.Th.) |
| ESI-MS: | m/z = 271 (M+H)⁺ |
| Rₜ(HPLC): | 1.06 min (Methode B) |

### Stufe 3: 7-Chlor-2-(tetrahydro-2H-pyran-4-yl)-1H-benzo[d]imidazol-5-ol

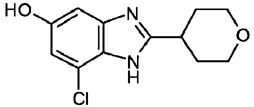

0.50 g (1.85 mmol) N-(2-Amino-6-chlor-4-hydroxyphenyl)tetrahydro-2H-pyran-4-carbox-amid wurden in 20 mL Methanol vorgelegt, mit einer Spatelspitze p-Toluolsulfonsäure Monohydrat versetzt und über Nacht gekocht. Der Reaktionsansatz wurde i.vac. eingeengt und chromatographisch gereinigt. Die Produkt enthaltenden Fraktionen wurden vereinigt und i.vac. eingeengt.

| | |
|---|---|
| Ausbeute: | 0.10 g (21% d.Th.) |
| ESI-MS: | m/z = 253 (M+H)⁺ |
| Rₜ(HPLC): | 0.75 min (Methode B) |

### Stufe 4: 7-Chlor-5-(6-chlorpyrimidin-4-yloxy)-2-(tetrahydro-2H-pyran-4-yl)-1H-benzo[d]imidazol

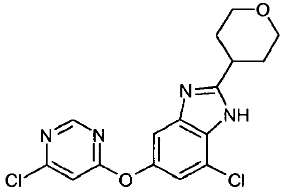

0.10 g (0.40 mmol) 7-Chlor-2-(tetrahydro-2H-pyran-4-yl)-1 H-benzo[d]imidazol-5-ol und 60 mg (0.40 mmol) 4,6-Dichlorpyrimidin wurden in 2.0 mL DMF vorgelegt. 61 mg (0.40 mmol) Kaliumcarbonat wurden hinzugegeben und über Nacht bei RT gerührt. Der Reaktionsansatz wurde filtriert und chromatographisch gereinigt. Die Produkt enthaltenden Fraktionen wurden vereinigt und i.vac. eingeengt.

| | |
|---|---|
| Ausbeute: | 80 mg (51% d.Th.) |
| ESI-MS: | m/z = 365 (M+H)⁺ |
| Rₜ(HPLC): | 1.12 min (Methode B) |

### Intermediat 43

### 6-(6-Chlorpyrimidin-4-yloxy)-4-methyl-2-(trifluormethyl)-1H-benzo[d]imidazol

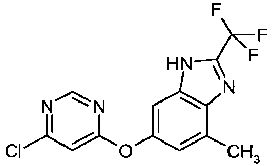

### Stufe 1: 7-Methyl-2-(trifluormethyl)-1H-benzo[d]imidazol-5-ol

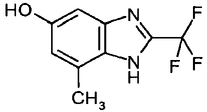

0.20 g (1.45 mmol) 3,4-Diamino-5-methylphenol wurden zusammen mit 1 mL (13.07 mmol) Trifluoressigsäure 1 h bei 100°C gerührt. Der Reaktionsansatz wurde abgekühlt und chromatographisch gereinigt. Die Produkt enthaltenden Fraktionen wurden vereinigt und i.vac. bis zum wässrigen Rückstand eingeengt. Dieser wurde mit einer wässrigen 1 M NaOH Lösung neutralisiert und mit Essigsäureethylester extrahiert. Die organische Phase wurde i.vac. eingeengt.

| | |
|---|---|
| Ausbeute: | 60 mg (19% d.Th.) |
| ESI-MS: | m/z = 217 (M+H)⁺ |
| Rₜ(HPLC): | 1.07 min (Methode B) |

### Stufe 2: 6-(6-Chlorpyrimidin-4-yloxy)-4-methyl-2-(trifluormethyl)-1H-benzo-[d]imidazol

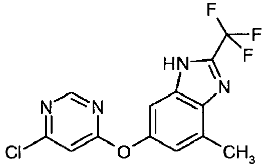

57 mg (0.26 mmol) 7-Methyl-2-(trifluormethyl)-1 H-benzo[d]imidazol-5-ol und 46 mg (0.30 mmol) 4,6-Dichlorpyrimidin wurden in 2.0 mL DMF vorgelegt. 0.11 g (0.78 mmol) Kaliumcarbonat wurden hinzugefügt und über Nacht bei 70°C gerührt. Der Reaktionsansatz wurde mit Eiswasser versetzt. Die Substanz wurde mit Essigsäureethylester extrahiert und i.vac. eingeengt. Das Rohprodukt wurde chromatographisch gereinigt. Die Produkt enthaltenden Fraktionen wurden vereinigt und i.vac. bis zum wässrigen Rückstand eingeengt. Dieser wurde mit einer wässrigen 4M NaOH Lösung neutralisiert und mit Essigsäureethylester extrahiert. Die organische Phase wurde i.vac. eingeengt.

| | |
|---|---|
| Ausbeute: | 24 mg (24% d.Th.) |
| ESI-MS: | m/z = 329 (M+H)⁺ |
| Rₜ(HPLC): | 1.41 min (Methode B) |

### Intermediat 44

### 6-(6-Chlorpyrimidin-4-yloxy)-4-methylbenzo[d]thiazol-2(3H)-on

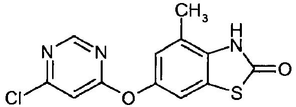

### Stufe 1: 6-Hydroxy-4-methylbenzo[d]thiazol-2(3H)-on

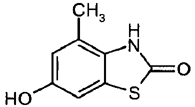

1.00 g (4.78 mmol) 6-Ethoxy-4-methylbenzo[d]thiazol-2(3H)-on und 7.00 g (60.57 mmol) Pyridinhydrochlorid wurden gut durchmischt und unter einer Stickstoff-Atmosphäre bei 180°C 15 h gerührt. Der Reaktionsansatz wurde auf Wasser gegossen und mit Essigsäureethylester extrahiert. Die organische Phase wurde mit Wasser gewaschen und i.vac. eingeengt. Der Rückstand wurde mit Diisopropylether verrührt und abgesaugt.

| | |
|---|---|
| Ausbeute: | 0.65 g (75% d.Th.) |
| ESI-MS: | m/z = 182 (M+H)⁺ |
| Rₜ(HPLC): | 0.89 min (Methode B) |

### Stufe 2: 6-(6-Chlorpyrimidin-4-yloxy)-4-methylbenzo[d]thiazol-2(3H)-on

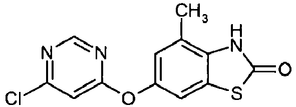

0.30 g (1.66 mmol) 6-Hydroxy-4-methylbenzo[d]thiazol-2(3H)-on und 0.25 g (1.68 mmol) 4,6-Dichlorpyrimidin wurden in 2.0 mL DMF vorgelegt. 0.25 g (1.81 mmol) Kaliumcarbonat wurden hinzugefügt und 2 h bei RT gerührt. Der Reaktionsansatz wurde mit Wasser versetzt und über Nacht gerührt. Der ausgefallene Niederschlag wurde abgesaugt, nachgewaschen und getrocknet.

| | |
|---|---|
| Ausbeute: | 0.37 g (75% d.Th.) |
| ESI-MS: | m/z = 294 (M+H)⁺ |
| Rₜ(HPLC): | 1.32 min (Methode B) |

### Intermediat 45

### 5-(6-Chlorpyrimidin-4-yloxy)-7-methyl-2-(tetrahydro-2H-pyran-4-yl)-1H-benzo[d]imidazol

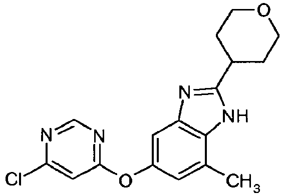

### Stufe 1: 4-(Benzyloxy)-2-methyl-6-nitroanilin

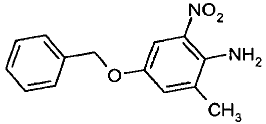

0.84 g (5.00 mmol) 4-Amino-3-methyl-5-nitrophenol wurde in 10 mL DMF vorgelegt, mit 0.78 g (5.50 mmol) Kaliumcarbonat versetzt und 10 min bei RT gerührt. Anschließend wurden 0.65 mL (5.50 mmol) Benzylbromid zugegeben und weitere 30 min bei RT und 1 h bei 60°C gerührt. Es wurden nochmals 0.33 mL Benzylbromid zugegben und bei 60°C 1 h gerührt. Der Reaktionsansatz wurde auf Eiswasser gegossen, mit Essigsäureethylester extrahiert und i.vac. eingeengt. Der Rückstand wurde chromatographisch gereinigt. Die Produkt enthaltenden Fraktionen wurden vereinigt und i.vac. bis zum wässrigen Rückstand eingeengt. Dieser wurde mit einer wässrigen 1 M NaOH Lösung neutralisiert und mit Essigsäureethylester extrahiert. Die organische Phase wurde i.vac. eingeengt.

| | |
|---|---|
| Ausbeute: | 0.23 g (18% d.Th.) |
| ESI-MS: | m/z = 259 (M+H)⁺ |
| Rₜ(HPLC): | 1.60 min (Methode B) |

### Stufe 2: N-(4-(Benzyloxy)-2-methyl-6-nitrophenyntetrahydro-2H-pyran-4-carbox-amid

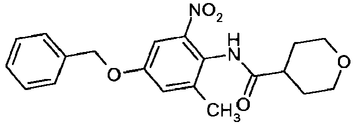

0.22 g (0.85 mmol) 4-(Benzyloxy)-2-methyl-6-nitroanilin wurden in 5 mL DCM vorgelegt. 0.17 mL (1.00 mmol) DIPEA und 0.10 mL (1.00 mmol) Tetrahydro-2H-pyran-4-carbonyl-chlorid wurden hinzugegeben und bei RT 2 h gerührt. Es wurden weitere 0.10 mL (1.00 mmol) Tetrahydro-2H-pyran-4-carbonylchlorid zugegeben und über Nacht bei RT nachgerührt. Der Reaktionsansatz wurde mit DCM verdünnt und mit Wasser gewaschen. Die organische Phase wurde i.vac. eingeengt.

| | |
|---|---|
| Ausbeute: | 0.34 g (quantitativ) |
| ESI-MS: | m/z = 371 (M+H)⁺ |
| Rₜ(HPLC): | 1.40 min (Methode B) |

### Stufe 3: 7-Methyl-2-(tetrahydro-2H-pyran-4-yl)-1H-benzo[d]imidazol-5-ol

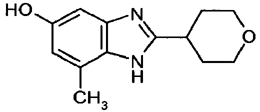

0.33 g (0.84 mmol) N-(4-(Benzyloxy)-2-methyl-6-nitrophenyl)tetrahydro-2H-pyran-4-carboxamid wurden in 10 mL Essigsäure vorgelegt und unter einer Wasserstoffatmosphäre und unter Zusatz von 0.10 g Palladium/Kohle (10%) bei 50°C und einem Wasserstoffdruck von 5 bar bis zur vollständigen Wasserstoffaufnahme hydriert. Der Katalysator wurde abfiltriert und das Filtrat i.vac. eingeengt.

| | |
|---|---|
| Ausbeute: | 0.35 g (quantitativ) |
| ESI-MS: | m/z = 233 (M+H)⁺ |
| Rₜ(HPLC): | 0.64 min (Methode B) |

### Stufe 4: 5-(6-Chlorpyrimidin-4-yloxy)-7-methyl-2-(tetrahydro-2H-pyran-4-yl)-1H-benzo[d]imidazol

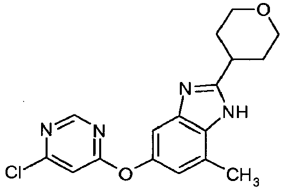

0.35 g (0.84 mmol) 7-Methyl-2-(tetrahydro-2H-pyran-4-yl)-1H-benzo[d]imidazol-5-ol und 0.14 mg (0.90 mmol) 4,6-Dichlorpyrimidin wurden in 5.0 mL DMF vorgelegt. 0.35 g (2.52 mmol) Kaliumcarbonat wurden hinzugegeben und über Nacht bei 60°C gerührt. Der Reaktionsansatz wurde auf Eiswasser gegossen, mit Essigsäureethylester extrahiert und i.vac. eingeengt. Der Rückstand wurde chromatographisch gereinigt. Die Produkt enthaltenden Fraktionen wurden vereinigt und i.vac. bis zum wässrigen Rückstand eingeengt. Dieser wurde mit einer wässrigen NaHCO₃-Lösung neutralisiert und mit

Essigsäureethylester extrahiert. Die organische Phase wurde getrocknet und i.vac. eingeengt.

| | |
|---|---|
| Ausbeute: | 42 mg (14% d.Th.) |
| ESI-MS: | m/z = 345 (M+H)⁺ |
| Rₜ(HPLC): | 0.96 min (Methode B) |

### Intermediat 46

### 6-(6-Chlorpyrimidin-4-yloxy)-2-(2-methoxymethyl)-4-methyl-1H-benzo[d]imidazol

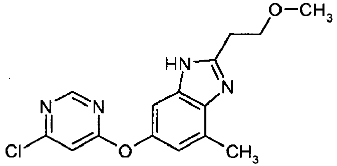

### Stufe1: 2-(2-Methoxyethyl)-4-methyl-1H-benzo[d]imidazol-6-ol

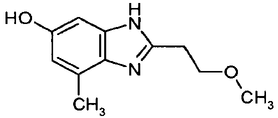

0.65 g (4.70 mmol) 3,4-Diamino-5-methylphenol wurden in wenig Methanol vorgelegt und 0.94 mL (10.00 mmol) 3-Methoxypropionsäure zugegeben. Zunächst wurde das Methanol abdestilliert und anschließend 2 h bei 140°C gerührt. Der Reaktionsansatz wurde in DMF/MeOH aufgenommen und chromatographisch gereinigt. Die Produkt enthaltenden Fraktionen wurden vereinigt und i.vac. bis zum wässrigen Rückstand eingeengt. Dieser wurde mit einer wässrigen 4M NaOH-Lösung neutralisiert und mit Essigsäureethylester extrahiert. Die organische Phase wurde getrocknet und i.vac. eingeengt und getrocknet.

| | |
|---|---|
| Ausbeute: | 0.16 g (17% d.Th.) |
| ESI-MS: | m/z = 207 (M+H)⁺ |
| Rₜ(HPLC): | 0.78 min (Methode B) |

### Stufe 2: 6-(6-Chlorpyrimidin-4-yloxy)-2-(2-methoxymethyl)-4-methyl-1H-benzo-[d]imidazol

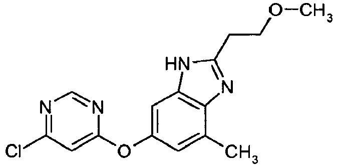

0.15 g (0.74 mmol) 2-(2-Methoxyethyl)-4-methyl-1H-benzo[d]imidazol-6-ol und 0.12 g (0.80 mmol) 4,6-Dichlorpyrimidin wurden in 5.0 mL DMF vorgelegt. 0.33 g (2.40 mmol) Kaliumcarbonat wurden hinzugefügt und über Nacht bei 50°C gerührt. Der Reaktionsansatz wurde mit Eiswasser versetzt, die Substanz wurde mit Essigsäureethylester extrahiert und i.vac. eingeengt. Der Rückstand wurde chromatographisch gereinigt. Die Produkt enthaltenden Fraktionen wurden vereinigt und i.vac. bis zum wässrigen Rückstand eingeengt. Dieser wurde mit einer wässrigen 1 M NaOH-Lösung neutralisiert und mit Essigsäureethylester extrahiert. Die organische Phase wurde getrocknet und i.vac. eingeengt.

| | |
|---|---|
| Ausbeute: | 33 mg (13% d.Th.) |
| ESI-MS: | m/z = 319 (M+H)⁺ |
| Rₜ(HPLC): | 2.71 min (Methode B) |

### Intermediat 47

### 6-(6-Chlorpyrimidin-4-yloxy)-2-methoxy-4-methyl-1H-benzo[d]imidazol

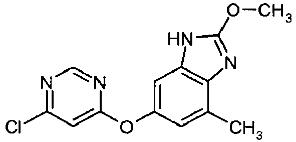

### Stufe 1: 2-Methoxy-4-methyl-1H-benzo[d]imidazol-6-ol

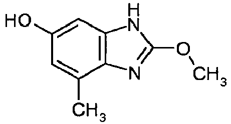

0.60 g (4.34 mmol) 3,4-Diamino-5-methylphenol wurden in 2 mL Essigsäure vorgelegt. 4.0 mL (0.03 mol) Orthokohlensäure-tetramethylester wurden hinzugegeben und bei RT 4h gerührt. Der Reaktionsansatz wurde chromatographisch gereinigt. Die Produkt enthaltenden Fraktionen wurden vereinigt und i.vac. bis zum wässrigen Rückstand eingeengt. Dieser wurde mit einer wässrigen 1 M NaOH-Lösung neutralisiert und mit Essigsäureethylester extrahiert. Die organische Phase wurde getrocknet und i.vac. eingeengt.

| | |
|---|---|
| Ausbeute: | 80 mg (10% d.Th.) |
| ESI-MS: | m/z = 179 (M+H)⁺ |
| Rₜ(HPLC): | 0.61 min (Methode B) |

### Stufe 2: 6-(6-Chlorpyrimidin-4-yloxy)-2-methoxy-4-methyl-1H-benzo[d]imidazol

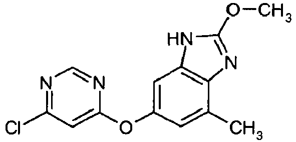

74 mg (0.42 mmol) 2-Methoxy-4-methyl-1H-benzo[d]imidazol-6-ol und 68 mg (0.44 mmol) 4,6-Dichlorpyrimidin wurden in 2.0 mL DMF vorgelegt. 0.12 g (0.88 mmol) Kaliumcarbonat wurden hinzugefügt und 4 h bei 50°C gerührt. Der Reaktionsansatz wurde mit Eiswasser versetzt, die Substanz wurde mit Essigsäureethylester extrahiert und i.vac. eingeengt.

| | |
|---|---|
| Ausbeute: | 120 mg (94% d.Th.) |
| ESI-MS: | m/z = 291 (M+H)⁺ |
| Rₜ(HPLC): | 1.16 min (Methode B) |

### Intermediat 48

### 6-(6-Chlor-2-trifluormethyl-pyrimidin-4-yloxy)-4-methyl-3H-benzooxazol-2-on

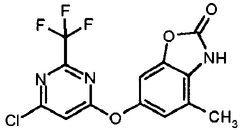

0.40 g (1.86 mmol) 4,6-Dichlor-2-trifluormethylpyrimidin, 0.31 g (1.88 mmol) 6-Hydroxy-2-benzoxazolinon und 0.28.g (2.03 mmol) Kaliumcarbonat in 2.0 mL DMF wurden 2 h bei RT gerührt. Der Reaktionsansatz wurde mit Wasser versetzt und 1 h bei RT gerührt. Der ausgefallene Niederschlag wurde abgesaugt, nachgewaschen und getrocknet.

| | |
|---|---|
| Ausbeute: | 0.42 g (65% d.Th.) |
| ESI-MS: | m/z = 346 (M+H)⁺ |
| Rₜ(HPLC): | 1.53 min (Methode B) |

### Intermediat 49

### 6-Chlor-4-(4-methyl-2-oxo-2,3-dihydro-benzooxazol-6-yloxy)-nicotinonitril

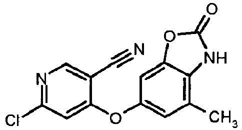

0.12 g (0.69 mmol) 4,6-Dichlor-nicotinonitril, 0.15 g (0.91 mmol) 6-Hydroxy-2-benzoxazolinon und 90 mg (0.80 mmol) Kalium-tert.-butylat in 0.8 mL NMP wurden 2 h bei 70°C gerührt. Der Reaktionsansatz wurde mit Eiswasser versetzt. Der ausgefallene Niederschlag wurde abgesaugt und getrocknet.

| | |
|---|---|
| Ausbeute: | 0.12 g (52% d.Th.) |
| ESI-MS: | m/z = 302 (M+H)⁺ |
| Rₜ(HPLC): | 1.31 min (Methode B) |

### Intermediat 50

### 6-(6-Chlor-pyrimidin-4-yloxy)-2-methyl-4-trifluoromethyl-1H-benzimidazol

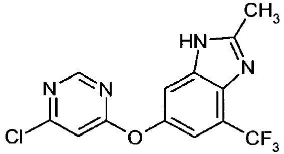

### Stufe 1: N-(4-Fluor-2-trifluormethyl-phenyl)-acetamid

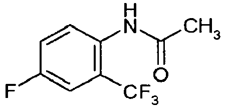

10.0 g (0.56 mol) 4-Fluor-2-trifluormethyl-phenylamin wurden über Nacht bei RT in 40 mL Essigsäureanhydrid gerührt. Der Reaktionsansatz wurde auf 300 g Eiswasser gegossen. Der ausgefallene Niederschlag wurde abgesaugt, mit 100 mL Wasser gewaschen und i.vac. getrocknet.

| | |
|---|---|
| Ausbeute: | 8.80 g (71% d.Th.) |
| ESI-MS: | m/z = 222 (M+H)⁺ |
| Rₜ(HPLC): | 3.0 min (Methode C) |

### Stufe 2: N-(4-Fluor-2-nitro-6-trifluormethyl-phenyl)-acetamid

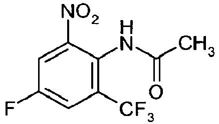

8.80 g (39.7 mmol) *N*-(4-Fluor-2-trifluormethyl-phenyl)-acetamid wurden unter Eiskühlung zu 60 mL 97%iger Schwefelsäure gegeben. Zu diesem Gemisch wurden bei 0°C langsam und unter ständigem Rühren 6.60 mL (95.3 mmol) Salpetersäure zugetropft. Nach beendeter Zugabe wurde 30 min bei 0°C gerührt. Anschließend wurde die Kühlung entfernt und das Reaktionsgemisch bis zur vollständigern Umsetzung weitere 1.5 h gerührt. Der Reaktionsansatz wurde vorsichtig auf 500 g Eis gegossen und 30 min gerührt. Der ausgefallene Niederschlag wurde abfiltriert, mit 150 mL Wasser gewaschen und i.vac. getrocknet.

| | |
|---|---|
| Ausbeute: | 8.60 g (81% d.Th.) |
| ESI-MS: | m/z = 267 (M+H)⁺ |
| Rₜ(HPLC): | 3.16 min (Methode C) |

### Stufe 3: N-(4-Methoxy-2-nitro-6-trifluormethyl-phenyl)-acetamid

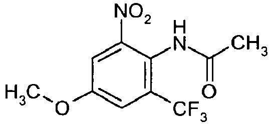

Ein gut gerührtes Gemisch aus 3.00 g (11.2 mmol) *N*-(4-Fluor-2-nitro-6-trifluormethylphenyl)-acetamid und 6.00 g (18.4 mmol) Cäsiumcarbonat in 90 mL Methanol wurde 10 min bei 100°C in der Mikrowelle erhitzt. Nach dem Abkühlen wurde das Lösungsmittel i.vac. entfernt und der verbliebene Rückstand mit 100 mL Wasser versetzt. Die Wasserphase wurde mit 10%iger Zitronensäure angesäuert und das Produkt mit 100 mL Ethylacetat extrahiert. Die organische Phase wurde getrocknet und i.vac. eingeengt. Der erhaltene Rückstand wurde mittels Flash-Chromatographie gereinigt. Die Produkt enthaltenden Fraktionen wurden vereinigt und i.vac. eingeengt.

| | |
|---|---|
| Ausbeute: | 550 mg (17% d.Th.) |
| ESI-MS: | m/z = 279 (M+H)⁺ |
| Rₜ(HPLC): | 1.18 min (Methode B) |

### Stufe 4: 6-Methoxy-2-methyl-4-trifluormethyl-1H-benzimidazol

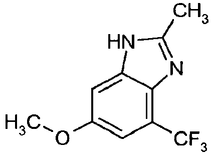

Ein Gemisch aus 0.55 g (1.98 mmol) *N*-(4-Methoxy-2-nitro-6-trifluoromethyl-phenyl)-acetamid und 100 mg Pd/C in 30 mL Essigsäure wurde 5 h unter einer Wasserstoff-Atmosphäre bei 80°C bis zur vollständigen Wasserstoffaufnahme hydriert. Der Katalysator wurde abfiltriert und das Filtrat i.vac. eingeengt.

| | |
|---|---|
| Ausbeute: | 450 mg (99% d.Th.) |
| ESI-MS: | m/z = 231 (M+H)⁺ |
| Rₜ(HPLC): | 2.6 min (Methode C) |

### Stufe 5: 2-Methyl-7-trifluormethyl-3H-benzimidazol-5-ol

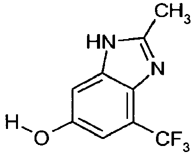

Ein gut gerührtes Gemisch aus 450 mg (1.95 mmol) 6-Methoxy-2-methyl-4-trifluormethyl-1H-benzimidazol und 2.50 g (21.6 mmol) Pyridinhydrochlorid wurde 15 min auf 200°C erhitzt. Nach dem Abkühlen auf RT wurde der Reaktionsansatz mit 3.0 mL DMF versetzt und mittels präparativer HPLC gereinigt. Die Produkt enthaltenden Fraktionen wurden vereinigt und i.vac. eingeengt.

| | |
|---|---|
| Ausbeute: | 330 mg (78% d.Th.) |
| ESI-MS: | m/z = 217 (M+H)⁺ |
| Rₜ(HPLC): | 2.0 min (Methode C) |

### Stufe 6: 6-(6-Chlor-pyrimidin-4-yloxy)-2-methyl-4-trifluoromethyl-1H-benzimidazol

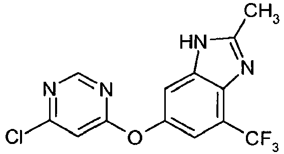

Zu 245 mg (1.60 mmol) 4,6-Dichlorpyrimidin und 330 mg (1.52 mmol) 2-Methyl-7-trifluormethyl-3H-benzimidazol-5-ol in 5.0 mL DMF wurden bei RT 221 mg (1.60 mmol) Kaliumcarbonat gegeben und das Gemisch über Nacht bei RT gerührt. Der Reaktionsansatz wurde auf Wasser gegossen. Der ausgefallene Niederschlag wurde abfiltriert, mit Wasser gewaschen und getrocknet.

| | |
|---|---|
| Ausbeute: | 500 mg (95% d.Th.) |
| ESI-MS: | m/z = 329/331 (Cl) (M+H)⁺ |
| Rₜ(HPLC): | 3.18 min (Methode C) |

### Intermediat 51

### 7-Methoxy-3-(4'-nitro-1'-oxy-3,4,5,6-tetrahydro-2H-[1,2']bipyridinyl-4-yl)-1,3,4,5-tetrahydro-benzo[d][1,3]diazepin-2-on

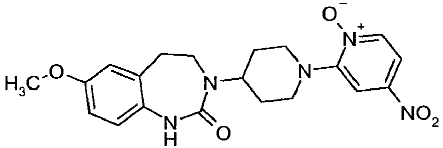

Ein Gemisch aus 160 mg (0.581 mmol) 7-Methoxy-3-piperidin-4-yl-1,3,4,5-tetrahydro-1,3-benzodiazepin-2-on, 100 mg (0.573 mmol) 2-Chlor-4-nitro-pyridin-N-oxid und 100 mg (0.724 mmol) Kaliumcarbonat in 2.0 mL DMF wurde 2 h bis zur vollständigen Umsetzung bei 60°C gerührt. Nach dem Abkühlen auf RT wurde der Reaktionsansatz auf Wasser gegossen. Der ausgefallene Niederschlag wurde abfiltriert, mit Wasser gewaschen und i.vac. getrocknet.

| | |
|---|---|
| Ausbeute: | 180 mg (75% d.Th.) |
| ESI-MS: | m/z = 414 (M+H)⁺ |

### Herstellung der Endverbindungen:

### Beispiel 1

### 7-Methoxy-3-{1-[6-(4-methyl-2-oxo-2,3-dihydro-benzoxazol-6-yloxy)-pyrimidin-4-yl]-piperidin-4-yl}-1,3,4,5-tetrahydro-benzo[d][1,3]diazepin-2-on

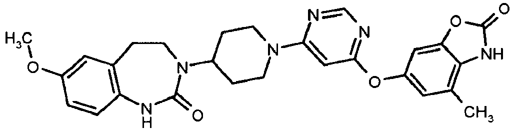

64 mg (0.23 mmol) 7-Methoxy-3-piperidin-4-yl-1,3,4,5-tetrahydro-1,3-benzodiazepin-2-on, 64 mg (0.23 mmol) 6-(6-Chlor-pyrimidin-4-yloxy)-4-methyl-3H-benzoxazol-2-on und 0.10 mL (0.58 mmol) DIPEA wurden in 1.8 mL DMF zusammen gegeben und 1 h bei RT gerührt. Anschließend wurden 40 mg (0.15 mmol) 7-Methoxy-3-piperidin-4-yl-1,3,4,5-tetrahydro-1,3-benzodiazepin-2-on zugefügt und über Nacht gerührt. Das Reaktionsgemisch wurde mittels präparativer HPLC-MS gereinigt. Die Produkt enthaltenden Fraktionen wurden vereinigt, das Acetonitril eingeengt, der Niederschlag abgesaugt und i.vac. getrocknet.

| | |
|---|---|
| Ausbeute: | 30 mg (25% d.Th.) |
| ESI-MS: | m/z = 517 (M+H)⁺ |
| Rₜ(HPLC): | 1.62 min (Methode B) |

### Beispiel 2

### 1-{1-[6-(3,4-Dimethyl-2-oxo-2,3-dihydro-benzoxazol-6-yloxy)pyrimidin-4-yl]-piperidin-4-yl}-1,3-dihydro-imidazo[4,5-b]pyridin-2-on

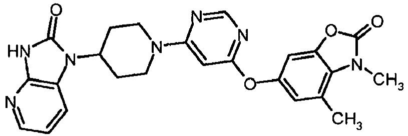

75 mg (0.34 mmol) 1-Piperidin-4-yl-1,3-dihydroimidazo[4,5-*b*]pyridin-2-on-dihydrochlorid, 100 mg (0.34 mmol) 6-(6-Chlor-pyrimidin-4-yloxy)-3,4-dimethyl-3H-benzoxazol-2-on und 0.15 mL (0.86 mmol) DIPEA wurden in 1.8 mL DMF zusammen gegeben und über Nacht bei RT gerührt. Das Reaktionsgemisch wurde mittels präparativer HPLC-MS gereinigt. Die Produkt enthaltenden Fraktionen wurden vereinigt, das Acetonitril eingeengt und der Niederschlag abgesaugt und i.vac. getrocknet.

| | |
|---|---|
| Ausbeute: | 82 mg (51% d.Th.) |
| ESI-MS: | m/z = 474(M+H)⁺ |
| Rₜ(HPLC): | 1.36 min (Methode B) |

### Beispiel 3

### 3-{1-[6-(3,4-Dimethyl-2-oxo-2,3-dihydro-benzoxazol-6-yloxy)-pyrimidin-4-yl]-piperidin-4-yl}-7-methoxy-1,3,4,5-tetrahydro-benzo[d][1,3]diazepin-2-on

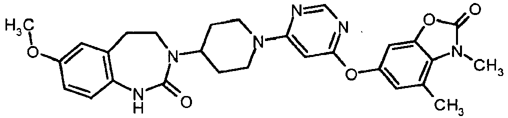

95 mg (0.35 mmol) 7-Methoxy-3-piperidin-4-yl-1,3,4,5-tetrahydro-1,3-benzodiazepin-2-on, 0.10 g (0.34 mmol) 6-(6-Chlor-pyrimidin-4-yloxy)-3,4-dimethyl-3H-benzoxazol-2-on und 0.15 mL (0.86 mmol) DIPEA wurden in 1.8 mL DMF zusammen gegeben und über Nacht bei RT gerührt. Das Reaktionsgemisch wurde mittels präparativer HPLC-MS gereinigt. Die Produkt enthaltenden Fraktionen wurden vereinigt, das Acetonitril eingeengt, der Niederschlag abgesaugt und i.vac. getrocknet.

| | |
|---|---|
| Ausbeute: | 81 mg (45% d.Th.) |
| ESI-MS: | m/z = 531 (M+H)⁺ |
| Rₜ(HPLC): | 1.52 min (Methode B) |

### Beispiel 4

### 1-{1-[6-(3,4-Dimethyl-phenoxy)-pyrimidin-4-yl]-piperidin-4-yl}-1,3-dihydro-imidazo[4,5-b]-pyridin-2-on

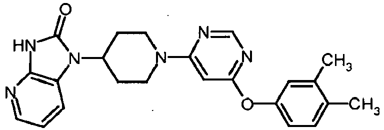

291 mg (1.00 mmol) 1-Piperidin-4-yl-1,3-dihydroimidazo[4,5-*b*]pyridin-2-on-dihydrochlorid, 276 mg (1.00 mmol) 4-Chlor-6-(3,4-dimethyl-phenoxy)-pyrimidin und 0.697 mL (4.00 mmol) DIPEA wurden in 3.0 mL DMF zusammen gegeben und 4 h bei 150°C gerührt. Das Reaktionsgemisch wurde mittels präparativer HPLC-MS gereinigt. Die Produkt enthaltenden Fraktionen wurden vereinigt, das Acetonitril eingeengt und der Rückstand mit 1N wässriger Natronlauge neutralisiert. Der ausgefallene Niederschlag wurde abgesaugt, mit Wasser nachgewaschen und i.vac. getrocknet.

| | |
|---|---|
| Ausbeute: | 285 mg (68% d.Th.) |
| ESI-MS: | m/z = 417 (M+H)⁺ |
| Rₜ(HPLC): | 1.47 min (Methode B) |

### Beispiel 5

### 1-{1-[6-(4-Methyl-2-oxo-2,3-dihydro-benzoxazol-6-yloxy)-pyrimidin-4-yl]-piperidin-4-yl)-1,3-dihydro-imidazo[4,5-b]pyridin-2-on

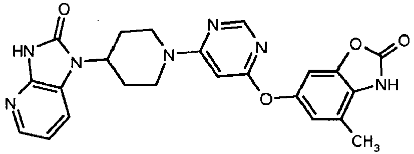

40 mg (0.18 mmol) 1-Piperidin-4-yl-1,3-dihydroimidazo[4,5-*b*]pyridin-2-on, 50 mg (0.18 mmol) 6-(6-Chlor-pyrimidin-4-yloxy)-4-methyl-3H-benzoxazol-2-on und 0.10 mL (0.58 mmol) DIPEA wurden in 1.8 mL DMF zusammen gegeben und 1 h bei RT gerührt. Anschließend wurden nochmals 40 mg (0.18 mmol) 1-Piperidin-4-yl-1,3-dihydroimidazo-[4,5-*b*]pyridin-2-on zugefügt und über Nacht gerührt. Das Reaktionsgemisch wurde mittels präparativer HPLC-MS gereinigt. Die Produkt enthaltenden Fraktionen wurden vereinigt, das Acetonitril eingeengt, der Niederschlag abgesaugt und i.vac. getrocknet.

| | |
|---|---|
| Ausbeute: | 45 mg (25% d.Th.) |
| ESI-MS: | m/z = 460 (M+H)⁺ |
| Rₜ(HPLC): | 1.22 min (Methode B) |

### Beispiel 6

### 1-{1-[6-(4-Methyl-2-oxo-2,3-dihydro-benzoxazol-6-ylamino)-pyrimidin-4-yl]-piperidin-4-yl}-1,3-dihydro-imidazo[4,5-b]pyridin-2-on

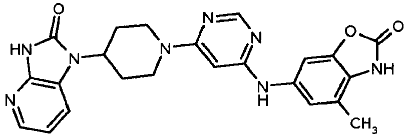

276 mg (1.26 mmol) 1-Piperidin-4-yl-1,3-dihydroimidazo[4,5-*b*]pyridin-2-on, 350 mg (0.987 mmol) 6-(6-Chlor-pyrimidin-4-ylamino)-4-methyl-3H-benzoxazol-2-on und 350 mg (2.53 mmol) Kaliumcarbonat in 2 mL NMP wurden 2 h bei 130°C gerührt. Nach dem Abkühlen auf RT wurde das Reaktionsgemisch mit DMF verdünnt und mittels präparativer HPLC gereinigt. Die Produkt enthaltenden Fraktionen wurden vereinigt und gefriergetrocknet.

| | |
|---|---|
| Ausbeute: | 50 mg (11% d.Th.) |
| ESI-MS: | m/z = 459 (M+H)⁺ |
| Rₜ(HPLC): | 0.96 min (Methode B) |

### Beispiel 7

### 7-Methoxy-3-{1-[6-(4-methyl-2-oxo-2,3-dihydro-benzoxazol-6-ylamino)-pyrimidin-4-yl]-piperidin-4-yl}-1,3,4,5-tetrahydro-benzo[d][1,3]diazepin-2-on

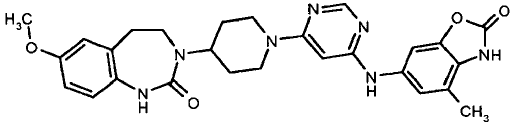

80 mg (0.29 mmol) 7-Methoxy-3-piperidin-4-yl-1,3,4,5-tetrahydro-1,3-benzodiazepin-2-on, 90 mg (0.26 mmol) 6-(6-Chlor-pyrimidin-4-ylamino)-4-methyl-3H-benzoxazol-2-on und 0.15 mL (0.87 mmol) DIPEA in 1.5 mL DMF wurden bei 150°C gerührt. Nach der Umsetzung der Reaktionspartner wurde der Reaktionsansatz mittels präparativer HPLC gereinigt. Die Produkt enthaltenden Fraktionen wurden vereinigt und eingeengt. Der Rückstand wurde getrocknet.

| | |
|---|---|
| Ausbeute: | 13 mg (25% d.Th.) |
| ESI-MS: | m/z = 514 (M-H)⁻ |
| Rₜ(HPLC): | 1.15 min (Methode B) |

### Beispiel 8

### 3-{1-[6-(2,7-Dimethyl-1H-benzimidazol-5-ylamino)-pyrimidin-4-yl]-piperidin-4-y}-7-methoxy-1,3,4,5-tetrahydro-benzo[d][1,3]diazepin-2-on

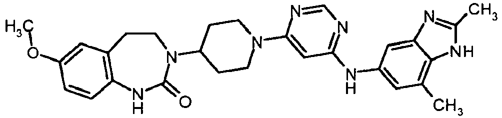

265 mg (0.962 mmol) 7-Methoxy-3-piperidin-4-yl-1,3,4,5-tetrahydro-1,3-benzodiazepin-2-on, 250 mg (0.913 mmol) (6-Chlor-pyrimidin-4-yl)-(2,7-dimethyl-1 H-benzimidazol-5-yl)-amin und 1.00 mL (5.81 mmol) DIPEA wurden in 1.5 mL DMF unter Rückfluss gekocht. Nach beendeter Umsetzung wurde der Reaktionsansatz mit etwas DMF verdünnt und mikrofiltriert. Das Filtrat wurde mittels präparativer HPLC gereinigt. Die Produkt enthaltenden Fraktionen wurden vereinigt und gefriergetrocknet.

| | |
|---|---|
| Ausbeute: | 170 mg (36 % d. Th.) |
| ESI-MS: | m/z = 513 (M+H)⁺ |
| Rₜ(HPLC): | 0.97 min (Methode B) |

### Beispiel 9

### 3-{1-[6-(2,7-Dimethyl-1H-benzimidazol-5-ylamino)-pyrimidin-4-yl]-piperidin-4-yl}-1,3-dihydro-imidazo[4,5-c]chinolin-2-on

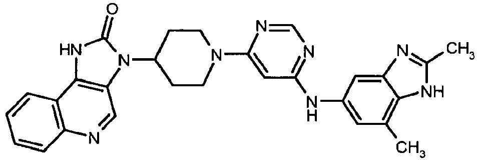

150 mg (0.548 mmol) (6-Chlor-pyrimidin-4-yl)-(2,7-dimethyl-1 H-benzimidazol-5-yl)-amin, 150 mg (0.559 mmol) 3-Piperidin-4-yl-1,3-dihydro-imidazo[4,5-c]chinolin-2-on und 1.00 mL (5.81 mmol) DIPEA wurden in 1.0 mL DMF unter Rückfluss gekocht. Nach beendeter Umsetzung wurde der Reaktionsansatz mit MeOH verdünnt und mittels präparativer HPLC gereinigt. Die Produkt enthaltenden Fraktionen wurden vereinigt und gefriergetrocknet.

| | |
|---|---|
| Ausbeute: | 20 mg (7% d.Th.) |
| ESI-MS: | m/z = 506 (M+H)⁺ |
| Rₜ(HPLC): | 0.74 min (Methode B) |

### Beispiel 10

### 3-{1-[6-(3,4-Dimethyl-2-oxo-2,3-dihydro-benzoxazol-6-ylamino)-pyrimidin-4-yl]-piperidin-4-yl}-7-methoxy-1,3,4,5-tetrahydro-benzo[d][1,3]diazepin-2-on

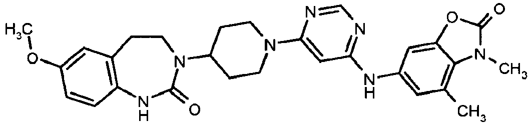

90 mg (0.33 mmol) 7-Methoxy-3-piperidin-4-yl-1,3,4,5-tetrahydro-1,3-benzodiazepin-2-on, 90 mg (0.31 mmol) 6-(6-Chlor-pyrimidin-4-ylamino)-3,4-dimethyl-3H-benzoxazol-2-on und 0.15 mL (0.87 mmol) DIPEA wurden in 1.5 mL DMF zusammen gegeben und bei 150°C gerührt. Nach beendeter Umsetzung wurde der Reaktionsansatz mittels präparativer HPLC gereinigt. Die Produkt enthaltenden Fraktionen wurden vereinigt und getrocknet.

| | |
|---|---|
| Ausbeute: | 60 mg (37% d.Th.) |
| ESI-MS: | m/z = 530 (M+H)⁺ |
| Rₜ(HPLC): | 3.30 min (Methode C) |

### Beispiel 11

### 7-Methoxy-3-{1-[6-(7-methyl-1H-indazol-5-ylamino)-pyrimidin-4-yl]-piperidin-4-yl}-1,3,4,5-tetrahydro-benzo[d][1,3]diazepin-2-on

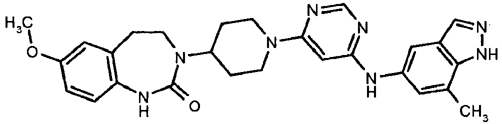

210 mg (0.763 mmol) 7-Methoxy-3-piperidin-4-yl-1,3,4,5-tetrahydro-1,3-benzodiazepin-2-on, 200 mg (0.770 mmol) (6-Chlor-pyrimidin-4-yl)-(7-methyl-1 H-indazol-5-yl)-amin und 3.50 mL (20.3 mmol) DIPEA wurden in 1.5 mL DMF zusammen gegeben und bei 150°C gerührt. Nach beendeter Umsetzung wurde der Reaktionsansatz mittels präparativer HPLC gereinigt. Die Produkt enthaltenden Fraktionen wurden vereinigt und gefriergetrocknet.

| | |
|---|---|
| Ausbeute: | 40 mg (11% d.Th.) |
| ESI-MS: | m/z = 497 (M-H)⁻ |
| R_{f}: | 0.5 (Kieselgel: DCM/MeOH/Cyc/NH₄OH = 70:15:15:2) |

### Beispiel 12

### 7-Methoxy-3-{1-[6-(1,2,7-trimethyl-1H-benzimidazol-5-ylamino)-pyrimidin-4-yl]-piperidin-4-yl}-1,3,4,5-tetrahydro-benzo[d][1,3]diazepin-2-on

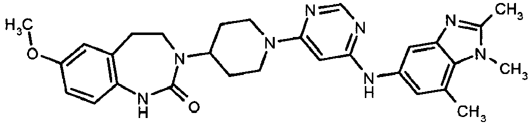

80 mg (0.29 mmol) 7-Methoxy-3-piperidin-4-yl-1,3,4,5-tetrahydro-1,3-benzodiazepin-2-on, 0.10 g (0.26 mmol) (6-lod-pyrimidin-4-yl)-(1,2,7-trimethyl-1H-benzimidazol-5-yl)-amin und 0.15 mL (0.87 mmol) DIPEA wurden in 1.5 mL DMF zusammen gegeben und bei 150°C gerührt. Der Reaktionsansatz wurde anschließend mittels präparativer HPLC gereinigt. Die Produkt enthaltenden Fraktionen wurden vereinigt, eingeengt und der Rückstand getrocknet. Dieser wurde in DMF aufgenommen und erneut mittels präparativer HPLC gereinigt. Die Produkt enthaltenden Fraktionen wurden vereinigt und gefriergetrocknet.

| | |
|---|---|
| Ausbeute: | 10 mg (7% d.Th.) |
| ESI-MS: | m/z = 527 (M+H)⁺ |
| Rₜ(HPLC): | 0.97 min (Methode B) |

### Beispiel 13

### 1-{1-[6-(2,7-Dimethyl-1H-benzimidazol-5-ylamino)-pyrimidin-4-yl]-piperidin-4-yl}-1,3-dihydro-imidazo[4,5-b]pyridin-2-on

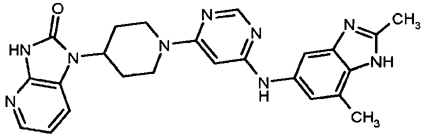

Zu 80 mg (0.3 mmol) (6-Chlor-pyrimidin-4-yl)-(2,7-dimethyl-1 H-benzimidazol-5-yl)-amin, 0.2 mL (1.1 mmol) DIPEA in 2 mL DMF wurden 85 mg (0.3 mmol) 1-Piperidin-4-yl-1,3-dihydroimidazo[4,5-b]pyridin-2-on-dihydrochlorid zugegeben und über Nacht bei 60°C gerührt, danach 3 h bei 120°C in der Mikrowelle erwärmt und dann nochmals 1 h 15 min bei 150°C in der Mikrowelle erwärmt. Danach wurde nochmals 30 mg 1-Piperidin-4-yl-1,3-dihydroimidazo[4,5-*b*]pyridin-2-on-dihydrochlorid und DIPEA zugesetzt und weitere 30 min bei 150°C in der Mikrowelle gerührt. Der Reaktionsansatz wurde dann mittels präparativer HPLC gereinigt. Die Produkt enthaltenden Fraktionen wurden vereinigt und gefriergetrocknet.

| | |
|---|---|
| Ausbeute: | 46 mg (31% d. Th.) |
| ESI-MS: | m/z = 456 (M+H)⁺ |
| Rₜ(HPLC): | 0.79 min (Methode B) |

### Beispiel 14

### 1-{1-[6-(3,4-Dimethyl-2-oxo-2,3-dihydro-benzoxazol-6-ylamino)-pyrimidin-4-yl]-piperidin-4-yl}-1,3-dihydro-imidazo[4,5-b]pyridin-2-on

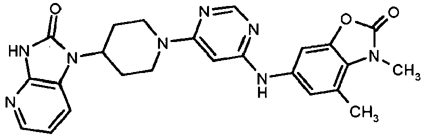

0.10 g (0.34 mmol) 1-Piperidin-4-yl-1,3-dihydroimidazo[4,5-*b*]pyridin-2-on-dihydrochlorid, 90 mg (0.31 mmol) 6-(6-Chlor-pyrimidin-4-ylamino)-3,4-dimethyl-3H-benzoxazol-2-on und 0.15 mL (0.87 mmol) DIPEA in 1.5 mL DMF wurden zusammen gegeben und bei 150°C gerührt. Nach beendeter Umsetzung wurde der Reaktionsansatz mit Wasser verdünnt. Der entstandene Niederschlag wurde abgesaugt, in DMF aufgenommen und mittels präparativer HPLC gereinigt. Die Produkt enthaltenden Fraktionen wurden vereinigt, eingeengt und der Rückstand getrocknet.

| | |
|---|---|
| Ausbeute: | 25 mg (17% d.Th.) |
| ESI-MS: | m/z = 473 (M+H)⁺ |
| Rₜ(HPLC): | 1.04 min (Methode B) |

### Beispiel 15

### N-(3,4-Dimethyl-2-oxo-2,3-dihydro-benzoxazol-6-yl)-N-{6-[4-(7-methoxy-2-oxo-1,2,4,5-tetrahydro-benzo[d][1,3]diazepin-3-yl)-piperidin-1-yl]-pyrimidin-4-yl}-acetamid

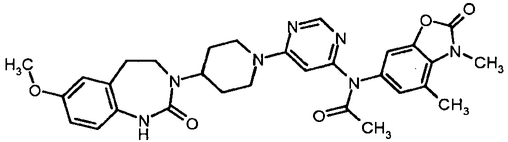

59 mg (0.21 mmol) 7-Methoxy-3-piperidin-4-yl-1,3,4,5-tetrahydro-1,3-benzodiazepin-2-on, 65 mg (0.19 mmol) N-(6-Chlor-pyrimidin-4-yl)-N-(3,4-dimethyl-2-oxo-2,3-dihydro-benzoxazol-6-yl)-acetamid und 81 mg (0.59 mmol) Kaliumcarbonat in 1.5 mL NMP wurden vereinigt und bei 80°C gerührt. Der Reaktionsansatz wurde mittels präparativer HPLC gereinigt. Die Produkt enthaltenden Fraktionen wurden vereinigt und eingeengt. Der erhaltene Feststoff wurde mit Diethylether gewaschen und getrocknet.

| | |
|---|---|
| Ausbeute: | 6 mg (5% d.Th.) |
| ESI-MS: | m/z = 572 (M+H)⁺ |
| Rₜ(HPLC): | 1.53 min (Methode F) |

### Beispiel 16

### 3-{1-[6-(2,7-Dimethyl-benzoxazol-5-ylamino)-pyrimidin-4-yl]-piperidin-4-y}-7-methoxy-1,3,4,5-tetrahydro-benzo[d][1,3]diazepin-2-on

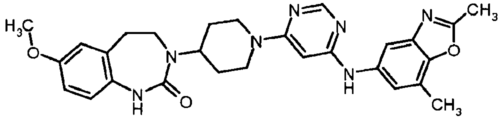

70 mg (0.25 mmol) 7-Methoxy-3-piperidin-4-yl-1,3,4,5-tetrahydro-1,3-benzodiazepin-2-on, 55 mg (0.20 mmol) (6-Chlor-pyrimidin-4-yl)-(2,7-dimethyl-benzoxazol-5-yl)-amin und 0.15 mL (0.87 mmol) DIPEA wurden in 1.5 mL DMF zusammen gegeben und 2.5 h bei 150°C gerührt. Der Reaktionsansatz wurde mittels präparativer HPLC gereinigt. Die das Produkt enthaltenden Fraktionen wurden vereinigt und gefriergetrocknet. Der Rückstand wurde mit MeOH und DMF verrührt, 1 h stehen gelassen und filtriert. Der Niederschlag wurde mit MeOH gewaschen und getrocknet.

| | |
|---|---|
| Ausbeute: | 25 mg (24% d.Th.) |
| ESI-MS: | m/z = 514 (M+H)⁺ |
| Rₜ(HPLC): | 1.25 min (Methode B) |

### Beispiel 17

### 3-{1-[6-(4-Hydroxy-3,5-dimethyl-phenylamino)-pyrimidin-4-yl]-piperidin-4-yl}-7-methoxy-1,3,4,5-tetrahydro-benzo[d][1,3]diazepin-2-on

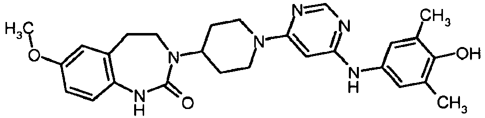

140 mg (0.508 mmol) 7-Methoxy-3-piperidin-4-yl-1,3,4,5-tetrahydro-1,3-benzodiazepin-2-on, 130 mg (0.521 mmol) 4-(6-Chlor-pyrimidin-4-ylamino)-2,6-dimethyl-phenol und 2.50 mL (14.5 mmol) DIPEA wurden vereinigt und über Nacht auf 150°C erhitzt. Der Reaktionsansatz wurde eingeengt, der Rückstand in DMF gelöst und mittels präparativer HPLC gereinigt. Die Produkt enthaltenden Fraktionen wurden vereinigt und gefriergetrocknet. Das gefriergetrocknete Produkt wurde mit MeOH verrührt, der Niederschlag abgesaugt und getrocknet.

| | |
|---|---|
| Ausbeute: | 35 mg (14% d.Th.) |
| ESI-MS: | m/z = 489 (M+H)⁺ |
| R_{f}: | 0.62 (Kieselgel: DCM/MeOH/Cyc/NH₄OH = 70:15:15:2) |

### Beispiel 18

### 4-Methyl-6-(2'-oxo-1,1',2',3-tetrahydrospiro[inden-2,3'-pyrrolo[2,3-b]pyridin]-5-ylamino)-pyrimidin-4-ylamino)benzo[d]oxazol-2(3H)-on

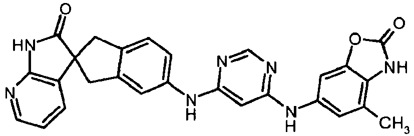

57 mg (0.23 mmol) 5-Amino-1,3-dihydrospiro[inden-2,3'-pyrrolo[2,3-b]pyridin]-2'(1'H)-on, 80 mg (0.23 mmol) 6-(6-Chlor-pyrimidin-4-ylamino)-4-methyl-3H-benzoxazol-2-on und 69 mg (0.50 mmol) Kaliumcarbonat wurden in 1.5 mL NMP vereint und 2 h bei 130°C gerührt. Anschließend wurde der Reaktionsansatz abgekühlt, mit 1 M wässriger Salzsäure-Lösung angesäuert und 1 h bei 50°C gerührt. Der Reaktionsansatz wurde mit Wasser verdünnt, der Niederschlag in DMF aufgenommen und mittels präparativer HPLC gereinigt. Die Produkt enthaltenden Fraktionen wurden vereinigt und gefriergetrocknet.

| | |
|---|---|
| Ausbeute: | 30 mg (27% d.Th.) |
| ESI-MS: | m/z = 492 (M+H)⁺ |
| Rₜ(HPLC): | 1.27 min (Methode B) |

### Beispiel 19

### 5-(6-(2,7-dimethyl-1H-benz[d]imidazol-5-ylamino)pyrimidin-4-ylamino)-1,3-dihydrospiro-[inden-2,3'-pyrrolo[2,3-b]pyridin]-2'(1'H)-on

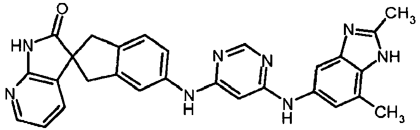

140 mg (0.557 mmol), 150 mg (0.548 mmol) (6-Chlor-pyrimidin-4-yl)-(2,7-dimethyl-1 H-benzimidazol-5-yl)-amin und 1.00 mL (5.81 mmol) DIPEA wurden mit 1.5 mL DMF zusammen gegeben und unter Rückfluss gekocht. Es wurden 20.0 mg (0.126 mmol) Benzolsulfonsäure zugegeben und weitere 3 h gerührt. Der Reaktionsansatz wurde mit DMF verdünnt und mittels präparativer HPLC gereinigt. Die Produkt enthaltenden Fraktionen wurden vereinigt und gefriergetrocknet.

| | |
|---|---|
| Ausbeute: | 15 mg (5% d.Th.) |
| ESI-MS: | m/z = 489 (M+H)⁺ |
| Rₜ(HPLC): | 0.90 min (Methode B) |

### Beispiel 20

### 1-{1-[6-(2,7-Dimethyl-benzoxazol-5-ylamino)-pyrimidin-4-yl]-piperidin-4-yl}-3-dihydro-imidazo[4,5-b]pyridin-2-on

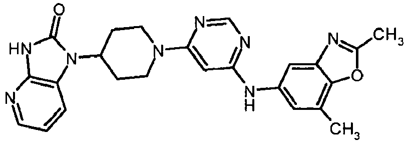

75 mg (0.26 mmol) 1-Piperidin-4-yl-1,3-dihydroimidazo[4,5-*b*]pyridin-2-on-dihydrochlorid, 55 mg (0.20 mmol) (6-Chlor-pyrimidin-4-yl)-(2,7-dimethyl-benzoxazol-5-yl)-amin und 0.15 mL (0.87 mmol) DIPEA wurden in 1.5 mL DMF zusammen gegeben und bei 150°C 2.5 h gerührt. Der Reaktionsansatz wurde mittels präparativer HPLC gereinigt. Die Produkt enthaltenden Fraktionen wurden vereinigt und gefriergetrocknet.

| | |
|---|---|
| Ausbeute: | 25 mg (27% d.Th.) |
| ESI-MS: | m/z = 457 (M+H)⁺ |
| Rₜ(HPLC): | 1.11 min (Methode B) |

### Beispiel 21

### 7-Methoxy-3-{1-[6-4-methyl-2-oxo-2,3-dihydro-benzoxazol-6-ylsulfanyl)-pyrimidin-4-yl]-piperidin-4-yl}-1,3,4,5-tetrahydro-benzo[d][1,3]diazepin-2-on

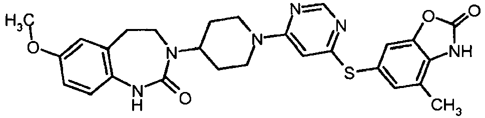

187 mg (0.681 mmol) 7-Methoxy-3-piperidin-4-yl-1,3,4,5-tetrahydro-1,3-benzodiazepin-2-on, 180 mg (0.490 mmol) 6-(6-Chlor-pyrimidin-4-ylsulfanyl)-4-methyl-3H-benzoxazol-2-on und 200 mg (1.45 mmol) Kaliumcarbonat wurden in 5.0 mL NMP zusammen gegeben und 2 h bei 130°C gerührt. Der Reaktionsansatz wurde über Nacht abgekühlt, filtriert und das Filtrat mittels präparativer HPLC gereinigt. Die Produkt enthaltenden Fraktionen wurden vereinigt, eingeengt und i.vac. getrocknet.

| | |
|---|---|
| Ausbeute: | 130 mg (50% d.Th.) |
| ESI-MS: | m/z = 533 (M+H)⁺ |
| Rₜ(HPLC): | 1.00 min (Methode B) |

### Beispiel 22

### 7-Methoxy-3-{1-[2-methyl-6-(4-methyl-2-oxo-2,3-dihydro-benzoxazol-6-yloxy)-pyrimidin-4-yl]-piperidin-4-yl}-1,3,4,5-tetrahydro-benzo[d][1,3]diazepin-2-on

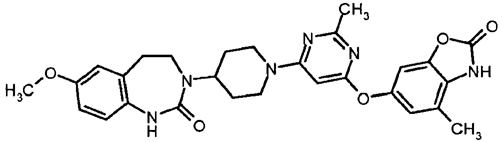

48 mg (0.17 mol) 7-Methoxy-3-piperidin-4-yl-1,3,4,5-tetrahydro-1,3-benzodiazepin-2-on, 50 mg (0.17 mmol) 6-(6-Chlor-2-methyl-pyrimidin-4-yloxy)-4-methyl-3H-benzoxazol-2-on und 0.10 mL (0.58 mmol) DIPEA wurden in 1.8 mL DMF zusammen gegeben und über Nacht bei 80°C gerührt. Der Reaktionsansatz wurde mit Wasser verdünnt und der ausgefallene Niederschlag abgesaugt und getrocknet.

| | |
|---|---|
| Ausbeute: | 62 mg (68% d.Th.) |
| ESI-MS: | m/z =531 (M+H)⁺ |
| Rₜ (HPLC): | 1.24 min (Methode B) |

### Beispiel 23

### 1-(6-(4-Methyl-2-oxo-2,3-dihydrobenz-[d]oxazol-6-yloxy)pyrimidin-4-yl)spiro[piperidin-4,4'-pyrido[2,3-d][1,3]oxazin]-2'(1'H)-on

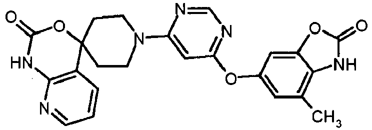

20 mg (0.078 mol) Spiro[piperidin-4,4'-pyrido[2,3-d][1,3]oxazin]-2'(1'H)-on Hydrochlorid, 20 mg (0.072 mmol) 6-(6-Chlor-pyrimidin-4-ylamino)-4-methyl-3H-benzoxazol-2-on und 0.10 mL (0.57 mmol) DIPEA wurden in 1.0 mL DMF zusammen gegeben und über Nacht bei RT gerührt. Der Reaktionsansatz wurde mittels präparativer HPLC gereinigt. Die Produkt enthaltenden Fraktionen wurden vereinigt und gefriergetrocknet.

| | |
|---|---|
| Ausbeute: | 3 mg (9% d.Th.) |
| ESI-MS: | m/z =461 (M+H)⁺ |
| Rₜ (HPLC): | 1.24 min (Methode B) |

### Beispiel 24

### 1-{1-[2-Methyl-6-(4-methyl-2-oxo-2,3-dihydro-benzoxazol-6-yloxy)-pyrimidin-4-yl]-piperidin-4-yl}-1,3-dihydro-imidazo[4,5-b]pyridin-2-on

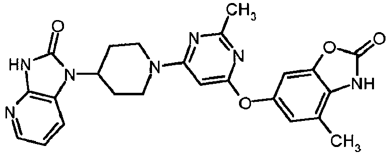

38 mg (0.17 mmol) 1-Piperidin-4-yl-1,3-dihydroimidazo[4,5-*b*]pyridin-2-on, 50 mg (0.17 mmol) 6-(6-Chlor-2-methyl-pyrimidin-4-yloxy)-4-methyl-3H-benzoxazol-2-on und 0.10 mL (0.57 mmol) DIPEA wurden in 1.8 mL DMF zusammen gegeben und über Nacht bei 80°C gerührt. Anschließend wurden nochmals 15 mg (0.069 mmol) 1-Piperidin-4-yl-1,3-dihydroimidazo[4,5-b]pyridin-2-on zugegeben und über Nacht bei RT weitergerührt. Der Reaktionsansatz wurde mit Wasser verdünnt, der ausgefallene Niederschlag abgesaugt und i.vac. getrocknet.

| | |
|---|---|
| Ausbeute: | 49 mg (60% d.Th.) |
| ESI-MS: | m/z =474 (M+H)⁺ |
| Rₜ (HPLC): | 1.09 min (Methode B) |

### Beispiel 25

### 7-Methoxy-3-{1-[6-(4-methyl-2-oxo-2,3-dihydro-benzoxazol-6-sulfinyl)-pyrimidin-4-yl]-piperidin-4-yl}-1,3,4,5-tetrahydro-benzo[d][1,3]diazepin-2-on

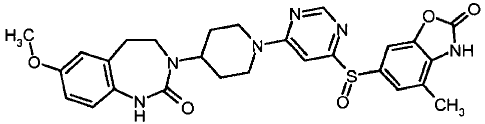

Unter Stickstoffatmosphäre wurde zu 60 mg (0.11 mmol) 7-Methoxy-3-{1-[6-(4-methyl-2-oxo-2,3-dihydro-benzoxazol-6-ylsulfanyl)-pyrimidin-4-yl]-piperidin-4-yl}-1,3,4,5-tetrahydro-benzo[d][1,3]diazepin-2-on in 5 mL Dichlormethan und 1 mL Methanol 28 mg (0.11 mmol) 3-Chlorperoxybenzoesäure (70%ig) in 5 mL Dichlormethan zugetropft. Nach beendeter Reaktion wurde der Reaktionsansatz eingeengt, in MeOH/DMF gelöst und mittels präparativer HPLC-MS aufgereinigt. Die Produkt enthaltende Fraktionen wurden vereinigt und gefriergetrocknet.

| | |
|---|---|
| Ausbeute: | 50 mg (73% d.Th.) |
| ESI-MS: | m/z = 549 (M+H)⁺ |
| Rₜ(HPLC): | 1.26 min (Methode B) |

### Beispiel 26

### 7-Methoxy-3-{1-[6-(4-methyl-2-oxo-2,3-dihydro-benzoxazol-6-sulfonyl)-pyrimidin-4-yl]-piperidin-4-yl}-1,3,4,5-tetrahydro-benzo[d][1,3]diazepin-2-on

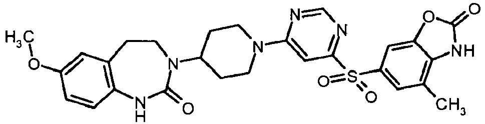

Unter einer Stickstoffatmosphäre wurde unter Eiskühlung zu 60 mg (0.11 mmol) 7-Methoxy-3-{1-[6-(4-methyl-2-oxo-2,3-dihydro-benzoxazol-6-ylsulfanyl)-pyrimidin-4-yl]-piperidin-4-yl}-1,3,4,5-tetrahydro-benzo[d][1,3]diazepin-2-on in 5 mL Dichlormethan 56 mg (0.23 mmol) 3-Chlorperoxybenzoesäure (70%ig) in 5 mL Dichlormethan zugetropft. Nach erfolgter Reaktion wurde der Reaktionsansatz mittels präparativer HPLC-MS aufgereinigt. Die Produkt enthaltende Fraktionen wurden vereinigt und gefriertrocknet.

| | |
|---|---|
| Ausbeute: | 19 mg (28% d.Th.) |
| ESI-MS: | m/z = 565 (M+H)⁺ |
| Rₜ(HPLC): | 1.42 min (Methode B) |

### Beispiel 27

### 7-Methoxy-3-{1-[6-(chinoxalin-2-yloxy)-pyrimidin-4-yl]-piperidin-4-yl}-1,3,4,5-tetrahydro-benzo[d][1,3]diazepin-2-on

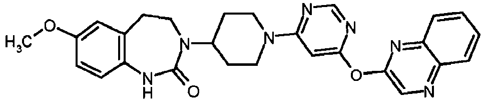

24 mg (0.087 mmol) 7-Methoxy-3-piperidin-4-yl-1,3,4,5-tetrahydro-1,3-benzodiazepin-2-on, 20 mg (0.077 mmol) 2-(6-Chlor-pyrimidin-4-yloxy)-chinoxalin und 0.050 mL (0.29 mmol) DIPEA wurden in 1.0 mL DMF zusammen gegeben und über Nacht bei 90°C gerührt. Das Reaktionsgemisch wurde mittels präparativer HPLC-MS gereinigt. Die Produkt enthaltenden Fraktionen wurden vereinigt und gefriergetrocknet.

| | |
|---|---|
| Ausbeute: | 10 mg (26% d.Th.) |
| ESI-MS: | m/z = 498 (M+H)⁺ |
| Rₜ(HPLC): | 1.48 min (Methode B) |

### Beispiel 28

### (6-{6-[4-(7-Methoxy-2-oxo-1,2,4.5-tetrahydro-benzo[d][1,3]diazepin-3-yl)-piperidin-1-yl]-pyrimidin-4-yloxy}-4-methyl-1H-benzimidazol-2-yl)-acetonitril

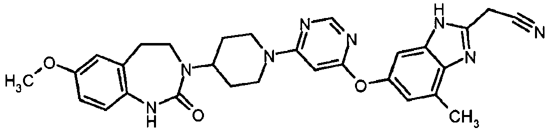

0.20 g (0.42 mmol) 3-{1-[6-(3,4-Diamino-5-methyl-phenoxy)-pyrimidin-4-yl]-piperidin-4-yl}-7-methoxy-1,3,4,5-tetrahydro-benzo[d][1,3]diazepin-2-on, 40 mg (0.46 mmol) Cyanessigsäure, 0.15 g (0.47 mmol) TBTU und 0.14 mL (1.00 mmol) TEA in 2.00 mL DMF wurden 4 h bei RT gerührt. Anschließend wurde 2.00 mL Eisessig zugesetzt und über Nacht bei RT gerührt. Weiterhin wurde der Reaktionsansatz 1 h bei 100°C gerührt, dann abgekühlt und mittels präparativer HPLC-MS gereinigt. Die Produkt enthaltenden Fraktionen wurden vereinigt und das Acetonitril eingeengt. Der Rückstand wurde mit 4N wässriger Natronlauge alkalisch gestellt, der ausgefallene Niederschlag abgesaugt, mit Wasser gewaschen und im HV getrocknet.

| | |
|---|---|
| Ausbeute: | 17 mg (8% d.Th.) |
| ESI-MS: | m/z = 539 (M+H)⁺ |
| Rₜ (HPLC): | 1.23 min (Methode B) |

### Beispiel 29

### 3-{1-[6-(2,7-Dimethyl-3H-benzimidazol-5-yloxy)-pyrimidin-4-yl]-piperidin-4-yl}-7-methoxy-1,3,4,5-tetrahydro-benzo[d][1,3]diazepin-2-on

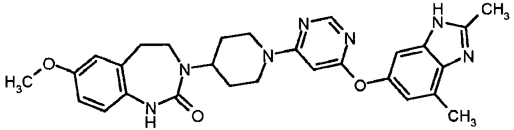

0.16 g (0.59 mmol) 7-Methoxy-3-piperidin-4-yl-1,3,4,5-tetrahydro-1,3-benzodiazepin-2-on, 0.15 g (0.55 mmol) 6-(6-Chlor-pyrimidin-4-yloxy)-2,4-dimethyl-1H-benzimidazol und 0.21 mL (1.2 mmol) DIPEA wurden in 2.0 mL DMF zusammen gegeben und über Nacht bei RT gerührt. Das Reaktionsgemisch wurde mittels präparativer HPLC-MS gereinigt. Die Produkt enthaltenden Fraktionen wurden vereinigt und das Acetonitril eingeengt. Der Rückstand wurde mit 4N wässriger Natronlauge alkalisch gestellt, der ausgefallene Niederschlag abgesaugt, mit Wasser gewaschen und im HV getrocknet.

| | |
|---|---|
| Ausbeute: | 70 mg (26% d.Th.) |

| | |
|---|---|
| ESI-MS: | m/z = 514 (M+H)⁺ |
| Rₜ(HPLC): | 1.15 min (Methode B) |

### Beispiel 30

### 1-(6-(2,4-Dimethyl-1H-benzo[d]imidazol-6-yloxy)pyrimidin-4-yl)spiro[piperidin-4,4'-pyrido-[2,3-d][1,3]oxazin]-2'(1'H)-on

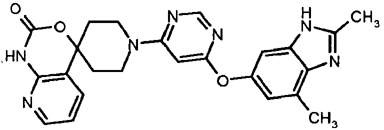

0.15 g (0.59 mmol) Spiro[piperidin-4,4'-pyrido[2,3-d][1,3]oxazin]-2'(1'H)-on Hydrochlorid, 0.15 g (0.55 mmol) 6-(6-Chlor-pyrimidin-4-yloxy)-2,4-dimethyl-1H-benzimidazol und 0.31 mL (1.8 mmol) DIPEA wurden in 1.5 mL DMF zusammen gegeben und zunächst über Nacht bei RT anschließend 2 h bei 50°C gerührt. Das Reaktionsgemisch wurde mittels präparativer HPLC-MS gereinigt. Die Produkt enthaltenden Fraktionen wurden vereinigt und das Acetonitril eingeengt. Der Rückstand wurde mit 4N wässriger Natronlauge alkalisch gestellt, der ausgefallene Niederschlag abgesaugt, mit Wasser gewaschen und im HV getrocknet.

| | |
|---|---|
| Ausbeute: | 130 mg (48% d.Th.) |
| ESI-MS: | m/z = 458 (M+H)⁺ |
| Rₜ(HPLC): | 0.96 min (Methode B) |

### Beispiel 31

### 7-Methoxy-3-{1-[6-(2-methyl-chinolin-4-yloxy)-pyrimidin-4-yl]-piperidin-4-yl}-1,3,4,5-tetrahydro-benzo[d][1,3]diazepin-2-on

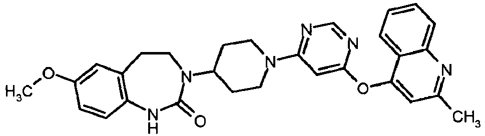

75 mg (0.27 mmol) 7-Methoxy-3-piperidin-4-yl-1,3,4,5-tetrahydro-1,3-benzodiazepin-2-on, 70 mg (0.26 mmol) 4-(6-Chlor-pyrimidin-4-yloxy)-2-methyl-chinolin und 0.10 mL (0.58 mmol) DIPEA wurden in 1.8 mL DMF zusammen gegeben und über Nacht bei 90°C gerührt. Das Reaktionsgemisch wurde mittels präparativer HPLC-MS gereinigt. Die Produkt enthaltenden Fraktionen wurden vereinigt und gefriergetrocknet.

| | |
|---|---|
| Ausbeute: | 35 mg (25% d.Th.) |
| ESI-MS: | m/z = 511 (M+H)⁺ |
| Rₜ(HPLC): | 1.22 min (Methode B) |

### Beispiel 32

### 7-Methoxy-3-{1-[6-(7-methyl-3H-benzimidazo)-5-yloxy)-pyrimidin-4-yl]-piperidin-4-yl}-1,3,4,5-tetrahydro-benzo[d][1,3]diazepin-2-on

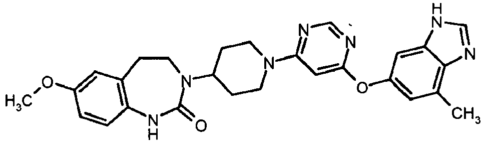

47 mg (0.17mmol) 7-Methoxy-3-piperidin-4-yl-1,3,4,5-tetrahydro-1,3-benzodiazepin-2-on, 45 mg (0.17 mmol) 6-(6-Chlor-pyrimidin-4-yloxy)-4-methyl-1H-benzoimidazol und 0.06 mL (0.34 mmol) DIPEA wurden in 2.0 mL DMF zusammen gegeben und 48 h bei RT gerührt. Das Reaktionsgemisch wurde mittels präparativer HPLC-MS gereinigt. Die Produkt enthaltenden Fraktionen wurden vereinigt und das Acetonitril eingeengt. Der Rückstand wurde mit 4N wässriger Natronlauge alkalisch gestellt, der ausgefallene Niederschlag abgesaugt, mit Wasser gewaschen und im HV getrocknet.

| | |
|---|---|
| Ausbeute: | 55 mg (65% d.Th.) |
| ESI-MS: | m/z = 500 (M+H)⁺ |
| Rₜ(HPLC): | 1.14 min (Methode B) |

### Beispiel 33

### 3-{1-[6-(2-Cyclopropylmethyl-7-methyl-3H-benzimidazol-5-yloxy)-pyrimidin-4-yl]-piperidin-4-yl}-7-methoxy-1,3,4,5-tetrahydro-benzo[d][1,3]diazepin-2-on

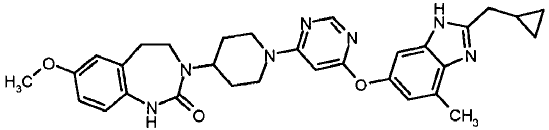

0.20 g (0.42 mmol) 3-{1-[6-(3,4-Diamino-5-methyl-phenoxy)-pyrimidin-4-yl]-piperidin-4-yl}-7-methoxy-1,3,4,5-tetrahydro-benzo[d][1,3]diazepin-2-on, 46 mg (0.46 mmol) Cyclopropyl-essigsäure, 151 mg (0.47 mmol) TBTU und 0.14 mL (1.00 mmol) TEA in 2.00 mL DMF wurden 4 h bei RT gerührt. Anschließend wurde 2.00 mL Eisessig zugesetzt und über Nacht bei RT gerührt. Weiterhin wurde der Reaktionsansatz 1 h bei 100°C gerührt, dann abgekühlt und mittels präparativer HPLC-MS gereinigt. Die Produkt enthaltenden Fraktionen wurden vereinigt und das Acetonitril eingeengt. Der Rückstand wurde mit 4N wässriger Natronlauge alkalisch gestellt, der ausgefallene Niederschlag abgesaugt, mit Wasser gewaschen und im HV getrocknet.

| | |
|---|---|
| Ausbeute: | 45 mg (20% d.Th.) |
| ESI-MS: | m/z = 554 (M+H)⁺ |
| Rₜ (HPLC): | 1.24 min (Methode B) |

### Beispiel 34

### 1-{1-[6-(2,7-Dimethyl-3H-benzimidazol-5-yloxy)-pyrimidin-4-yl]-piperidin-4-yl}-1,3-dihydro-imidazo[4,5-b]pyridin-2-on

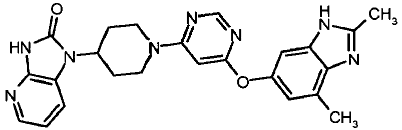

0.13 g (0.59 mmol) 1-Piperidin-4-yl-1,3-dihydroimidazo[4,5-*b*]pyridin-2-on, 150 mg (0.55 mmol) 6-(6-Chlor-pyrimidin-4-yloxy)-2,4-dimethyl-1 H-benzimidazol und 0.21 mL (1.20 mmol) DIPEA wurden in 2.00 mL DMF zusammen gegeben und über Nacht bei RT gerührt. Das Reaktionsgemisch wurde mittels präparativer HPLC-MS gereinigt. Die Produkt enthaltenden Fraktionen wurden vereinigt und das Acetonitril eingeengt. Der Rückstand wurde mit 1 N wässriger Natronlauge alkalisch gestellt, der ausgefallene Niederschlag abgesaugt, mit Wasser gewaschen und im HV getrocknet.

| | |
|---|---|
| Ausbeute: | 72 mg (27% d.Th.) |
| ESI-MS: | m/z = 457 (M+H)⁺ |

### Beispiel 35

### 7-Methoxy-3-(1-{6-[7-methyl-2-(2,2,2-trifluorethyl)-3H-benzoimidazol-5-yloxy]-pyrimidin-4-yl}-piperidin-4-yl)-1,3,4,5-tetrahydro-benzo[d][1,3]diazepin-2-on

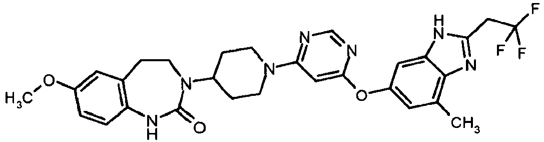

0.20 g (0.42 mmol) 3-{1-[6-(3,4-Diamino-5-methyl-phenoxy)-pyrimidin-4-yl]-piperidin-4-yl}-7-methoxy-1,3,4,5-tetrahydro-benzo[d][1,3]diazepin-2-on, 60 mg (0.46 mmol) 3,3,3-Trifluorpropionsäure, 0.15 g (0.47 mmol) TBTU und 0.14 mL (1.0 mmol) TEA in 2.0 mL DMF wurden 4 h bei RT gerührt. Anschließend wurde 2.0 mL Eisessig zugesetzt und über Nacht bei RT gerührt. Weiterhin wurde der Reaktionsansatz 1 h bei 100°C gerührt, dann abgekühlt und mittels präparativer HPLC-MS gereinigt. Die das Produkt enthaltenden Fraktionen wurden vereinigt und das Acetonitril eingeengt. Der Rückstand wurde mit 4N wässriger Natronlauge alkalisch gestellt, der ausgefallene Niederschlag abgesaugt, mit Wasser gewaschen und im HV getrocknet.

| | |
|---|---|
| Ausbeute: | 13 mg (5% d.Th.) |
| ESI-MS: | m/z = 582 (M+H)⁺ |
| Rₜ (HPLC): | 1.31 min (Methode B) |

### Beispiel 36

### 3-{1-[6-(2,6-Dimethylpyridin-4-yloxy)pyrimidin-4-yl]piperidin-4-yl}-7-methoxy-4,5-dihydro-1H-benzo[d][1,3]diazepin-2(3H)-on

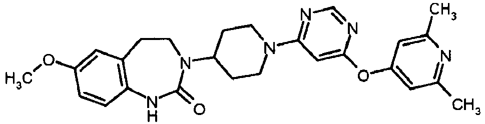

62 mg (0.23 mmol) 7-Methoxy-3-piperidin-4-yl-1,3,4,5-tetrahydro-1,3-benzodiazepin-2-on, 50 mg (0.21 mmol) 6-Chlor-N-(2,6-dimethylpyridin-4-yl)pyrimidin-4-amin und 0.10 mL (0.57 mmol) DIPEA in 1.0 mL DMF wurden 1 h bei 90°C gerührt. Das Reaktionsgemisch wurde mit Wasser versetzt und der entstandene Niederschlag abgesaugt und getrocknet.

| | |
|---|---|
| Ausbeute: | 72 mg (68% d.Th.) |
| ESI-MS: | m/z = 475 (M+H)⁺ |
| Rₜ (HPLC): | 1.11 min (Methode B) |

### Beispiel 37

### 3-{1-[2-Amino-6-(4-methyl-2-oxo-2,3-dihydrobenzofuran-6-yloxy)piperidin-4-yloxy)-methoxy-4,5-dihydro-1H-benzo[d][1,3]diazepin-2(3H)-on

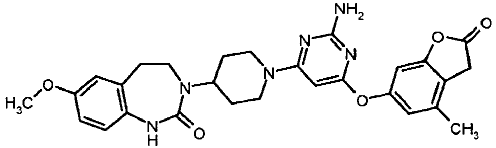

78 mg (0.28 mmol) 7-Methoxy-3-piperidin-4-yl-1,3,4,5-tetrahydro-1,3-benzodiazepin-2-on, 80 mg (0.23 mmol) 6-(2-Amino-6-chlorpyrimidin-4-yloxy)-4-methylbenzo[d]oxazol-2(3H)-on und 0.10 mL (0.57 mmol) DIPEA in 1.0 mL DMF wurden 1 h bei 90°C gerührt. Das Reaktionsgemisch wurde mittels präparativer HPLC-MS gereinigt. Die Produkt enthaltenden Fraktionen wurden vereinigt und gefriergetrocknet.

| | |
|---|---|
| Ausbeute: | 43 mg (34% d.Th.) |
| ESI-MS: | m/z = 532 (M+H)⁺ |
| Rₜ (HPLC): | 1.19 min (Methode B) |

### Beispiel 38

### 7-Methoxy-3-{1-[6-(2-(methoxymethyl)-4-methyl-1H-benzo[d]imidazol-6-yloxy)pyrimidin-4-yl]piperidin-4-yl}-4,5-dihydro-1H-benzo[d][1,3]diazepin-2(3H)-on

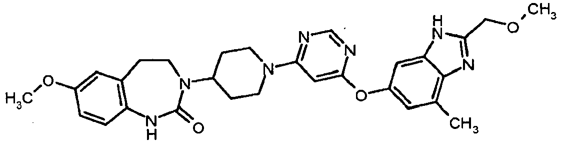

52 mg (0.19 mmol) 7-Methoxy-3-piperidin-4-yl-1,3,4,5-tetrahydro-1,3-benzodiazepin-2-on, 50 mg (0.16 mmol) 6-(6-Chlorpyrimidin-4-yloxy)-2-(methoxymethyl)-4-methyl-1 H-benzo-[d]imidazol und 0.07 mL (0.40 mmol) DIPEA in 2.0 mL DMF wurden über das Wochenende bei RT gerührt. Das Reaktionsgemisch wurde mittels präparativer HPLC-MS gereinigt. Die Produkt enthaltenden Fraktionen wurden teilweise i.vac. eingeengt und mit 4M Natronlauge neutralisiert. Der ausgefallene Niederschlag wurde abgesaugt, nachgewaschen und getrocknet.

| | |
|---|---|
| Ausbeute: | 30 mg (29% d.Th.) |
| ESI-MS: | m/z = 544 (M+H)⁺ |
| Rₜ (HPLC): | 1.16 min (Methode B) |

### Beispiel 39

### 1-{6-[2-(Methoxymethyl)-4-methyl-1H-benzo[d]imidazol-6-yloxyrimidin-4-yl}spiro-(piperidin-4,4'-pyrido[2,3-d][1,3]oxazin)-2'(1'H)-on

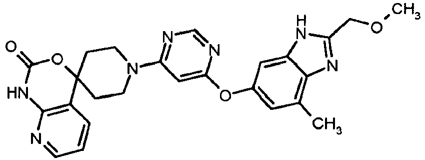

49 mg (0.19 mmol) Spiro[piperidin-4,4'-pyrido[2,3-d][1,3]oxazin]-2'(1'H)-on Hydrochlorid, 50 mg (0.16 mmol) 6-(6-Chlorpyrimidin-4-yloxy)-2-(methoxymethyl)-4-methyl-1H-benzo-[d]imidazol und 0.10 mL (0.57 mmol) DIPEA in 2.0 mL DMF wurden über das Wochenende bei 40°C gerührt. Das Reaktionsgemisch wurde mittels präparativer HPLC-MS gereinigt. Die Produkt enthaltenden Fraktionen wurden teilweise i.vac. eingeengt und mit 4M Natronlauge neutralisiert. Der ausgefallene Niederschlag wurde abgesaugt, nachgewaschen und getrocknet.

| | |
|---|---|
| Ausbeute: | 37 mg (40% d.Th.) |
| ESI-MS: | m/z = 488 (M+H)⁺ |
| Rₜ (HPLC): | 0.99 min (Methode B) |

### Beispiel 40

### 6-{2-Methoxy-6-[4-(7-methoxy-2-oxo-4,5-dihydro-1H-benzo[d][1,3]diazepin-3(2H)-yl)-piperidin-1-yl]pyrimidin-4-yloxy}-4-methylbenzo[d]oxazol-2(3H)-on

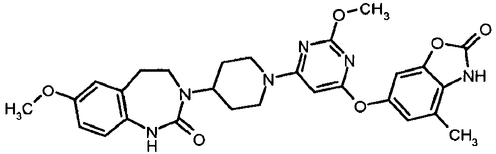

45 mg (0.16 mmol) 7-Methoxy-3-piperidin-4-yl-1,3,4,5-tetrahydro-1,3-benzodiazepin-2-on, 50 mg (0.15 mmol) 6-(6-Chlor-2-methoxypyrimidin-4-yloxy)-4-methylbenzo[d]oxazol-2(3H)-on und 0.10 mL (0.57 mmol) DIPEA in 1.0 mL DMF wurden 3 h bei 90°C gerührt. Das Reaktionsgemisch wurde mittels präparativer HPLC-MS gereinigt. Die Produkt enthaltenden Fraktionen wurden vereinigt und gefriergetrocknet.

| | |
|---|---|
| Ausbeute: | 20 mg (21% d.Th.) |
| ESI-MS: | m/z = 547 (M+H)⁺ |
| Rₜ (HPLC): | 1.42 min (Methode B) |

### Beispiel 41

### 1-[2-Methoxy-6-(4-methyl-2-oxo-2,3-dihydrobenzo[d]oxazol-6-yloxy)-pyrimidin-4-yl]spiro-[piperidin-4,4'-pyrido[2,3-d][1,3]oxazin]-2'(1'H)-on

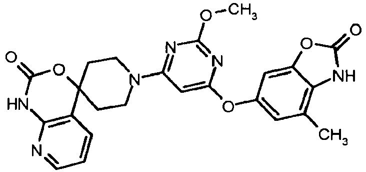

58 mg (0.23 mmol) Spiro[piperidin-4,4'-pyrido[2,3-d][1,3]oxazin]-2'(1'H)-on Hydrochlorid, 70 mg (0.20 mmol) 6-(6-Chlor-2-methoxypyrimidin-4-yloxy)-4-methylbenzo[d]oxazol-2(3H)-on und 0.10 mL DIPEA (0.57 mmol) in 1.0 mL DMF wurden 3 h bei 90°C gerührt. Das Reaktionsgemisch wurde mit Wasser versetzt, der Niederschlag abgesaugt, nachgewaschen und getrocknet. Der Rückstand wurde mittels präparativer HPLC-MS gereinigt. Die Produkt enthaltenden Fraktionen wurden vereinigt und gefriergetrocknet.

| | |
|---|---|
| Ausbeute: | 45 mg (43% d.Th.) |
| ESI-MS: | m/z = 491 (M+H)⁺ |
| Rₜ (HPLC): | 1.26 min (Methode B) |

### Beispiel 42

### 3-{1-[6-(2-Cyclopropyl-4-methyl-1H-benzo[d]imidazol-6-yloxy)pyrimidin-4-yl]piperidin-4-yl}-7-methoxy-4,5-dihydro-1H-benzo[d][1,3]diazepin-2(3H)-on

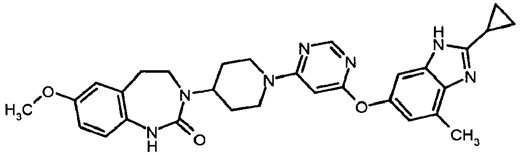

44 mg (0.16 mmol) 7-Methoxy-3-piperidin-4-yl-1,3,4,5-tetrahydro-1,3-benzodiazepin-2-on, 40 mg (0.13 mmol) 6-(6-Chlorpyrimidin-4-yloxy)-2-cyclopropyl-4-methyl-1H-benzo[d]-imidazol und 0.06 mL (0.32 mmol) DIPEA in 2.0 mL DMF wurden über Nacht bei RT gerührt. Das Reaktionsgemisch wurde mittels präparativer HPLC-MS gereinigt. Die das Produkt enthaltenden Fraktionen wurden teilweise i.vac. eingeengt und mit 4M Natronlauge neutralisiert. Der ausgefallene Niederschlag wurde abgesaugt, nachgewaschen und getrocknet.

| | |
|---|---|
| Ausbeute: | 26 mg (30% d.Th.) |
| ESI-MS: | m/z = 540 (M+H)⁺ |
| Rₜ (HPLC): | 1.17 min (Methode B) |

### Beispiel 43

### 1-(1-(6-(2-Cyclopropyl-4-methyl-1H-benzo[d]imidazol-6-yloxy)pyrimidin-4-yl)piperidin-4-yl)-1H-imidazo[4,5-b]pyridin-2(3H)-on

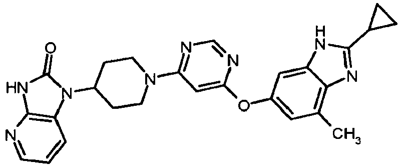

35 mg (0.16 mmol) 1-Piperidin-4-yl-1,3-dihydroimidazo[4,5-*b*]pyridin-2-on, 40 mg (0.13 mmol) 6-(6-Chlorpyrimidin-4-yloxy)-2-cyclopropyl-4-methyl-1H-benzo[d]imidazol und 0.06 mL (0.32 mmol) DIPEA in 2.0 mL DMF wurden über Nacht bei RT und 2 h bei 40°C gerührt. Das Reaktionsgemisch wurde mittels präparativer HPLC-MS gereinigt. Die Produkt enthaltenden Fraktionen wurden teilweise i.vac. eingeengt und mit 1 M Natronlauge neutralisiert. Der ausgefallene Niederschlag wurde abgesaugt, nachgewaschen und getrocknet.

| | |
|---|---|
| Ausbeute: | 30 mg (39% d.Th.) |
| ESI-MS: | m/z = 483 (M+H)⁺ |
| Rₜ (HPLC): | 1.06 min (Methode B) |

### Beispiel 44

### 1-(6-(2-Cyclopropyl-4-methyl-1H-benzo[d]imidazol-6-yloxy)pyrimidin-4-yl)spiro[piperidin-4,4'-pyrido[2,3-d][1,3]oxazin]-2'(1'H)-on

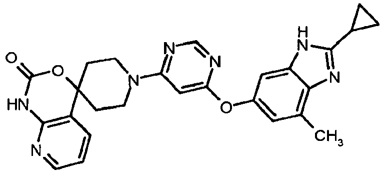

41 mg (0.16 mmol) Spiro[piperidin-4,4'-pyrido[2,3-d][1,3]oxazin]-2'(1'H)-on Hydrochlorid, 40 mg (0.13 mmol) 6-(6-Chlorpyrimidin-4-yloxy)-2-cyclopropyl-4-methyl-1 H-benzo[d]-imidazol und 0.08 mL (0.48 mmol) DIPEA in 2.0 mL DMF wurden über Nacht bei 40°C gerührt. Das Reaktionsgemisch wurde mittels präparativer HPLC-MS gereinigt. Die Produkt enthaltenden Fraktionen wurden teilweise i.vac. eingeengt und mit 4M Natronlauge neutralisiert. Der ausgefallene Niederschlag wurde abgesaugt, nachgewaschen und getrocknet.

| | |
|---|---|
| Ausbeute: | 30 mg (39% d.Th.) |
| ESI-MS: | m/z = 484 (M+H)⁺ |
| Rₜ (HPLC): | 1.04 min (Methode B) |

### Beispiel 45

### 6-(2-Cyclopropyl-6-(4-(7-methoxy-2-oxo-4,5-dihydro-1H-benzo[d][1,3]diazepin-3(2H)-yl)piperidin-1-yl)pyrimidin-4-yloxy)-4-methylbenzo[d]oxazol-2(3H)-on

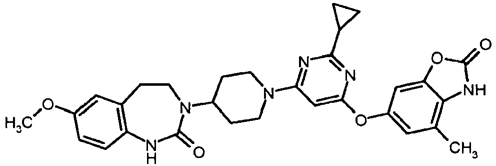

39 mg (0.14 mmol) 7-Methoxy-3-piperidin-4-yl-1,3,4,5-tetrahydro-1,3-benzodiazepin-2-on, 39 mg (0.12 mmol) 6-(6-Chlor-2-cyclopropylpyrimidin-4-yloxy)-4-methylbenzo[d]oxazol-2(3H)-on und 0.10 mL (0.57 mmol) DIPEA in 1.0 mL DMF wurden 2 h bei 90°C gerührt. Das Reaktionsgemisch wurde mit Wasser versetzt. Der ausgefallene Niederschlag wurde abgesaugt, nachgewaschen und getrocknet.

| | |
|---|---|
| Ausbeute: | 45 mg (63% d.Th.) |
| ESI-MS: | m/z = 557 (M+H)⁺ |
| Rₜ (HPLC): | 1.43 min (Methode B) |

### Beispiel 46

### 6-(6-Chlor-2-(4-(7-methoxy-2-oxo-4,5-dihydro-1H-benzo[d][1,3]diazepin-3(2H)-yl)piperidin-1-yl)pyrimidin-4-yloxy)-4-methylbenzo[d]oxazol-2(3H)-on

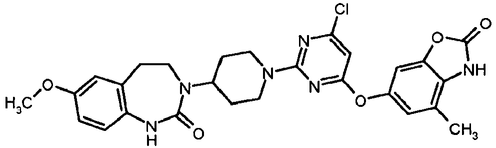

180 mg (0.65 mmol) 7-Methoxy-3-piperidin-4-yl-1,3,4,5-tetrahydro-1,3-benzodiazepin-2-on, 205 mg (0.66 mmol) 6-(2,6-Dichlorpyrimidin-4-yloxy)-4-methylbenzo[d]oxazol-2(3H)-on und 0.25 mL (1.44 mmol) DIPEA in 5.0 mL DMF wurden 2 h bei RT gerührt. Das Reaktionsgemisch wurde mit Wasser versetzt. Der ausgefallene Niederschlag wurde abgesaugt, nachgewaschen und getrocknet. Die Substanz wurde mittels präparativer HPLC-MS gereinigt. Die Produkt enthaltenden Fraktionen wurden teilweise i.vac. eingeengt und mit Natronlauge neutralisiert. Der ausgefallene Niederschlag wurde abgesaugt, nachgewaschen und getrocknet.

| | |
|---|---|
| Ausbeute: | 115 mg (32% d.Th.) |
| ESI-MS: | m/z = 551/553 (Cl) (M+H)⁺ |
| Rₜ (HPLC): | 1.64 min (Methode B) |

### Beispiel 47

### 6-(6-(4-(7-Methoxy-2-oxo-4,5-dihydro-1H-benzo[d][1,3]diazepin-3(2H)-yl)piperidin-1-yl)pyrimidin-4-yloxy)benzo[d]oxazol-2(3H)-on

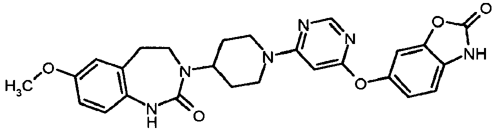

100 mg (0.36 mmol) 7-Methoxy-3-piperidin-4-yl-1,3,4,5-tetrahydro-1,3-benzodiazepin-2-on, 90 mg (0.34 mmol) 6-(6-Chlorpyrimidin-4-yloxy)benzo[d]oxazol-2(3H)-on und 0.07 mL (0.41 mmol) DIPEA in 2.0 mL DMF wurden über Nacht bei RT und 4 h bei 40°C gerührt. Das Reaktionsgemisch wurde mittels präparativer HPLC-MS gereinigt. Die Produkt enthaltenden Fraktionen wurden teilweise i.vac. eingeengt. Der ausgefallene Niederschlag wurde abgesaugt, nachgewaschen und getrocknet.

| | |
|---|---|
| Ausbeute: | 65 mg (38% d.Th.) |
| ESI-MS: | m/z = 503 (M+H)⁺ |
| Rₜ (HPLC): | 1.28 min (Methode B) |

### Beispiel 48

### N-(4-(6-(4-(7-Methoxy-2-oxo-4,5-dihydro-1H-benzo[d][1,3]diazepin-3(2H)-yl)piperidin-1-yl)pyrimidin-4-yloxy)-2-methylphenyl)acetamid

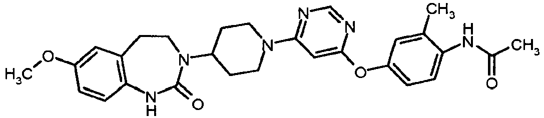

80 mg (0.17 mmol) 3-(1-(6-(4-Amino-3-methylphenoxy)pyrimidin-4-yl)piperidin-4-yl)-7-methoxy-4,5-dihydro-1H-benzo[d][1,3]diazepin-2(3H)-on, 0.02 mL (0.35 mmol) AcOH, 120 mg (0.37 mmol) TBTU und 0.06 mL (0.43 mmol) TEA in 2.0 mL DMF wurden 2 h bei RT gerührt. Zu dem Reaktionsgemisch wurde Wasser zugegeben. Der Niederschlag wurde abgesaugt und mittels präparativer HPLC-MS gereinigt. Die Produkt enthaltenden Fraktionen wurden vereinigt und gefriergetrocknet.

| | |
|---|---|
| Ausbeute: | 20 mg (23% d.Th.) |
| ESI-MS: | m/z = 517 (M+H)⁺ |
| Rₜ (HPLC): | 1.28 min (Methode B) |

### Beispiel 49

### 3-(1-(6-(4-Amino-3-meth)phenoxy)pyrimidin-4-yl)piperidin-4-yl)-7-methoxy-4,5-dihydro-1H-benzo[d][1,3]diazepin-2(3H)-on

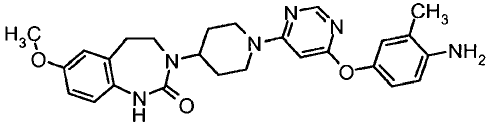

930 mg (1.84 mmol) 7-Methoxy-3-(1-(6-(3-methyl-4-nitrophenoxy)pyrimidin-4-yl)piperidin-4-yl)-4,5-dihydro-1H-benzo[d][1,3]diazepin-2(3H)-on wurden in 20 mL THF und 20 mL MeOH vorgelegt und unter einer Wasserstoffatmosphäre unter Zusatz von 150 mg Pd/C (10%ig) bei RT und einem Wasserstoffdruck von 3 bar bis zur vollständigen Wasserstoffaufnahme hydriert. Der Katalysator wurde abfiltriert. Vom Filtrat wurde alles bis auf ein Restvolumen von 15 mL abdestilliert. Es wurde DIPE zugegeben. Der Niederschlag wurde abgesaugt, nachgewaschen und getrocknet.

| | |
|---|---|
| Ausbeute: | 680 mg (78% d.Th.) |
| ESI-MS: | m/z = 475 (M+H)⁺ |
| Rₜ(HPLC): | 1.25 min (Methode B) |

### Beispiel 50

### 3-(1-(6-(7-Chlor-2-methyl-1H-benzo[d]imidazol-5-yloxy)pyrimidin-4-yl)piperidin-4-yl)-7-methoxy-4,5-dihydro-1H-benzo[d][1,3]diazepin-2(3H)-on

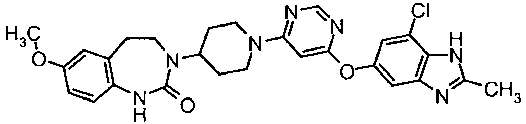

100 mg (0.36 mmol) 7-Methoxy-3-piperidin-4-yl-1,3,4,5-tetrahydro-1,3-benzodiazepin-2-on, 110 mg (0.37 mmol) 7-Chlor-5-(6-chlorpyrimidin-4-yloxy)-2-methyl-1 H-benzo[d]-imidazol und 0.14 mL (0.81 mmol) DIPEA in 2.0 mL DMF wurden 3 h bei RT und 4 h bei 40°C gerührt. Das Reaktionsgemisch wurde mit Wasser versetzt. Der ausgefallene Niederschlag wurde abgesaugt, nachgewaschen und getrocknet. Die Substanz wurde mittels präparativer HPLC-MS gereinigt. Die Produkt enthaltenden Fraktionen wurden vereint und gefriergetrocknet.

| | |
|---|---|
| Ausbeute: | 90 mg (46% d.Th.) |
| ESI-MS: | m/z = 534/536 (Cl) (M+H)⁺ |
| Rₜ(HPLC): | 1.29 min (Methode B) |

### Beispiel 51

### 1-(6-(7-Chlor-2-methyl-1H-benzo[d]imidazol-5-yloxy)pyrimidin-4-yl)spiro[piperidin-4,4'-pyrido[2,3-d][1,3]oxazin]-2'(1'H)-on

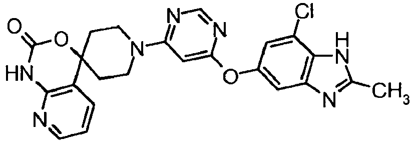

90 mg (0.35 mmol) Spiro[piperidin-4,4'-pyrido[2,3-d][1,3]oxazin]-2'(1'H)-on Hydrochlorid, 110 mg (0.37 mmol) 7-Chlor-5-(6-chlorpyrimidin-4-yloxy)-2-methyl-1 H-benzo[d]imidazol und 0.14 mL (0.81 mmol) DIPEA in 2.0 mL DMF wurden 3 h bei RT und 4 h bei 40°C gerührt. Das Reaktionsgemisch wurde mittels präparativer HPLC-MS gereinigt. Die Produkt enthaltenden Fraktionen wurden vereinigt und gefriergetrocknet.

| | |
|---|---|
| Ausbeute: | 50 mg (30% d.Th.) |
| ESI-MS: | m/z = 478/480 (Cl) (M+H)⁺ |
| Rₜ (HPLC): | 1.07 min (Methode B) |

### Beispiel 52

### (S)-1-(1-(6-(4-Methyl-2-(tetrahydrofuran-2-yl)-1H-benzo[d]imidazol-6-yloxy)pyrimidin-4-yl)-piperidin-4-yl)-1H-imidazo[4,5-b]pyridin-2(3H)-on

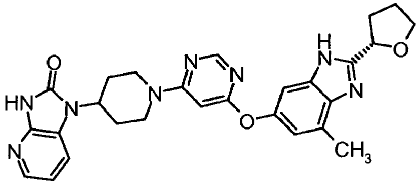

50 mg (0.23 mmol) 1-Piperidin-4-yl-1,3-dihydroimidazo[4,5-*b*]pyridin-2-on, 70 mg (0.21 mmol) (*S*)-6-(6-Chlorpyrimidin-4-yloxy)-4-methyl-2-(tetrahydrofuran-2-yl)-1H-benzo[d]-imidazol und 0.08 mL (0.46 mmol) DIPEA in 2.0 mL DMF wurden über Nacht bei RT und 2 h bei 40°C gerührt. Das Reaktionsgemisch wurde mittels präparativer HPLC-MS gereinigt. Die Produkt enthaltenden Fraktionen wurden teilweise i.vac. eingeengt und mit 1 M Natronlauge neutralisiert. Der ausgefallene Niederschlag wurde abgesaugt, nachgewaschen und getrocknet.

| | |
|---|---|
| Ausbeute: | 36 mg (31% d.Th.) |
| ESI-MS: | m/z = 513 (M+H)⁺ |
| Rₜ (HPLC): | 1.13 min (Methode B) |

### Beispiel 53

### (S)-7-Methoxy-3-(1-(6-(4-methyl-2-(tetrahydrofuran-2-yl)-1H-benzo[d]imidazol-6-yloxy)-pyrimidin-4-yl)piperidin-4-yl)-4,5-dihydro-1H-benzo[d][1,3]diazepin-2(3H)-on

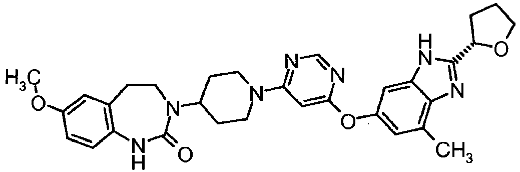

63 mg (0.23 mmol) 7-Methoxy-3-piperidin-4-yl-1,3,4,5-tetrahydro-1,3-benzodiazepin-2-on, 70 mg (0.21 mmol) (*S*)-6-(6-Chlorpyrimidin-4-yloxy)-4-methyl-2-(tetrahydrofuran-2-yl)-1H-benzo[d]imidazol und 0.08 mL (0.46 mmol) DIPEA in 2.0 mL DMF wurden über Nacht bei RT und 2 h bei 40°C gerührt. Das Reaktionsgemisch wurde mittels präparativer HPLC-MS gereinigt. Die Produkt enthaltenden Fraktionen wurden teilweise i.vac. eingeengt und mit 4M Natronlauge neutralisiert. Der ausgefallene Niederschlag wurde abgesaugt, nachgewaschen und getrocknet.

| | |
|---|---|
| Ausbeute: | 22 mg (17% d.Th.) |
| ESI-MS: | m/z = 570 (M+H)⁺ |
| Rₜ (HPLC): | 3.15 min (Methode C) |

### Beispiel 54

### (S)-1-(6-(4-Methyl-2-(tetrahydrofuran-2-yl)-1H-benzo[d]imidazol-6-yloxy)pyrimidin-4-yl)-spiro[piperidin-4,4'-pyrido[2,3-d][1,3]oxazin]-2'(1'H)-on

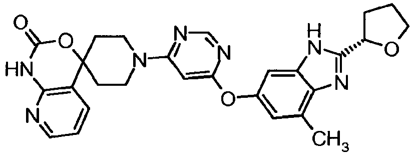

59 mg (0.23 mmol) Spiro[piperidin-4,4'-pyrido[2,3-d][1,3]oxazin]-2'(1'H)-on Hydrochlorid, 70 mg (0.21 mmol) (*S*)-6-(6-Chlorpyrimidin-4-yloxy)-4-methyl-2-(tetrahydrofuran-2-yl)-1H-benzo[d]imidazol und 0.12 mL (0.70 mmol) DIPEA in 2.0 mL DMF wurden über Nacht bei 40°C gerührt. Das Reaktionsgemisch wurde mittels präparativer HPLC-MS gereinigt. Die Produkt enthaltenden Fraktionen wurden teilweise i.vac. eingeengt und mit 4M Natronlauge neutralisiert. Der ausgefallene Niederschlag wurde abgesaugt, nachgewaschen und getrocknet.

| | |
|---|---|
| Ausbeute: | 21 mg (18% d.Th.) |
| ESI-MS: | m/z = 514 (M+H)⁺ |
| Rₜ (HPLC): | 1.09 min (Methode E |

### Beispiel 55

### 7-Methoxy-3-(1-(6-(2-methyl-1H-benzo[d]imidazol-5-yloxy)pyrimidin-4-yl)piperidin-4-yl)-4,5-dihydro-1H-benzo[d][1,3]diazepin-2(3H)-on

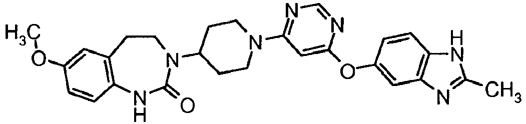

90 mg (0.17 mmol) 3-(1-(6-(4-Chlor-2-methyl-1 H-benzo[d]imidazol-5-yloxy)pyrimidin-4-yl)-piperidin-4-yl)-7-methoxy-4,5-dihydro-1H-benzo[d][1,3]diazepin-2(3H)-on und 0.10 mL (0.71 mmol) TEA wurden in 10 mL Methanol vorgelegt und unter Wasserstoff-Atmosphäre und unter Zusatz von 10 mg Pd/C (10%ig) bei 50°C und einem Wasserstoffdruck von 3 bar bis zur beendeten Wasserstoffaufnahme hydriert. Der Katalysator wurde abfiltriert und das Filtrat i.vac. eingeengt. Der Rückstand wurde mittels präparativer HPLC-MS gereinigt. Die Produkt enthaltenden Fraktionen wurden vereinigt und gefriergetrocknet.

| | |
|---|---|
| Ausbeute: | 10 mg (12% d.Th.) |
| ESI-MS: | m/z = 500 (M+H)⁺ |
| Rₜ(HPLC): | 2.12 (Methode J) |

### Beispiel 56

### 3-(1-(6-(4-Chlor-2-methyl-1H-benzo[d]imidazol-5-yloxy)pyrimidin-4-yl)piperidin-4-yl)-7-methoxy-4,5-dihydro-1H-benzo[d][1,3]diazepin-2(3H)-on

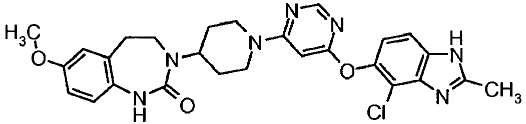

100 mg (0.36 mmol) 7-Methoxy-3-piperidin-4-yl-1,3,4,5-tetrahydro-1,3-benzodiazepin-2-on, 110 mg (0.37 mmol) 4-Chlor-5-(6-chlorpyrimidin-4-yloxy)-2-methyl-1 H-benzo[d]-imidazol und 0.14 mL (0.81 mmol) DIPEA in 2.0 mL DMF wurden 3 h bei RT und 4 h bei 40°C gerührt. Das Reaktionsgemisch wurde mit Wasser versetzt. Der ausgefallene Niederschlag wurde abgesaugt, nachgewaschen und getrocknet. Das Filtrat wurde nochmals abgesaugt, nachgewaschen und getrocknet.

| | |
|---|---|
| Ausbeute: | 190 mg (98% d.Th.) |
| ESI-MS: | m/z = 534/536 (Cl) (M+H)⁺ |
| Rₜ(HPLC): | 1.25 min (Methode B) |

### Beispiel 57

### 7-(6-(4-(7-Methoxy-2-oxo-4,5-dihydro-1H-benzo[d][1,3]diazepin-3(2H)-yl)piperidin-1-yl)-pyrimidin-4-yloxy)-5-methyl-2H-benzo[b][1,4]oxazin-3(4H)-on

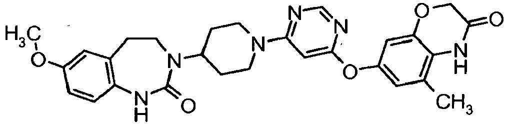

70 mg (0.25 mmol) 7-Methoxy-3-piperidin-4-yl-1,3,4,5-tetrahydro-1,3-benzodiazepin-2-on, 65 mg (0.20 mmol) 7-(6-Chlorpyrimidin-4-yloxy)-5-methyl-2H-benzo[b][1,4]oxazin-3(4H)-on und 50 µL (0.27 mmol) DIPEA in 2.0 mL DMF wurden über Nacht bei RT und 3 h bei 40°C gerührt. Das Reaktionsgemisch wurde mit Wasser und Methanol versetzt. Der ausgefallene Niederschlag wurde abgesaugt, nachgewaschen und getrocknet. Das Filtrat wurde nochmals abgesaugt, nachgewaschen und getrocknet.

| | |
|---|---|
| Ausbeute: | 69 mg (62% d.Th.) |
| ESI-MS: | m/z = 531 (M+H)⁺ |
| Rₜ(HPLC): | 3.65 (Methode C) |

### Beispiel 58

### 1-(6-(7-Methyl-2-(tetrahydrofuran-3-yl)-1H-benzo[d]imidazol-5-yloxy)pyrimidin-4-yl)spiro-[piperidin-4,4'-pyrido[2,3-d][1,3]oxazin]-2'(1'H)-on

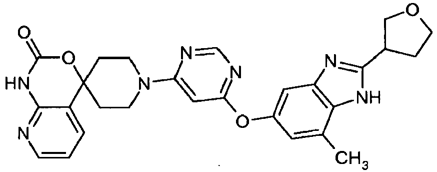

138 mg (0.54 mmol) Spiro[piperidin-4,4'-pyrido[2,3-d][1,3]oxazin]-2'(1'H)-on Hydrochlorid, 180 mg (0.54 mmol) 5-(6-Chlorpyrimidin-4-yloxy)-7-methyl-2-(tetrahydrofuran-3-yl)-1H-benzo[d]imidazol und 280 µL (1.62 mmol) DIPEA in 5.0 mL DMF wurden über Nacht bei 60°C gerührt. Der Reaktionsansatz wurde mit Eiswasser versetzt. Der ausgefallene Niederschlag wurde abgesaugt und chromatographisch gereinigt. Die Produkt enthaltenden Fraktionen wurden vereinigt und i.vac. bis zum wässrigen Rückstand eingeengt. Dieser wurde mit einer wässrigen NaHCO₃-Lösung neutralisiert und mit Essigsäureethylester extrahiert. Die organische Phase wurde getrocknet und i.vac. eingeengt.

| | |
|---|---|
| Ausbeute: | 50 mg (18% d.Th.) |
| ESI-MS: | m/z = 514 (M+H)⁺ |
| Rₜ(HPLC): | 2.88 min (Methode L) |

### Beispiel 59

### (S)-1-(6-(7-Methyl-2-(tetrahydrofuran-3-yl)-1H-benzo[d]imidazol-5-yloxy)pyrimidin-4-yl)-spiro[piperidin-4,4'-pyrido[2,3-d][1,3]oxazin]-2'(1'H)-on

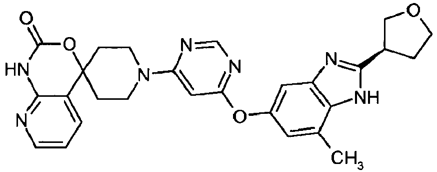

30 mg (60 mmol) 1-(6-(7-Methyl-2-(tetrahydrofuran-3-yl)-1H-benzo[d]imidazol-5-yloxy)-pyrimidin-4-yl)spiro[piperidin-4,4'-pyrido[2,3-d][1,3]oxazin]-2'(1'H)-on wurden mit einer chiralen HPLC (Methode Daicel OJH, 250 mm x 4.6 mm, 4 ml/min, 10 min, 25% MeOH mit DEA) aufgetrennt. Die Produkt enthaltenden Fraktionen wurden vereinigt und i.vac. eingeengt. Der Rückstand wurde in MeOH/H₂O gelöst und lyophilisiert.

| | |
|---|---|
| Ausbeute: | 10 mg |
| ESI-MS: | m/z = 514 (M+H)⁺ |
| Rₜ(HPLC): | 4.29 min (Methode Daicel OJH, 250 mm x 4.6 mm, 4 ml/min, 10min, 25% MeOH mit DEA) |

### Beispiel 60

### (R)-1-(6-(7-Methyl-2-(tetrahydrofuran-3-yl)-1H-benzo[d]imidazol-5-yloxy)pyrimidin-4-yl)-spiro[piperidin-4,4'-pyrido[2,3-d][1,3]oxazin]-2'(1'H)-on

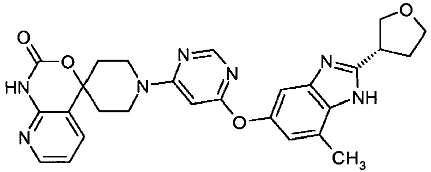

30 mg (60 mmol) 1-(6-(7-Methyl-2-(tetrahydrofuran-3-yl)-1 H-benzo[d]imidazol-5-yloxy)-pyrimidin-4-yl)spiro[piperidin-4,4'-pyrido[2,3-d][1,3]oxazin]-2'(1'H)-on wurden mit einer chiralen HPLC (Methode Daicel OJH, 250 mm x 4.6 mm, 4 ml/min, 10 min, 25% MeOH mit DEA) aufgetrennt. Die Produkt enthaltenden Fraktionen wurden vereinigt und i.vac. eingeengt. Der Rückstand wurde in MeOH/H₂O gelöst und lyophilisiert.

| | |
|---|---|
| Ausbeute: | 8 mg |
| ESI-MS: | m/z = 514 (M+H)⁺ |
| Rₜ(HPLC): | 5.53 min (Methode Daicel OJH, 250 mm x 4.6 mm, 4 ml/min, 10 min, 25% MeOH mit DEA) |

### Beispiel 61

### 1-(6-(7-Chlor-2-(tetrahydrofuran-3-yl)-1H-benzo[d]imidazol-5-yloxy)pyrimidin-4-yl)spiro-[piperidin-4,4'-pyrido[2,3-d][1,3]oxazin]-2'(1'H)-on

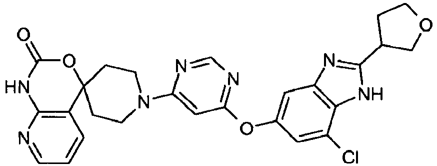

36 mg (0.14 mmol) Spiro[piperidin-4,4'-pyrido[2,3-d][1,3]oxazin]-2'(1'H)-on Hydrochlorid, 50 mg (0.14 mmol) 7-Chlor-5-(6-chlorpyrimidin-4-yloxy)-2-(tetrahydrofuran-3-yl)-1 H-benzo[d]imidazol und 50 µL (0.30 mmol) DIPEA in 2.0 mL DMF wurden über Nacht bei RT gerührt. Der Reaktionsansatz wurde chromatographisch gereinigt. Die Produkt enthaltenden Fraktionen wurden vereinigt und lyophilisiert.

| | |
|---|---|
| Ausbeute: | 38 mg (51% d.Th.) |
| ESI-MS: | m/z = 534 (M+H)⁺ |
| Rₜ(HPLC): | 1.04 min (Methode B) |

### Beispiel 62

### 1-(6-(7-Chlor-2-(tetrahydro-2H-pyran-4-yl)-1H-benzo[d]imidazol-5-yloxy)pyrimidin-4-yl)-spiro(piperidin-4,4'-pyrido[2,3-d][1,3]oxazin]-2'(1'H)-on

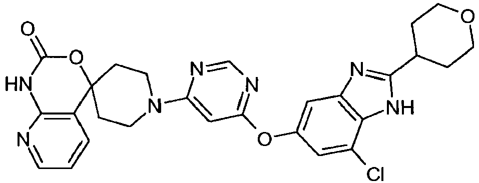

49 mg (0.19 mmol) Spiro[piperidin-4,4'-pyrido[2,3-d][1,3]oxazin]-2'(1'H)-on Hydrochlorid, 70 mg (0.19 mmol) 7-Chlor-5-(6-chlorpyrimidin-4-yloxy)-2-(tetrahydro-2H-pyran-4-yl)-1H-benzo[d]imidazol und 70 µL (0.40 mmol) DIPEA in 2.0 mL DMF wurden über 3 h bei RT und 3 h bei 40°C gerührt. Der Reaktionsansatz wurde chromatographisch gereinigt. Die Produkt enthaltenden Fraktionen wurden vereinigt und i.vac. eingeengt.

| | |
|---|---|
| Ausbeute: | 8 mg (8% d.Th.) |
| ESI-MS: | m/z = 548 (M+H)⁺ |
| Rₜ(HPLC): | 1.07 min (Methode B) |

### Beispiel 63

### 1-(6-(7-Methyl-2-(trifluormethyl)-1H-benzo[d]imidazol-5-yloxy)pyrimidin-4-yl)spiro-[piperidin-4,4'-pyrido[2,3-d][1,3]oxazin]-2'(1'H)-on

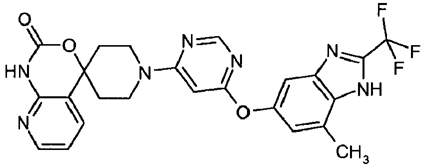

21 mg (0.08 mmol) Spiro[piperidin-4,4'-pyrido[2,3-d][1,3]oxazin]-2'(1'H)-on Hydrochlorid, 22 mg (0.07 mmol) 5-(6-Chlorpyrimidin-4-yloxy)-7-methyl-2-(trifluormethyl)-1H-benzo[d]-imidazol und 40 µL (0.24 mmol) DIPEA in 2.0 mL DMF wurden über das Wochenende bei 50°C gerührt. Der Reaktionsansatz wurde chromatographisch gereinigt. Die Produkt enthaltenden Fraktionen wurden vereinigt und i.vac. bis zum wässrigen Rückstand eingeengt. Dieser wurde mit einer wässrigen 1 M NaOH Lösung neutralisiert. Der ausgefallene Niederschlag wurde abgesaugt, nachgewaschen und getrocknet.

| | |
|---|---|
| Ausbeute: | 18 mg (44% d.Th.) |
| ESI-MS: | m/z = 512 (M+H)⁺ |
| Rₜ(HPLC): | 1.30 min (Methode B) |

### Beispiel 64

### 1-(6-(4-Methyl-2-oxo-2,3-dihydrobenzo[d]thiazol-6-yloxy)pyrimidin-4-yl)spiro[piperidin-4,4'-pyrido[2,3-d][1,3]oxazin]-2'(1'H)-on

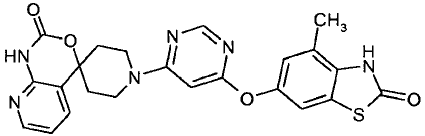

90 mg (0.35 mmol) Spiro[piperidin-4,4'-pyrido[2,3-d][1,3]oxazin]-2'(1'H)-on Hydrochlorid, 105 mg (0.36 mmol) 6-(6-Chlorpyrimidin-4-yloxy)-4-methylbenzo[d]thiazol-2(3H)-on und 200 µL (1.16 mmol) DIPEA in 2.0 mL DMF wurden 14 h bei 60°C gerührt. Der Reaktionsansatz wurde abgekühlt, mit Wasser versetzt und 30 min gerührt. Der ausgefallene Niederschlag wurde abgesaugt, mit Methanol verrührt, nochmals abgesaugt und getrocknet.

| | |
|---|---|
| Ausbeute: | 120 mg (72% d.Th.) |
| ESI-MS: | m/z = 477 (M+H)⁺ |

| | |
|---|---|
| Rₜ(HPLC): | 3.25 min (Methode C) |

### Beispiel 65

### 1-(6-(7-Methyl-2-(tetrahydro-2H-pyran-4-yl)-1H-benzo[d]imidazol-5-yloxy)pyrimidin-4-yl)-spiro[piperidin-4,4'-pyrido[2,3-d][1,3]oxazin]-2'(1'H)-on

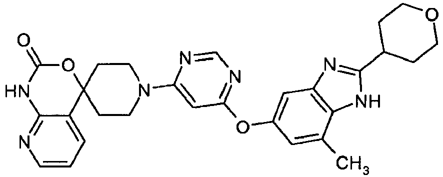

33 mg (0.13 mmol) Spiro[piperidin-4,4'-pyrido[2,3-d][1,3]oxazin]-2'(1'H)-on Hydrochlorid, 40 mg (0.12 mmol) 7-Methyl-5-(6-chlorpyrimidin-4-yloxy)-2-(tetrahydro-2H-pyran-4-yl)-1 H-benzo[d]imidazol und 70 µL (0.39 mmol) DIPEA in 2.0 mL DMF wurden über Nacht bei 60°C gerührt. Der Reaktionsansatz wurde auf Eiswasser gegossen und der ausgefallene Niederschlag wurde abgesaugt. Der Niederschlag wurde gelöst und anschließend chromatographisch gereinigt. Die Produkt enthaltenden Fraktionen wurden vereinigt und i.vac. bis zum wässrigen Rückstand eingeengt. Dieser wurde mit einer wässrigen NaHCO₃ Lösung neutralisiert. Der ausgefallene Niederschlag wurde abgesaugt, nachgewaschen und getrocknet.

| | |
|---|---|
| Ausbeute: | 35 mg (51% d.Th.) |
| ESI-MS: | m/z = 528 (M+H)⁺ |
| Rₜ(HPLC): | 0.96 min (Methode B) |

### Beispiel 66

### 1-(6-(2-(2-Methoxyethyl)-4-methyl-1H-benzo[d]imidazol-6-yloxy)pyrimidin-4-yl)spiro-[piperidin-4,4'-pyrido[2,3-d][1,3]oxazin]-2'(1'H)-on

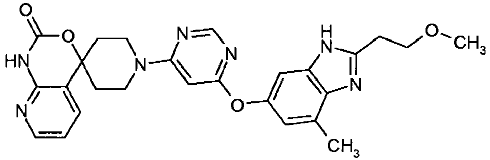

29 mg (0.11 mmol) Spiro[piperidin-4,4'-pyrido[2,3-d][1,3]oxazin]-2'(1'H)-on Hydrochlorid, 29 mg (0.09 mmol) 6-(6-Chlorpyrimidin-4-yloxy)-2-(2-methoxyethyl)-4-methyl-1H-benzo-[d]imidazol und 50 µL (0.39 mmol) DIPEA in 2.0 mL DMF wurden über Nacht bei 50°C gerührt. Der Reaktionsansatz wurde chromatographisch gereinigt. Die Produkt enthaltenden Fraktionen wurden vereinigt und i.vac. bis zum wässrigen Rückstand eingeengt. Dieser wurde mit einer wässrigen 1 M NaOH Lösung neutralisiert. Der ausgefallene Niederschlag wurde abgesaugt, nachgewaschen und getrocknet.

| | |
|---|---|
| Ausbeute: | 55 mg (51% d.Th.) |
| ESI-MS: | m/z = 502 (M+H)⁺ |
| Rₜ(HPLC): | 2.72 min (Methode C) |

### Beispiel 67

### 1-(6-(2-Methoxy-7-methyl-1H-benzo[d]imidazol-5-yloxy)pyrimidin-4-yl)spiro[piperidin-4,4'-pyrido[2,3-d][1,3]oxazin]-2'(1'H)-on

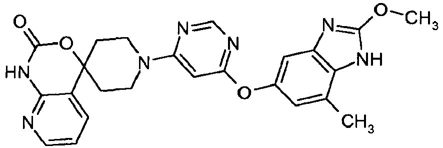

64 mg (0.25 mmol) Spiro[piperidin-4,4'-pyrido[2,3-d][1,3]oxazin]-2'(1'H)-on Hydrochlorid, 57 mg (0.20 mmol) 5-(6-Chlorpyrimidin-4-yloxy)-2-methoxy-7-methyl)-1H-benzo[d]-imidazol und 130 µL (0.75 mmol) DIPEA in 2.0 mL DMF wurden über Nacht bei 60°C gerührt. Der Reaktionsansatz wurde chromatographisch gereinigt. Die Produkt enthaltenden Fraktionen wurden vereinigt und i.vac. bis zum wässrigen Rückstand eingeengt. Dieser wurde mit einer wässrigen 1 M NaOH Lösung neutralisiert. Der ausgefallene Niederschlag wurde abgesaugt, nachgewaschen und getrocknet.

| | |
|---|---|
| Ausbeute: | 20 mg (17% d.Th.) |
| ESI-MS: | m/z = 474 (M+H)⁺ |
| Rₜ(HPLC): | 1.10 min (Methode B) |

### Beispiel 68

### 1-(6-(7-Methyl-1H-indazol-5-yloxy)pyrimidin-4-yl)spiro[piperidin-4,4'-pyrido[2,3-d]-[1,3]oxazin]-2'(1'H)-on

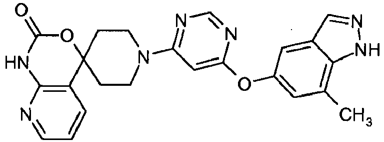

60 mg (0.23 mmol) Spiro[piperidin-4,4'-pyrido[2,3-d][1,3]oxazin]-2'(1'H)-on Hydrochlorid, 60 mg (0.23 mmol) 5-(6-Chlorpyrimidin-4-yloxy)-7-methyl-1 H-indazol und 100 µL (0.58 mmol) DIPEA in 0.8 mL DMF wurden über das Wochenende bei RT gerührt. Der Reaktionsansatz wurde chromatographisch gereinigt. Die Produkt enthaltenden Fraktionen wurden vereinigt und i.vac. bis zum wässrigen Rückstand eingeengt. Dieser wurde mit einer wässrigen gesättigten NaHCO₃ Lösung neutralisiert. Der ausgefallene Niederschlag wurde abgesaugt und getrocknet.

| | |
|---|---|
| Ausbeute: | 40 mg (38% d.Th.) |
| ESI-MS: | m/z = 444 (M+H)⁺ |
| Rₜ(HPLC): | 3.06 min (Methode C) |

### Beispiel 69

### 5-Methyl-7-(6-(2'-oxo-1',2'-dihydrospiro[piperidin-4,4'-pyrido[2,3-d][1,3]oxazin]-1-yl)-pyrimidin-4-yloxy)-2H-benzo[b][1,4]oxazin-3(4H)-on

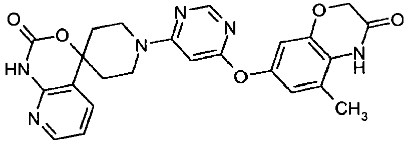

72 mg (0.28 mmol) Spiro[piperidin-4,4'-pyrido[2,3-d][1,3]oxazin]-2'(1'H)-on Hydrochlorid, 70 mg (0.22 mmol) 7-(6-Chlorpyrimidin-4-yloxy)-5-methyl-2H-benzo[b][1,4]oxazin-3(4H)-on und 100 µL (0.58 mmol) DIPEA in 2.0 mL DMF wurden über Nacht bei RT gerührt. Der Reaktionsansatz wurde mit Methanol und Wasser versetzt. Der ausgefallene Niederschlag wurde abgesaugt, mit Methanol gewaschen und getrocknet.

| | |
|---|---|
| Ausbeute: | 75 mg (73% d.Th.) |
| ESI-MS: | m/z = 475 (M+H)⁺ |
| Rₜ(HPLC): | 1.18 min (Methode B) |

### Beispiel 70

### (R)-1-(6-(4-Methyl-2-(tetrahydrofuran-2-yl)-1H-benzo[d]imidazol-6-yloxy)pyrimidin-4-yl)-spiro[piperidin-4,4'-pyrido[2,3-d][1,3]oxazin]-2'(1'H)-on

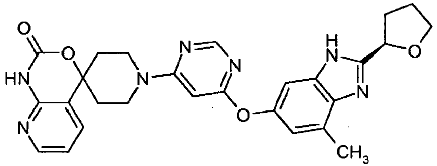

166 mg (0.65 mmol) Spiro[piperidin-4,4'-pyrido[2,3-d][1,3]oxazin]-2'(1'H)-on Hydrochlorid, 205 mg (0.62 mmol) (R)-6-(6-Chlorpyrimidin-4-yloxy)-4-methyl-2-(tetrahydrofuran-2-yl)-1 H-benzo[d]imidazol und 320 µL (1.86 mmol) DIPEA in 5.0 mL DMF wurden über Nacht bei 50°C gerührt. Der Reaktionsansatz wurde chromatographisch gereinigt. Die Produkt enthaltenden Fraktionen wurden vereinigt und i.vac. bis zum wässrigen Rückstand eingeengt. Dieser wurde mit einer wässrigen NaHCO₃-Lösung neutralisiert. Der ausgefallene Niederschlag wurde abgesaugt, gewaschen und getrocknet.

| | |
|---|---|
| Ausbeute: | 110 mg (35% d.Th.) |
| ESI-MS: | m/z = 514 (M+H)⁺ |
| Rₜ(HPLC): | 0.98 min (Methode B) |

### Beispiel 71

### 4-Methyl-6-(2-methyl-6-(2'-oxo-1',2'-dihydrospiro[piperidin-4,4'-pyrido[2,3-d][1,3]oxazin]-1-yl)pyrimidin-4-yloxy)benzo[d]oxazol-2(3H)-on

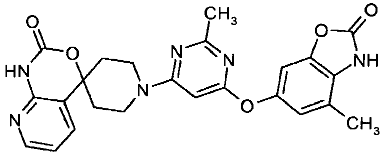

90 mg (0.35 mmol) Spiro[piperidin-4,4'-pyrido[2,3-d][1,3]oxazin]-2'(1'H)-on Hydrochlorid, 90 mg (0.31 mmol) 6-(6-Chlor-2-methylpyrimidin-4-yloxy)-4-methylbenzo[d]oxazol-2(3H)-on und 200 µL (1.16 mmol) DIPEA in 1.5 mL DMF wurden über Nacht bei RT gerührt. Es wurden nochmals 90 mg (0.35 mmol) Spiro[piperidin-4,4'-pyrido[2,3-d][1,3]oxazin]-2'(1'H)-on Hydrochlorid und 200 µL (1.16 mmol) DIPEA hinzugegeben und 8 h bei 60°C gerührt. Der Reaktionsansatz wurde mit Wasser versetzt und über Nacht gerührt. Anschließend wurde DCM hinzugegeben und weitere 30 min gerührt. DCM wurde entfernt und die wässrige Phase abgesaugt. Der Niederschlag wurde mit EtOH gewaschen und getrocknet.

| | |
|---|---|
| Ausbeute: | 100 mg (68% d.Th.) |
| ESI-MS: | m/z = 475 (M+H)⁺ |
| Rₜ(HPLC): | 1.04 min (Methode B) |

### Beispiel 72

### 6-(6-(4-(7-Methoxy-2-oxo-4,5-dihydro-1H-benzo[d][1,3]diazepin-3(2H)-yl)piperidin-1-yl)-pyrimidin-4-yloxy)-4-methylbenzo[d]thiazol-2(3H)-on

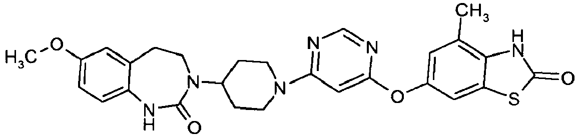

100 mg (0.36 mmol) 7-Methoxy-3-(piperidin-4-yl)-4,5-dihydro-1H-benzo[d][1,3]diazepin-2(3H)-on, 110 mg (0.36 mmol) 6-(6-Chlorpyrimidin-4-yloxy)-4-methylbenzo[d]thiazol-2(3H)-on und 140 µL (0.81 mmol) DIPEA in 2.0 mL DMF wurden 14 h bei 60°C gerührt. Der Reaktionsansatz wurde abgekühlt, mit Wasser versetzt und 30 min gerührt. Der ausgefallene Niederschlag wurde abgesaugt und getrocknet.

| | |
|---|---|
| Ausbeute: | 160 mg (72% d.Th.) |
| ESI-MS: | m/z = 533 (M+H)⁺ |
| Rₜ(HPLC): | 1.37 min (Methode B) |

### Beispiel 73

### 7-Methoxy-3-(1-(6-(4-methyl-2-(tetrahydrofuran-3-yl)-1H-benzo[d]imidazol-6-yloxy) pyrimidin-4-yl)piperidin-4-yl)-4,5-dihydro-1H-benzo[d][1,3]diazepin-2(3H)-on

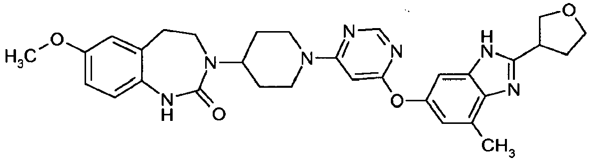

28 mg (0.10 mmol) 7-Methoxy-3-(piperidin-4-yl)-4,5-dihydro-1H-benzo[d][1,3]diazepin-2(3H)-on, 33 mg (0.10 mmol) 6-(6-Chlorpyrimidin-4-yloxy)-4-methyl-2-(tetrahydrofuran-3-yl)-1H-benzo[d]imidazol und 30 µL (0.20 mmol) DIPEA in 2.0 mL DMF wurden über das Wochenende bei 50°C gerührt. Der Reaktionsansatz wurde chromatographisch gereinigt. Die Produkt enthaltenden Fraktionen wurden vereinigt und i.vac. bis zum wässrigen Rückstand eingeengt. Dieser wurde mit einer wässrigen 4 M NaOH Lösung neutralisiert. Der ausgefallene Niederschlag wurde abgesaugt, gewaschen und getrocknet.

| | |
|---|---|
| Ausbeute: | 16 mg (28% d.Th.) |
| ESI-MS: | m/z = 570 (M+H)⁺ |
| Rₜ(HPLC): | 3.22 min (Methode C) |

### Beispiel 74

### 1-(6-(4-Methyl-2-oxo-2,3-dihydrobenzo[d]oxazol-6-yloxy)-2-(trifluormethyl)pyrimidin-4-yl)-spiro[piperidin-4,4'-pyrido[2,3-d][1,3]oxazin]-2'(1'H)-on

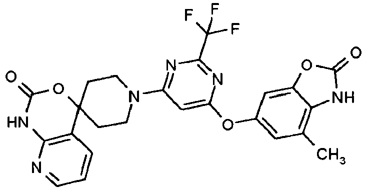

90 mg (0.35 mmol) Spiro[piperidin-4,4'-pyrido[2,3-d][1,3]oxazin]-2'(1'H)-on Hydrochlorid, 125 mg (0.36 mmol) 6-(6-Chlor-2-trifluormethyl-pyrimidin-4-yloxy)-4-methyl-3H-benzo-oxazol-2-on und 0.20 mL (1.16 mmol) DIPEA in 2.0 mL NMP wurden 14 h bei 60°C gerührt. Nach dem Erkalten wurde der Reaktionsansatz mit Wasser versetzt und 30 min gerührt. Der ausgefallene Niederschlag wurde abgesaugt und getrocknet.

| | |
|---|---|
| Ausbeute: | 0.18 mg (97% d.Th.) |
| ESI-MS: | m/z = 529 (M+H)⁺ |
| Rₜ(HPLC): | 1.40 min (Methode B) |

### Beispiel 75

### 4-(4-Methyl-2-oxo-2,3-dihydrobenzo[d]oxazol-6-yloxy)-6-(2'-oxo-1',2'-dihydrospiro-[piperidin-4,4'-pyrido[2,3-d][1,3]oxazin]-1-yl)nicotinonitril

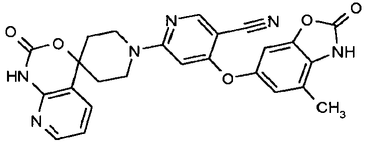

40 mg (0.16 mmol) Spiro[piperidin-4,4'-pyrido[2,3-d][1,3]oxazin]-2'(1'H)-on Hydrochlorid, 40 mg (0.12 mmol) 6-Chlor-4-(4-methyl-2-oxo-2,3-dihydro-benzooxazol-6-yloxy)-nicotino-nitril und 0.08 mL (0.47 mmol) DIPEA in 0.80 mL NMP wurden 1 h bei 140°C gerührt. Der Reaktionsansatz wurde mittels präparativer HPLC-MS gereinigt. Die Produkt enthaltenden Fraktionen wurden teilweise i.vac. eingeengt und mit wässriger NaHCO₃-Lösung neutralisiert. Der ausgefallene Niederschlag wurde abgesaugt und getrocknet.

| | |
|---|---|
| Ausbeute: | 47 mg (36% d.Th.) |
| ESI-MS: | m/z = 485 (M+H)⁺ |
| Rₜ(HPLC): | 1.34 min (Methode B) |

### Beispiel 76

### 4-Methyl-6-(2-methyl-6-(2-oxo-2,3-dihydro-1H-spiro[[1,8]naphthyridin-4,4'-piperidin]-1'-yl)pyrimidin-4-yloxy)benzo[d]oxazol-2(3H)-on

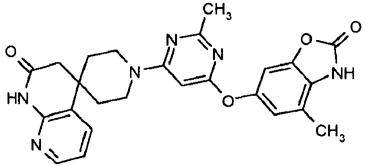

26 mg (0.12 mmol) 1H-spiro[[1,8]Naphthyridin-4,4'-piperidin]-2(3H)-on, 35 mg (0.12 mmol) 6-(6-Chlor-2-methylpyrimidin-4-yloxy)-4-methylbenzo[d]oxazol-2(3H)-on und 42 µL (0.24 mmol) DIPEA in 2.0 mL DMF wurden 4 Tage bei 60°C gerührt. Der Reaktionsansatz wurde mittels präparativer HPLC-MS gereinigt. Die Produkt enthaltenden Fraktionen wurden vereinigt und lyophilisiert.

| | |
|---|---|
| Ausbeute: | 19 mg (34% d.Th.) |
| ESI-MS: | m/z = 4739 (M+H)⁺ |
| Rₜ(HPLC): | 1.40 min (Methode B) |

### Beispiel 77

### 4-Methyl-6-(2-methyl-6-(2'-oxo-1',2'-dihydrospiro[piperidin-4,3'-pyrrolo[2,3-b]pyridinl-1-yl-pyrimidin-4-yloxy)benzo[d]oxazol-2(3H)-on

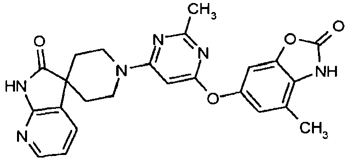

36 mg (0.15 mmol) Spiro[piperidin-4,3'-pyrrolo[2,3-b]pyridin]-2'(1'H)-on, 44 mg (0.15 mmol) 6-(6-Chlor-2-methylpyrimidin-4-yloxy)-4-methylbenzo[d]oxazol-2(3H)-on und 91 µL (0.536 mmol) DIPEA in 2.0 mL DMF wurden 48 h bei RT gerührt. Der Reaktionsansatz wurde mittels präparativer HPLC-MS gereinigt. Die Produkt enthaltenden Fraktionen wurden vereinigt und lyophilisiert.

| | |
|---|---|
| Ausbeute: | 0.13 mg (18% d.Th.) |
| ESI-MS: | m/z = 459 (M+H)⁺ |
| Rₜ(HPLC): | 1.06 min (Methode B) |

### Beispiel 78

### 1-{1-[6-(2,7-Dimethyl-3H-benzimidazol-5-yloxy)-pyrimidin-4-yl]-spiro[piperidin-4,4'-pyrido-[2,3-d][1,3]oxazin]-2'(1'H)-on

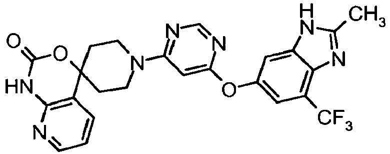

128 mg (0.500 mmol) Spiro[piperidin-4,4'-pyrido[2,3-d][1,3]oxazin]-2'(1'H)-on Hydrochlorid, 167 mg (0.500 mmol) 6-(6-Chlor-pyrimidin-4-yloxy)-2-methyl-4-trifluormethyl-1 H-benzimidazol und 0.261 mL (1.50 mmol) DIPEA in 1.5 mL DMF wurden 10 h bei RT gerührt. Das Reaktionsgemisch wurde mittels präparativer HPLC-MS gereinigt. Die Produkt enthaltenden Fraktionen wurden vereinigt und gefriergetrocknet.

| | |
|---|---|
| Ausbeute: | 65 mg (25% d.Th.) |
| ESI-MS: | m/z = 512 (M+H)⁺ |
| Rₜ(HPLC): | 3.0 min (Methode C) |

### Beispiel 79

### 7-Methoxy-3-{1-[6-(2-methyl-7-trifluormethyl-3H-benzimidazol-5-yloxy)-pyrimidin-4-yl]-piperidin-4-yl}1,3,4,5-tetrahydro-benzo[d][1,3]diazepin-2-on

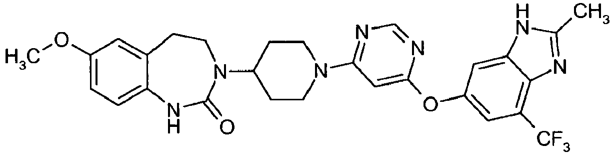

140 mg (0.510 mmol) 7-Methoxy-3-piperidin-4-yl-1,3,4,5-tetrahydro-1,3-benzodiazepin-2-on, 167 mg (0.500 mmol) 6-(6-Chlor-pyrimidin-4-yloxy)-2-methyl-4-trifluormethyl-1 H-benzimidazol und 0.261 mL (1.50 mmol) DIPEA in 1.5 mL DMF wurden 10 h bei RT gerührt. Das Reaktionsgemisch wurde mittels präparativer HPLC-MS gereinigt. Die Produkt enthaltenden Fraktionen wurden vereinigt und gefriergetrocknet.

| | |
|---|---|
| Ausbeute: | 106 mg (37% d.Th.) |
| ESI-MS: | m/z = 568 (M+H)⁺ |
| Rₜ(HPLC): | 3.5 min (Methode C) |

### Beispiel 80

### 7-Methoxy-3-[4'-(4-methyl-2-oxo-2,3-dihydro-benzooxazol-6-yloxy)-1'-oxy-3,4,5,6-tetra-hydro-2H-[1,2']bipyridinyl-4-yl]-1,3,4,5-tetrahydro-benzo[d][1,3]diazepin-2-on

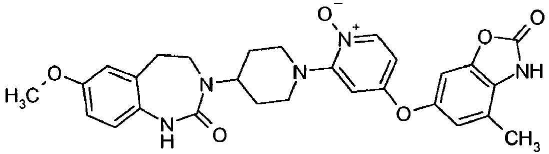

Ein Gemisch aus 75 mg (0.18 mmol) 7-Methoxy-3-(4'-nitro-1'-oxy-3,4,5,6-tetrahydro-2H-[1,2']bipyridinyl-4-yl)-1,3,4,5-tetrahydro-benzo[d][1,3]diazepin-2-on, 35 mg (0.21 mmol) 6-Hydroxy-4-methyl-3H-benzoxazol-2-on und 50 mg (0.44 mmol) Kalium-*tert*-butylat in 1.0 mL NMP wurde 20 min in der Mikrowelle bei 150°C gerührt. Der Reaktionsansatz wurde nach dem Abkühlen auf RT filtriert und mittels präparativer HPLC-MS gereinigt. Die Produkt enthaltenden Fraktionen wurden vereinigt und gefriergetrocknet.

| | |
|---|---|
| Ausbeute: | 3.0 mg (3% d.Th.) |
| ESI-MS: | m/z = 532 (M+H)⁺ |
| Rₜ(HPLC): | 1.31 min (Methode B) |

Die nachfolgenden Beispiele beschreiben die Herstellung pharmazeutischer Anwendungsformen, die als Wirkstoff eine beliebige Verbindung der allgemeinen Formel **I** enthalten:

### Beispiel I

Kapseln zur Pulverinhalation mit 1 mg Wirkstoff

### Zusammensetzung:

1 Kapsel zur Pulverinhalation enthält:

| | |
|---|---|
| Wirkstoff | 1.0 mg |
| Milchzucker | 20.0 mg |
| Hartgelatinekapseln | 50.0 mg |
| | 71.0 mg |

### Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel II

Inhalationslösung für Respimat^{®} mit 1 mg Wirkstoff

### Zusammensetzung:

### 1 Hub enthält:

| | |
|---|---|
| Wirkstoff | 1.0 mg |
| Benzalkoniumchlorid | 0.002 mg |
| Dinatriumedetat | 0.0075 mg |
| Wasser gereinigt ad | 15.0 µl |

### Herstellungsverfahren:

Der Wirkstoff und Benzalkoniumchlorid werden in Wasser gelöst und in Respimat^{®}-Kartuschen abgefüllt.

### Beispiel III

Inhalationslösung für Vernebler mit 1 mg Wirkstoff

### Zusammensetzung:

### 1 Fläschchen enthält:

| | |
|---|---|
| Wirkstoff | 0.1 g |
| Natriumchlorid | 0.18 g |
| Benzalkoniumchlorid | 0.002 g |
| Wasser gereinigt ad | 20.0 ml |

### Herstellungsverfahren:

Wirkstoff, Natriumchlorid und Benzalkoniumchlorid werden in Wasser gelöst.

### Beispiel IV

Treibgas-Dosieraerosol mit 1 mg Wirkstoff

### Zusammensetzung:

### 1 Hub enthält:

| | |
|---|---|
| Wirkstoff | 1.0 mg |
| Lecithin | 0.1 % |
| Treibgas ad | 50.0 µl |

### Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

### Beispiel V

Nasalspray mit 1 mg Wirkstoff

### Zusammensetzung:

| | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

### Herstellungsverfahren:

Der Wirkstoff und die Hilfsstoffe werden in Wasser gelöst und in ein entsprechendes Behältnis abgefüllt.

### Beispiel VI

Injektionslösung mit 5 mg Wirksubstanz pro 5 ml

### Zusammensetzung:

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel VII

Injektionslösung mit 100 mg Wirksubstanz pro 20 ml

### Zusammensetzung:

| | |
|---|---|
| Wirksubstanz | 100 mg |
| Monokaliumdihydrogenphosphat = KH₂PO₄ | 12 mg |
| Dinatriumhydrogenphosphat = Na₂HPO₄*2H₂O | 2 mg |
| Natriumchlorid | 180 mg |
| Human-Serum-Albumin | 50 mg |
| Polysorbat 80 | 20 mg |
| Wasser für Injektionszwecke ad | 10 ml |

### Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

### Beispiel VIII

Lyophilisat mit 10 mg Wirksubstanz

### Zusammensetzung:

| | |
|---|---|
| Wirksubstanz | 10 mg |
| Mannit | 300 mg |
| Human-Serum-Albumin | 20 mg |
| Wasser für Injektionszwecke ad | 2 ml |

### Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

Lösungsmittel für Lyophilisat:

| | |
|---|---|
| Polysorbat 80 = Tween 80 | 20 mg |
| Mannit | 200 mg |
| Wasser für Injektionszwecke ad | 10 ml |

### Herstellung:

Polysorbat 80 und Mannit in Wasser für Injektionszwecke (Wfl) auflösen; in Ampullen abfüllen.

### Beispiel IX

Tabletten mit 20 mg Wirksubstanz

### Zusammensetzung:

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

### Herstellung:

Wirksubstanz, Lactose und Maisstärke homogen mischen; mit einer wässrigen Lösung von Povidon granulieren; mit Magnesiumstearat mischen; auf einer Tablettenpresse abpressen; Tablettengewicht 200 mg.

### Beispiel X

Kapseln mit 20 mg Wirksubstanz

### Zusammensetzung:

| | |
|---|---|
| Wirksubstanz | 20 mg |
| Maisstärke | 80 mg |
| Kieselsäure, hochdispers | 5 mg |
| Magnesiumstearat | 2.5 mg |

### Herstellung:

Wirksubstanz, Maisstärke und Kieselsäure homogen mischen; mit Magnesiumstearat mischen; Mischung auf einer Kapselfüllmaschine in Hartgelatine-Kapseln Größe 3 abfüllen.

### Beispiel XI

Zäpfchen mit 50 mg Wirksubstanz

### Zusammensetzung:

| | |
|---|---|
| Wirksubstanz | 50 mg |
| Hartfett (Adeps solidus) q.s. ad | 1700 mg |

### Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgießen.

### Beispiel XII

Injektionslösung mit 10 mg Wirksubstanz pro 1 mL

### Zusammensetzung:

| | |
|---|---|
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

## Patentansprüche

1. Verbindungen der allgemeinen Formel 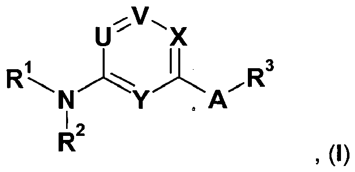 in der
**A** -NH, -N(C(O)-C₁₋₃-Alkyl)-, S, O, SO, SO₂,
**R¹** und **R²** zusammen mit dem Stickstoffatom, an das sie gebunden sind, eine Gruppe ausgewählt aus 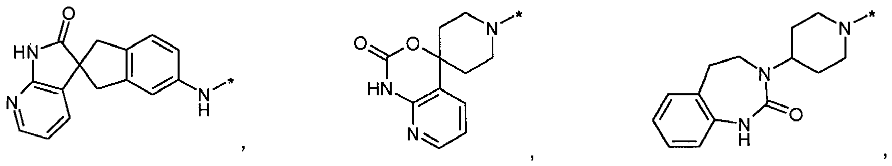 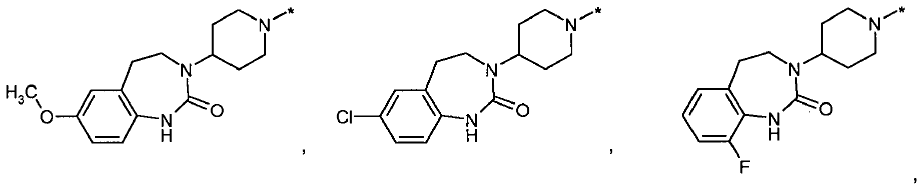 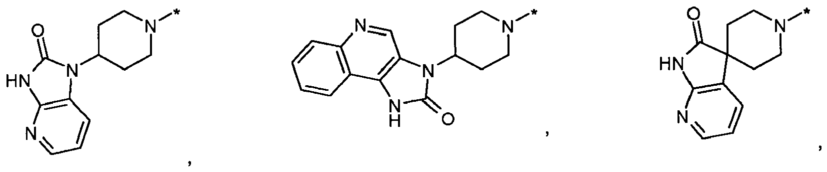 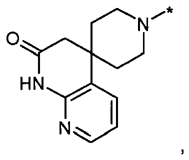 bedeutet,
**R³** eine Gruppe ausgewählt aus 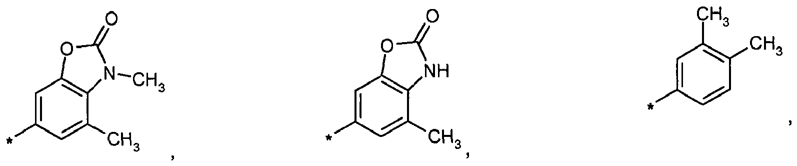 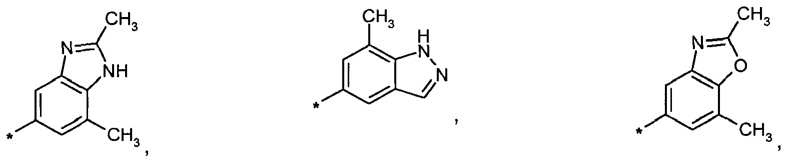 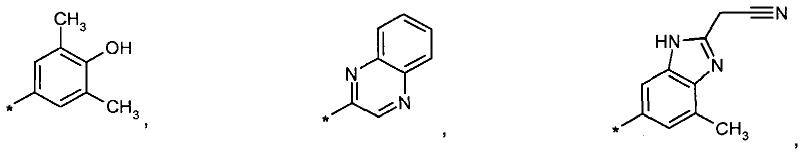 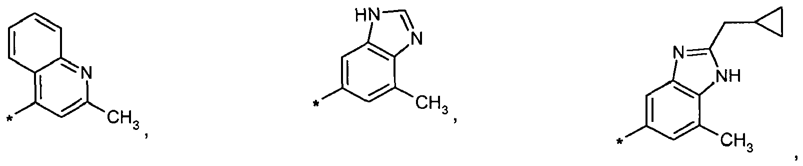 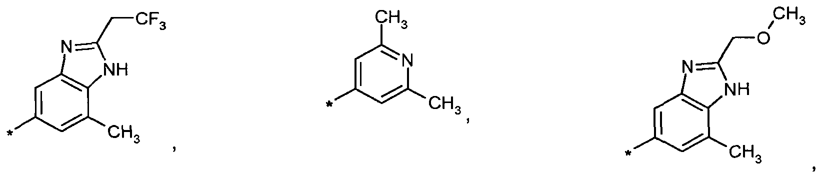 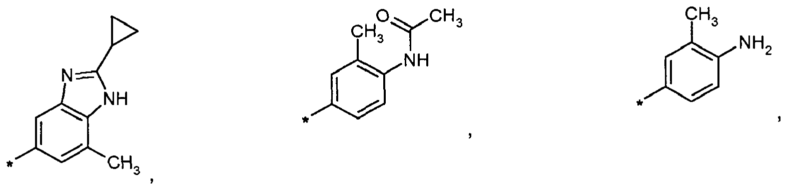 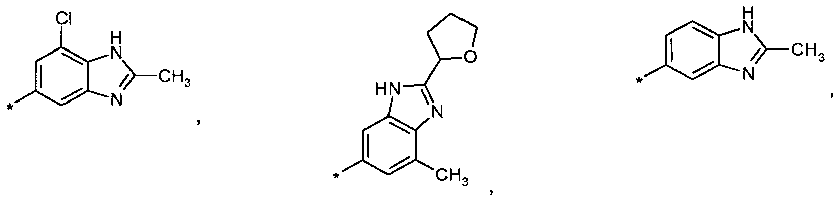 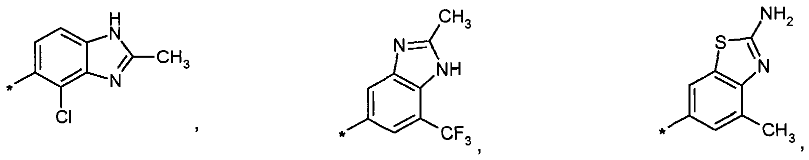 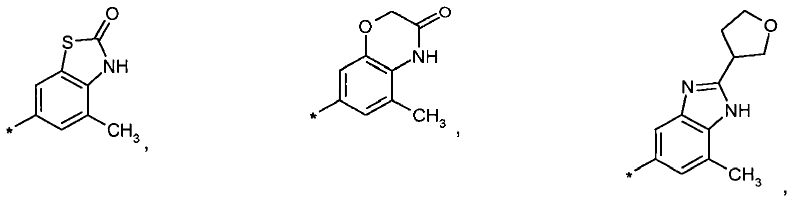 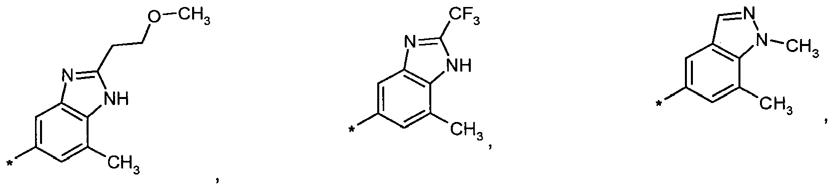 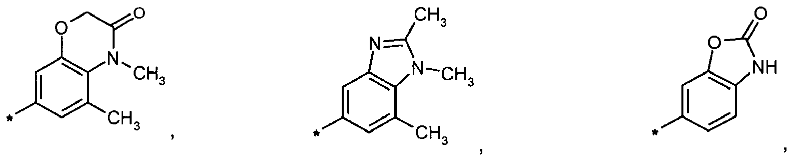 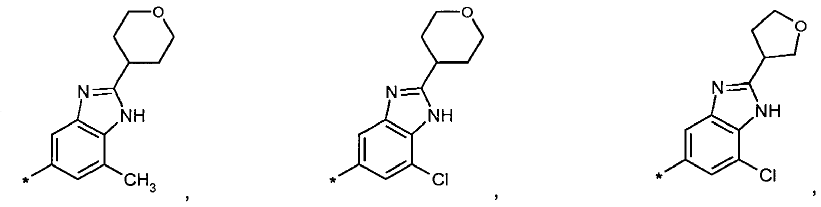 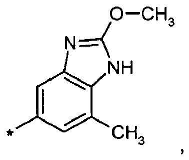 bedeutet,
**U-V-X** eine Gruppe ausgewählt aus
-N=(C-**R⁵**)-N=, -N=(C-**R⁵**)-(C-**R⁶**)=, -(N-Oxid)=(C-**R⁵**)-(C-**R⁶**)=, und
**Y** N oder CH,
**R⁵**
(a) H,
(b) -N**R^{5.1}R^{5.2}**,
(c) Halogen, C₃₋₆-Cycloalkyl-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
**R^{5.1}** H,
**R^{5.2}** H,
**R⁶**
(a) H,
(b) -CN
bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

2. Verbindungen der allgemeinen Formel **I** gemäß Anspruch 1, in denen **A, R¹**, **R²** und **R³** wie in Anspruch 1 definiert sind und der Ring 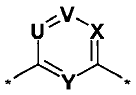 eine Gruppe ausgewählt aus 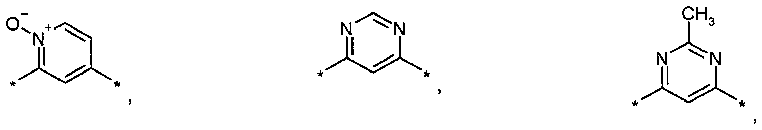 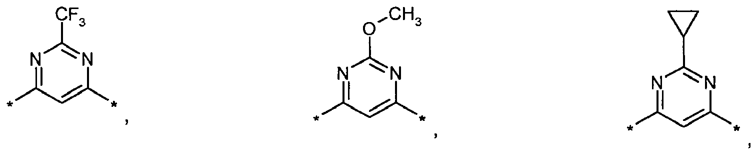 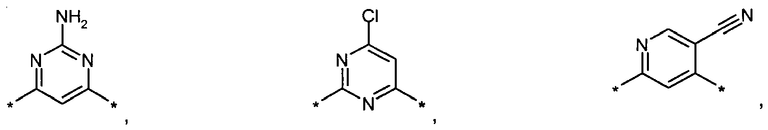 bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

3. Verbindungen der allgemeinen Formel **I** gemäß Anspruch 1, in denen **U, V, X, Y, R¹**, **R²** und **R³** wie in Anspruch 1 oder 2 definiert sind und
**A** -O- oder -NH- bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

4. Verbindungen der allgemeinen Formel **Ia** gemäß Anspruch 1 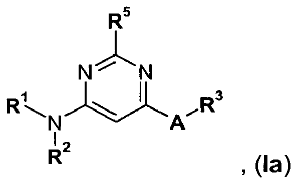 in der
**A** -NH- oder -O-,
**R¹** und **R²** zusammen mit dem Stickstoffatom, an das sie gebunden sind, eine Gruppe ausgewählt aus 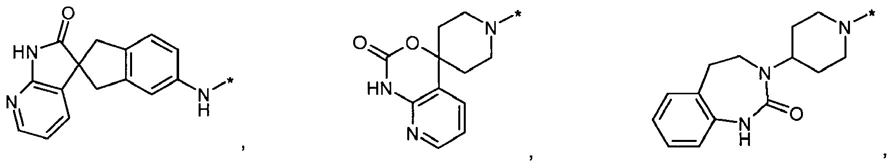 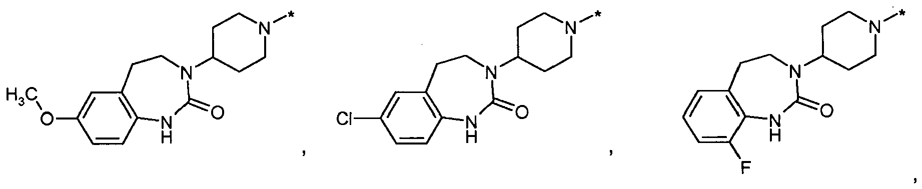 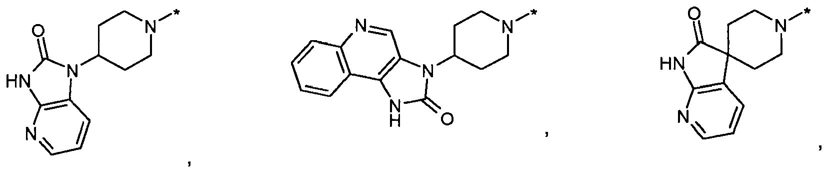 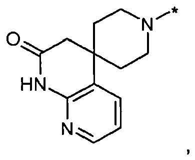
**R³** eine Gruppe ausgewählt aus 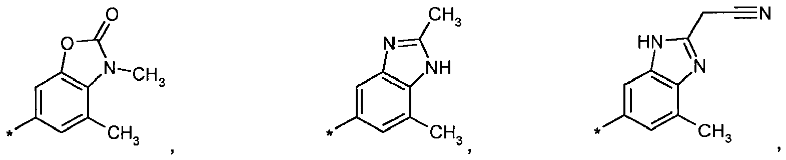 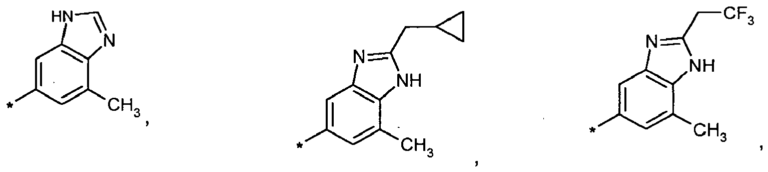 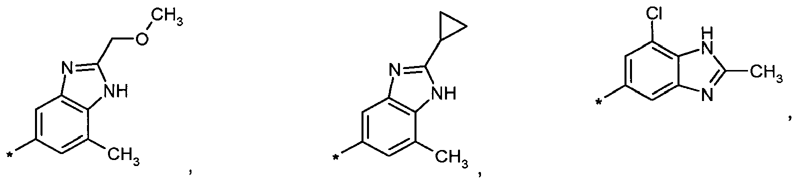 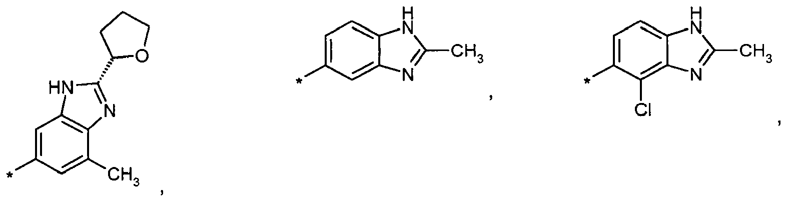 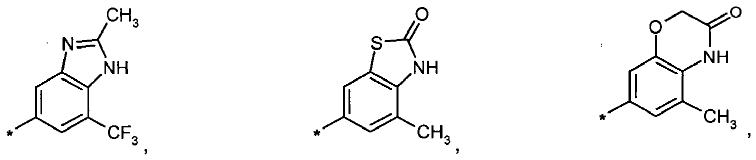 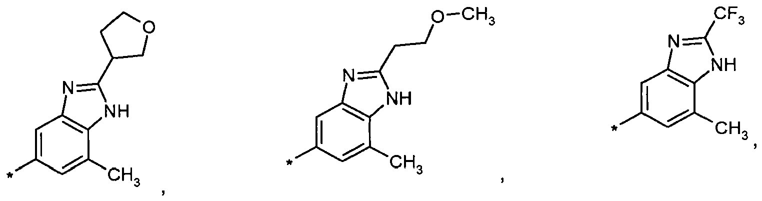 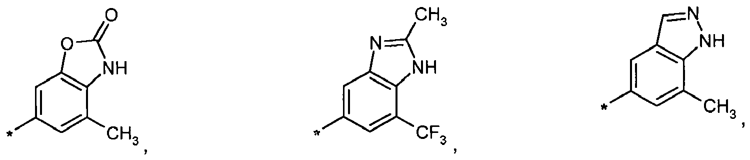 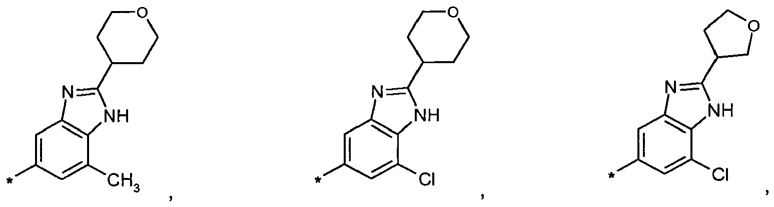 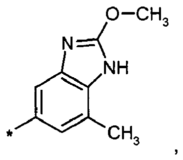
**R⁵** H, Cl, CH₃, CF₃, -O-CH₃ oder Cyclopropyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

5. Folgende Verbindungen der allgemeinen Formel **I** gemäß Anspruch 1:
| **Nr.** | **Struktur** |
|---|---|
| (1) | 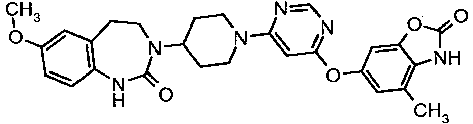 |
| (2) | 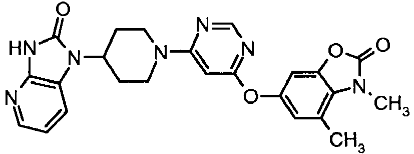 |
| (3) | 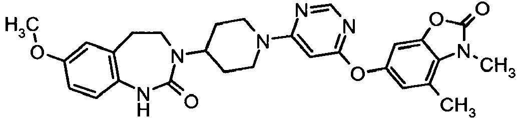 |
| (4) | 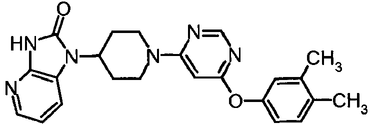 |
| (5) | 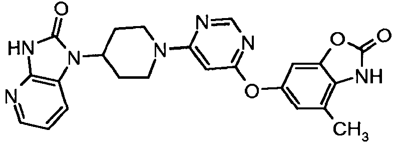 |
| (6) | 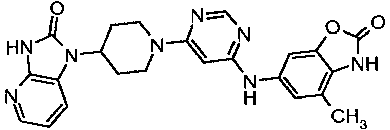 |
| (7) | 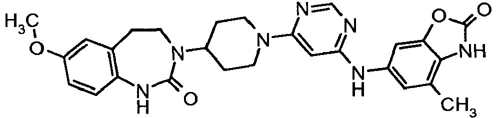 |
| (8) | 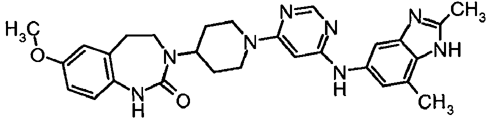 |
| (9) | 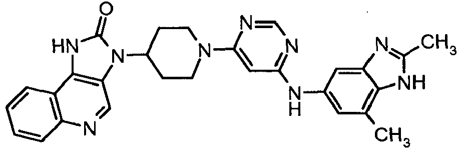 |
| (10) | 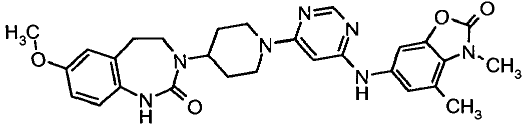 |
| (11) | 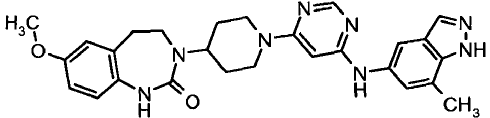 |
| (12) | 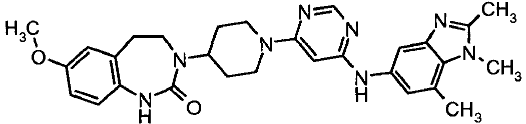 |
| (13) | 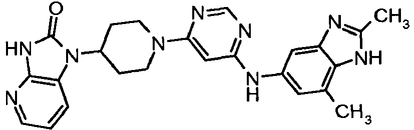 |
| (14) | 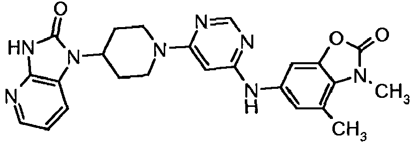 |
| (15) | 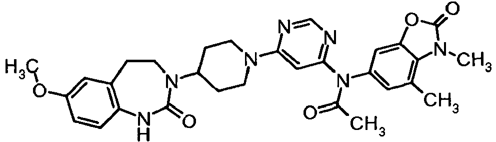 |
| (16) | 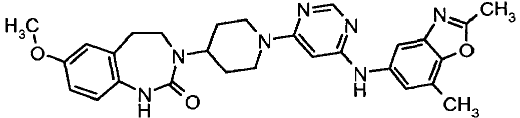 |
| (17) | 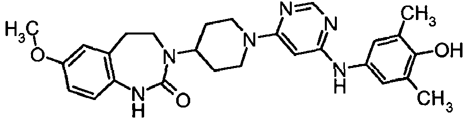 |
| (18) | 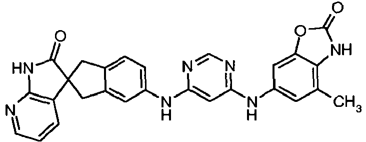 |
| (19) | 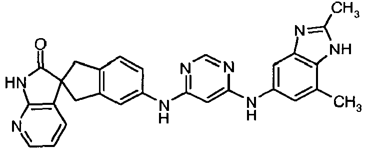 |
| (20) | 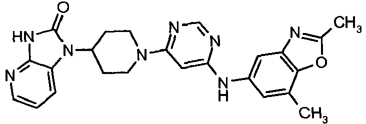 |
| (21) | 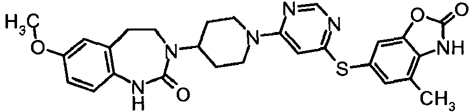 |
| (22) | 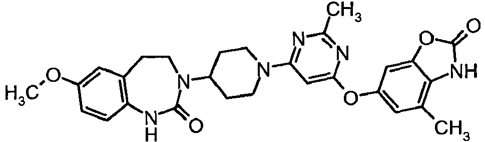 |
| (23) | 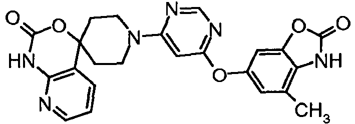 |
| (24) | 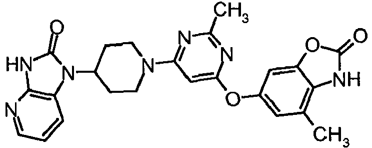 |
| (25) | 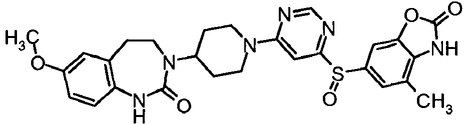 |
| (26) | 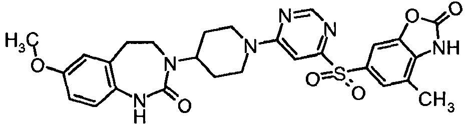 |
| (27) | 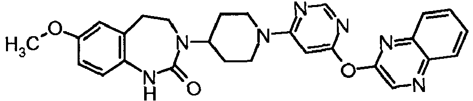 |
| (28) | 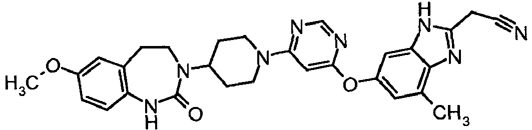 |
| (29) | 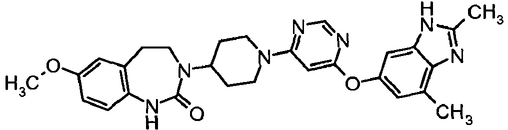 |
| (30) | 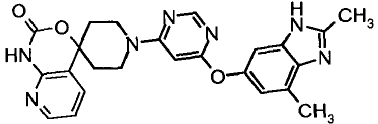 |
| (31) | 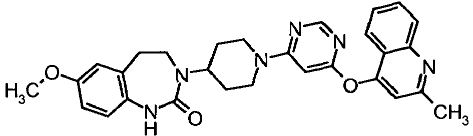 |
| (32) | 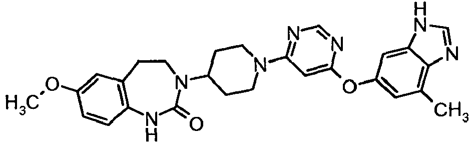 |
| (33) | 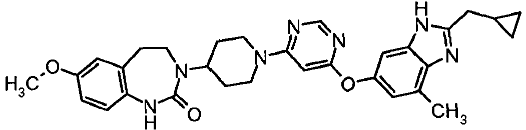 |
| (34) | 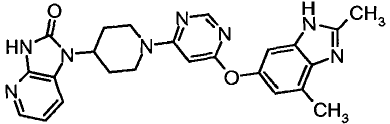 |
| (35) | 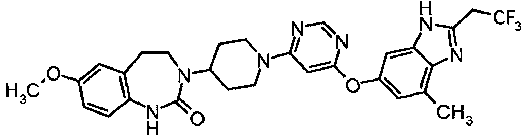 |
| (36) | 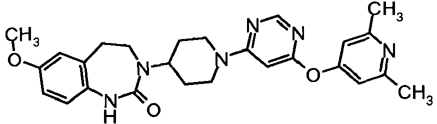 |
| (37) | 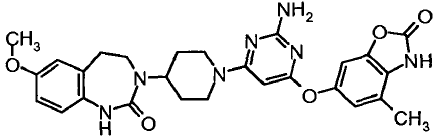 |
| (38) | 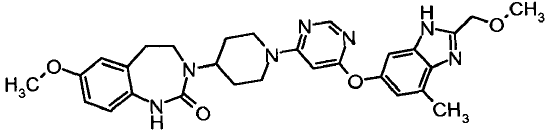 |
| (39) | 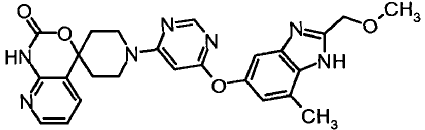 |
| (40) | 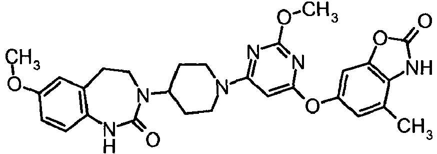 |
| (41) | 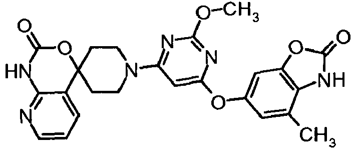 |
| (42) | 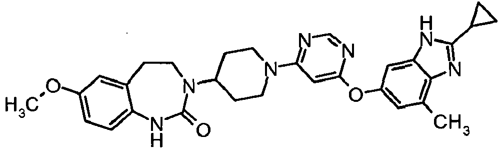 |
| (43) | 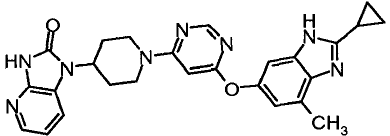 |
| (44) | 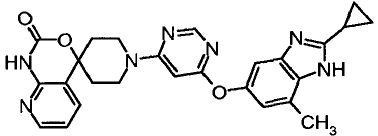 |
| (45) | 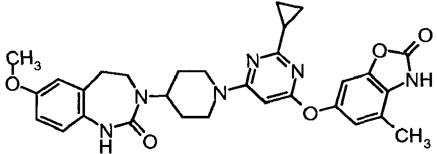 |
| (46) | 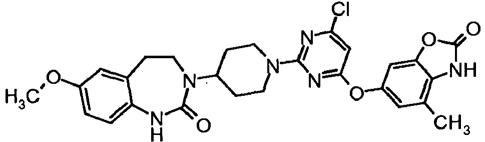 |
| (47) | 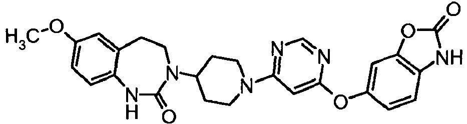 |
| (48) | 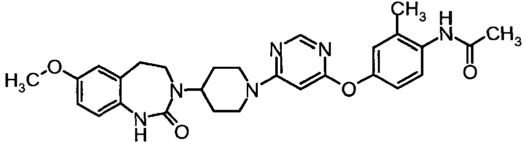 |
| (49) | 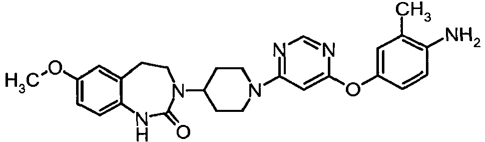 |
| (50) | 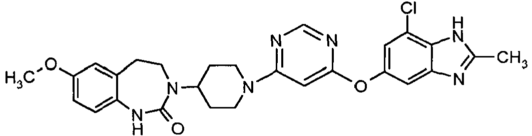 |
| (51) | 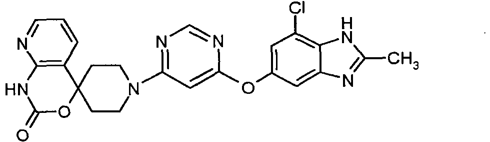 |
| (52) | 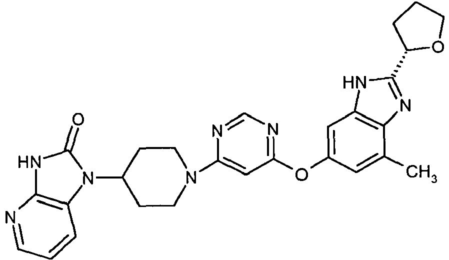 |
| (53) | 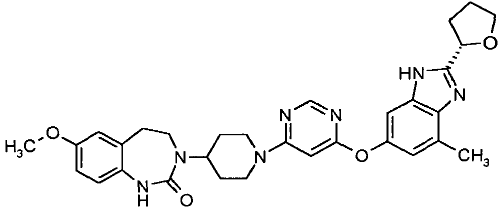 |
| (54) | 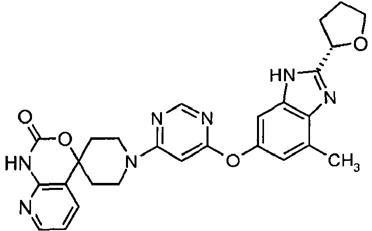 |
| (55) | 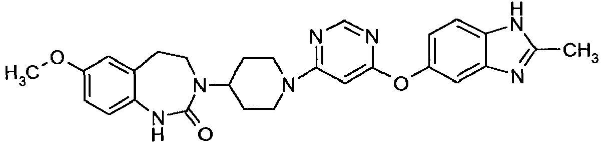 |
| (56) | 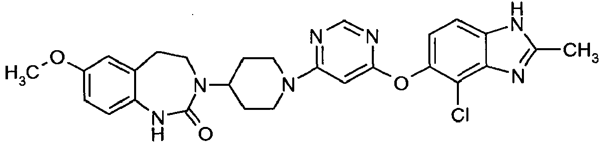 |
| (57) | 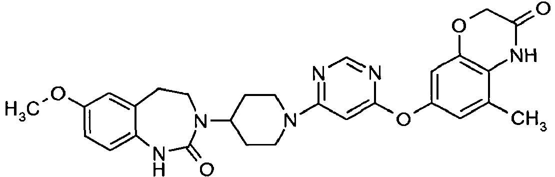 |
| (58) | 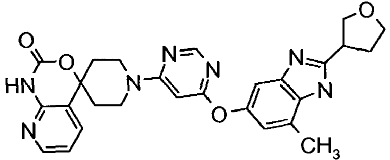 |
| (59) | 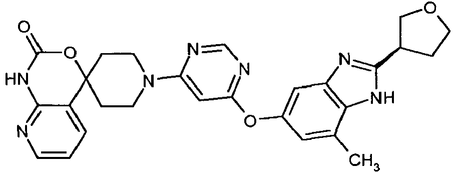 |
| (60) | 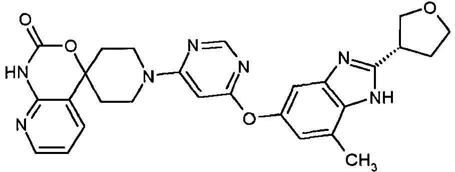 |
| (61) | 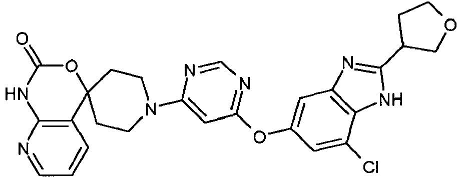 |
| (62) | 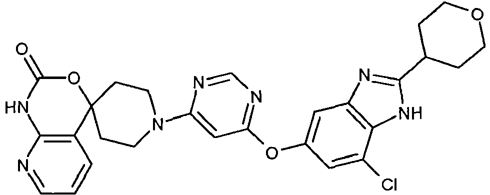 |
| (63) | 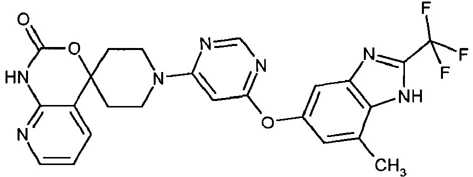 |
| (64) | 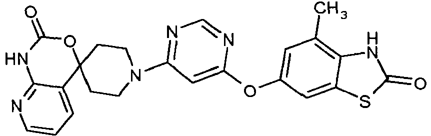 |
| (65) | 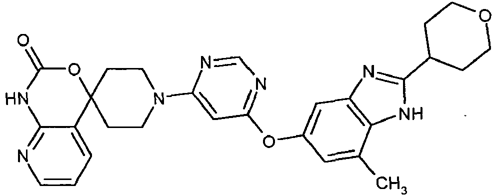 |
| (66) | 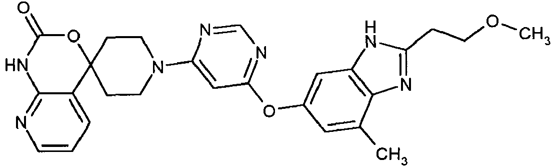 |
| (67) | 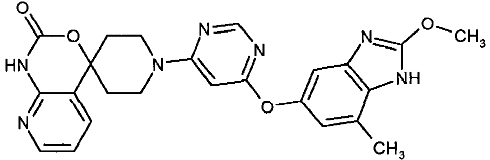 |
| (68) | 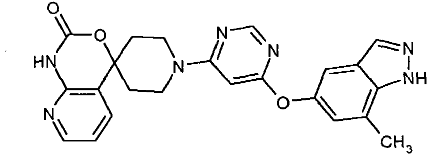 |
| (69) | 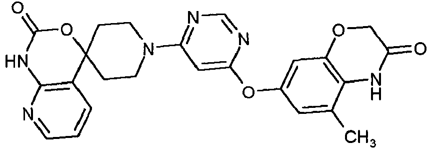 |
| (70) | 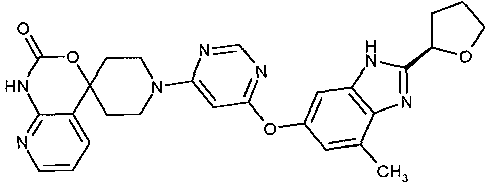 |
| (71) | 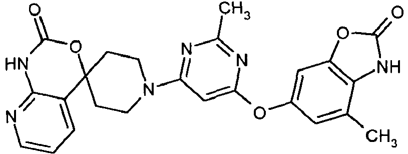 |
| (72) | 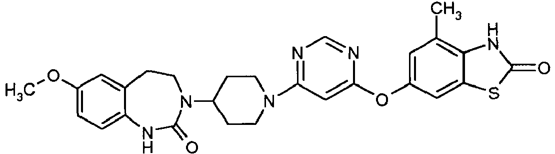 |
| (73) | 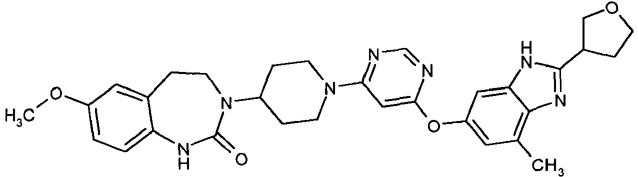 |
| (74) | 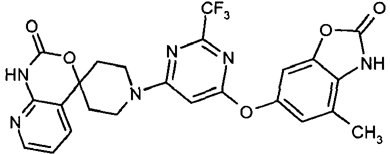 |
| (75) | 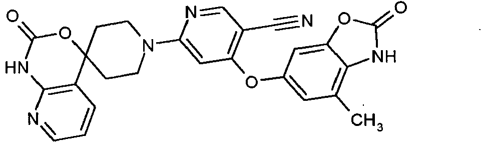 |
| (76) | 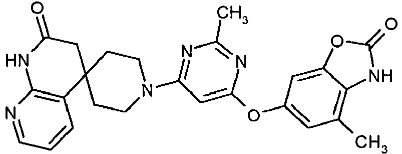 |
| (77) | 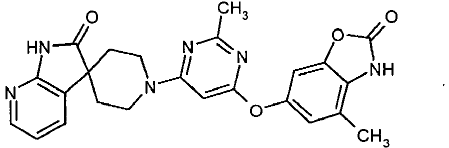 |
| (78) | 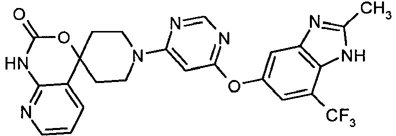 |
| (79) | 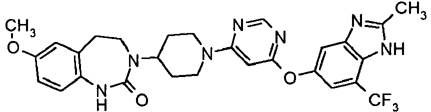 |
| (80) | 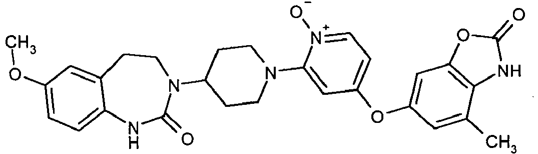 |
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

6. Arzneimittel, enthaltend eine Verbindung nach einem der Ansprüche 1 bis 5 neben gegebenenfalls einem oder mehreren inerten Trägerstoffen und/oder Verdünnungsmitteln.

7. Verbindung nach einem der Ansprüche 1 bis 5 zur Verwendung bei der akuten und prophylaktischen Behandlung von Kopfschmerzen, insbesondere Migräne- bzw. Cluster-Kopfschmerz.

8. Verbindung nach einem der Ansprüche 1 bis 5 zur Verwendung bei der Behandlung von nicht insulinabhängigem Diabetes mellitus ("NIDDM"), des complex regional pain syndrome (CRPS1), cardiovaskulären Erkrankungen, Morphintoleranz, Clostridiumtoxinbedingten Durchfallerkrankungen, Erkrankungen der Haut, insbesondere thermische und strahlenbedingte Hautschäden inklusive Sonnenbrand, Lichen, Prurigo, pruriginöse Toxidermien sowie schwerer Juckreiz, entzündlichen Erkrankungen, z.B. entzündlichen Gelenkerkrankungen (Osteoarthritis, rheumatoide Arthritis, neurogene Arthritis), generalisiertem Weichteilrheumatismus (Fibromyalgie), neurogenen Entzündungen der oralen Mucosa, entzündlichen Lungenerkrankungen, allergische Rhinitis, Asthma, COPD, Erkrankungen, die mit einer überschießenden Gefäßerweiterung und dadurch bedingter verringerter Gewebedurchblutung einhergehen, z.B. Schock und Sepsis, chronische Schmerzerkrankungen, wie z.B. diabetische Neuropathien, durch Chemotherapien induzierten Neuropathien, HIV-induzierten Neuropathien, postherpetischen Neuropathien durch Gewebetrauma induzierten Neuropathien, trigeminalen Neuralgien, temporomandibulären Dysfunktionen, CRPS (complex regional pain syndrome), Rückenschmerzen, viszeralen Erkrankungen, wie z.B. irritable bowel syndrome (IBS), inflammatory bowel syndrome, zur Linderung von Schmerzzuständen, oder zur präventiven oder akut-therapeutisch Behandlung der Symptomatik menopausaler, durch Gefäßerweiterung und erhöhten Blutfluss verursachter Hitzewallungen östrogendefizienter Frauen sowie hormonbehandelter Prostatakarzinompatienten und Kastraten.

9. Verbindung nach einem der Ansprüche 1 bis 5 zur Verwendung bei der Behandlung von Migräne- und Cluster-Kopfschmerz.

10. Verbindung zur Verwendung gemäß Anspruch 9, **dadurch gekennzeichnet, dass** die Behandlung akut oder prophylaktisch sein kann.

11. Verbindung nach einem der Ansprüche 1 bis 5 zur Verwendung bei der Behandlung des irritable bowel syndroms (IBS).

12. Verbindung nach einem der Ansprüche 1 bis 5 zur Verwendung bei der präventiven und akut-therapeutischen Behandlung von Hitzewallungen östrogendefizienter Frauen.

## Claims

1. Compounds of general formula 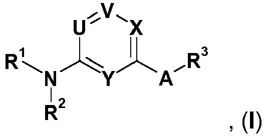 wherein
**A** denotes -NH, -N(C(O)-C₁₋₃-alkyl), S, O, SO, SO₂,
**R¹** and **R²** together with the nitrogen atom to which they are attached denote a group selected from 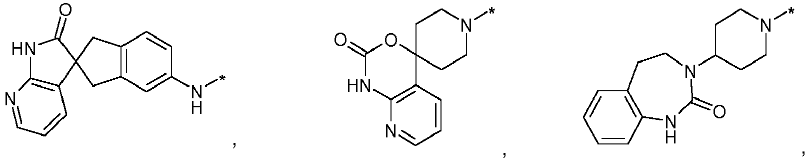 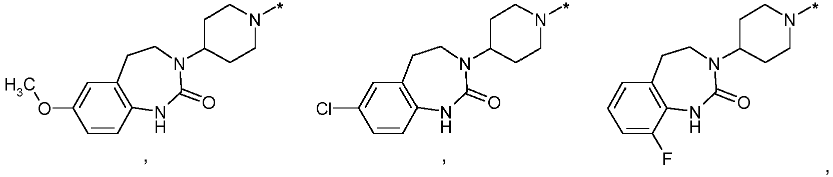 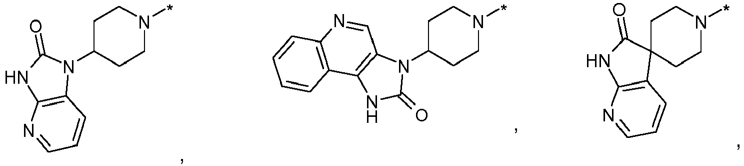 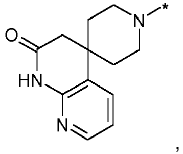
**R³** denotes a group selected from 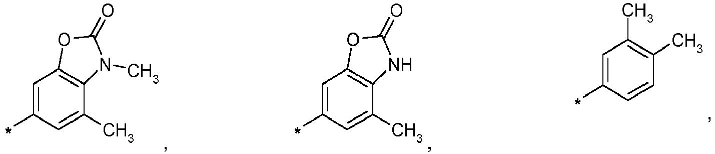 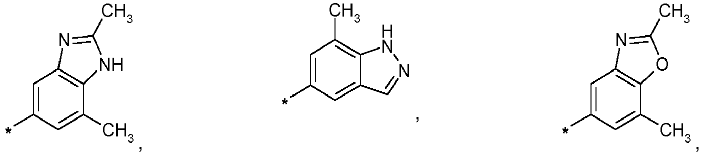 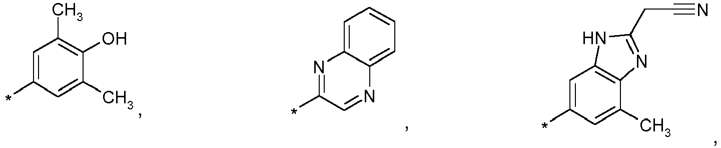 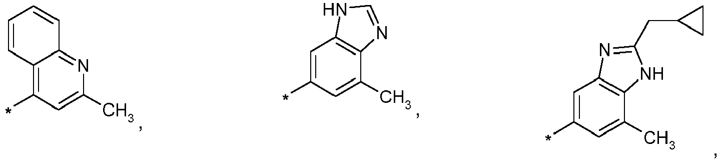 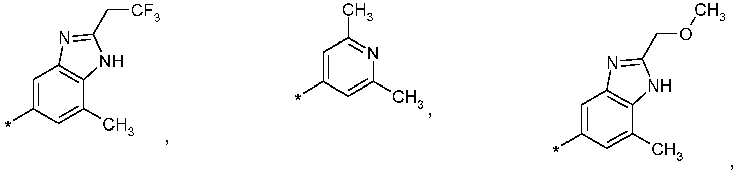 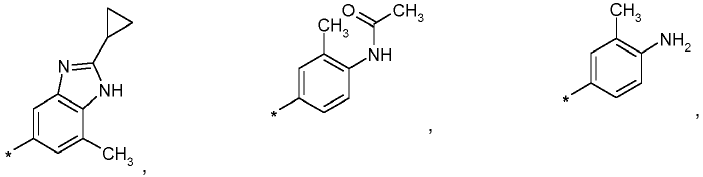 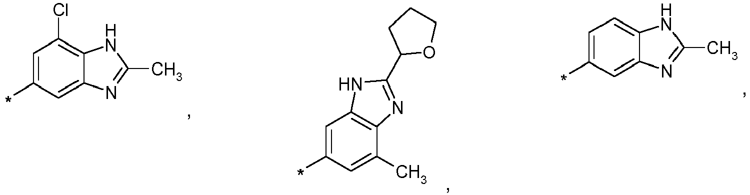 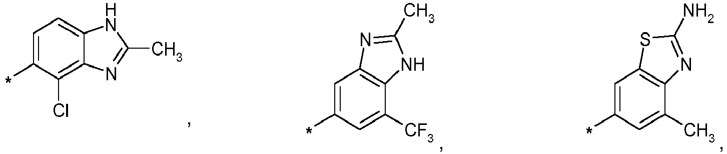 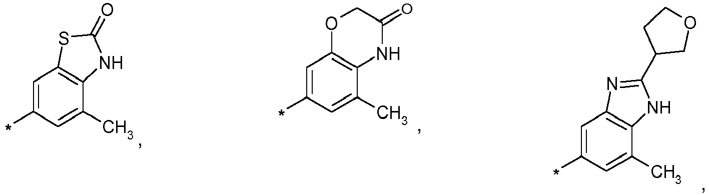 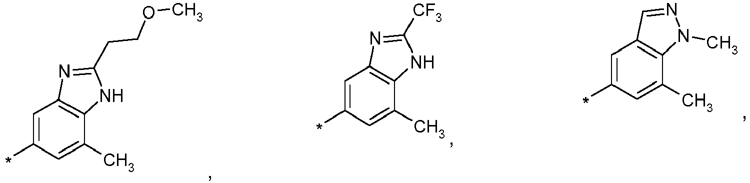 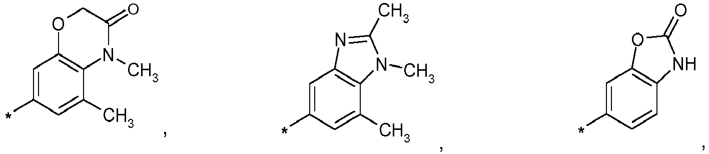 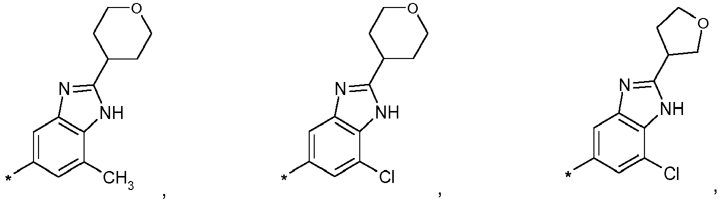 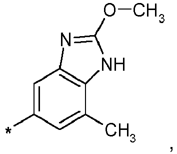
**U-V-X** denotes a group selected from
-N=(C-**R⁵**)-N=, -N=(C-**R⁵**)-(C-**R⁶**)=, -(N-oxide)=(C-**R⁵**)-(C-**R⁶**)=, and
**Y** denotes N or CH,
**R⁵** denotes
(a) H,
(b) -N**R^{5.1}R^{5.2}**,
(c) halogen, C₃₋₆-cycloalkyl-,
(d) a C₁₋₃-alkyl or C₁₋₃-alkyl-O- group wherein each methylene group is substituted by up to two fluorine atoms and each methyl group is substituted by up to three fluorine atoms,
**R^{5.1}** denotes H,
**R^{5.2}** denotes H,
**R⁶** denotes
(a) H,
(b) -CN,
the tautomers, the diastereomers, the enantiomers, the hydrates, the mixtures thereof and the salts thereof as well as the hydrates of the salts, particularly the physiologically acceptable salts thereof with inorganic or organic acids or bases.

2. Compounds of general formula **I** according to claim 1, wherein **A, R¹**, **R²** and **R³** are defined as in claim 1 and the ring 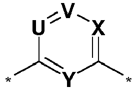 denotes a group selected from 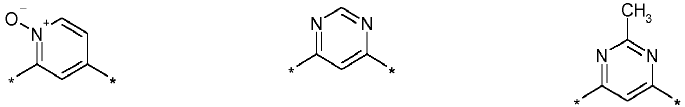 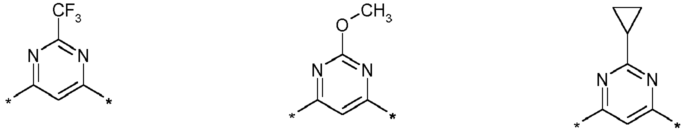 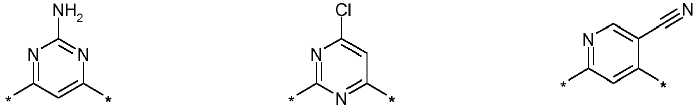 the tautomers, the diastereomers, the enantiomers, the hydrates, the mixtures thereof and the salts thereof as well as the hydrates of the salts, particularly the physiologically acceptable salts thereof with inorganic or organic acids or bases.

3. Compounds of general formula **I** according to claim 1, wherein **U, V, X, Y, R¹**, **R²** and **R³** are defined as in claim 1 or 2 and
**A** denotes -O- or -NH-,
the tautomers, the diastereomers, the enantiomers, the hydrates, the mixtures thereof and the salts thereof as well as the hydrates of the salts, particularly the physiologically acceptable salts thereof with inorganic or organic acids or bases.

4. Compounds of general formula **Ia** according to claim 1, 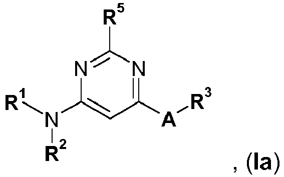 wherein
**A** denotes -NH- or -O-,
**R¹** and **R²** together with the nitrogen atom to which they are attached denote a group selected from 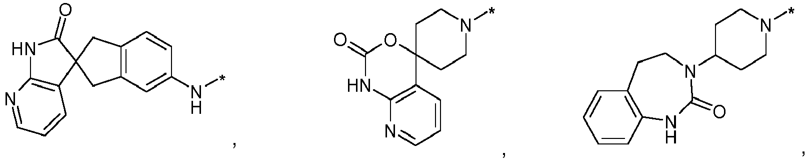 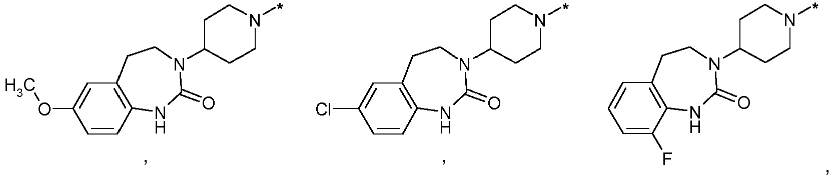 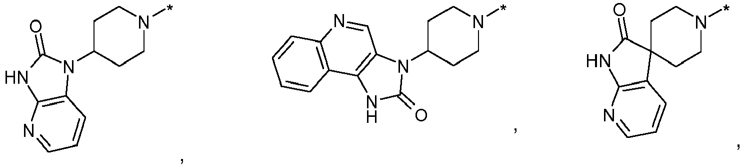 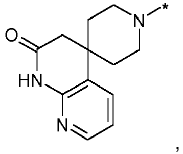
**R³** denotes a group selected from 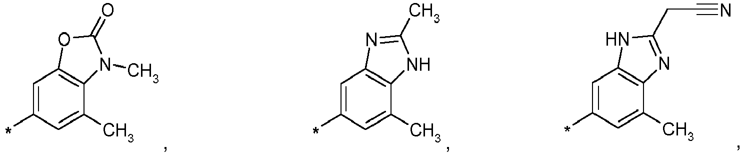 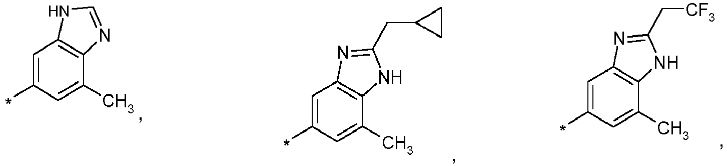 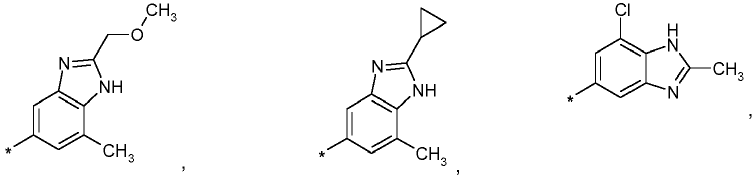 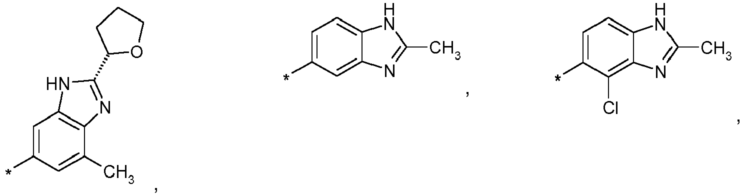 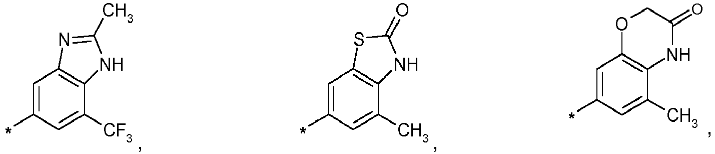 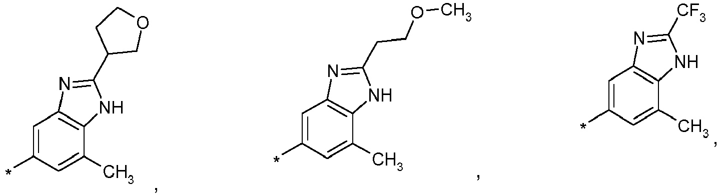 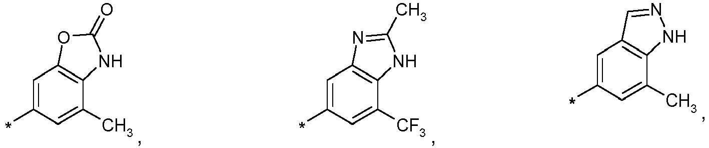 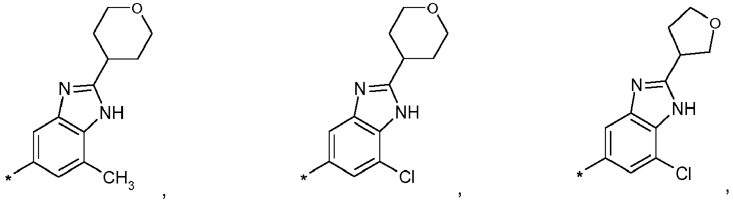 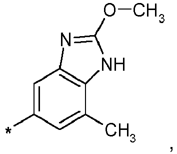
**R⁵** denotes H, Cl, CH₃, CF₃, -O-CH₃ or cyclopropyl,
the tautomers, the diastereomers, the enantiomers, the hydrates, the mixtures thereof and the salts thereof as well as the hydrates of the salts, particularly the physiologically acceptable salts thereof with inorganic or organic acids or bases.

5. The following compounds of general formula **I** according to claim 1:
| **No.** | **Structure** |
|---|---|
| (1) | 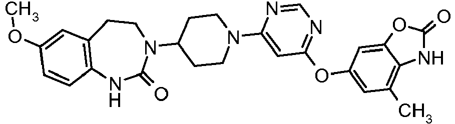 |
| (2) | 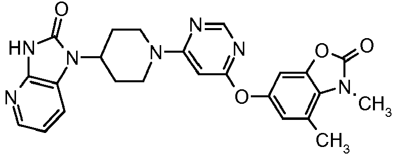 |
| (3) | 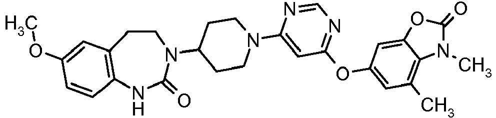 |
| (4) | 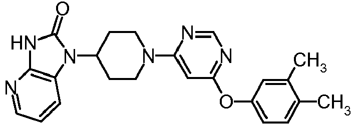 |
| (5) | 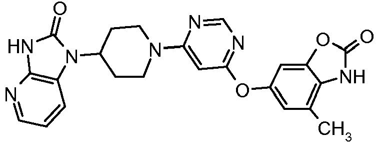 |
| (6) | 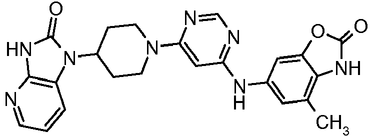 |
| (7) | 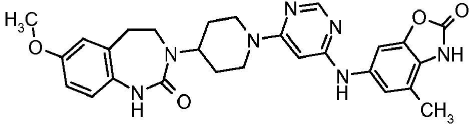 |
| (8) | 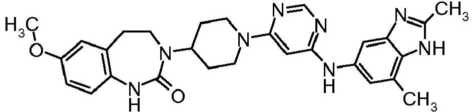 |
| (9) | 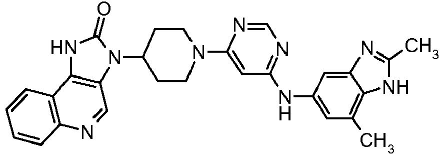 |
| (10) | 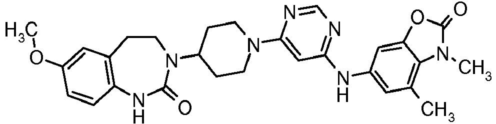 |
| (11) | 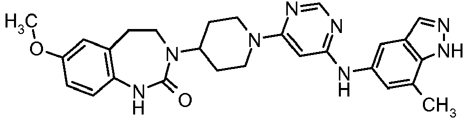 |
| (12) | 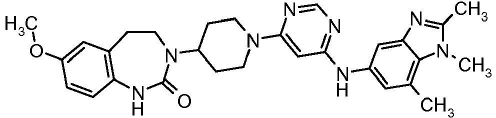 |
| (13) | 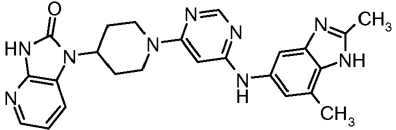 |
| (14) | 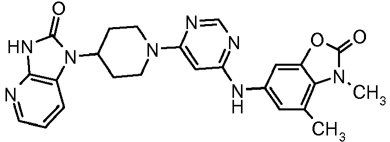 |
| (15) | 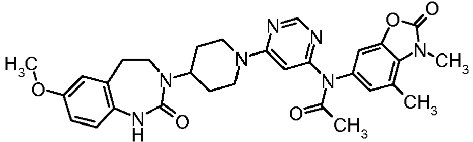 |
| (16) | 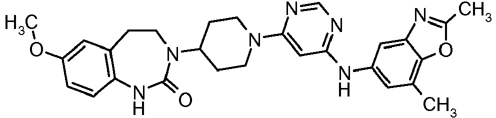 |
| (17) | 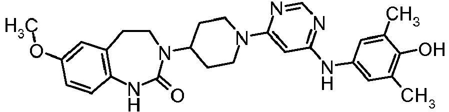 |
| (18) | 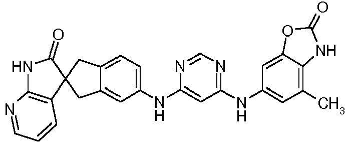 |
| (19) | 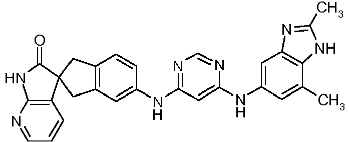 |
| (20) | 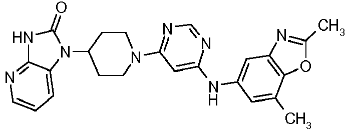 |
| (21) | 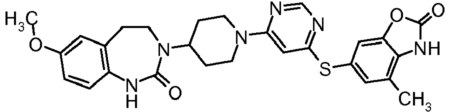 |
| (22) | 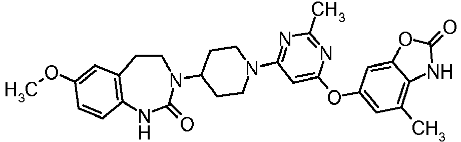 |
| (23) | 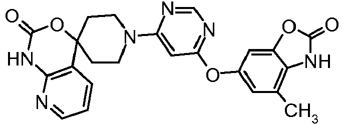 |
| (24) | 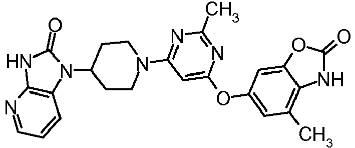 |
| (25) | 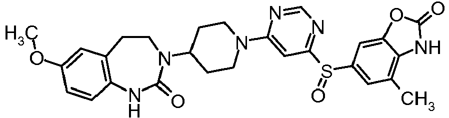 |
| (26) | 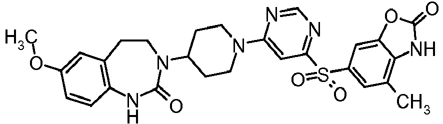 |
| (27) | 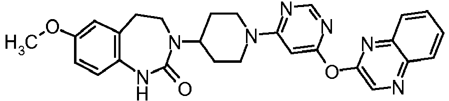 |
| (28) | 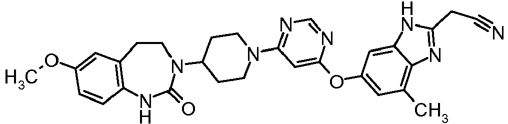 |
| (29) | 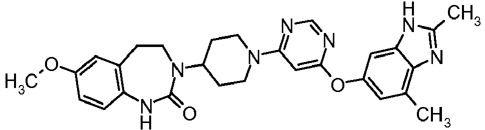 |
| (30) | 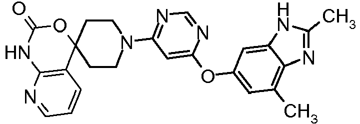 |
| (31) | 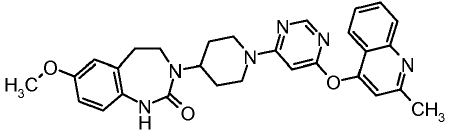 |
| (32) | 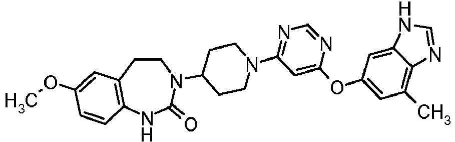 |
| (33) | 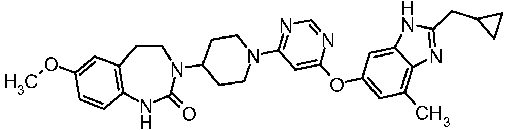 |
| (34) | 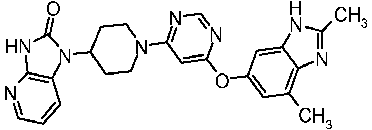 |
| (35) | 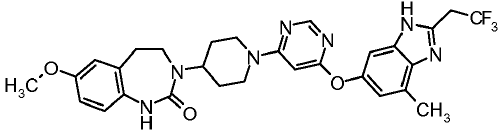 |
| (36) | 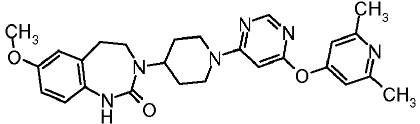 |
| (37) | 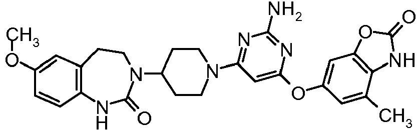 |
| (38) | 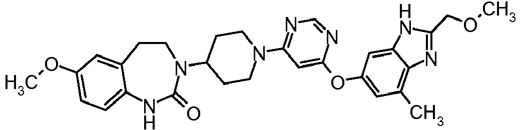 |
| (39) | 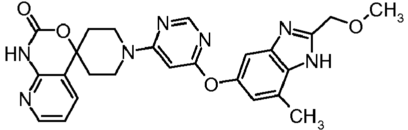 |
| (40) | 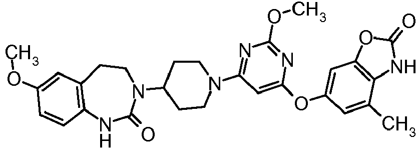 |
| (41) | 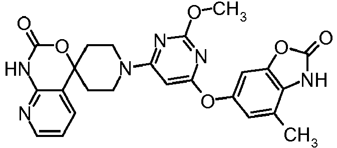 |
| (42) | 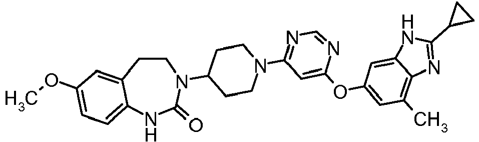 |
| (43) | 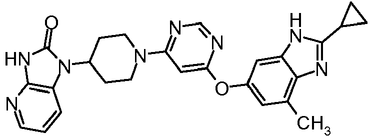 |
| (44) | 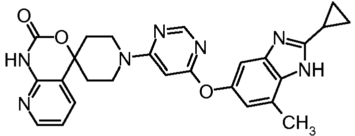 |
| (45) | 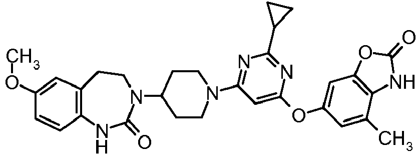 |
| (46) | 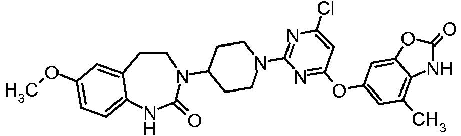 |
| (47) | 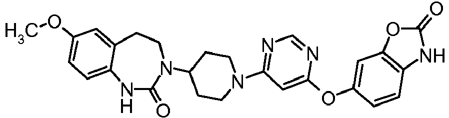 |
| (48) | 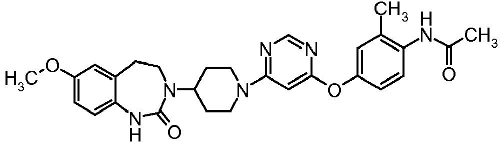 |
| (49) | 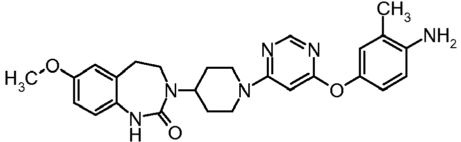 |
| (50) | 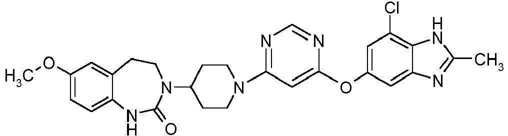 |
| (51) | 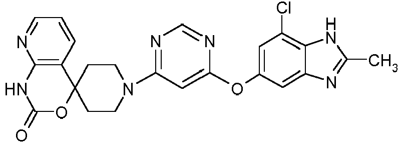 |
| (52) | 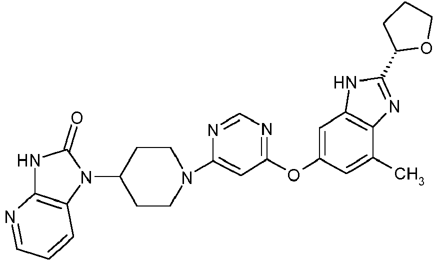 |
| (53) | 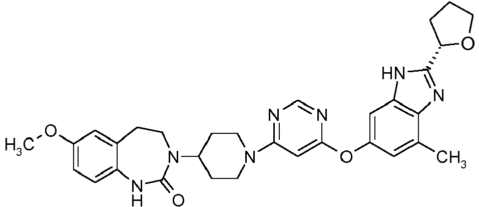 |
| (54) | 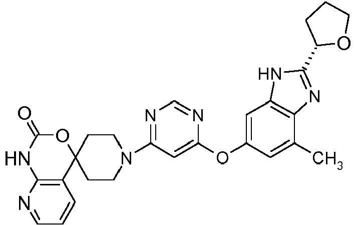 |
| (55) | 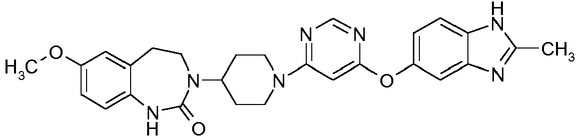 |
| (56) | 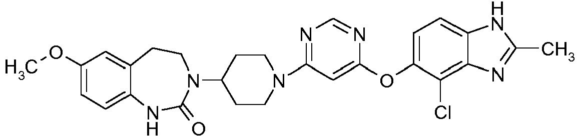 |
| (57) | 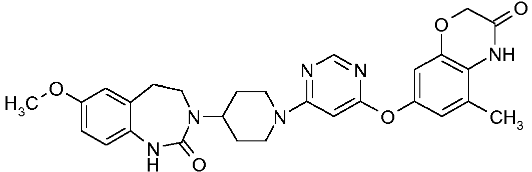 |
| (58) | 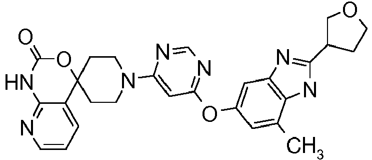 |
| (59) | 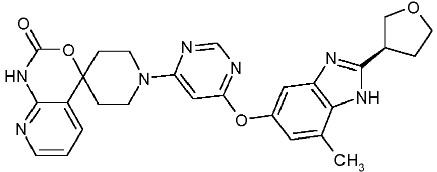 |
| (60) | 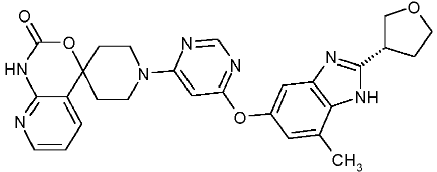 |
| (61) | 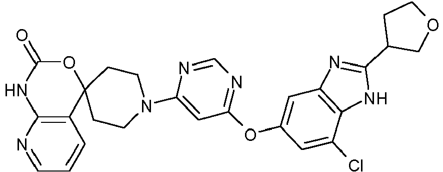 |
| (62) | 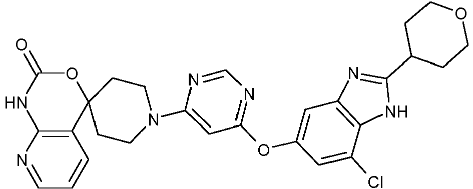 |
| (63) | 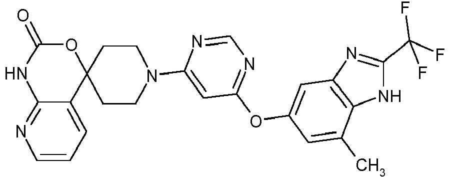 |
| (64) | 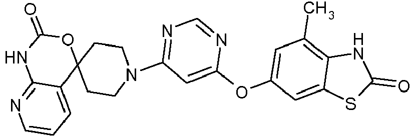 |
| (65) | 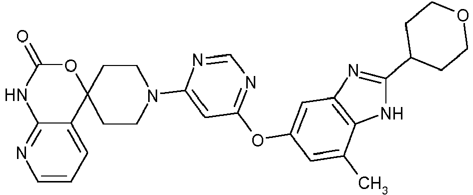 |
| (66) | 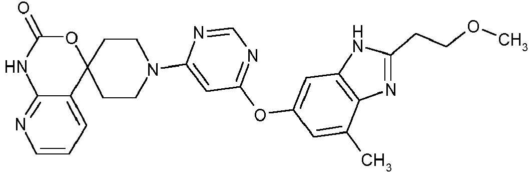 |
| (67) | 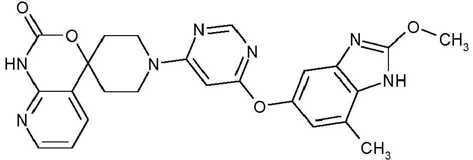 |
| (68) | 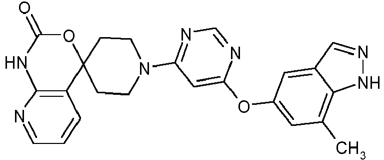 |
| (69) | 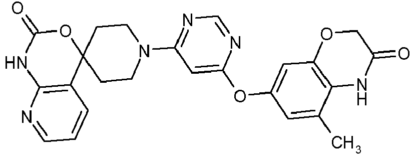 |
| (70) | 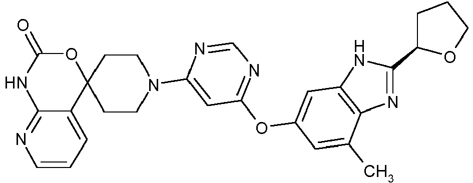 |
| (71) | 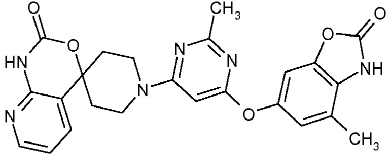 |
| (72) | 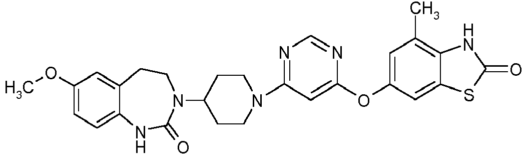 |
| (73) | 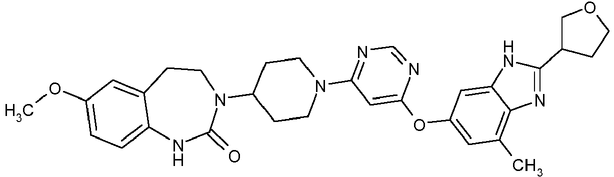 |
| (74) | 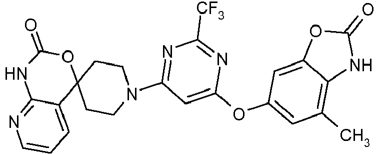 |
| (75) | 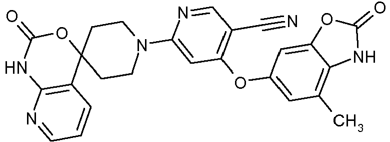 |
| (76) | 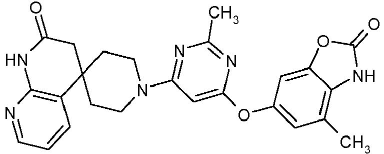 |
| (77) | 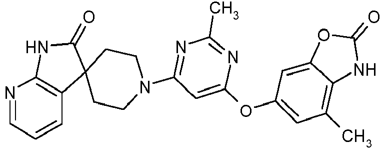 |
| (78) | 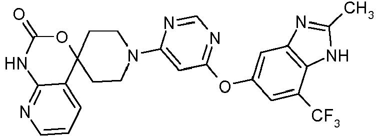 |
| (79) | 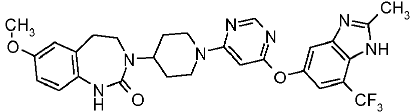 |
| (80) | 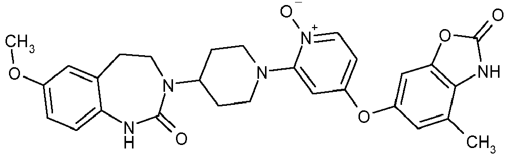 |
the tautomers, the diastereomers, the enantiomers, the hydrates, the mixtures thereof and the salts thereof as well as the hydrates of the salts, particularly the physiologically acceptable salts thereof with inorganic or organic acids or bases.

6. Medicaments containing a compound according to one of claims 1 to 5 optionally together with one or more inert carriers and/or diluents.

7. Compound according to one of claims 1 to 5 for use in the acute and prophylactic treatment of headaches, particularly migraine or cluster headaches.

8. Use of a compound according to one of claims 1 to 5 for use in the treatment of non-insulin-dependent diabetes mellitus ("NIDDM"), complex regional pain syndrome (CRPS1), cardiovascular diseases, morphine tolerance, diarrhoea caused by clostridium toxin, skin diseases, particularly thermal and radiation-induced skin damage including sunburn, lichen, pruritis, pruritic toxidermies and severe itching, inflammatory diseases, e.g. inflammatory diseases of the joints (osteoarthritis, rheumatoid arthritis, neurogenic arthritis), generalised soft-tissue rheumatism (fibromyalgia), neurogenic inflammation of the oral mucosa, inflammatory lung diseases, allergic rhinitis, asthma, COPD, diseases accompanied by excessive vasodilatation and resultant reduced blood supply to the tissues, e.g. shock and sepsis, chronic pain, e.g. diabetic neuropathies, neuropathies induced by chemotherapy, HIV-induced neuropathies, postherpetic neuropathies, neuropathies induced by tissue trauma, trigeminal neuralgias, temporomandibular dysfunctions, CRPS (complex regional pain syndrome), back pain, visceral complaints, such as e.g. irritable bowel syndrome (IBS), inflammatory bowel syndrome, for relieving pain, or for preventive or acute therapeutic treatment of the symptoms of menopausal hot flushes caused by vasodilatation and increased blood flow in oestrogen-deficient women and hormone-treated patients with prostate carcinoma and castrated men.

9. Use of a compound according to one of claims 1 to 5 for use in the treatment of migraine and cluster headaches.

10. Compound for use according to claim 9, **characterised in that** the treatment may be acute or prophylactic.

11. Compound according to one of claims 1 to 5 for use in the treatment of irritable bowel syndrome (IBS).

12. Compound according to one of claims 1 to 5 for use in the preventative and acute-therapeutic treatment of hot flushes in oestrogen-deficient women.

## Revendications

1. Composés de formule générale 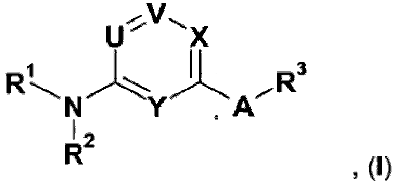 dans laquelle
A représente -NH, -N(C(O)-C₁-₃-alkyl)-, S, O, SO, SO₂,
R¹ et R² représentent conjointement avec l'atome d'azote auxquels ils sont liés un groupe choisi parmi 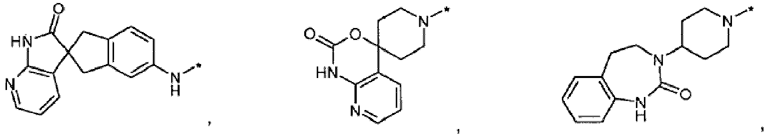 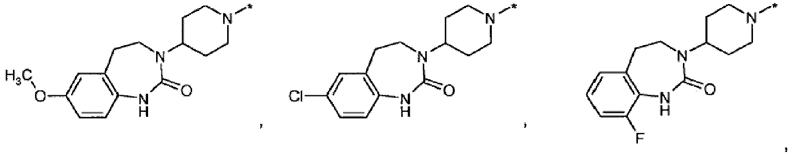 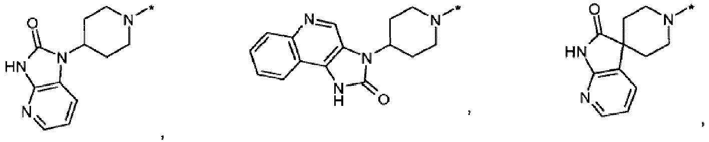 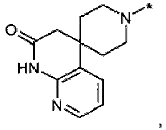
R³ représente un groupe choisi parmi 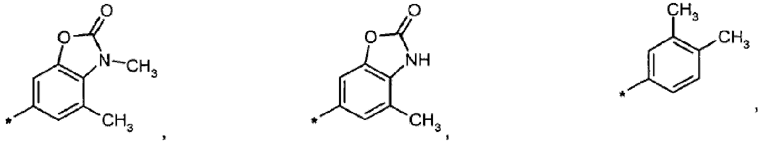 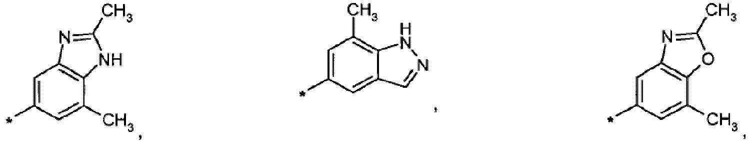 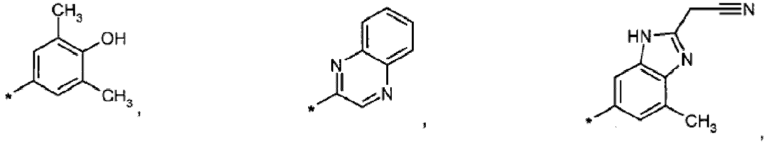 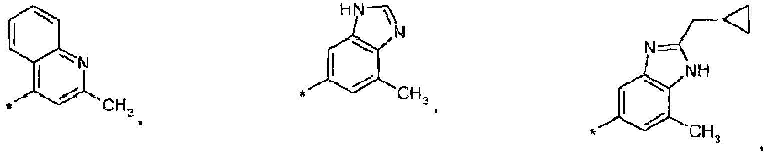 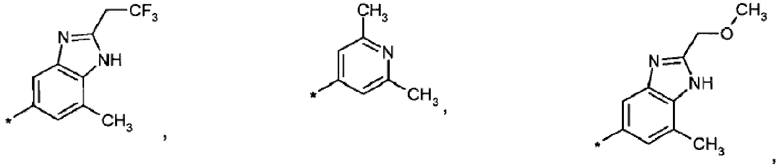 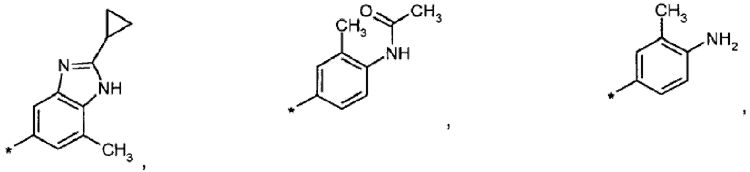 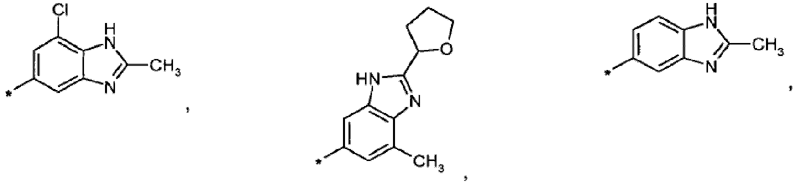 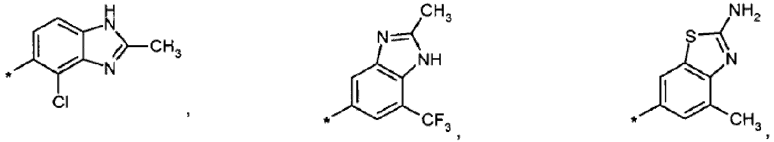 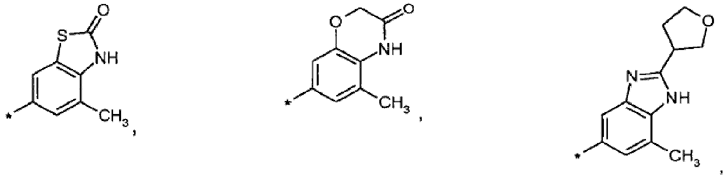 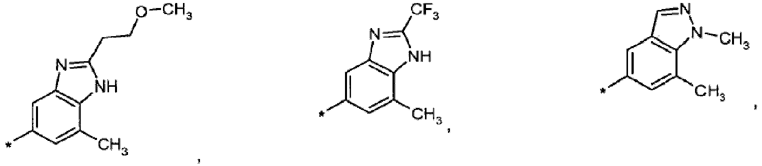 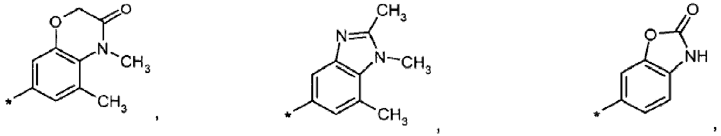 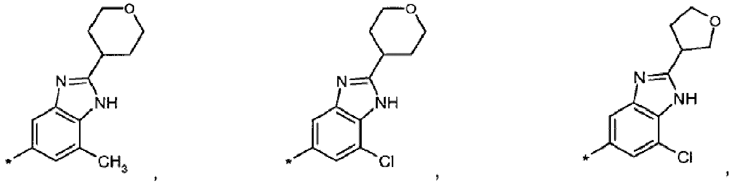 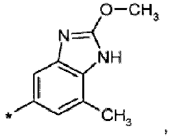
U-V-X représente un groupe choisi parmi
-N= (C-R⁵) -N=, -N= (C-R⁵) - (C-R⁶) =, -(N-oxyde)=(C-R⁵)-(C-R⁶) =, et
Y représente N ou CH,
R⁵ représente
(a) H,
(b) -NR^{5.1}R^{5.2},
(c) un atome d'halogène, un groupe C₃₋₆-cycloalkyle, (d) un groupe C₁₋₃-alkyle ou C₁₋₃-alkyl-O- dans lequel chaque groupe méthylène est substitué par jusqu'à deux atomes de fluor et chaque groupe méthyle est substitué par jusqu' à trois atomes de fluor,
R^{5.1} représente H,
R^{5.2} représente H,
R⁶ représente
(a)H,
(b) -CN,
leurs tautomères, leurs diastéréomères, leurs énantiomères, leurs hydrates, leurs mélanges et leurs sels et les hydrates des sels, en particulier leurs sels physiologiquement acceptables avec des acides ou des bases inorganiques ou organiques.

2. Composés de formule générale I selon la revendication I, dans laquelle A, R¹, R² et R³ sont tels que définis dans la revendication 1 et le cycle 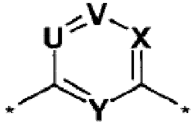 représente un groupe choisi parmi 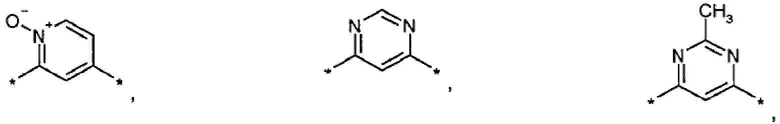 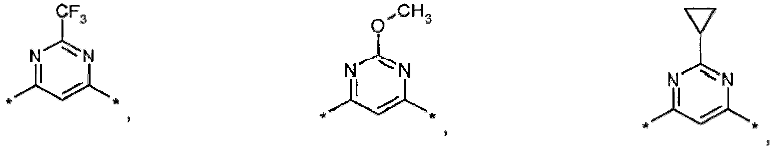 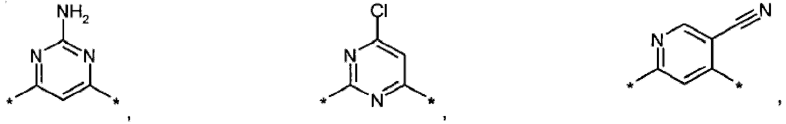 leurs tautomères, leurs diastéréomères, leurs énantiomères, leurs hydrates, leurs mélanges et leurs sels et les hydrates des sels, en particulier leurs sels physiologiquement acceptables avec des acides ou des bases inorganiques ou organiques.

3. Composés de formule générale I selon la revendication 1, dans lesquels U, V, X, Y, R¹, R² et R³ sont tels que définis dans la revendication 1 ou 2 et
A représente -O- ou -NH-,
leurs tautomères, leurs diastéréomères, leurs énantiomères, leurs hydrates, leurs mélanges et leurs sels et les hydrates des sels, en particulier leurs sels physiologiquement acceptables avec des acides ou des bases inorganiques ou organiques.

4. Composés de formule générale la selon la revendication 1 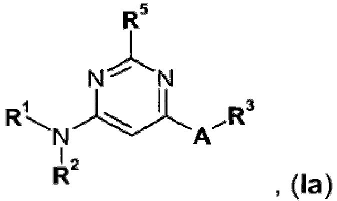 dans laquelle
A représente -NH- ou -O-
R¹ et R² représentent conjointement avec l'atome d'azote auxquels ils sont liés un groupe choisi parmi 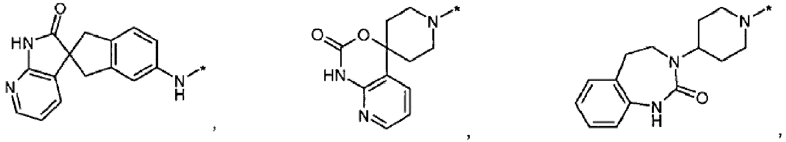 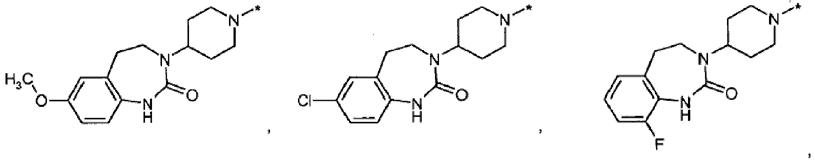 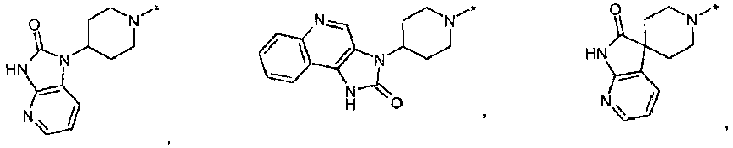 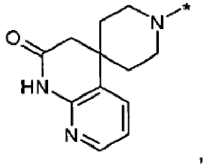
R³ représente un groupe choisi parmi 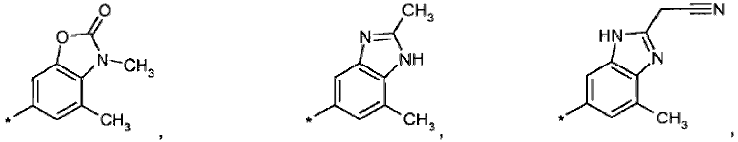 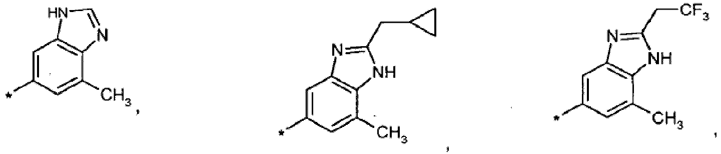 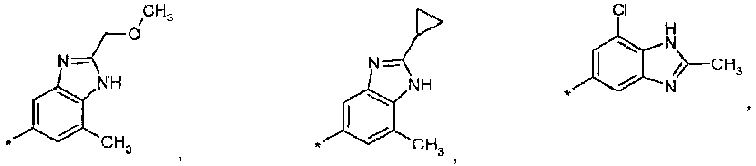 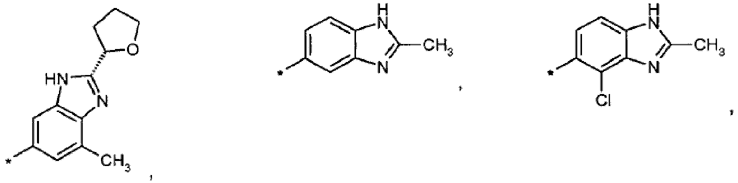 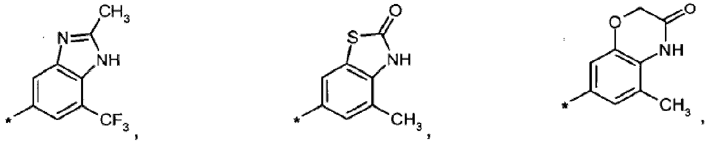 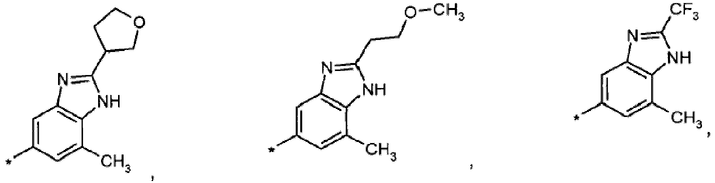 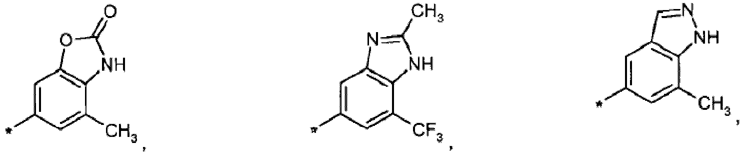 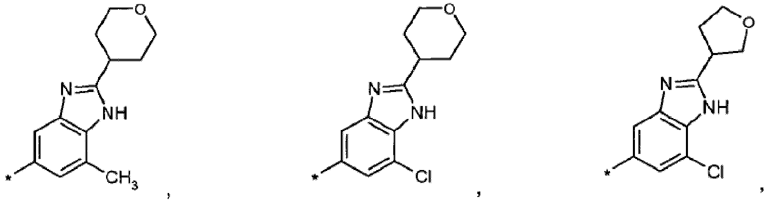 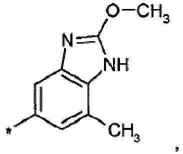
R⁵ représente H, Cl, CH₃, CF₃, -O-CH₃ ou un groupe cyclopropyle,
leurs tautomères, leurs diastéréomères, leurs énantiomères, leurs hydrates, leurs mélanges et leurs sels et les hydrates des sels, en particulier leurs sels physiologiquement acceptables avec des acides ou des bases inorganiques ou organiques.

5. Composés de formule générale I suivants selon la revendication 1 :
| N° | Structure |
|---|---|
| (1) | 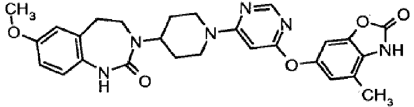 |
| (2) | 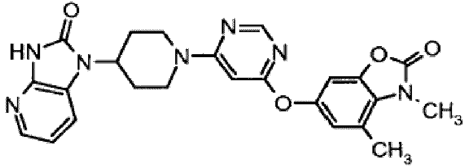 |
| (3) | 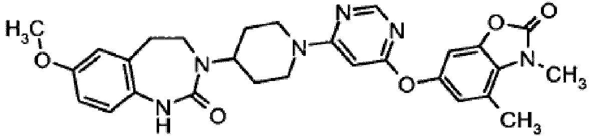 |
| (4) | 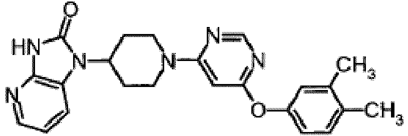 |
| (5) | 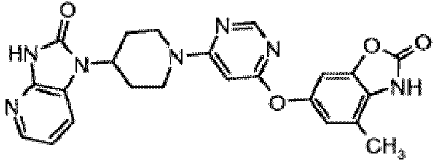 |
| (6) | 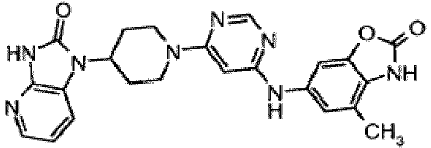 |
| (7) | 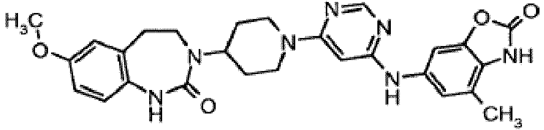 |
| (8) | 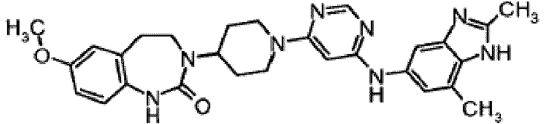 |
| (9) | 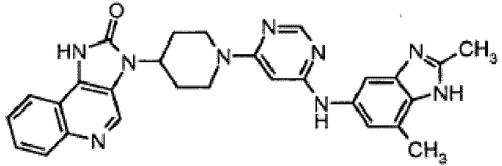 |
| (10) | 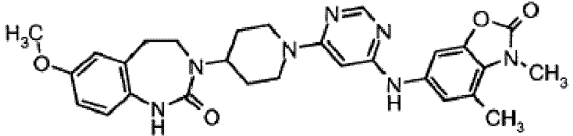 |
| (11) | 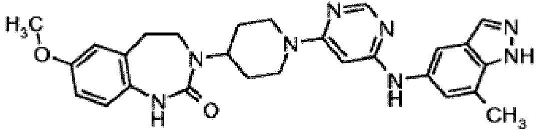 |
| (12) | 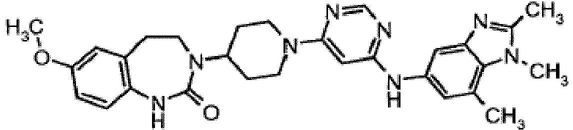 |
| (13) | 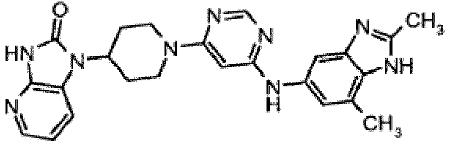 |
| (14) | 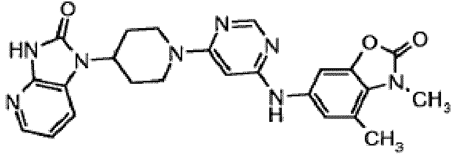 |
| (15) | 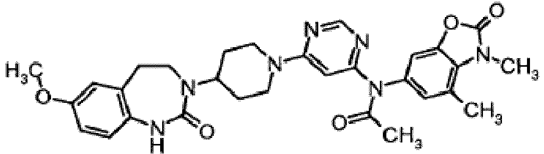 |
| (16) | 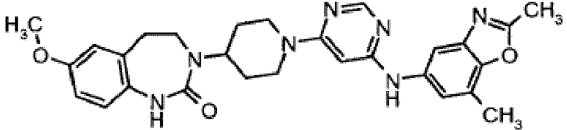 |
| (17) | 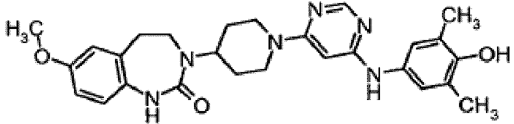 |
| (18) | 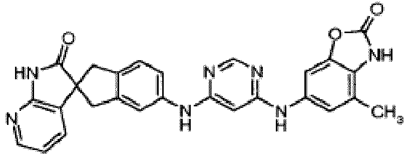 |
| (19) | 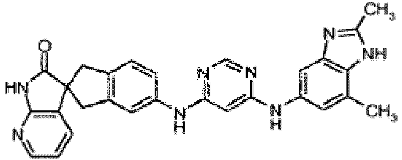 |
| (20) | 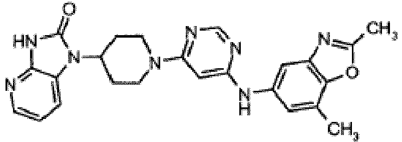 |
| (21) | 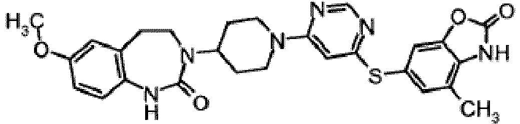 |
| (22) | 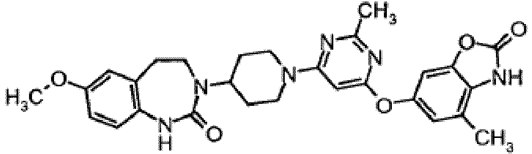 |
| (23) | 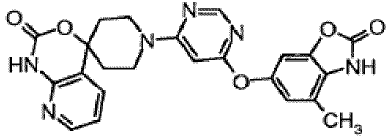 |
| (24) | 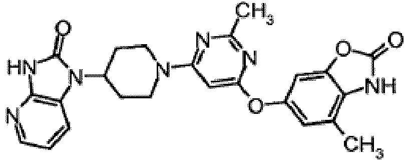 |
| (25) | 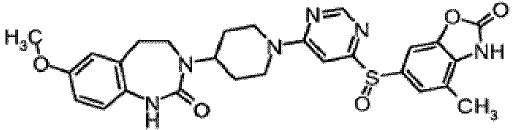 |
| (26) | 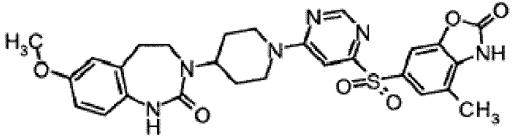 |
| (27) | 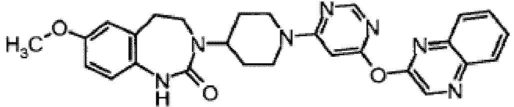 |
| (28) | 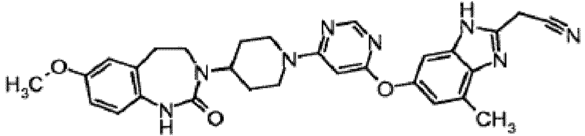 |
| (29) | 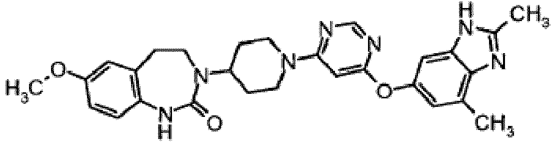 |
| (30) | 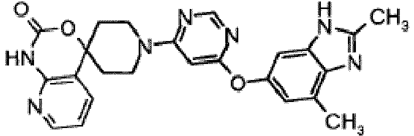 |
| (31) | 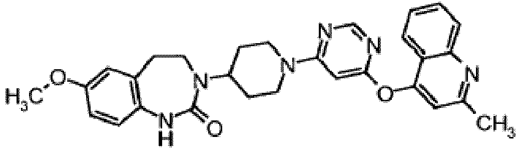 |
| (32) | 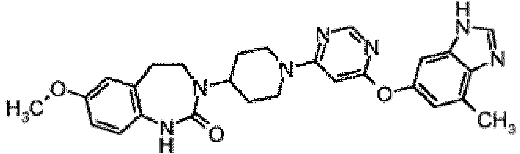 |
| (33) | 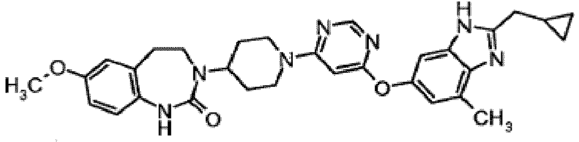 |
| (34) | 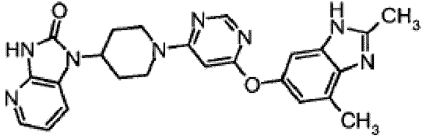 |
| (35) | 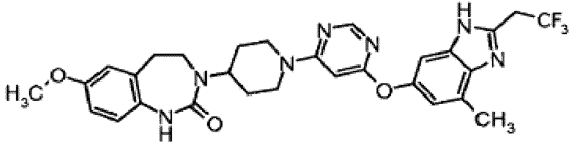 |
| (36) | 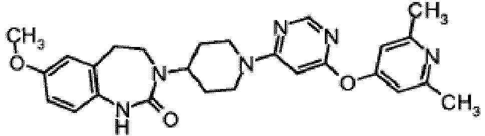 |
| (37) | 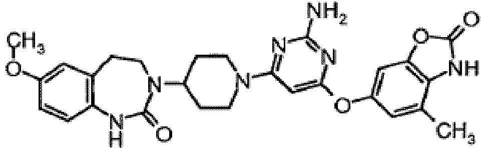 |
| (38) | 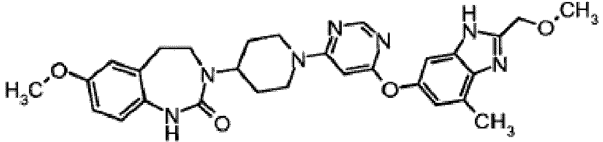 |
| (39) | 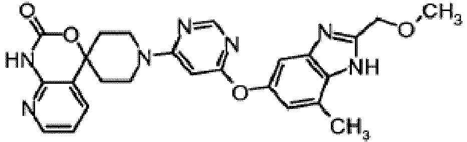 |
| (40) | 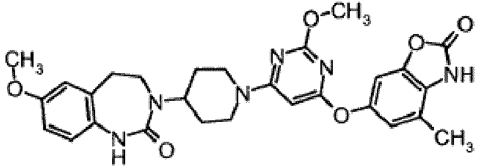 |
| (41) | 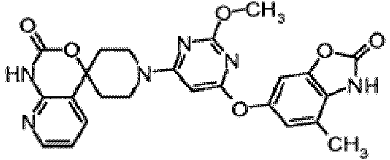 |
| (42) | 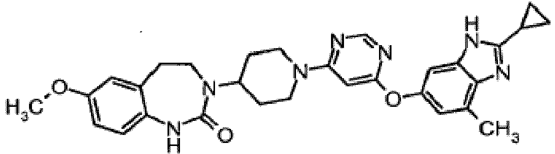 |
| (43) | 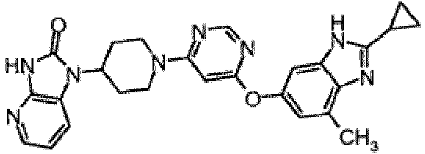 |
| (44) | 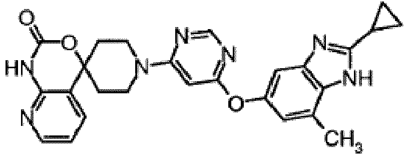 |
| (45) | 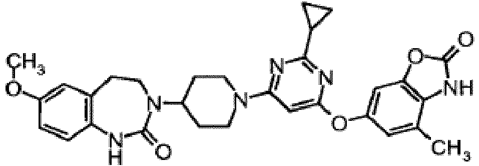 |
| (46) | 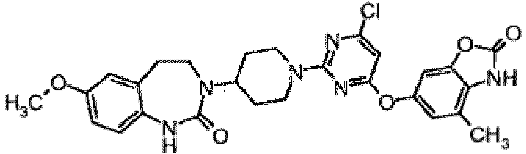 |
| (47) | 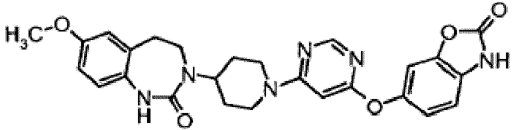 |
| (48) | 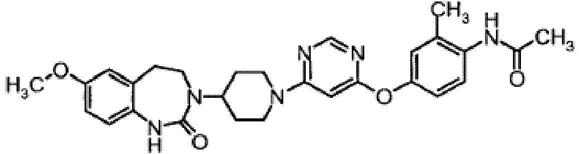 |
| (49) | 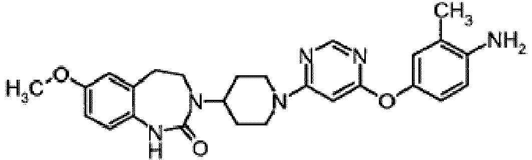 |
| (50) | 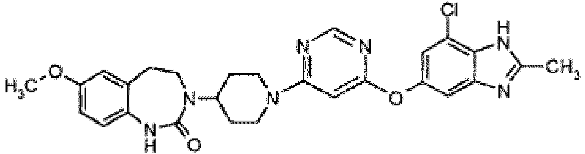 |
| (51) | 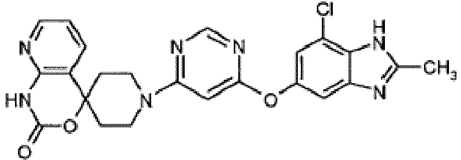 |
| (52) | 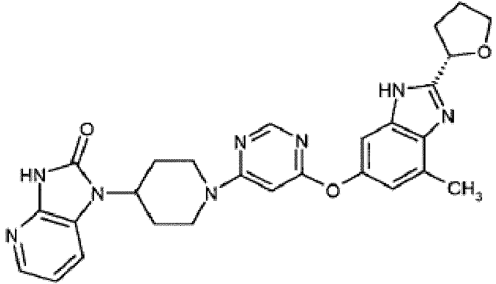 |
| (53) | 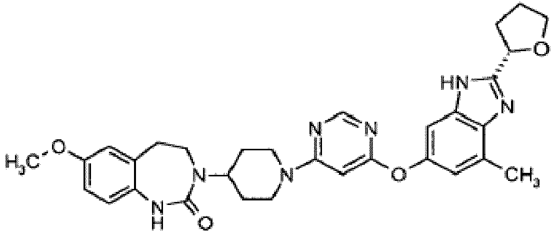 |
| (54) | 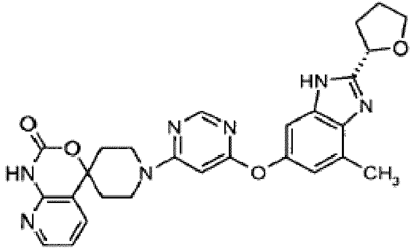 |
| (55) | 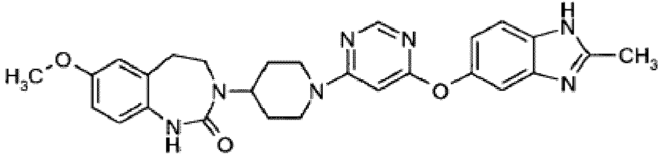 |
| (56) | 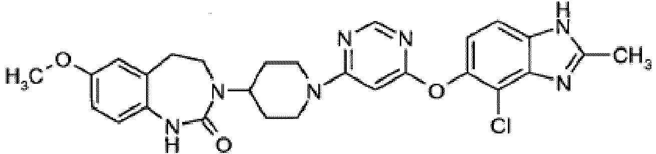 |
| (57) | 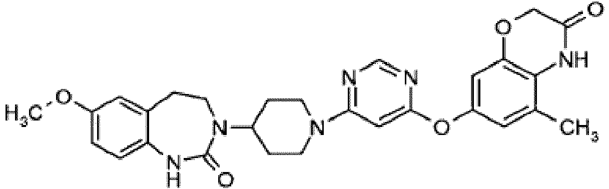 |
| (58) | 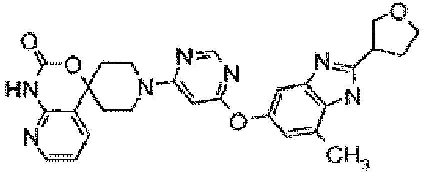 |
| (59) | 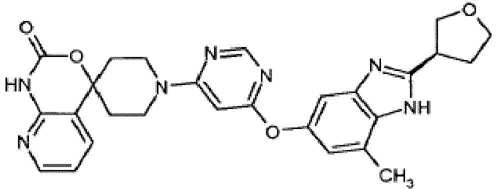 |
| (60) | 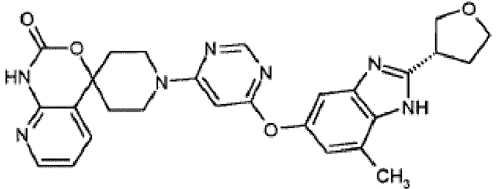 |
| (61) | 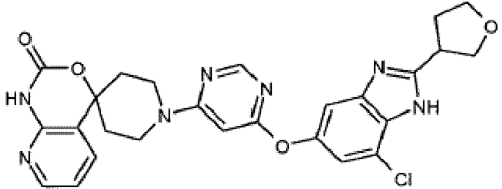 |
| (62) | 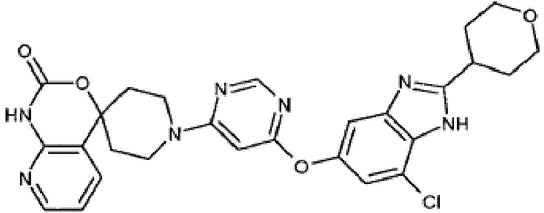 |
| (63) | 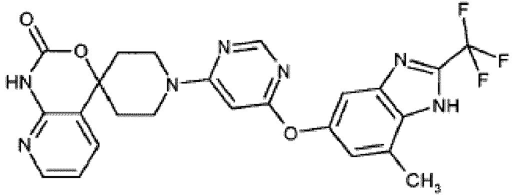 |
| (64) | 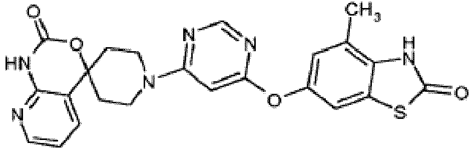 |
| (65) | 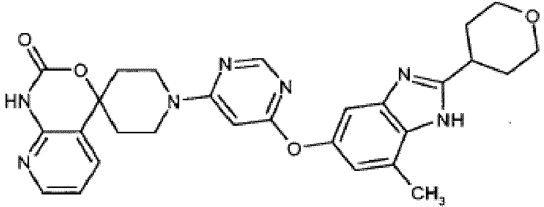 |
| (66) | 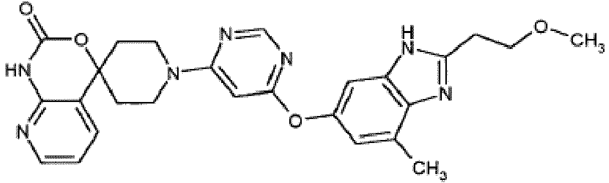 |
| (67) | 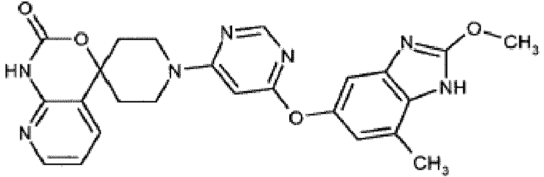 |
| (68) | 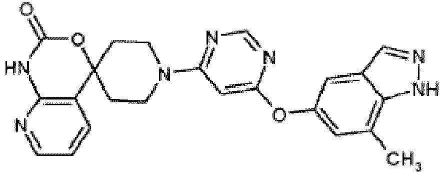 |
| (69) | 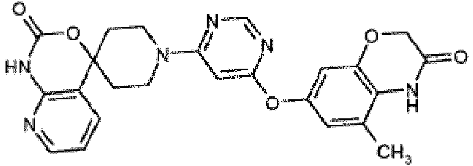 |
| (70) | 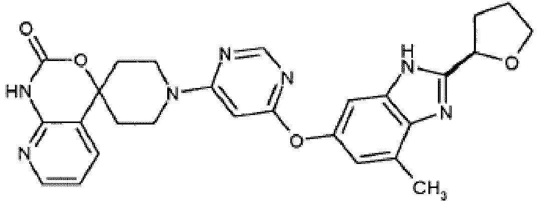 |
| (71) | 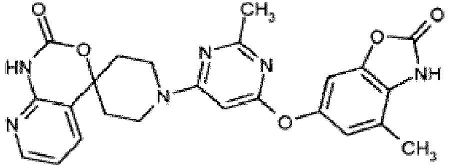 |
| (72) | 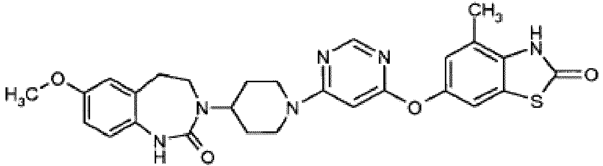 |
| (73) | 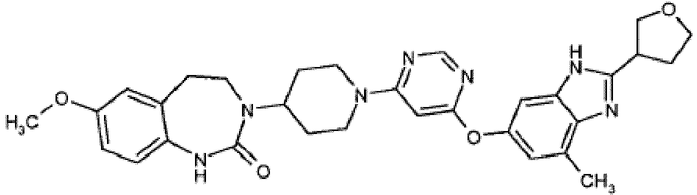 |
| (74) | 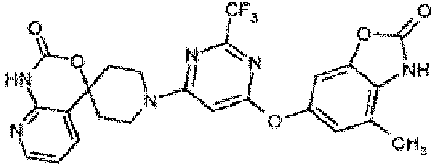 |
| (75) | 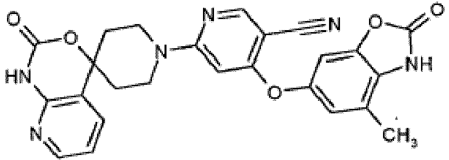 |
| (76) | 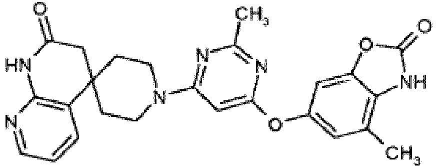 |
| (77) | 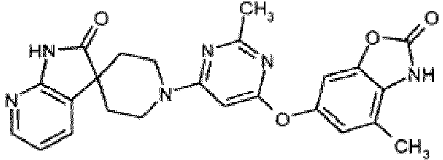 |
| (78) | 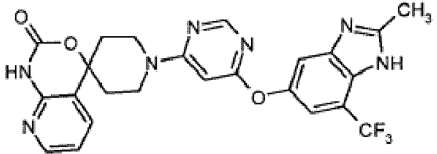 |
| (79) | 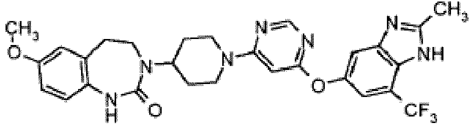 |
| (80) | 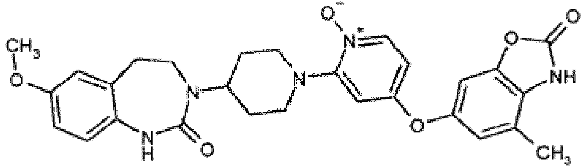 |
leurs tautomères, leurs diastéréomères, leurs énantiomères, leurs hydrates, leurs mélanges et leurs sels et les hydrates des sels, en particulier leurs sels physiologiquement acceptables avec des acides ou des bases inorganiques ou organiques.

6. Médicament, contenant un composé selon l'une des revendications 1 à 5, avec éventuellement un ou plusieurs véhicules et/ou diluants inertes.

7. Composé selon l'une des revendications 1 à 5, pour son utilisation dans le traitement aigu et prophylactique des céphalées, en particulier des migraines ou des algies vasculaires de la face.

8. Composé selon l'une des revendications 1 à 5, pour son utilisation dans le traitement du diabète sucré non insulinodépendant (« NIDDM »), du syndrome de douleur régionale complexe (CRPS1), de maladies cardiovasculaires, de la tolérance à la morphine, des diarrhées dues à la toxine de *Clostridium,* des maladies de la peau, en particulier des lésions cutanées thermiques et provoquées par un rayonnement comprenant les coups de soleil, le lichen, le prurigo, les toxidermies prurigineuses et le prurit sévère, des maladies inflammatoires, par exemple des maladies inflammatoires articulaires (arthrose, arthrite rhumatoïde, arthrite neurogène), des rhumatismes généralisés des parties molles (fibromyalgie), des inflamations neurogènes des muqueuses buccales, des maladies inflammatoires pulmonaires, de la rhinite allergique, de l'asthme, de la BPCO, de maladies qui s'accompagnent d'une vasodilatation excessive et d'une réduction de l'irrigation sanguine tissulaire qui en résulte, par exemple choc et septicémie, des affections chroniques douloureuses telles que les neuropathies diabétiques, des neuropathies induites par chimiothérapie, des neuropathies induites par le VIH, des neuropathies post-herpétiques, des neuropathies induites par un traumatisme tissulaire, des névralgies du trijumeau, des dysfonctionnements temporo-mandibulaires, du CRPS (syndrome de douleur régionale complexe), des douleurs dorsales, des maladies viscérales, par exemple du syndrome du côlon irritable (SCI), du syndrome intestinal inflammatoire, pour l'atténuation des états douloureux ou pour le traitement préventif ou thérapeutique aigu des symptômes de bouffées de chaleur de la ménopause provoquées par une vasodilatation ou une augmentation du débit sanguin des femmes présentant un déficit en oestrogènes et des patients souffrant d'un cancer de la prostate et sujets ayant subi une castration traités par des hormones.

9. Composé selon l'une des revendications 1 à 5, pour son utilisation dans le traitement des migraines ou des algies vasculaires de la face.

10. Composé pour son utilisation selon la revendication 9, **caractérisé en ce que** le traitement peut être aigu ou prophylactique.

11. Composé selon l'une des revendications 1 à 5, pour son utilisation dans le traitement du syndrome du côlon irritable (SCI).

12. Composé selon l'une des revendications 1 à 5, pour son utilisation dans le traitement préventif et thérapeutique aigu des bouffées de chaleur des femmes présentant un déficit en oestrogènes.
